# EUROPEAN PATENT SPECIFICATION

(11) **EP 3 833 438 B1**
(45) Date of publication and mention of the grant of the patent: **07.02.2024**
(21) Application number: 19759133.2
(22) Date of filing: 07.08.2019
(51) Int. Cl.: A61P 35/00, C07D 401/14, C07D 403/14, C07D 405/14, C07D 409/14, C07D 413/14, C07F 9/00, A61K 31/4184, C07F 9/6561

(54) **SUBSTITUTED BENZIMIDAZOLES AS PAD4 INHIBITORS**
SUBSTITUIERTE BENZIMIDAZOLE ALS PAD4-INHIBITOREN
BENZIMIDAZOLES SUBSTITUÉS EN TANT QU'INHIBITEURS DE PAD4

(30) Priority: 08.08.2018 US 201862715858 P
(43) Date of publication of application: 16.06.2021
(73) Proprietor: Bristol-Myers Squibb Company, Princeton, NJ 08543 (US)
(72) Inventor: GARDNER, Daniel S., Princeton, NJ 08543 (US); DUNCIA, John V., Princeton, NJ 08543 (US); SANTELLA, Joseph B., Springfield, PA 19064 (US); NGU, Khehyong, Princeton, NJ 08543 (US); ANNUNZIATO, Christopher, North Lawrence, NJ 08648 (US)
(74) Representative: Dehns
(86) International application number: PCT/US2019/045424
(87) International publication number: WO 2020/033488

(56) References cited:
- WO-A1-2014/015905
- WO-A1-2016/185279
- WO-A1-2017/100594
- WO-A1-2018/022897

## Description

### BACKGROUND OF THE INVENTION

PAD4 is a member of the peptidylarginine deiminase (PAD) family of enzymes capable of catalysing the citrullination of arginine into citrulline within peptide sequences. PAD4 is responsible for the deimination or citrullination of a variety of proteins *in vitro* and *in vivo,* with consequences of diverse functional responses in a variety of diseases (Jones J.E. et al, Curr. Opin. Drug Discov. Devel., 12(5), (2009), 616-627). Examples of exemplar diseases include rheumatoid arthritis, diseases with neutrophilic contributions to pathogenesis (for example vasculitis, systemic lupus erythematosus, ulcerative colitis) in addition to oncology indications. PAD4 inhibitors also have wider applicability as tools and therapeutics for human disease through epigenetic mechanisms.

Inhibitors of PAD4 have utility against Rheumatoid Arthritis (RA). RA is an auto-immune disease affecting approximately 1% of the population *(*Wegner N. et al, Immunol. Rev., 233(1) (2010), 34-54*).* It is characterized by inflammation of articular joints leading to debilitating destruction of bone and cartilage. A weak genetic association between PAD4 polymorphisms and susceptibility to RA has been suggested, albeit inconsistently, in a number of population studies (Kochi Y. et al, Ann. Rheum. Dis., 70, (2011),512-515*).* PAD4 (along with family member PAD2) has been detected in synovial tissue where it is responsible for the deimination of a variety of joint proteins. This process is presumed to lead to a break of tolerance to, and initiation of immune responses to, citrullinated substrates such as fibrinogen, vimentin and collagen in RA joints. These anti-citrullinated protein antibodies (ACPA) contribute to disease pathogenesis and may also be used as a diagnostic test for RA (e.g. the commercially available CCP2 or cyclic citrullinated protein 2 test). In addition, increased citrullination may also offer additional direct contributions to disease pathogenesis through its ability to affect directly the function of several joint and inflammatory mediators (e.g. fibrinogen, anti-thrombin, multiple chemokines). In a smaller subset of RA patients, anti-PAD4 antibodies can be measured and may correlate with a more erosive form of the disease.

PAD4 inhibitors are also useful for the reduction of pathological neutrophil activity in a variety of diseases. Studies suggest that the process of Neutrophil Extracellular Trap (NET) formation, an innate defense mechanism by which neutrophils are able to immobilize and kill pathogens, is associated with histone citrullination and is deficient in PAD4 knockout mice (Neeli I. et al, J. Immunol., 180, (2008), 1895-1902 and Li P. et al, J. Exp. Med., 207(9), (2010), 1853-1862*).* PAD4 inhibitors may therefore have applicability for diseases where NET formation in tissues contributes to local injury and disease pathology. Such diseases include, but are not limited to, small vessel vasculitis (Kessenbrock K. et al, Nat. Med., 15(6), (2009), 623-625*),* systemic lupus erythematosus *(*Hakkim A. et al, Proc. Natl. Acad. Sci. USA, 107(21), (2010), 9813-9818 and Villanueva E. et al, J. Immunol., 187(1), (2011), 538-52*),* ulcerative colitis *(*Savchenko A. et al, Pathol. Int., 61(5), (2011), 290-7*),* cystic fibrosis, asthma *(*Dworski R. et al, J. Allergy Clin. Immunol., 127(5), (2011), 1260-6*),* deep vein thrombosis *(*Fuchs T. et al, Proc. Natl. Acad. Sci. USA, 107(36), (2010), 15880-5*),* periodontitis *(*Vitkov L. et al, Ultrastructural Pathol., 34(1), (2010), 25-30*),* sepsis *(*Clark S.R. et al, Nat. Med., 13(4), (2007), 463-9*),* appendicitis *(*Brinkmann V. et al, Science, 303, (2004), 1532-5*),* and stroke. In addition, there is evidence that NETs may contribute to pathology in diseases affecting the skin, e.g., in cutaneous lupus erythematosus *(*Villanueva E. et al, J. Immunol., 187(1), (2011), 538-52*)* and psoriasis *(*Lin A.M. et al., J. Immunol., 187(1), (2011), 490-500*),* so a PAD4 inhibitor may show benefit to tackle NET skin diseases, when administered by a systemic or cutaneous route. PAD4 inhibitors may affect additional functions within neutrophils and have wider applicability to neutrophilic diseases.

Studies have demonstrated efficacy of tool PAD inhibitors (for example chloro-amidine) in a number of animal models of disease, including collagen-induced arthritis *(*Willis V.C. et al, J. Immunol., 186(7), (2011), 4396-4404*),* dextran sulfate sodium (DSS)-induced experimental colitis *(*Chumanevich A.A. et al, Am. J. Physiol. Gastrointest. Liver Physiol., 300(6), (2011), G929-G938*),* spinal cord repair *(*Lange S. et al, Dev. Biol., 355(2), (2011), 205-14*),* and experimental autoimmune encephalomyelitis (EAE). The DSS colitis report also demonstrates that chloro-amidine drives apoptosis of inflammatory cells both *in vitro* and *in vivo,* suggesting that PAD4 inhibitors may be effective more generally in widespread inflammatory diseases.

PAD4 inhibitors are also useful in the treatment of cancers (Slack J.L. et al, Cell. Mol. Life Sci., 68(4), (2011), 709-720*).* Over-expression of PAD4 has been demonstrated in numerous cancers (ChangX. et al, BMC Cancer, 9, (2009), 40). An antiproliferative role has been suggested for PAD4 inhibitors from the observation that PAD4 citrullinates arginine residues in histones at the promoters of p53-target genes such as p21, which are involved in cell cycle arrest and induction of apoptosis (Li P. et al, Mol. Cell Biol., 28(15), (2008), 4745-4758*).*

The aforementioned role of PAD4 in deiminating arginine residues in histones may be indicative of a role for PAD4 in epigenetic regulation of gene expression. PAD4 is the primary PAD family member observed to be resident in the nucleus as well as the cytoplasm. Early evidence that PAD4 may act as a histone demethyliminase as well as a deiminase is inconsistent and unproven. However, it may reduce histone arginine methylation (and hence epigenetic regulation associated with this mark) indirectly *via* depletion of available arginine residues by conversion to citrulline. PAD4 inhibitors are useful as epigenetic tools or therapeutics for affecting expression of varied target genes in additional disease settings. Through such mechanisms, PAD4 inhibitors may also be effective in controlling citrullination levels in stem cells and may therefore therapeutically affect the pluripotency status and differentiation potential of diverse stem cells including, but not limited to, embryonic stem cells, neural stem cells, haematopoietic stem cells and cancer stem cells. Accordingly, there remains an unmet need to identify and develop PAD4 inhibitors for the treatment of PAD4-mediated disorders.

WO 2018/022897 discloses compounds that are useful as inhibitors of PAD4, compositions thereof, and methods of treating PAD4-related disorders. WO 2016/185279 discloses benzoimidazole derivatives that are useful as PAD4 inhibitors, and may be useful in the treatment of various disorders. WO 2014/015905 discloses 2-(azaindol-2-yl)benzimidazole compounds that are useful as PAD4 inhibitors and may be useful in the treatment of various disorders. WO2017/100594 discloses substituted benzimidazole derivatives, useful as inhibitors of PAD4 for the treatment of PAD4-mediated diseases.

### SUMMARY OF THE INVENTION

It has now been found that compounds of Formula (I) are useful as inhibitors of PAD4: 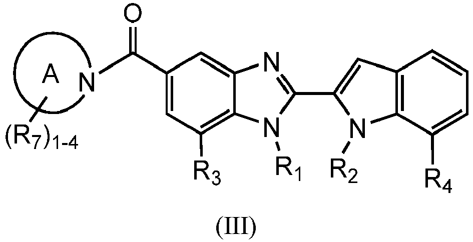 or a pharmaceutically acceptable salt thereof, wherein each of Ring A, R₁, R₂, R₃, R₄, and R₇, along with other variables is as defined herein.

In some embodiments, a provided compound demonstrates selectivity for PAD4 with respect to PAD2. The present invention also provides pharmaceutically acceptable compositions comprising a provided compound. Provided compounds are useful in treatment of various disorders associated with PAD4. Such disorders are described in detail, herein, and include, for example rheumatoid arthritis, vasculitis, systemic lupus erythematosus, ulcerative colitis, cancer, cystic fibrosis, asthma, cutaneous lupus erythematosus, and psoriasis.

### DETAILED DESCRIPTION OF THE INVENTION

### 1. General Description of Certain Aspects of the Invention

In some embodiments, such compounds include those of the formulae described herein, or a pharmaceutically acceptable salt thereof, wherein each variable is as defined herein and described in embodiments. Such compounds have the structure of Formula (III): 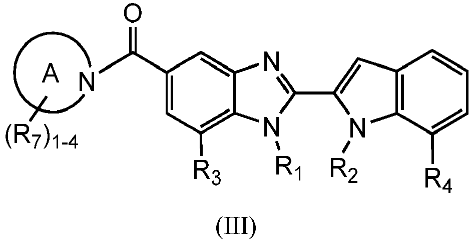 or a pharmaceutically acceptable salt thereof, wherein: 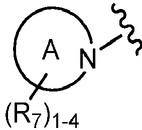 is selected from 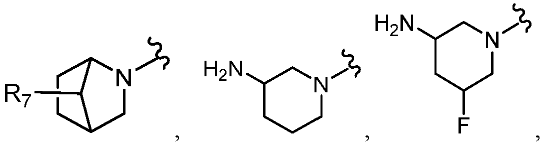 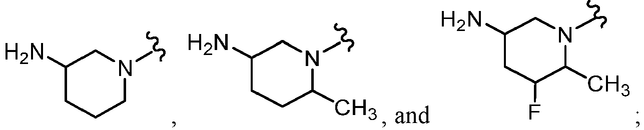
R₁ is selected from -CH₃, -CD₃, and -CH₂-5-6 membered heterocyclyl comprising carbon
   atoms and 1-3 heteroatoms selected from N, NH, and NC₁₋₃alkyl;
R₂ is selected from CH₃, CH₃CH₂, and -CH₂-cyclopropyl substituted with 0-3 Rₑ;
R₃ is selected from H, F, Cl, Br, and -OC₁₋₄ alkyl;
R4 is selected from 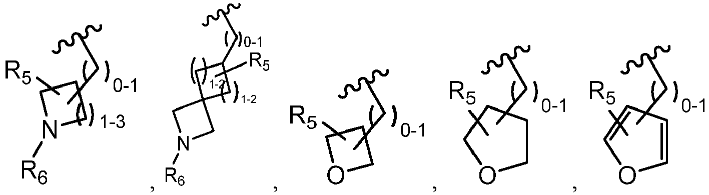 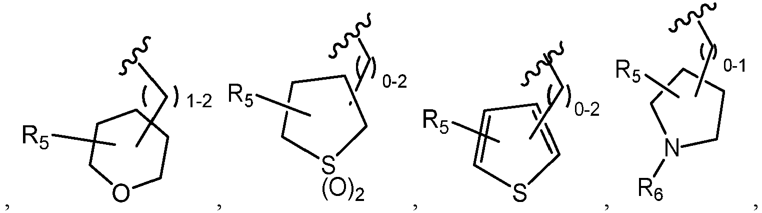 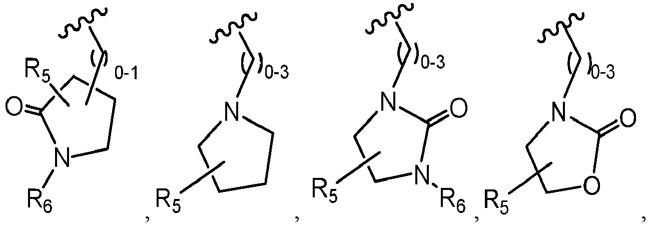 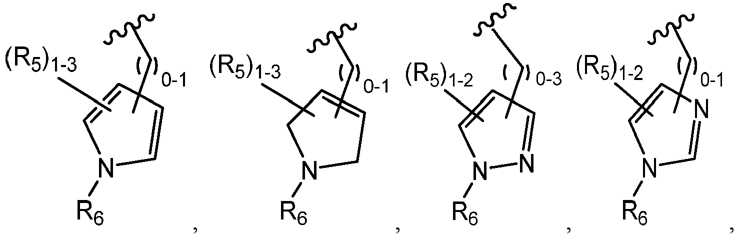 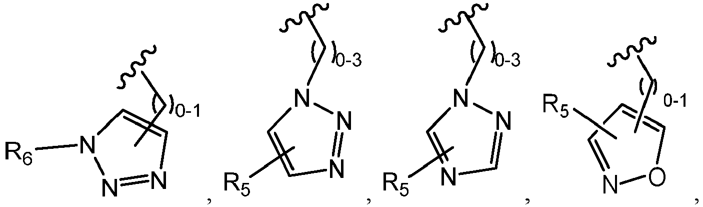 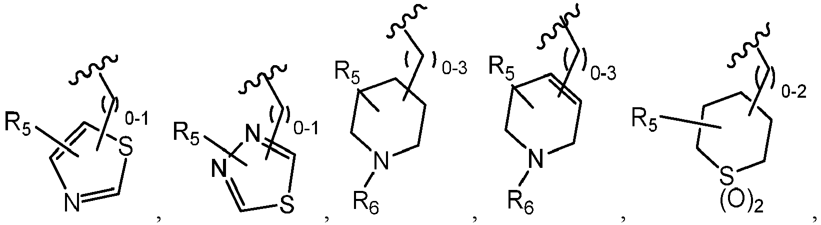 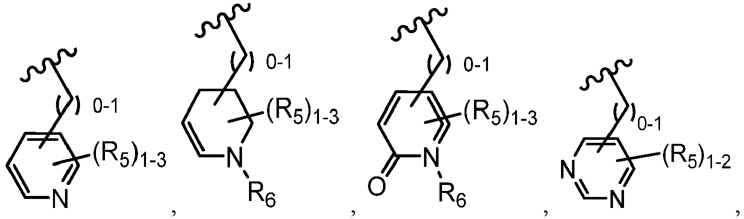 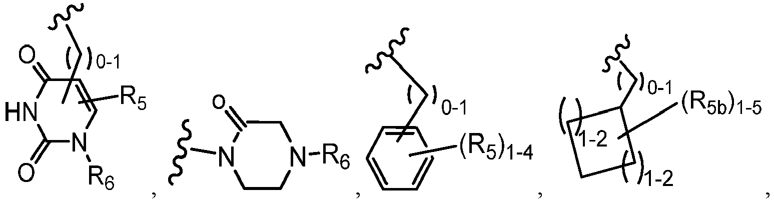 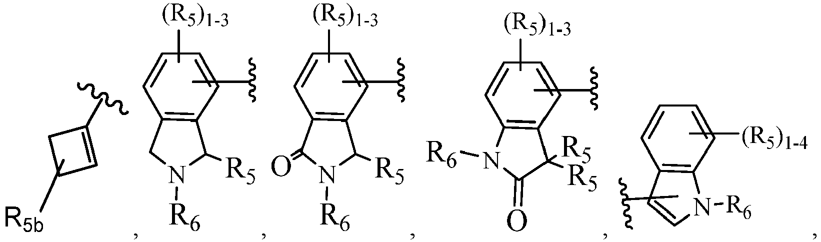 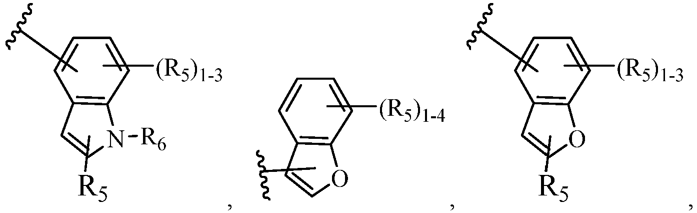 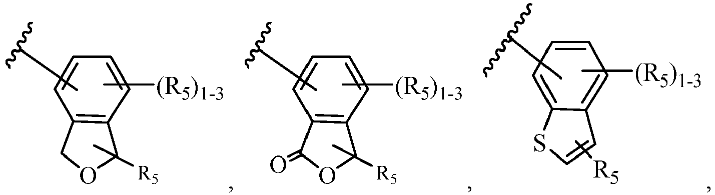 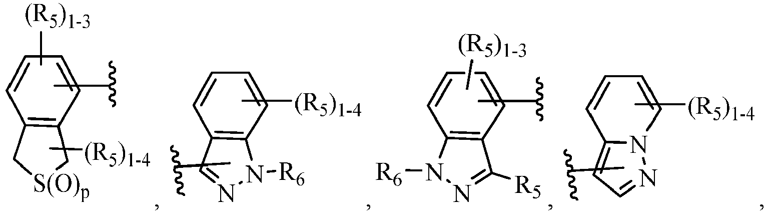 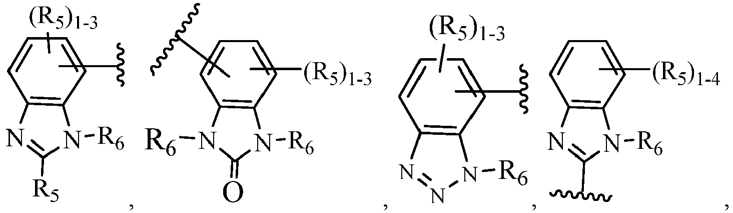 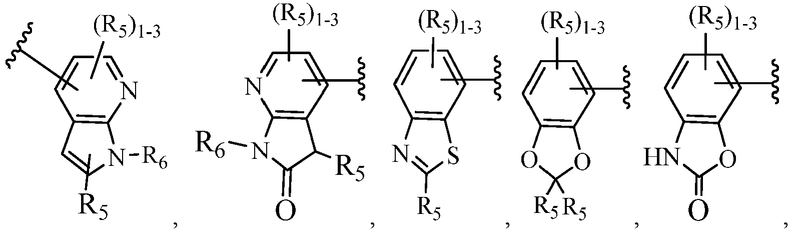 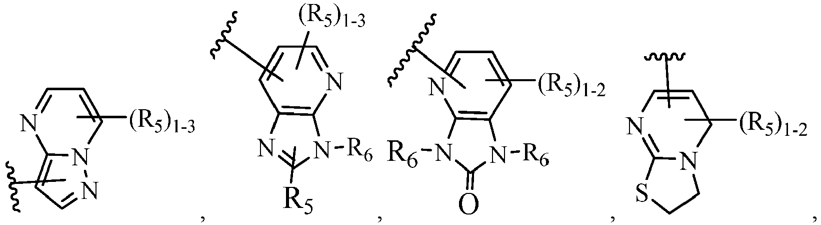 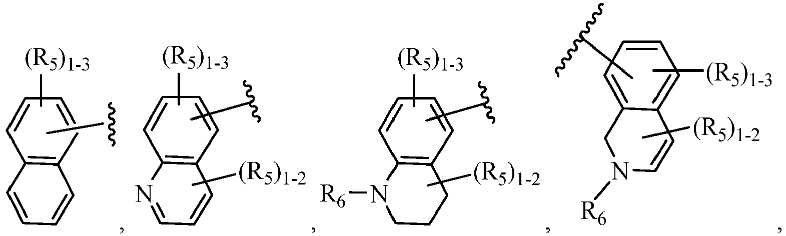 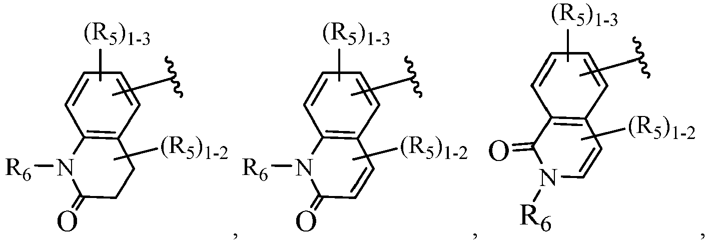 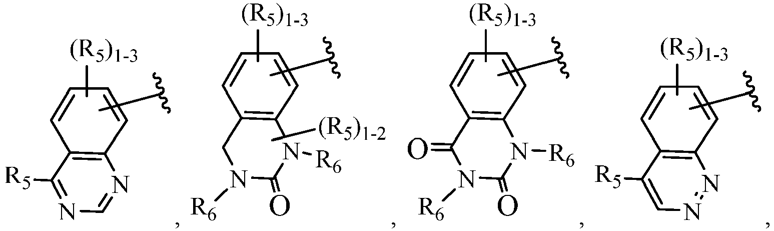 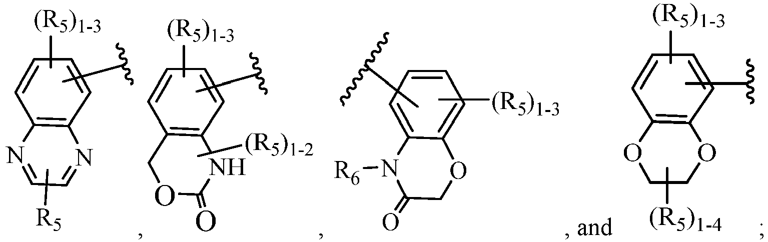
R₅, at each occurrence, is independently selected from H, F, Cl, Br, C₁₋₄alkyl substituted
   with 0-5 Rₑ, C₂₋₄alkenyl, C₂₋₄alkynyl,
   nitro, -(CH₂)ᵣOR_{b}, -CN, -NRₐRₐ, -(CH₂)ᵣNRₐC(=O)R_{b}, -NRₐC(=O)NRₐRₐ, - C(=O)OR_{b}, -C(=O)R_{b}, -OC(=O)R_{b}, -C(=O)NRₐRₐ, -P(=O)(C₁₋₄alkyl)₂, -S(O)*ₚ*R_{c}, -S(O)*ₚ*NRₐRₐ, -NRₐS(O)*ₚ*R_{c}, -C₃₋₆ cycloalkyl substituted with 0-4 Rₑ, aryl substituted with 0-4 Rₑ, and heterocyclyl substituted with 0-4 Rₑ;
R_{5b}, at each occurrence, is independently selected from OR_{b}, -C(=O)OR_{b}, -C(=O)NRₐRₐ, - NRₐRₐ, -NRₐC(=O)R_{b}, -NRₐC(=O)OR_{b}, -S(=O)ₚR_{c}, and -NRₐS(=O)pR_{c};
R₆, at each occurrence, is independently selected from H, C₁₋₃alkyl substituted with 0-4 Rₑ, -C(=O)R_{b}, -C(=O)(CH₂)ᵣOR_{b}, -C(=O)(CH₂)ᵣNRₐRₐ, -S(O)ₚR_{c}, -S(O)ₚNRₐRₐ, - (CH₂)ᵣ-aryl substituted with 0-4 Rₑ, and -(CH₂)ᵣ-heterocyclyl substituted with 0-4 Rₑ;
R₇ is selected from H, F, Cl, CN, C₁₋₃ alkyl, =N-OR_{b}, -(CH₂)ᵣOR_{b}, -(CH₂)ᵣNRₐRₐ, - NRₐC(=NH)C₁₋₃alkyl, -NRₐC(=O)OR_{b}, a carbocyclyl, and heterocyclyl;
Rₐ, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ;
R_{c}, at each occurrence, is independently selected from C₁₋₆ alkyl substituted with 0-5 Rₑ, C₂₋₆alkenyl substituted with 0-5 Rₑ, C₂₋₆ alkynyl substituted with 0-5 Rₑ, -(CH₂)ᵣ-C₃₋₆ carbocyclyl substituted with 0-5 Rₑ, and -(CH₂)ᵣ-heterocyclyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, -(CH₂)ᵣOC₁₋₄alkyl, -(CH₂)ᵣOC₂₋₄alkenyl, -(CH₂)ᵣOC₂₋₄alkynyl, and NH₂;
Rε, at each occurrence, is independently selected from H, F, Cl, Br, CN, OH, C₁₋₅ alkyl optionally substituted with OH, C₃₋₆ cycloalkyl, and phenyl;
p, at each occurrence, is independently selected from zero, 1, and 2; and
r, at each occurrence, is independently selected from zero, 1, 2, 3, and 4.

### 2. Definitions

Throughout the specification and the appended claims, a given chemical formula or name shall encompass all stereo and optical isomers and racemates thereof where such isomers exist. Unless otherwise indicated, all chiral (enantiomeric and diastereomeric) and racemic forms are within the scope of the invention. Many geometric isomers of C=C double bonds, C=N double bonds, ring systems, and the like can also be present in the compounds, and all such stable isomers are contemplated in the present invention. Cis- and trans- (or *E*- and *Z*-) geometric isomers of the compounds of the present invention are described and may be isolated as a mixture of isomers or as separated isomeric forms. The present compounds can be isolated in optically active or racemic forms. Optically active forms may be prepared by resolution of racemic forms or by synthesis from optically active starting materials. All processes used to prepare compounds of the present invention and intermediates made therein are considered to be part of the present invention. When enantiomeric or diastereomeric products are prepared, they may be separated by conventional methods, for example, by chromatography or fractional crystallization. Depending on the process conditions the end products of the present invention are obtained either in free (neutral) or salt form. Both the free form and the salts of these end products are within the scope of the invention. If so desired, one form of a compound may be converted into another form. A free base or acid may be converted into a salt; a salt may be converted into the free compound or another salt; a mixture of isomeric compounds of the present invention may be separated into the individual isomers. Compounds of the present invention, free form and salts thereof, may exist in multiple tautomeric forms, in which hydrogen atoms are transposed to other parts of the molecules and the chemical bonds between the atoms of the molecules are consequently rearranged. It should be understood that all tautomeric forms, insofar as they may exist, are included within the invention.

As used herein, the term "alkyl" or "alkylene" is intended to include both branched and straight-chain saturated aliphatic hydrocarbon groups having the specified number of carbon atoms. For examples, "C₁ to C₁₂ alkyl" or "C₁₋₁₂ alkyl" (or alkylene), is intended to include C₁, C₂, C₃, C₄, C₅, C₆, C₇, C₈, C₉, C₁₀, C₁₁ and C₁₂ alkyl groups; "C₄ to C₁₈ alkyl" or "C₄₋₁₈ alkyl" (or alkylene), is intended to include C₄, C₅, C₆, C₇, C₈, C₉, C₁₀, C₁₁, C₁₂, C₁₃, C₁₄, C₁₅, C₁₆, C₁₇, and C₁₈ alkyl groups. Additionally, for example, "C₁ to C₆ alkyl" or "C₁₋₆ alkyl" denotes alkyl having 1 to 6 carbon atoms. Alkyl group can be unsubstituted or substituted with at least one hydrogen being replaced by another chemical group. Example alkyl groups include, but are not limited to, methyl (Me), ethyl (Et), propyl (*e.g.,* n-propyl and isopropyl), butyl (*e.g*., n-butyl, isobutyl, *t*-butyl), and pentyl (*e.g*., n-pentyl, isopentyl, neopentyl). When "C₀ alkyl" or "C₀ alkylene" is used, it is intended to denote a direct bond.

"Alkenyl" or "alkenylene" is intended to include hydrocarbon chains of either straight or branched configuration having the specified number of carbon atoms and one or more, preferably one to two, carbon-carbon double bonds that may occur in any stable point along the chain. For example, "C₂ to C₆ alkenyl" or "C₂₋₆ alkenyl" (or alkenylene), is intended to include C₂, C₃, C₄, C₅, and C₆ alkenyl groups. Examples of alkenyl include, but are not limited to, ethenyl, 1-propenyl, 2-propenyl, 2-butenyl, 3-butenyl, 2-pentenyl, 3, pentenyl, 4-pentenyl, 2-hexenyl, 3-hexenyl, 4-hexenyl, 5-hexenyl, 2-methyl-2-propenyl, and 4-methyl-3-pentenyl.

"Alkynyl" or "alkynylene" is intended to include hydrocarbon chains of either straight or branched configuration having one or more, preferably one to three, carbon-carbon triple bonds that may occur in any stable point along the chain. For example, "C₂ to C₆ alkynyl" or "C₂₋₆ alkynyl" (or alkynylene), is intended to include C₂, C₃, C₄, C₅, and C₆ alkynyl groups; such as ethynyl, propynyl, butynyl, pentynyl, and hexynyl.

The term "alkoxy" or "alkyloxy" refers to an -O-alkyl group. For example, "C₁ to C₆ alkoxy" or "C₁₋₆ alkoxy" (or alkyloxy), is intended to include C₁, C₂, C₃, C₄, C₅, and C₆ alkoxy groups. Example alkoxy groups include, but are not limited to, methoxy, ethoxy, propoxy (e.g., n-propoxy and isopropoxy), and t-butoxy. Similarly, "alkylthio" or "thioalkoxy" represents an alkyl group as defined above with the indicated number of carbon atoms attached through a sulphur bridge; for example methyl-S- and ethyl-S-.

"Halo" or "halogen" includes fluoro, chloro, bromo, and iodo. "Haloalkyl" is intended to include both branched and straight-chain saturated aliphatic hydrocarbon groups having the specified number of carbon atoms, substituted with 1 or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" that is intended to include both branched and straight-chain saturated aliphatic hydrocarbon groups having the specified number of carbon atoms, substituted with 1 or more fluorine atoms.

The term "cycloalkyl" refers to cyclized alkyl groups, including mono-, bi- or poly-cyclic ring systems. For example, "C₃ to C₆ cycloalkyl" or "C₃₋₆ cycloalkyl" is intended to include C₃, C₄, C₅, and C₆ cycloalkyl groups. Example cycloalkyl groups include, but are not limited to, cyclopropyl, cyclobutyl, cyclopentyl, cyclohexyl, and norbornyl. Branched cycloalkyl groups such as 1-methylcyclopropyl and 2-methylcyclopropyl are included in the definition of "cycloalkyl". The term "cycloalkenyl" refers to cyclized alkenyl groups. C₄₋₆ cycloalkenyl is intended to include C₄, C₅, and C₆ cycloalkenyl groups. Example cycloalkenyl groups include, but are not limited to, cyclobutenyl, cyclopentenyl, and cyclohexenyl.

As used herein, "carbocycle", "carbocyclyl", or "carbocyclic residue" is intended to mean any stable 3-, 4-, 5-, 6-, 7-, or 8-membered monocyclic or bicyclic or 7-, 8-, 9-, 10-, 11-, 12-, or 13-membered bicyclic or tricyclic hydrocarbon ring, any of which may be saturated, partially unsaturated, unsaturated or aromatic. Examples of such carbocycles include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, [3.3.0]bicyclooctane, [4.3.0]bicyclononane, [4.4.0]bicyclodecane (decalin), [2.2.2]bicyclooctane, fluorenyl, phenyl, naphthyl, indanyl, adamantyl, anthracenyl, and tetrahydronaphthyl (tetralin). As shown above, bridged rings are also included in the definition of carbocycle (e.g., [2.2.2]bicyclooctane). Preferred carbocycles, unless otherwise specified, are cyclopropyl, cyclobutyl, cyclopentyl, cyclohexyl, phenyl, indanyl, and tetrahydronaphthyl. When the term "carbocycle" is used, it is intended to include "aryl." A bridged ring occurs when one or more, preferably one to three, carbon atoms link two non-adjacent carbon atoms. Preferred bridges are one or two carbon atoms. It is noted that a bridge always converts a monocyclic ring into a tricyclic ring. When a ring is bridged, the substituents recited for the ring may also be present on the bridge.

As used herein, the term "bicyclic carbocycle" or "bicyclic carbocyclic group" is intended to mean a stable 9- or 10-membered carbocyclic ring system that contains two fused rings and consists of carbon atoms. Of the two fused rings, one ring is a benzo ring fused to a second ring; and the second ring is a 5- or 6-membered carbon ring which is saturated, partially unsaturated, or unsaturated. The bicyclic carbocyclic group may be attached to its pendant group at any carbon atom which results in a stable structure. The bicyclic carbocyclic group described herein may be substituted on any carbon if the resulting compound is stable. Examples of a bicyclic carbocyclic group are, but not limited to, naphthyl, 1,2-dihydronaphthyl, 1,2,3,4-tetrahydronaphthyl, and indanyl. "Carbocycle", "carbocyclyl", or "carbocyclic residue" can also refer to spiro compounds, for example, a spiro[3.3]heptane.

"Aryl" groups refer to monocyclic or bicyclic aromatic hydrocarbons, including, for example, phenyl, and naphthyl. Aryl moieties are well known and described, for example, in Lewis, R.J., ed., Hawley's Condensed Chemical Dictionary, 15th Edition, John Wiley & Sons, Inc., New York (2007). "C₆₋₁₀ aryl" refers to phenyl and naphthyl.

As used herein, the term "heterocycle", "heterocyclyl", or "heterocyclic group" is intended to mean a stable 3-, 4-, 5-, 6-, or 7-membered monocyclic or bicyclic or 7-, 8-, 9-, 10-, 11-, 12-, 13-, or 14-membered polycyclic heterocyclic ring that is saturated, partially unsaturated, or fully unsaturated, and that contains carbon atoms and 1, 2, 3 or 4 heteroatoms independently selected from the group consisting of N, O and S; and including any polycyclic group in which any of the above-defined heterocyclic rings is fused to a benzene ring. The nitrogen and sulfur heteroatoms may optionally be oxidized (*i.e.,* N→O and S(O)ₚ, wherein p is 0, 1 or 2). The nitrogen atom may be substituted or unsubstituted (*i.e*., N or NR wherein R is H or another substituent, if defined). The heterocyclic ring may be attached to its pendant group at any heteroatom or carbon atom that results in a stable structure. The heterocyclic rings described herein may be substituted on carbon or on a nitrogen atom if the resulting compound is stable. A nitrogen in the heterocycle may optionally be quaternized. It is preferred that when the total number of S and O atoms in the heterocycle exceeds 1, then these heteroatoms are not adjacent to one another. It is preferred that the total number of S and O atoms in the heterocycle is not more than 1. When the term "heterocycle" is used, it is intended to include heteroaryl.

Examples of heterocycles include, but are not limited to, acridinyl, azetidinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinnolinyl, decahydroquinolinyl, 2*H*,6*H*-1,5,2-dithiazinyl, dihydrofuro[2,3-*b*]tetrahydrofuran, furanyl, furazanyl, imidazolidinyl, imidazolinyl, imidazolyl, 1*H*-indazolyl, imidazolopyridinyl, indolenyl, indolinyl, indolizinyl, indolyl, 3*H*-indolyl, isatinoyl, isobenzofuranyl, isochromanyl, isoindazolyl, isoindolinyl, isoindolyl, isoquinolinyl, isothiazolyl, isothiazolopyridinyl, isoxazolyl, isoxazolopyridinyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinylperimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridooxazolyl, pyridoimidazolyl, pyridothiazolyl, pyridinyl, pyrimidinyl, pyrrolidinyl, pyrrolinyl, 2-pyrrolidonyl, 2*H*-pyrrolyl, pyrrolyl, quinazolinyl, quinolinyl, 4*H*-quinolizinyl, quinoxalinyl, quinuclidinyl, tetrazolyl, tetrahydrofuranyl, tetrahydroisoquinolinyl, tetrahydroquinolinyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridinyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl. Also included are fused ring and spiro compounds containing, for example, the above heterocycles.

Examples of 5- to 10-membered heterocycles include, but are not limited to, pyridinyl, furanyl, thienyl, pyrrolyl, pyrazolyl, pyrazinyl, piperazinyl, piperidinyl, imidazolyl, imidazolidinyl, indolyl, tetrazolyl, isoxazolyl, morpholinyl, oxazolyl, oxadiazolyl, oxazolidinyl, tetrahydrofuranyl, thiadiazinyl, thiadiazolyl, thiazolyl, triazinyl, triazolyl, benzimidazolyl, 1*H*-indazolyl, benzofuranyl, benzothiofuranyl, benztetrazolyl, benzotriazolyl, benzisoxazolyl, benzoxazolyl, oxindolyl, benzoxazolinyl, benzthiazolyl, benzisothiazolyl, isatinoyl, isoquinolinyl, octahydroisoquinolinyl, tetrahydroisoquinolinyl, tetrahydroquinolinyl, isoxazolopyridinyl, quinazolinyl, quinolinyl, isothiazolopyridinyl, thiazolopyridinyl, oxazolopyridinyl, imidazolopyridinyl, and pyrazolopyridinyl.

Examples of 5- to 6-membered heterocycles include, but are not limited to, pyridinyl, furanyl, thienyl, pyrrolyl, pyrazolyl, pyrazinyl, piperazinyl, piperidinyl, imidazolyl, imidazolidinyl, indolyl, tetrazolyl, isoxazolyl, morpholinyl, oxazolyl, oxadiazolyl, oxazolidinyl, tetrahydrofuranyl, thiadiazinyl, thiadiazolyl, thiazolyl, triazinyl, and triazolyl. Also included are fused ring and spiro compounds containing, for example, the above heterocycles.

As used herein, the term "bicyclic heterocycle" or "bicyclic heterocyclic group" is intended to mean a stable 9- or 10-membered heterocyclic ring system which contains two fused rings and consists of carbon atoms and 1, 2, 3, or 4 heteroatoms independently selected from the group consisting of N, O and S. Of the two fused rings, one ring is a 5- or 6-membered monocyclic aromatic ring comprising a 5-membered heteroaryl ring, a 6-membered heteroaryl ring or a benzo ring, each fused to a second ring. The second ring is a 5- or 6-membered monocyclic ring which is saturated, partially unsaturated, or unsaturated, and comprises a 5-membered heterocycle, a 6-membered heterocycle or a carbocycle (provided the first ring is not benzo when the second ring is a carbocycle).

The bicyclic heterocyclic group may be attached to its pendant group at any heteroatom or carbon atom which results in a stable structure. The bicyclic heterocyclic group described herein may be substituted on carbon or on a nitrogen atom if the resulting compound is stable. It is preferred that when the total number of S and O atoms in the heterocycle exceeds 1, then these heteroatoms are not adjacent to one another. It is preferred that the total number of S and O atoms in the heterocycle is not more than 1.

Examples of a bicyclic heterocyclic group are, but not limited to, quinolinyl, isoquinolinyl, phthalazinyl, quinazolinyl, indolyl, isoindolyl, indolinyl, 1*H*-indazolyl, benzimidazolyl, 1,2,3,4-tetrahydroquinolinyl, 1,2,3,4-tetrahydroisoquinolinyl, 5,6,7,8-tetrahydro-quinolinyl, 2,3-dihydro-benzofuranyl, chromanyl, 1,2,3,4-tetrahydro-quinoxalinyl, and 1,2,3,4-tetrahydro-quinazolinyl.

As used herein, the term "aromatic heterocyclic group" or "heteroaryl" is intended to mean stable monocyclic and polycyclic aromatic hydrocarbons that include at least one heteroatom ring member such as sulfur, oxygen, or nitrogen. Heteroaryl groups include, without limitation, pyridyl, pyrimidinyl, pyrazinyl, pyridazinyl, triazinyl, furyl, quinolyl, isoquinolyl, thienyl, imidazolyl, thiazolyl, indolyl, pyrroyl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, purinyl, carbazolyl, benzimidazolyl, indolinyl, benzodioxolanyl, and benzodioxane. Heteroaryl groups are substituted or unsubstituted. The nitrogen atom is substituted or unsubstituted (*i.e.,* N or NR wherein R is H or another substituent, if defined). The nitrogen and sulfur heteroatoms may optionally be oxidized (*i.e.,* N→O and S(O)ₚ, wherein p is 0, 1 or 2).

Examples of 5- to 6-membered heteroaryls include, but are not limited to, pyridinyl, furanyl, thienyl, pyrrolyl, pyrazolyl, pyrazinyl, imidazolyl, imidazolidinyl, tetrazolyl, isoxazolyl, oxazolyl, oxadiazolyl, oxazolidinyl, thiadiazinyl, thiadiazolyl, thiazolyl, triazinyl, and triazolyl.

Bridged rings are also included in the definition of heterocycle. A bridged ring occurs when one or more, preferably one to three, atoms (*i.e.,* C, O, N, or S) link two non-adjacent carbon or nitrogen atoms. Examples of bridged rings include, but are not limited to, one carbon atom, two carbon atoms, one nitrogen atom, two nitrogen atoms, and a carbon-nitrogen group. It is noted that a bridge always converts a monocyclic ring into a tricyclic ring. When a ring is bridged, the substituents recited for the ring may also be present on the bridge.

The term "counter ion" is used to represent a negatively charged species such as chloride, bromide, hydroxide, acetate, and sulfate or a positively charged species such as sodium (Na⁺), potassium (K⁺), ammonium (RₙNHₘ+ where n=0-4 and m=0-4) and the like.

When a dotted ring is used within a ring structure, this indicates that the ring structure may be saturated, partially saturated or unsaturated.

As used herein, the term "amine protecting group" means any group known in the art of organic synthesis for the protection of amine groups which is stable to an ester reducing agent, a disubstituted hydrazine, R4-M and R7-M, a nucleophile, a hydrazine reducing agent, an activator, a strong base, a hindered amine base and a cyclizing agent. Such amine protecting groups fitting these criteria include those listed in Wuts, P.G.M. et al., Protecting Groups in Organic Synthesis, 4th Edition, Wiley (2007) and The Peptides: Analysis, Synthesis, Biology, Vol. 3, Academic Press, New York (1981). Examples of amine protecting groups include, but are not limited to, the following: (1) acyl types such as formyl, trifluoroacetyl, phthalyl, and p-toluenesulfonyl; (2) aromatic carbamate types such as benzyloxycarbonyl (Cbz) and substituted benzyloxycarbonyls, 1-(p-biphenyl)-1-methylethoxycarbonyl, and 9-fluorenylmethyloxycarbonyl (Fmoc); (3) aliphatic carbamate types such as tert-butyloxycarbonyl (Boc), ethoxycarbonyl, diisopropylmethoxycarbonyl, and allyloxycarbonyl; (4) cyclic alkyl carbamate types such as cyclopentyloxycarbonyl and adamantyloxycarbonyl; (5) alkyl types such as triphenylmethyl and benzyl; (6) trialkylsilane such as trimethylsilane; (7) thiol containing types such as phenylthiocarbonyl and dithiasuccinoyl; and (8) alkyl types such as triphenylmethyl, methyl, and benzyl; and substituted alkyl types such as 2,2,2-trichloroethyl, 2-phenylethyl, and t-butyl; and trialkylsilane types such as trimethylsilane.

As referred to herein, the term "substituted" means that at least one hydrogen atom is replaced with a non-hydrogen group, provided that normal valencies are maintained and that the substitution results in a stable compound. Ring double bonds, as used herein, are double bonds that are formed between two adjacent ring atoms (*e.g*., C=C, C=N, or N=N).

In cases wherein there are nitrogen atoms (*e.g*., amines) on compounds of the present invention, these may be converted to N-oxides by treatment with an oxidizing agent (*e.g.*, mCPBA and/or hydrogen peroxides) to afford other compounds of this invention. Thus, shown and claimed nitrogen atoms are considered to cover both the shown nitrogen and its N-oxide (N→O) derivative.

When any variable occurs more than one time in any constituent or formula for a compound, its definition at each occurrence is independent of its definition at every other occurrence. Thus, for example, if a group is shown to be substituted with 0-3 R, then said group may optionally be substituted with up to three R groups, and at each occurrence R is selected independently from the definition of R.

When a bond to a substituent is shown to cross a bond connecting two atoms in a ring, then such substituent may be bonded to any atom on the ring. When a substituent is listed without indicating the atom in which such substituent is bonded to the rest of the compound of a given formula, then such substituent may be bonded via any atom in such substituent.

Combinations of substituents and/or variables are permissible only if such combinations result in stable compounds.

The phrase "pharmaceutically acceptable" is employed herein to refer to those compounds, materials, compositions, and/or dosage forms that are, within the scope of sound medical judgment, suitable for use in contact with the tissues of human beings and animals without excessive toxicity, irritation, allergic response, and/or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic groups such as amines; and alkali or organic salts of acidic groups such as carboxylic acids. The pharmaceutically acceptable salts include the conventional non-toxic salts or the quaternary ammonium salts of the parent compound formed, for example, from non-toxic inorganic or organic acids. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

Salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Allen, Jr., L.V., ed., Remington: The Science and Practice of Pharmacy, 22nd Edition, Pharmaceutical Press, London, UK (2012).

The present invention is intended to include all isotopes of atoms occurring in the present compounds. Isotopes include those atoms having the same atomic number but different mass numbers. By way of general example and without limitation, isotopes of hydrogen include deuterium (symbol D or 2H) and tritium (symbol T or 3H). For example, a methyl group may be represented by CH₃ or CD₃. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

The term "solvate" means a physical association of a compound of this invention with one or more solvent molecules, whether organic or inorganic. This physical association includes hydrogen bonding. In certain instances the solvate will be capable of isolation, for example when one or more solvent molecules are incorporated in the crystal lattice of the crystalline solid. The solvent molecules in the solvate may be present in a regular arrangement and/or a non-ordered arrangement. The solvate may comprise either a stoichiometric or nonstoichiometric amount of the solvent molecules. "Solvate" encompasses both solution-phase and isolable solvates. Exemplary solvates include, but are not limited to, hydrates, ethanolates, methanolates, and isopropanolates. Methods of solvation are generally known in the art.

The terms "measurable affinity" and "measurably inhibit," as used herein, means a measurable change in PAD4 activity between a sample comprising a compound of the present invention, or composition thereof, and PAD4, and an equivalent sample comprising PAD4 in the absence of said compound, or composition thereof.

Abbreviations as used herein, are defined as follows: "1 x" for once, "2 x" for twice, "3 x" for thrice, "°C" for degrees Celsius, "eq" for equivalent or equivalents, "g" for gram or grams, "mg" for milligram or milligrams, "L" for liter or liters, "mL" for milliliter or milliliters, "µL" for microliter or microliters, "N" for normal, "M" for molar, "mmol" for millimole or millimoles, "min" for minute or min, "h" for hour or h, "rt" for room temperature, "RT" for retention time, "atm" for atmosphere, "psi" for pounds per square inch, "conc." for concentrate, "aq" for "aqueous", "sat" or "sat'd " for saturated, "MW" for molecular weight, "mp" for melting point, "MS" or "Mass Spec" for mass spectrometry, "ESI" for electrospray ionization mass spectroscopy, "HR" for high resolution, "HRMS" for high resolution mass spectrometry, "LCMS" for liquid chromatography mass spectrometry, "HPLC" for high pressure liquid chromatography, "RP HPLC" for reverse phase HPLC, "TLC" or "tlc" for thin layer chromatography, "NMR" for nuclear magnetic resonance spectroscopy, "nOe" for nuclear Overhauser effect spectroscopy, "¹H" for proton, "δ" for delta, "s" for singlet, "d" for doublet, "t" for triplet, "q" for quartet, "m" for multiplet, "br" for broad, "Hz" for hertz, and "α", "β", "R", "S", "E", "Z" and "ee" are stereochemical designations familiar to one skilled in the art. As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio.
- AcOH or HOAc: acetic acid
- ACN: acetonitrile
- Alk: Alkyl
- AlMe₃: Trimethylaluminum
- BBr₃: boron tribromide
- Bn: benzyl
- Boc: *tert*-butyloxycarbonyl
- BOP reagent: benzotriazol-1-yloxytris(dimethylamino)phosphonium hexafluorophosphate
- Bu: butyl
- *i*-Bu: isobutyl
- *t*-Bu: *tert*-butyl
- *t*-BuOH: *tert*-butanol
- Cbz: carbobenzyloxy
- CDCl₃: deutero-chloroform
- CD₃OD: deutero-methanol
- CH₂Cl₂: dichloromethane
- CH₃CN: acetonitrile
- CHCl₃: chloroform
- DCM: dichloromethane
- DIEA, DIPEA or: diisopropylethylamine
- Hunig's base DMF: dimethyl formamide
- DMSO: dimethyl sulfoxide
- Et: ethyl
- Et₃N or TEA: triethylamine
- Et₂O: diethyl ether
- EtOAc: ethyl acetate
- EtOH: ethanol
- HCl: hydrochloric acid
- HPLC: high-performance liquid chromatography
- K₂CO₃: potassium carbonate
- K₂HPO₄: potassium hydrogenphosphate
- LCMS: liquid chromatography mass spectrometry
- LiHMDS: lithium bis(trimethylsilyl)amide
- LG: leaving group
- Me: methyl
- MeOH: methanol
- MgSO₄: magnesium sulfate
- MsOH or MSA: methylsulfonic acid
- NaCl: sodium chloride
- Na₂CO₃: sodium carbonate
- NaHCO₃: sodium bicarbonate
- NaOH: sodium hydroxide
- Na₂SO₄: sodium sulfate
- NH₃: ammonia
- NH₄Cl: ammonium chloride
- NH₄OAc: ammonium acetate
- Pd(OAc)₂: palladium(II) acetate
- Pd(dppf)Cl₂: [1,1'-Bis(diphenylphosphino)ferrocene]*palladium*(II) dichloride
- Pd(PPh₃)₄: tetrakis(triphenylphosphine)palladium(0)
- PG: protecting group
- Ph: phenyl
- Pr: propyl
- *i*-Pr: isopropyl
- *i*-PrOH or IPA: isopropanol
- Rt: retention time
- SiO₂: silica oxide
- SFC: supercritical fluid chromatography
- TBAI: Tetrabutylammonium iodide
- TEA: triethylamine
- TFA: trifluoroacetic acid
- TFAA: Trifluoroacetic anhydride
- THF: tetrahydrofuran
- TiCl₄: titanium tetrachloride
- T3P: 1-propanephosphonic acid cyclic anhydride

### 3. Description of Exemplary Compounds

In a first aspect, the present invention provides a compound of Fomula (III): 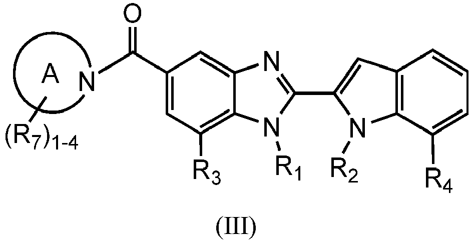 or a pharmaceutically acceptable salt thereof, wherein: 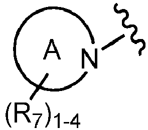 is selected from 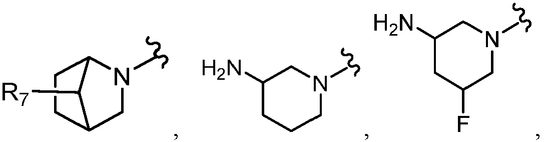 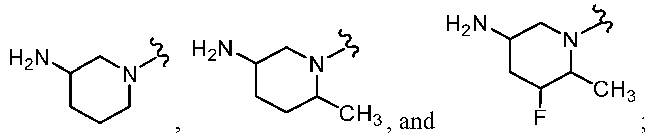
R₁ is selected from -CH₃, -CD₃, and -CH₂-5-6 membered heterocyclyl comprising carbon atoms and 1-3 heteroatoms selected from N, NH, and NC₁₋₃alkyl;
R₂ is selected from CH₃, CH₃CH₂, and -CH₂-cyclopropyl substituted with 0-3 Rₑ;
R₃ is selected from H, F, Cl, Br, and -OC₁₋₄ alkyl;
R₄ is selected from 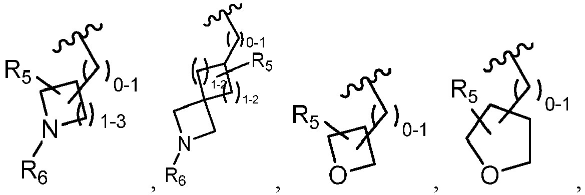 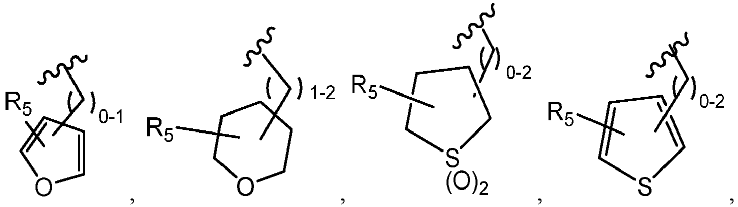 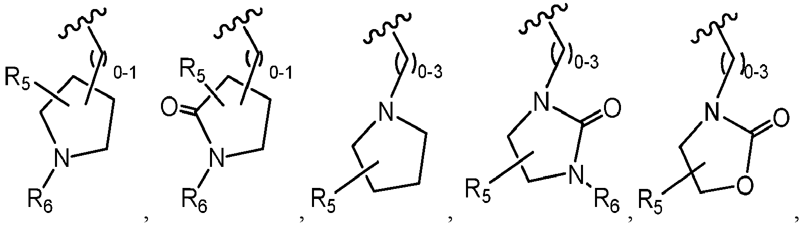 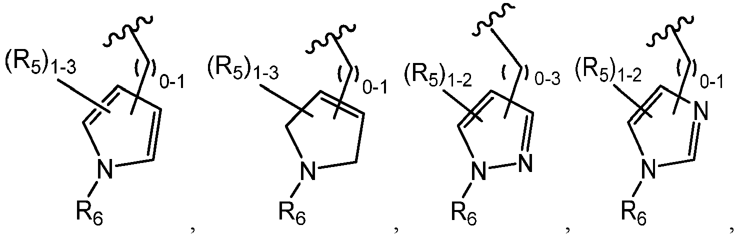 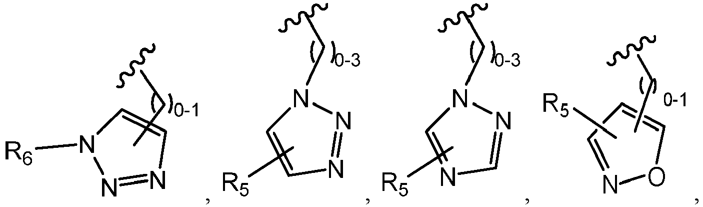 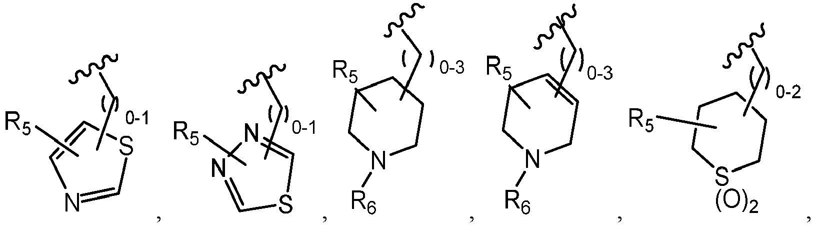 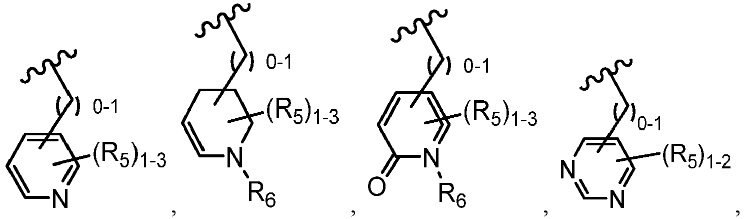 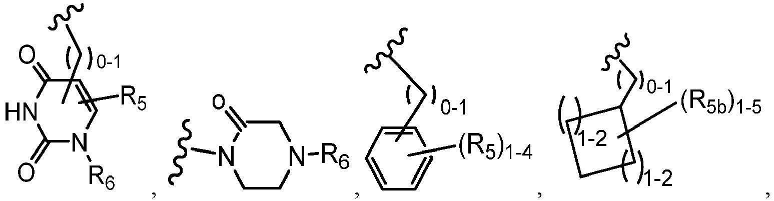 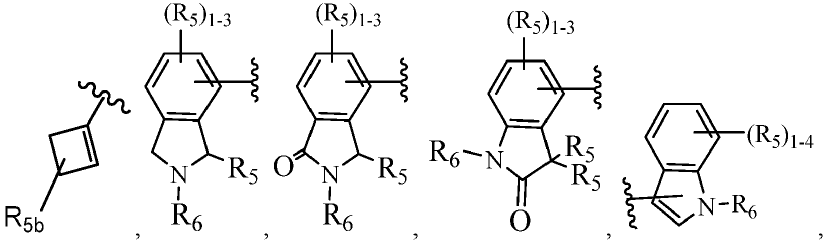 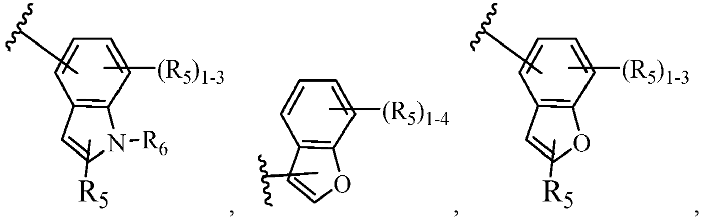 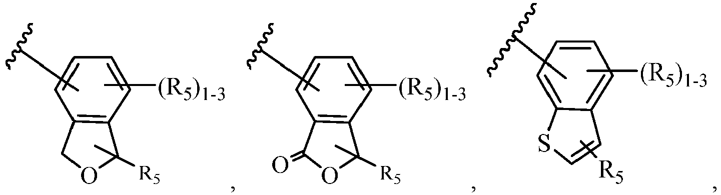 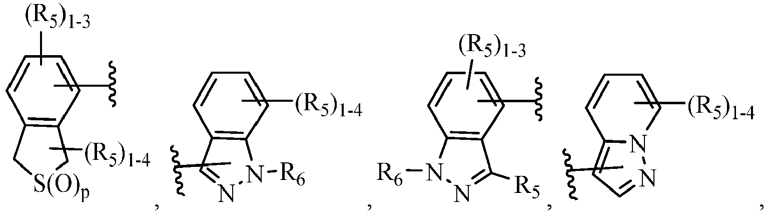 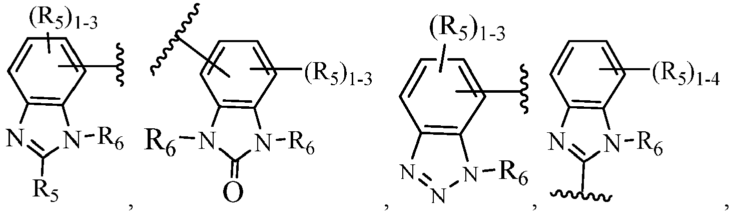 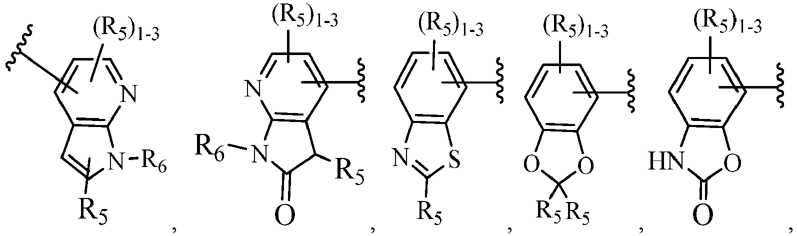 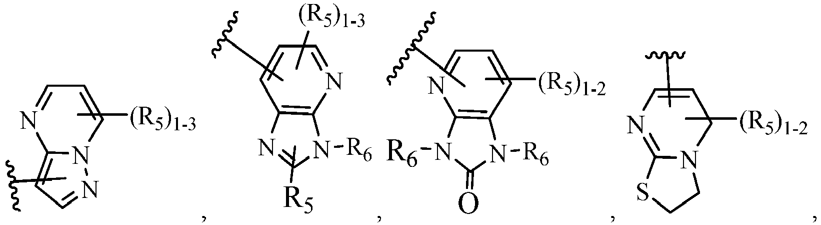 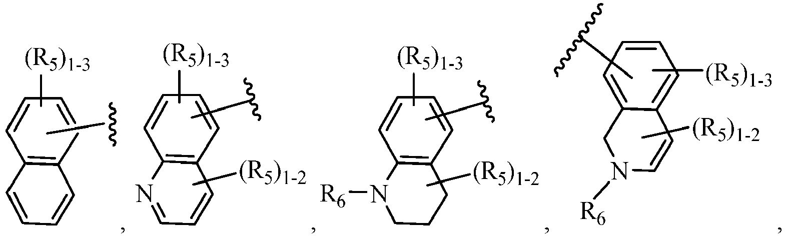 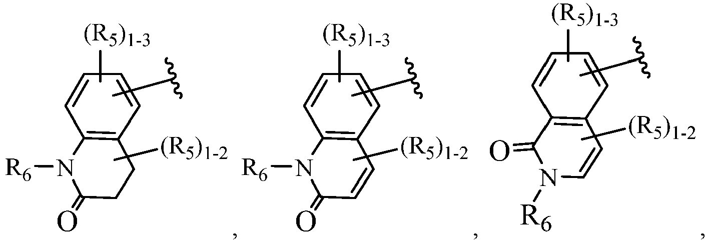 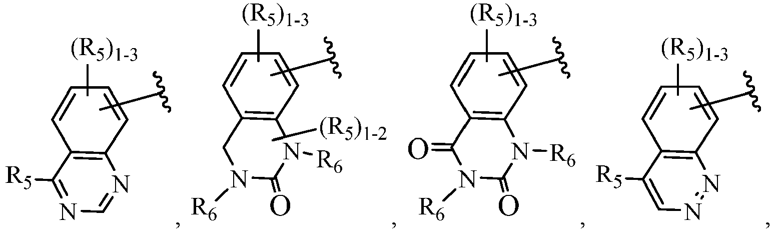 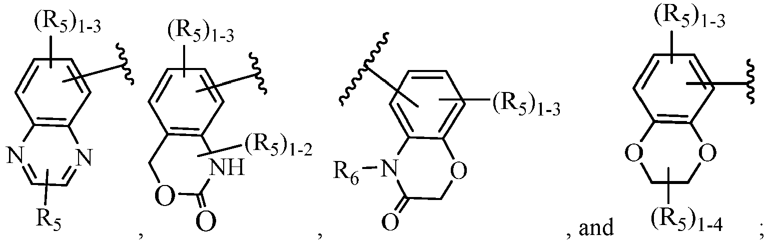
R₅, at each occurrence, is independently selected from H, F, Cl, Br, C₁₋₄alkyl substituted with 0-5 Rₑ, C₂₋₄alkenyl, C₂₋₄alkynyl, nitro, --(CH₂)ᵣOR_{b}, -CN, -NRₐRₐ, --(CH₂)ᵣNRₐC(=O)R_{b}, -NRₐC(=O)NRₐRₐ, -C(=O)OR_{b}, -C(=O)R_{b}, -OC(=O)R_{b}, - C(=O)NRₐRₐ, -P(=O)(C₁₋₄alkyl)₂, -S(O)*p*R_{c}, -S(O)*ₚ*NRₐRₐ, -NRₐS(O)*ₚ*R_{c}, -C3-6 cycloalkyl substituted with 0-4 Rₑ, aryl substituted with 0-4 Rₑ, and heterocyclyl substituted with 0-4 Rₑ;
R_{5b}, at each occurrence, is independently selected from OR_{b}, -C(=O)OR_{b}, -C(=O)NRₐRₐ, - NRₐRₐ, -NRₐC(=O)R_{b}, -NRₐC(=O)OR_{b}, -S(=O)pR_{c}, and -NRₐS(=O)pR_{c};
R₆, at each occurrence, is independently selected from H, C₁₋₃alkyl substituted with 0-4 Rₑ, -C(=O)R_{b}, -C(=O)(CH₂)ᵣOR_{b}, -C(=O)(CH₂)ᵣNRₐRₐ, -S(O)ₚR_{c}, -S(O)ₚNRₐRₐ, - (CH₂)ᵣ-aryl substituted with 0-4 Rₑ, and -(CH₂)ᵣ-heterocyclyl substituted with 0-4 Rₑ;
R₇ is selected from H, F, Cl, CN, C₁₋₃ alkyl, =N-OR_{b}, -(CH₂)ᵣOR_{b}, -(CH₂)ᵣNRₐRₐ, - NRₐC(=NH)C₁₋₃alkyl, -NRₐC(=O)OR_{b}, a carbocyclyl, and heterocyclyl;
Rₐ, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ;
R_{c}, at each occurrence, is independently selected from C₁₋₆ alkyl substituted with 0-5 Rₑ, C₂₋₆alkenyl substituted with 0-5 Rₑ, C₂₋₆ alkynyl substituted with 0-5 Rₑ, -(CH₂)ᵣ-C₃₋₆ carbocyclyl substituted with 0-5 Rₑ, and -(CH₂)ᵣ-heterocyclyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, -(CH₂)ᵣOC₁₋₄alkyl, -(CH₂)ᵣOC₂₋₄alkenyl, -(CH₂)ᵣOC₂₋₄alkynyl, and NH₂;
Rε, at each occurrence, is independently selected from H, F, Cl, Br, CN, OH, C₁₋₅ alkyl optionally substituted with OH, C₃₋₆ cycloalkyl, and phenyl;
p, at each occurrence, is independently selected from zero, 1, and 2; and
r, at each occurrence, is independently selected from zero, 1, 2, 3, and 4.

In a second aspect, the present invention provides a compound of Formula (III), or a pharmaceutically acceptable salt thereof, within the scope of the first aspect, wherein: 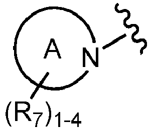 is selected from 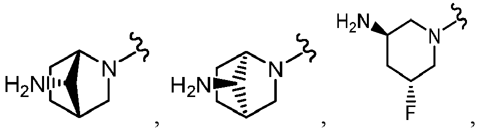 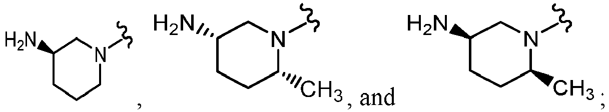
R₁ is selected from -CH₃ and -CD₃;
R₂ is selected from -CH₃ and -CH₂-cyclopropyl substituted with 0-2 F or Cl;
R₃ is selected from H, F and -OC₁₋₄ alkyl;
R₄ is selected from 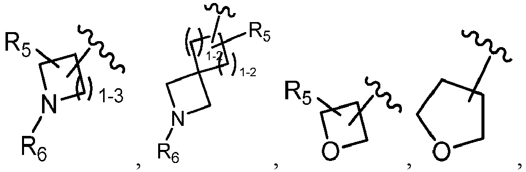 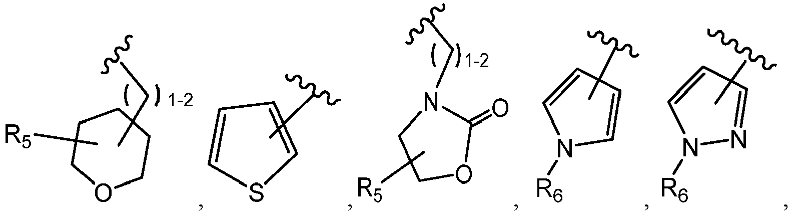 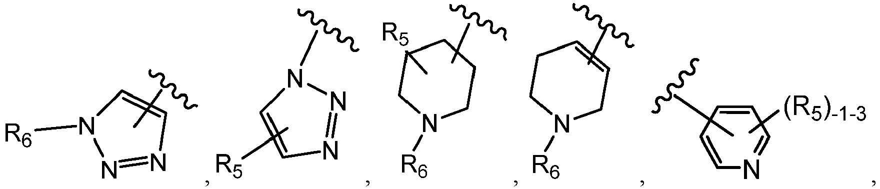 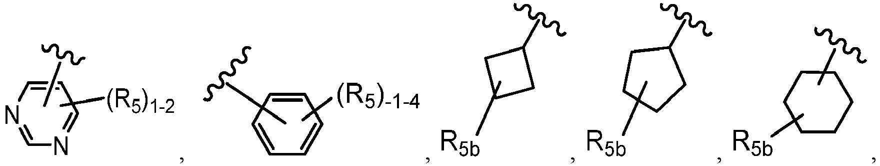 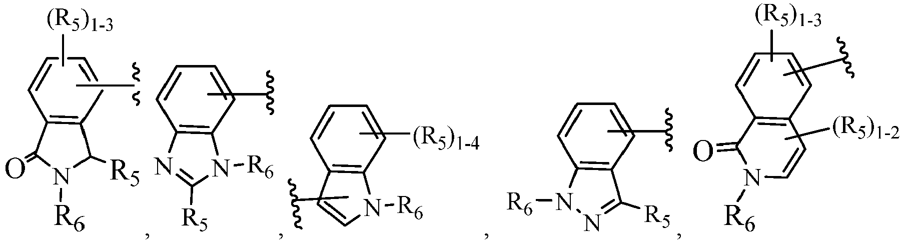 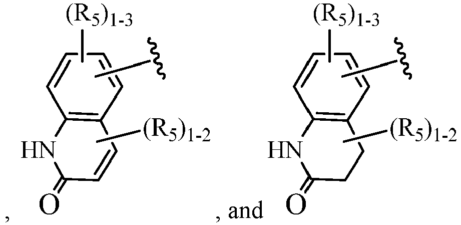
R₅, at each occurrence, is independently selected from H, F, Cl, Br,
   C₁₋₄alkyl, -(CH2)ᵣOR_{b}, -CN, -NRₐRₐ, -(CH2)ᵣNRₐC(=O)Rb, -NRₐC(=O)NRₐRₐ, -
   C(=O)OR_{b}, -C(=O)R_{b}, -OC(=O)R_{b}, -
   C(=O)NRₐRₐ, -S(O)*ₚ*R_{c}, -S(O)*ₚ*NRₐRₐ, -NRₐS(O)*ₚ*R_{c}, C₃₋₆cycloalkyl substituted
   with 0-4 Rₑ, aryl substituted with 0-4 Rₑ, and heterocyclyl substituted with 0-4 Rₑ;
R_{5b}, at each occurrence, is independently selected from OH, -C(=O)OR_{b}, -C(=O)NRₐRₐ, - NRₐRₐ, -NRₐC(=O)R_{b}, and -NRₐC(=O)OR_{b};
R₆, at each occurrence, is independently selected from H, C₁₋₃alkyl, -S(O)ₚR_{c}, -C(=O)R_{b}, -C(=O)OR_{b}, -C(=O)(CH₂)ᵣNRₐRₐ, -S(O)ₚNRₐRₐ, aryl substituted with 0-4 Rₑ, and heterocyclyl substituted with 0-4 Rₑ;
Rₐ, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, -(CH₂)ᵣ-C₃₋₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, -(CH₂)ᵣ-C₃₋₁₀carbocyclyl substituted with 0-5 Rₑ, and -(CH₂)ᵣ-heterocyclyl substituted with 0-5 Rₑ;
R_{c}, at each occurrence, is independently selected from C₁₋₆ alkyl substituted with 0-5 Rₑ, C₂₋₆alkenyl substituted with 0-5 Rₑ, C₂₋₆ alkynyl substituted with 0-5 Rₑ, -(CH₂)ᵣ-C₃₋₆ carbocyclyl substituted with 0-5 Rₑ, and -(CH₂)ᵣ-heterocyclyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, -(CH₂)ᵣOC₁₋₄alkyl, -(CH₂)ᵣOC₂₋₄alkenyl, -(CH₂)ᵣOC₂₋₄alkynyl, and NH₂;
Rε, at each occurrence, is independently selected from H, F, Cl, Br, CN, OH, C₁₋₅ alkyl optionally substituted with OH, C₃₋₆ cycloalkyl, and phenyl;
p, at each occurrence, is independently selected from zero, 1, and 2; and
r, at each occurrence, is independently selected from zero, 1, 2, 3, and 4.

In a third aspect, the present invention provides a compound of Formula (III), or a pharmaceutically acceptable salt thereof, within the scope of the first aspect, wherein: R₁ is -CH₃;
R₂ is selected from -CH₃ and -CH₂-cyclopropyl; R₃ is -OC₁₋₄ alkyl;
R₄ is selected from 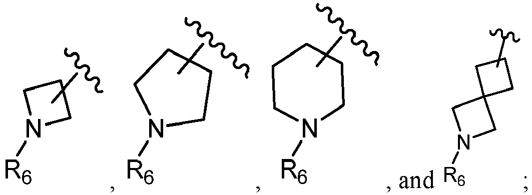
R₆ is selected from H, C₁₋₃ alkyl, -C(=O)R_{b}, -C(=O)OR_{b}, -C(=O)NRₐRₐ, and - C(=O)CH₂NRₐRₐ, and -S(O)ₚR_{c};
Rₐ, at each occurrence, is independently selected from H, C₁₋₆ alkyl substituted with 0-5 Rₑ, -(CH₂)ᵣ-C₃₋₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ;
R_{c}, at each occurrence, is independently C₁₋₆ alkyl substituted with 0-5 Rₑ, -(CH₂)ᵣ-C₃₋₆ carbocyclyl substituted with 0-5 Rₑ, or -(CH₂)ᵣ-heterocyclyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl;
Rε, at each occurrence, is independently selected from H, F, Cl, Br, CN, OH, C₁₋₅ alkyl optionally substituted with OH, C₃₋₆ cycloalkyl, and phenyl;
p, at each occurrence, is independently selected from zero, 1, and 2; and
r, at each occurrence, is independently selected from zero, 1, 2, 3, and 4.

In a fourth aspect, the present invention provides a compound of Formula (III), or a pharmaceutically acceptable salt thereof, within the scope of the first aspect, wherein:
R₄ is selected from 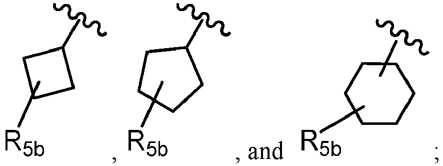
R_{5b}, at each occurrence, is independently selected from OH, -C(=O)OR_{b}, and - C(=O)NRₐRₐ, -NRₐRₐ, -NRₐC(=O)R_{b};
Rₐ, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl;
Rε, at each occurrence, is independently selected from H, F, Cl, Br, CN, OH, C₁₋₅ alkyl optionally substituted with OH, C₃₋₆ cycloalkyl, and phenyl;
p, at each occurrence, is independently selected from zero, 1, and 2; and
r, at each occurrence, is independently selected from zero, 1, 2, 3, and 4.

In an fifth aspect, the present invention provides a compound of Formula (III), or a pharmaceutically acceptable salt thereof, within the scope of the fourth aspect, wherein:
R₄ is 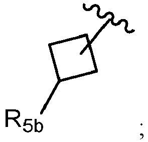
R_{5b} is selected from -C(=O)OR_{b}, -C(=O)NRₐRₐ, and -NHC(=O)R_{b};
Rₐ, at each occurrence, is independently selected from H and C₁₋₆ alkyl substituted with 0-5 Rₑ, or Rₐ and Rₐ together with the nitrogen atom to which they are both attached form a heterocyclic ring selected from 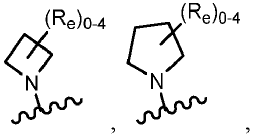 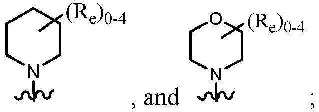
R_{b} is selected from H and C₁₋₆ alkyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl; and
Rε, at each occurrence, is independently selected from H, F, Cl, Br, CN, OH, C₁₋₅ alkyl optionally substituted with OH, C₃₋₆ cycloalkyl, and phenyl.

In a sixth aspect, the present invention provides a compound of Formula (III), or a pharmaceutically acceptable salt thereof, within the scope of the first aspect, wherein:
R₄ is selected from 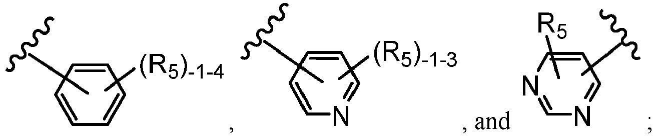
R₅, at each occurrence, is independently selected from H, F, Cl, Br, C₁₋₄alkyl substituted with 0-5 Rₑ, C₂₋₄alkenyl, C₂₋₄alkynyl, -(CH₂)₀₋₁OR_{b}, -CN, -NRₐRₐ, -(CH₂)₀₋₁-NHC(=O)R_{b}, -NRₐC(=O)NRₐRₐ, -C(=O)OR_{b}, -C(=O)R_{b}, -OC(=O)R_{b}, - C(=O)NRₐRₐ, -S(O)*ₚ*R_{c}, -S(O)ₚNRₐRₐ, -NRₐS(O)*ₚ*R_{c}, C₃₋₆ cycloalkyl, heterocyclyl, and aryl, wherein said alkyl, cycloalkyl, heterocyclyl, or aryl is substituted with 0-4 Rₑ;
Rₐ, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, -(CH₂)ᵣ-C₃₋₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ;
R_{c}, at each occurrence, is independently selected from C₁₋₆ alkyl substituted with 0-5 Rₑ, C₂₋₆alkenyl substituted with 0-5 Rₑ, C₂₋₆ alkynyl substituted with 0-5 Rₑ, -(CH₂)ᵣ-C₃₋₆ carbocyclyl substituted with 0-5 Rₑ, and -(CH₂)ᵣ-heterocyclyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl;
Rε, at each occurrence, is independently selected from H, F, Cl, Br, CN, OH, C₁₋₅ alkyl optionally substituted with OH, C₃₋₆ cycloalkyl, and phenyl;
p, at each occurrence, is independently selected from zero, 1, and 2; and
r, at each occurrence, is independently selected from zero, 1, 2, 3, and 4.

In a seventh aspect, the present invention provides a compound of Formula (III), or a pharmaceutically acceptable salt thereof, within the scope of the first aspect, wherein:
R₂ is selected from -CH₃ and -CH₂-cyclopropyl substituted with 0-2 F or Cl;
R₄, at each occurrence, is selected from 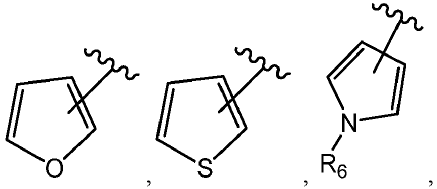 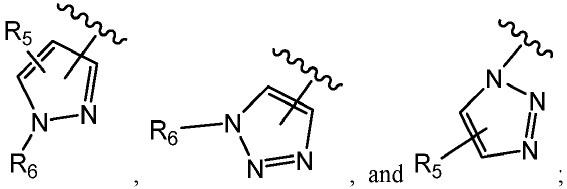
R₅, at each occurrence, is independently selected from H, F, Cl, Br, C₁₋₄alkyl substituted with 0-5 Rₑ, C₂₋₄alkenyl, C₂₋₄alkynyl, nitro, -OR_{b}, -CN, -NRₐRₐ, -S(O)*ₚ*R_{c}, - S(O)*ₚ*NRₐRₐ, -NRₐS(O)*ₚ*R_{c}, -(CH₂)ᵣ-NRₐC(=O)R_{b}, -NRₐC(=O)NRₐRₐ, C(=O)OR_{b}, -C(=O)R_{b}, -OC(=O)R_{b}, -C(=O)NRₐRₐ, P(=O)(OC₁₋₄alkyl)₂, C₃₋₆ cycloalkyl substituted with 0-4 Rₑ, aryl substituted with 0-4 Rₑ, heterocyclyl, substituted with 0-4 Rₑ;
R₆, at each occurrence, is independently selected from H, C₁₋₃alkyl substituted with 0-4 Rₑ, and heterocyclyl substituted with 0-4 Rₑ;
Rₐ, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, -(CH₂)ᵣ-C₃₋₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ;
R_{c}, at each occurrence, is independently selected from C₁₋₆ alkyl substituted with 0-5 Rₑ, C₂₋₆alkenyl substituted with 0-5 Rₑ, C₂₋₆ alkynyl substituted with 0-5 Rₑ, -(CH₂)ᵣ-C₃₋₆ carbocyclyl substituted with 0-5 Rₑ, and -(CH₂)ᵣ-heterocyclyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, -(CH₂)ᵣOC₁₋₄alkyl, -(CH₂)ᵣOC₂₋₄alkenyl, -(CH₂)ᵣOC₂₋₄alkynyl, and NH₂;
Rε, at each occurrence, is independently selected from H, F, Cl, Br, CN, OH, C₁₋₅ alkyl optionally substituted with OH, C₃₋₆ cycloalkyl, and phenyl;
p, at each occurrence, is independently selected from zero, 1, and 2; and
r, at each occurrence, is independently selected from zero, 1, 2, 3, and 4.

In a eighth aspect, the present invention provides a compound of Formula (III), or a pharmaceutically acceptable salt thereof, within the scope of the seventh aspect, wherein:
R₂ is selected from -CH₃ and -CH₂-cyclopropyl substituted with 0-2 F and Cl;
R₄ is 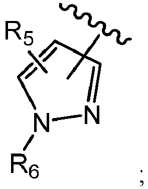
R₅, at each occurrence, is independently selected from H, F, Cl, Br, C₁₋₄alkyl, aryl substituted with 0-4 Rₑ, and heterocyclyl substituted with 0-4 Rₑ;
R₆, at each occurrence, is independently selected from H, C₁₋₃alkyl substituted with 0-4 Rₑ, and heterocyclyl substituted with 0-4 Rₑ;
Rₑ, at each occurrence, is independently selected from C₁₋₆ alkyl, F, Cl, Br, CN, -OH, and -(CH₂)ᵣOC₂₋₄alkynyl; and
r, at each occurrence, is independently selected from zero, 1 and 2.

In a ninth aspect, the present invention provides a compound of Formula (III), or a pharmaceutically acceptable salt thereof, within the scope of the first aspect, wherein:
R₄ is selected from 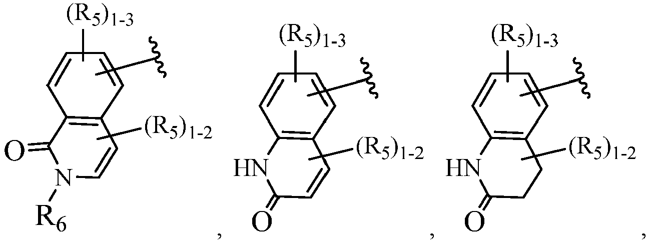 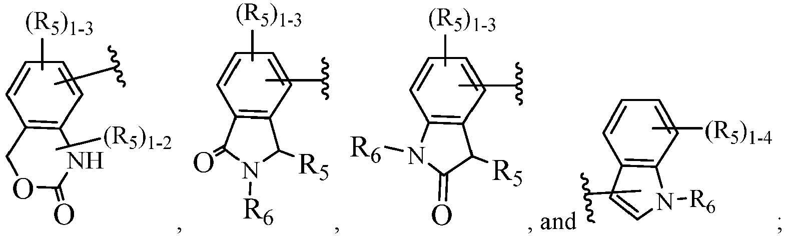
R₅, at each occurrence, is independently selected from H, F, Cl, Br, and C₁₋₄alkyl substituted with 0-5 Rₑ, OR_{b}, -CN, -C(=O)R_{b}, -C(=O)OR_{b}, -OC(=O)R_{b}, - C(=O)NRₐRₐ;
R₆ is selected from H and C₁₋₃alkyl;
Rₐ, at each occurrence, is independently selected from H and C₁₋₄alkyl substituted with 0-5 Rₑ;
R_{b}, at each occurrence, is independently selected from H and C₁₋₄alkyl substituted with 0-5 Rₑ: and
Rₑ, at each occurrence, is independently selected from C₁₋₆ alkyl F, Cl, Br, CN, NO₂, =O, -C(=O)OH, -C(=O)OC₁₋₄alkyl, -(CH₂)ᵣOH, and -(CH₂)ᵣOC₁₋₄alkyl; and
r, at each occurrence, is independently selected from zero, 1 and 2.

In a tenth aspect, the present invention provides a compound of Formula (III), or a pharmaceutically acceptable salt thereof, within the scope of the first aspect, wherein: 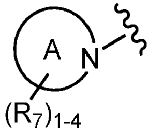 is selected from 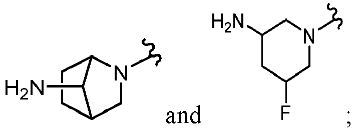
R₃ is selected from H, F, Cl, and -OC₁₋₄ alkyl;
R₄ is selected from 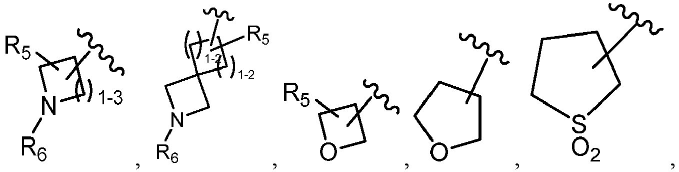 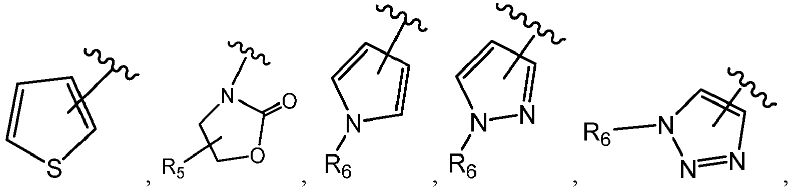 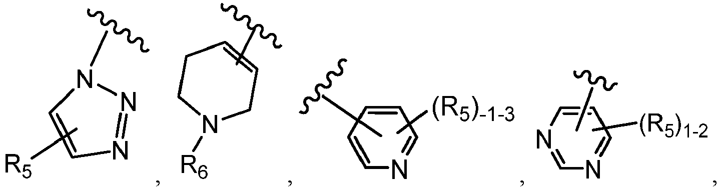 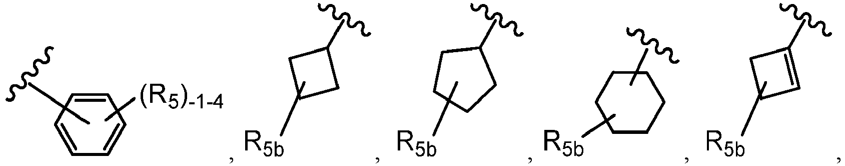 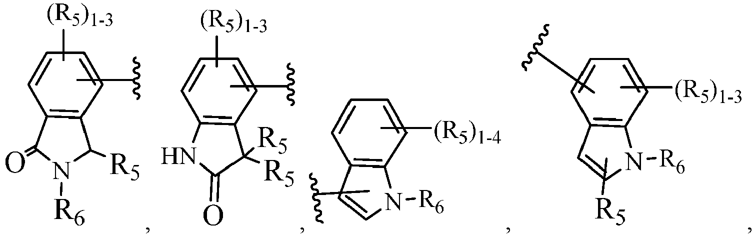 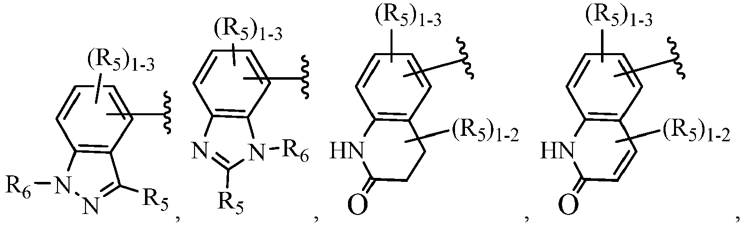 and 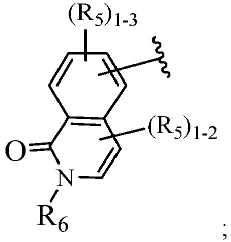
R₅, at each occurrence, is independently selected from H, F, Cl, Br, C₁₋₄alkyl substituted with 0-3 Rₑ, -OR_{b}, -CN, -NRₐRₐ, -NRₐC(=O)R_{b}, -NRₐC(=O)NRₐRₐ, -C(=O)OR_{b}, - C(=O)R_{b}, -OC(=O)R_{b}, -C(=O)NRₐRₐ, -S(O)*ₚ*R_{c}, -S(O)ₚNRₐRₐ, -NRₐS(O)*ₚ*R_{c}, C₃₋₆cycloalkyl substituted with 0-4 Rₑ, aryl substituted with 0-4 Rₑ, and heterocyclyl substituted with 0-4 Rₑ;
R_{5b}, at each occurrence, is independently selected from OH, -C(=O)OR_{b}, -C(=O)NRₐRₐ, and -NRₐC(=O)R_{b};
R₆, at each occurrence, is independently selected from H, C₁₋₃alkyl, -C(=O)R_{b}, - C(=O)(CH₂)ᵣOR_{b}, -C(=O)(CH₂)ᵣNRₐRₐ, -S(O)ₚR_{c}, -S(O)ₚNRₐRₐ, aryl substituted with 0-4 Rₑ, and heterocyclyl substituted with 0-4 Rₑ;
Rₐ, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, - (CH₂)ᵣ-C₃₋₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, -(CH₂)ᵣ-C₃₋₁₀carbocyclyl substituted with 0-5 Rₑ, and -(CH₂)ᵣ-heterocyclyl substituted with 0-5 Rₑ;
R_{c}, at each occurrence, is independently selected from C₁₋₆ alkyl substituted with 0-5 Rₑ, C₂₋₆alkenyl substituted with 0-5 Rₑ, C₂₋₆ alkynyl substituted with 0-5 Rₑ, -(CH₂)ᵣ-C₃₋₆ carbocyclyl substituted with 0-5 Rₑ, and -(CH₂)ᵣ-heterocyclyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, , F, Cl, Br, CN, NO₂, =O, -C(=O)OH, -C(=O)OC₁₋₄alkyl, -(CH₂)ᵣOH, and -(CH₂)ᵣOC₁₋₄alkyl;
Rε, at each occurrence, is independently selected from H, F, Cl, Br, CN, OH, C₁₋₅ alkyl optionally substituted with OH, C₃₋₆ cycloalkyl, and phenyl;
p, at each occurrence, is independently selected from zero, 1, and 2; and
r, at each occurrence, is independently selected from zero, 1, 2, 3, and 4.

In an eleventh aspect, the present invention provides a compound of Formula (III), or a pharmaceutically acceptable salt thereof, within the scope of the first aspect, wherein: 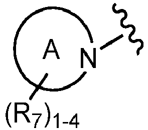 is selected from 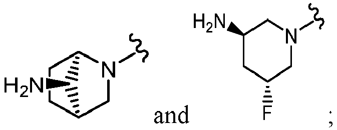
R₁ is selected from -CH₃, -CD₃, and -CH₂-5-6 membered heterocyclyl comprising carbon atoms and 1-3 heteroatoms selected from N, NH, and NC₁₋₃alkyl;
R₂ is selected from -CH₃ and -CH₂-cyclopropyl;
R₃ is selected from H, F, and -OC₁₋₄ alkyl;
R₄ is selected from 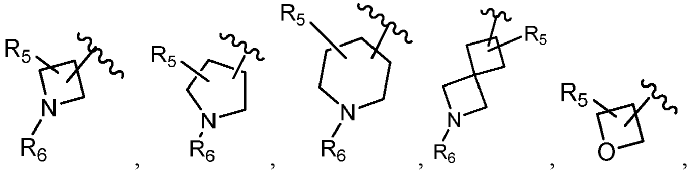 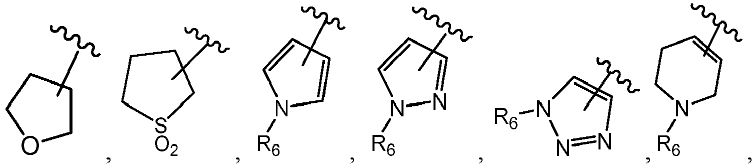 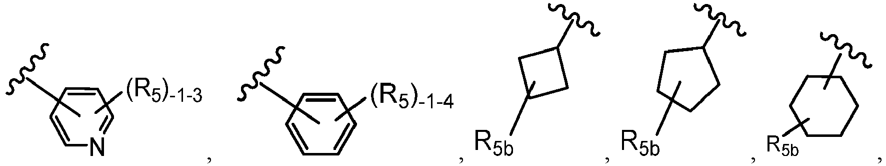 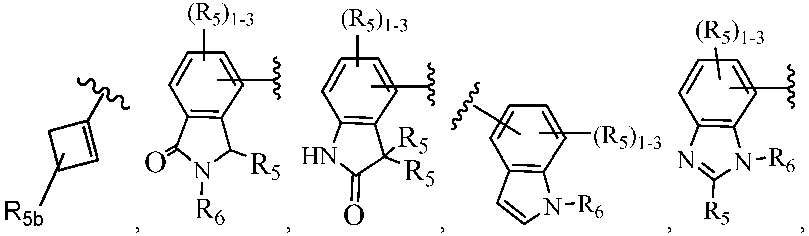 and 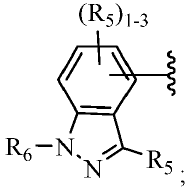
R₅, at each occurrence, is independently selected from H, F, Cl, Br, C₁₋₄alkyl substituted with 0-3 Rₑ, -OR_{b}, -CN, -NRₐRₐ;
R_{5b}, at each occurrence, is independently selected from OH, -C(=O)OR_{b}, -C(=O)NRₐRₐ, and -NRₐC(=O)R_{b};
R₆, at each occurrence, is independently selected from H, C₁₋₃alkyl, -C(=O)R_{b}, - C(=O)(CH₂)ᵣOR_{b}, -C(=O)(CH₂)ᵣNRₐRₐ, -S(O)ₚR_{c}, -S(O)ₚNRₐRₐ, aryl substituted with 0-4 Rₑ, and heterocyclyl substituted with 0-4 Rₑ;
Rₐ, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, -(CH₂)ᵣ-C₃₋₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁₋₆ alkyl substituted with 0-5 Rₑ, C₂₋₆ alkenyl substituted with 0-5 Rₑ, C₂₋₆ alkynyl substituted with 0-5 Rₑ, -(CH₂)ᵣ-C₃₋₁₀carbocyclyl substituted with 0-5 Rₑ, and -(CH₂)ᵣ-heterocyclyl substituted with 0-5 Rₑ;
R_{c}, at each occurrence, is independently selected from C₁₋₆ alkyl substituted with 0-5 Rₑ, C₂₋₆alkenyl substituted with 0-5 Rₑ, C₂₋₆ alkynyl substituted with 0-5 Rₑ, -(CH₂)ᵣ-C₃₋₆ carbocyclyl substituted with 0-5 Rₑ, and -(CH₂)ᵣ-heterocyclyl substituted with 0-5 Rₑ;
Rₑ, at each occurrence, is independently selected from C₁₋₆ alkyl substituted with 0-5 Rε, C₂₋₆ alkenyl, C₂₋₆ alkynyl, -(CH₂)ᵣ-C₃₋₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl;
Rε, at each occurrence, is independently selected from H, F, Cl, Br, CN, OH, C₁₋₅ alkyl optionally substituted with OH, C₃₋₆ cycloalkyl, and phenyl;
p, at each occurrence, is independently selected from zero, 1, and 2; and
r, at each occurrence, is independently selected from zero, 1, 2, 3, and 4.

In a twelfth aspect, the present invention provides a compound of Formula (V): 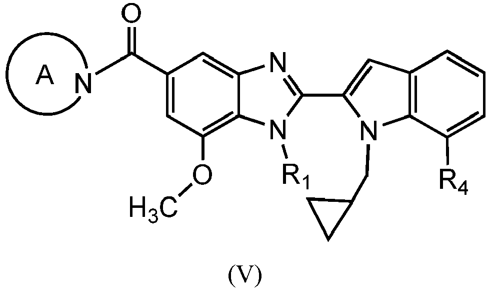
or a pharmaceutically acceptable salt thereof, within the scope of the first aspect, wherein: 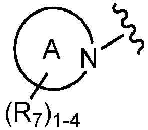 is selected from 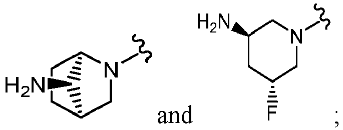
R₁ is selected from -CH₃ and 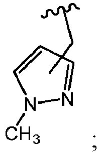
R₄ is selected from 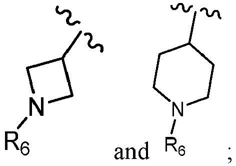
R₆, at each occurrence, is independently selected from -C(=O)R_{b}, -C(=O)(CH₂)₁₋₃OR_{b}, and -C(=O)NRₐRₐ;
Rₐ, at each occurrence, is independently selected from H, C₁₋₃ alkyl substituted with 0-3 Rₑ, C₃₋₆ cycloalkyl, phenyl substituted with 0-3 Rₑ, and pyridyl;
R_{b} is selected from Hand C₁₋₃ alkyl substituted with 0-3 Rₑ; and
Rₑ, at each occurrence, is independently selected from F, Cl, -OH, -OC₁₋₄alkyl, and phenyl.

In a thirteenth aspect, the present invention provides a compound of Formula (VI): 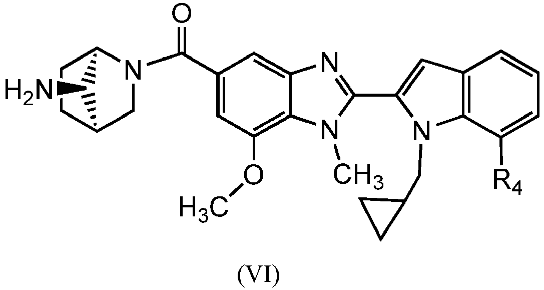
or a pharmaceutically acceptable salt thereof, wherein:
R₄ is selected from 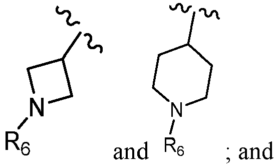
R₆, at each occurrence, is independently selected from -C(=O)CH₃, C(=O)(CH₂)₁₋₂OH, - C(=O)CH₂OCH₂CF₃, -C(=O)(CH₂)₁₋₃OCH₃, -C(=O)(CH₂)₁₋₃-phenyl, - C(=O)(CH₂)₁₋₃-pyridyl, -C(=O)(CH₂)₁₋₃-tetrazolyl, -C(=O)NH₂, -C(=O)NHC₁₋₃alkyl, -C(=O)NH-pyridyl, -C(=O)NH-cyclopropyl, and -C(=O)NH-phenyl substituted with 0-1 F, Cl, C₁₋₂ alkyl, and OC₁₋₂ alkyl.

In a fourteenth aspect, the present invention provides a compound of Formula (VII): 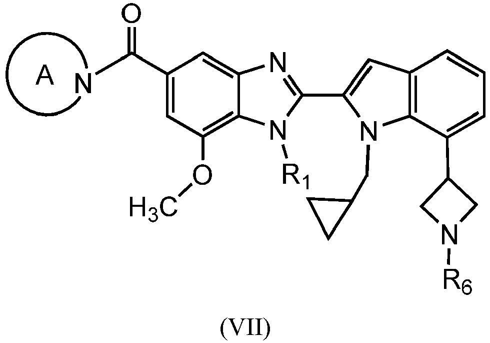
or a pharmaceutically acceptable salt thereof, wherein: 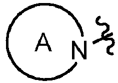 is selected from 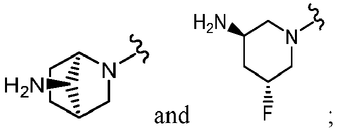
R₁ is selected from -CH₃ and 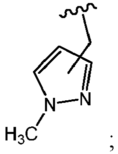
R₆ is selected from -C(=O)R_{b} and -C(=O)NRₐRₐ;
Rₐ, at each occurrence, is independently selected from H, C₁-₃ alkyl substituted with 0-3 Rₑ, C₃₋₆ cycloalkyl, phenyl substituted with 0-3 Rₑ, and pyridyl;
R_{b} is C₁-₃ alkyl substituted with 0-3 Rₑ; and
Rₑ, at each occurrence, is independently selected from F, Cl, -OH, -OC₁₋₄alkyl, and phenyl.

In a fifteenth aspect, the present invention provides a compound of Formula (VIII): 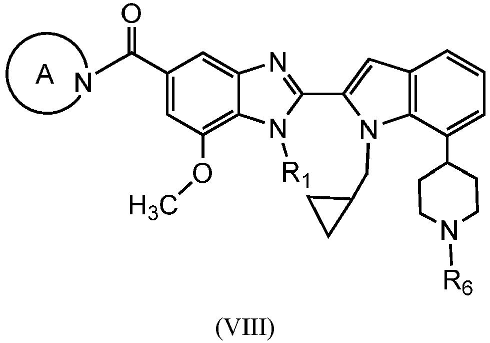
or a pharmaceutically acceptable salt thereof, wherein: 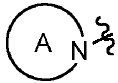 is selected from 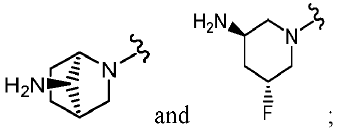
R₁ is selected from -CH₃ and 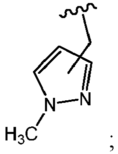
R₆, at each occurrence, is independently selected from -C(=O)R_{b} and -C(=O)NRₐRₐ;
Rₐ, at each occurrence, is independently selected from H, C₁-₃ alkyl substituted with 0-3 Rₑ, C₃₋₆ cycloalkyl, phenyl substituted with 0-3 Rₑ, and pyridyl;
R_{b} is C₁-₃ alkyl substituted with 0-3 Rₑ; and
Rₑ, at each occurrence, is independently selected from F, Cl, -OH, -OC₁₋₄alkyl substituted with 0-3 F and Cl, and phenyl.

In a sixteenth aspect, the present invention provides a compound of Formula (IX): 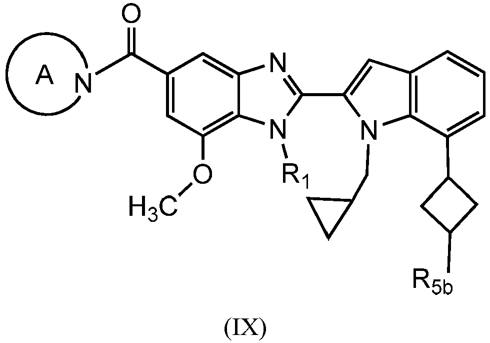
or a pharmaceutically acceptable salt thereof, wherein: 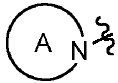 is selected from 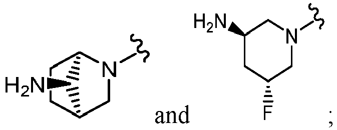
R₁ is selected from -CH₃ and 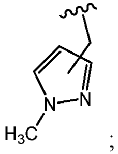
R_{5b} is selected from OH, -C(=O)NRₐRₐ, -C(=O)OR_{b}, NHC(=O)R_{b}, and NH₂,
Rₐ, at each occurrence, is independently selected from H, CH₃, and CD₃; or Rₐ and Rₐ together with the nitrogen atom to which they are both attached form a heterocyclic ring substituted with 0-2 OH; and
R_{b} is selected from H, CH₃, and CD₃.

In a seventeenth aspect, the present invention provides a compound of Formula (X): 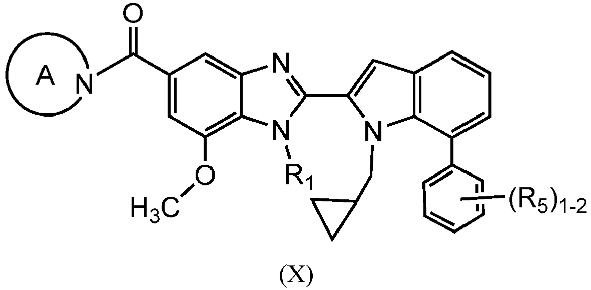
or a pharmaceutically acceptable salt thereof, wherein: 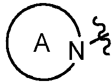 is selected from 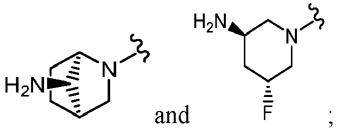
R₁ is selected from -CH₃ and 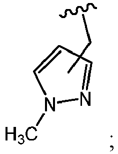
R₅, at each occurrence, is independently selected from H, F, C₁-₄alkyl substituted with 0-3 Rₑ, and -OR_{b};
R_{b} is H and C₁-₂ alkyl; and
Rₑ, at each occurrence, is independently selected from F, Cl, and -OH.

In a eighteenth aspect, the present invention provides a compound of Formula (XI): 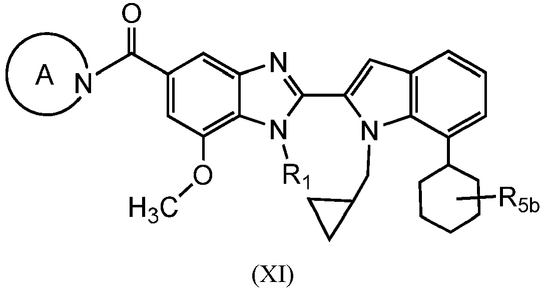
or a pharmaceutically acceptable salt thereof, wherein: 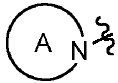 is selected from 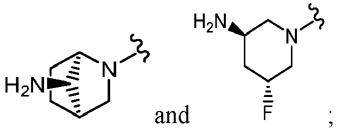
R₁ is selected from -CH₃ and 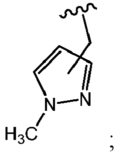 and
R_{5b}, is selected from -OH, -NH₂, and CONH₂.

As defined above and described herein, R₁ is selected from -CH₃ -CD₃, and -CH₂-5-6 membered heterocyclyl comprising carbon atoms and 1-3 heteroatoms selected from N, NH, and NC₁₋₃alkyl. In some embodiments, R₁ is -CH₃. In some embodiments, R₁ is -CD₃. In some embodiments, R₁ is

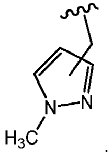

As defined above and described herein, R₂ is selected from CH₃, CH₃CH₂, and -CH₂-cyclopropyl substituted with 0-3 Rₑ.In some embodiments, R₂ is methyl. In some embodiments, R₂ is ethyl. In some embodiments, R₂ is -CH₂-cyclopropyl. In certain embodiments, R₂ is selected from those functional groups depicted in the examples below.

As defined above and described herein, R₃ is selected from H, F, Cl, Br, and - OC₁₋₄ alkyl. In some embodiments, R₃ is H, F, Cl, Br. In some embodiments, R₃ is F. In some embodiments, R₃ is H. In some embodiments, R₃ is -OCH₃. In some embodiments, R₃ is -OCH₂CH₃. In some embodiments, R₃ is -OCH₂CH₂CH₃. In certain embodiments, R₃ is selected from those functional groups depicted in the examples below.

As defined above and described herein, each R₄ is 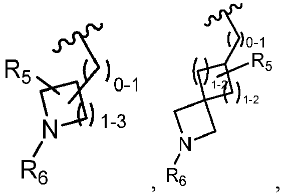 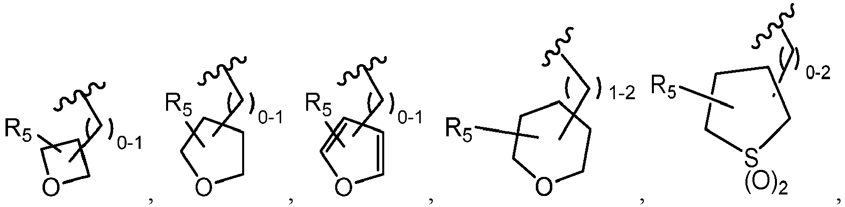 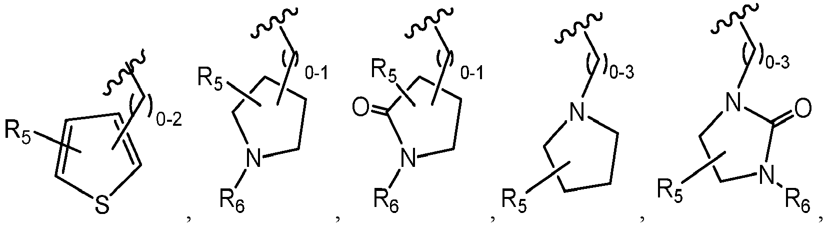 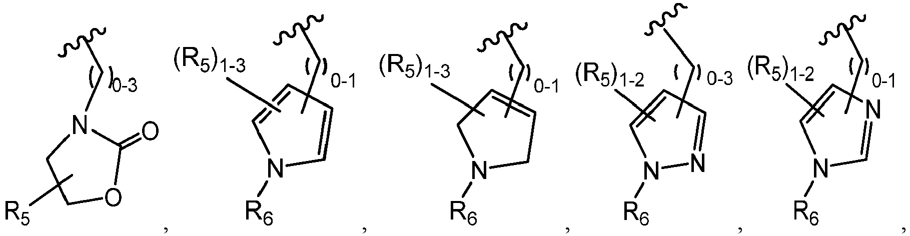 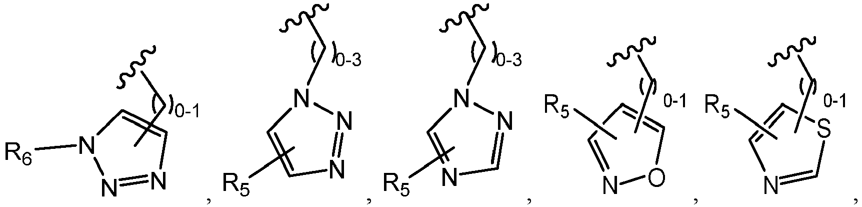 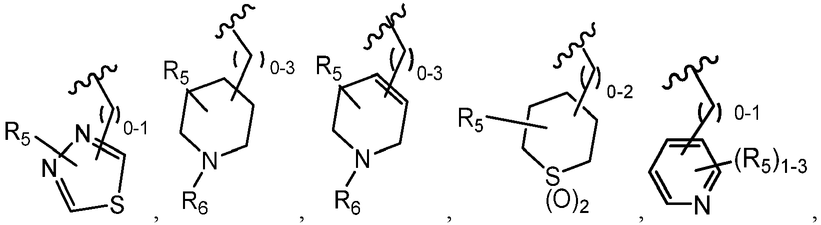 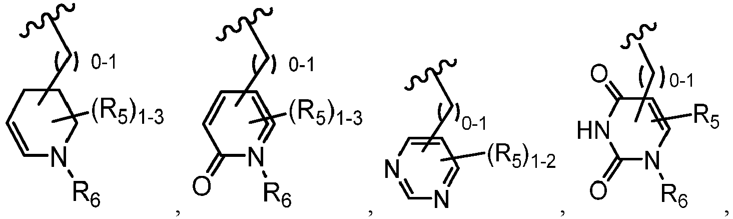 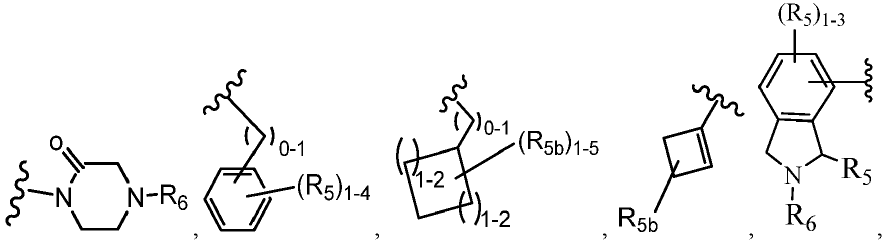 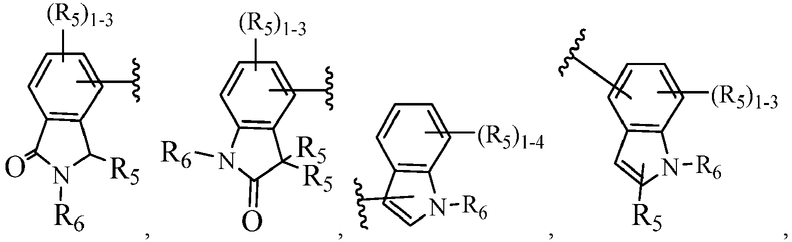 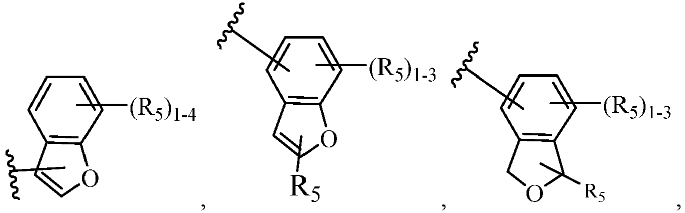 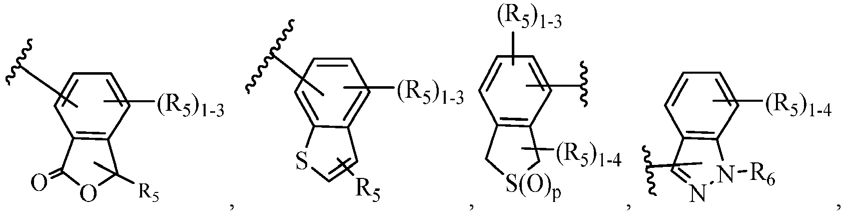 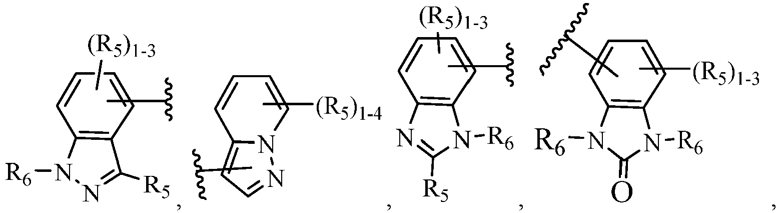 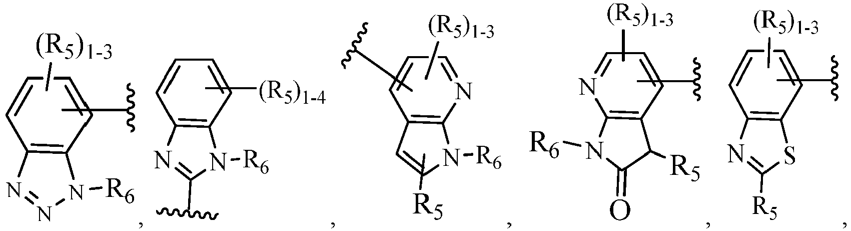 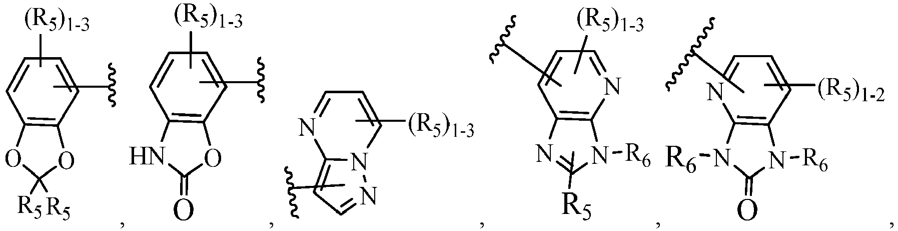 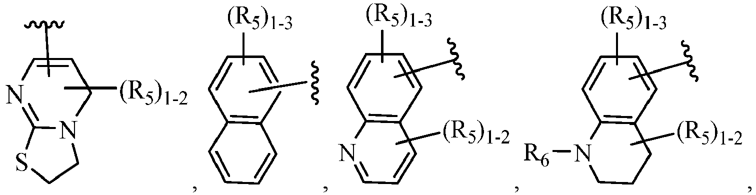 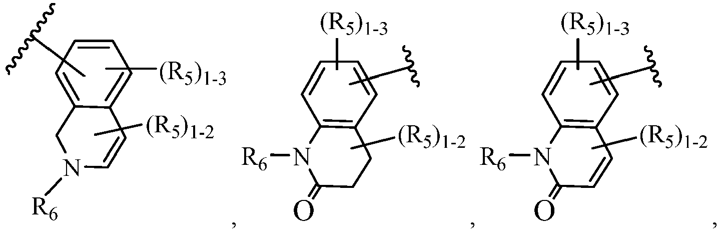 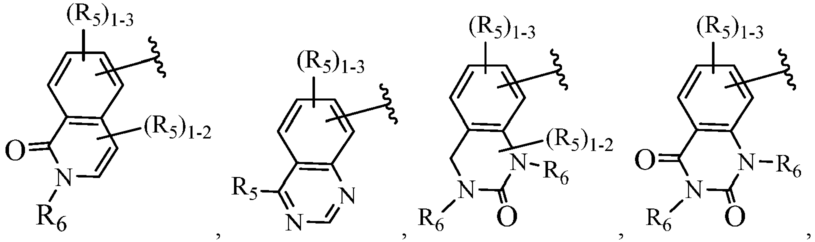 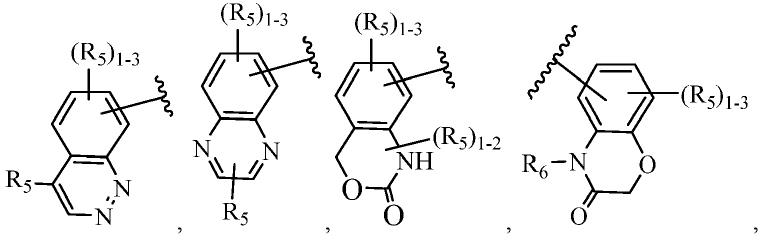 and 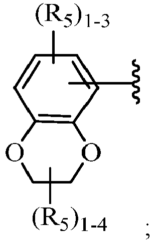 In some embodiments, R₄ is 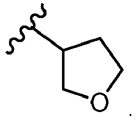 In some embodiments, R₄ is 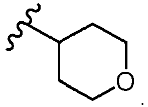 In some embodiments, R₄ is 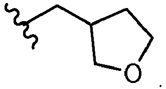 In some embodiments, R₄ is 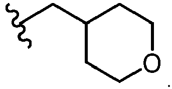 In some embodiments, R₄ is 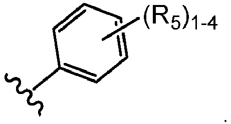 In some embodiments, R₄ is 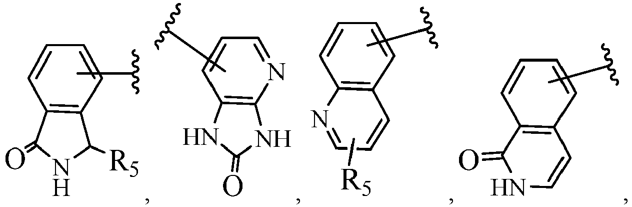 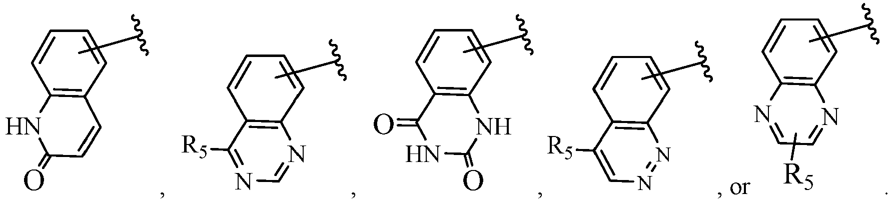 In certain embodiments, R₄ is selected from those functional groups depicted in the examples below.

As defined above and described herein, R₅ is H, F, Cl, Br, CN, C₁-₄alkyl substituted with 0-5 Rₑ, C₂-₄alkenyl, C₂-₄alkynyl, nitro, -S(O)*ₚ*R_{c}, - S(O)*ₚ*NRₐRₐ, -NRₐS(O)*ₚ*R_{c}, -(CHR_{d})ᵣOR_{b}, -(CH₂)ᵣNRₐRₐ, -NRₐC(=O)R_{b}, NRₐC(=O)OR_{b} - NRₐC(=O)NRₐRₐ, -C(=O)R_{b}, -C(=O)OR_{b}, C(=O)NRₐRₐ, -OC(=O)R_{b}, C₃₋₆cycloalkyl substituted with 0-4 Rₑ, aryl substituted with 0-4 Rₑ, and heterocyclyl substituted with 0-4 Rₑ

In some embodiments, R₅ is H, F, Cl, CN, C₁-₄alkyl, C₁-₄alkyl (substituted with OH, NH₂, and COOH), SC₁₋₄alkyl, S(O)₂C₁₋₄alkyl, S(O)₂NH-cyclopropyl, -(CH₂)₀₋₁NHS(O)₂C₁₋₄alkyl, N(R_{d})S(O)₂C₂₋₄alkenyl, -(CH₂)₀₋₁OH, OC₁₋₄alkyl, -(CH₂)₀₋₁NH₂, - (CH₂)₀₋₁NHC(=O)C₁₋₄alkyl, -NR_{d}C(=O)C₂₋₄alkenyl, -NHC(=O)C₂₋₄alkynyl, -(CH₂)₀₋₁C(=O)OH, -C(=O)OC₁₋₄alkyl, -NHC(=O)OC₁₋₄alkyl, -NHC(=O)O(CH₂)₂OC₁₋₄alkyl, - NHC(=O)OCH₂-cyclopropyl, -NHC(=O)NH₂, C(=O)NHC₁₋₄alkyl, CONH(CH₂)₁₋₂C(=O)OH, -(CH₂)₀₋₁C(=O)NH₂, -(CH₂)₀₋₁C(=O)NHC₁₋₄alkyl, C(=O)NH-pyridine, - C(=O)NH(CH₂)₂N(C₁₋₄alkyl)₂, -C(=O)NH(CH₂)₂OH, -C(=O)NH(CH₂)₂S(O)₂C₁₋₄alkyl, and -OC(=O)C₁₋₄alkyl.

In some embodiments, R₅ is

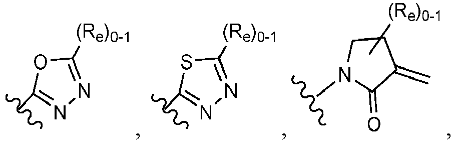

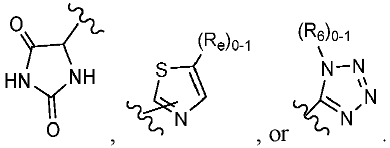

In some embodiments, R₅ is F. In some embodiments, R₅ C₁-₄alkyl. In some embodiments, R₅ is -OH or -OC₁₋₃alkyl. In some embodiments, R₅ is -NHS(O)₂C₂₋₄alkenyl. In certain embodiments, R₅ is selected from those functional groups depicted in the examples below.

As defined above and described herein, R₆ is H, C₁-₃alkyl substituted with 0-4 Rₑ, -S(O)ₚR_{c}, -C(=O)R_{b}, -(CH₂)ᵣ-C(=O)NRₐRₐ, -C(=O)(CH₂)ᵣNRₐC(=O)R_{b}, -C(=O)OR_{b}, - S(O)ₚNRₐRₐ, aryl substituted with 0-4 Rₑ, or heterocyclyl substituted with 0-4 Rₑ.

In some embodiments, R₆ is H. In some embodiments, R₆ is methyl or isopropyl. In some embodiments, R₆ is -(CH₂)₂C(=O)NH₂. In some embodiments, R₆ is - (CH₂)₂OH. In some embodiments, R₆ is C(=O)C₁₋₄alkyl. In certain embodiments, R₆ is selected from those functional groups depicted in the examples below.

As defined above and described herein, R₇ is H, F, Cl, C₁₋₃alkyl, -NRₐRₐ, or - NRₐC(=O)OR_{b}. In some embodiments, R₇ is NH₂. In some embodiments, R₇ is F.

As defined above, Ring A including its substituent R₇ is 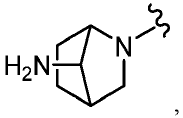 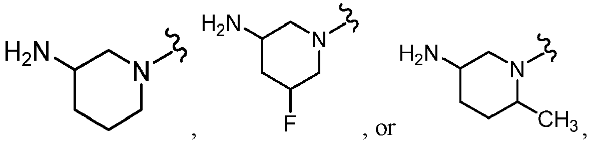 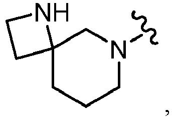 In some embodiments, Ring A is 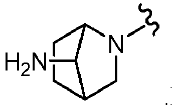 In some embodiments, Ring A is

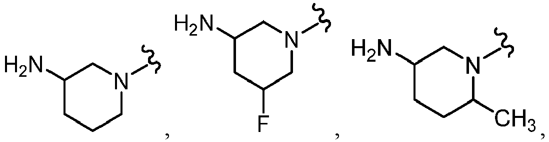

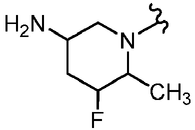

In some embodiments, Ring A is 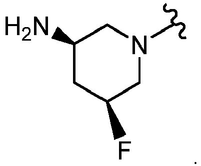 In some embodiments, Ring A is 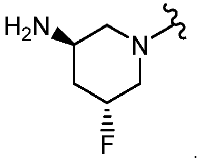 In some embodiments, Ring A is

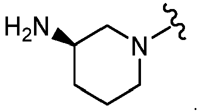

As defined above and described herein, r is 0-4. In some embodiments, r is 0. In some embodiments, r is 1. In some embodiments, r is 2. In some embodiments, r is 3. In some embodiments, r is 4.

In some embodiments, Ring A is 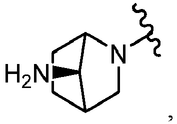 R₁ is -CH₃, R₂ is cyclopropylmethyl, R₃ is H, F, or -OCH₃, R₄ is 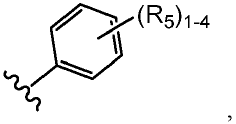 and R₅ is H, F, Cl, CN, C₁-₄alkyl, C₁-₄alkyl substituted with OH, NH₂, and COOH, SC₁₋₄alkyl, S(O)₂C₁₋₄alkyl, S(O)₂NH-cyclopropyl, -(CH₂)₀₋₁NHS(O)₂C₁₋₄alkyl, N(R_{d})S(O)₂C₂₋₄alkenyl, - (CH₂)₀₋₁OH, OC₁₋₄alkyl, -(CH₂)₀₋₁NH₂, -(CH₂)₀₋₁NHC(=O)C₁₋₄alkyl, -NR_{d}C(=O)C₂₋₄alkenyl, -NHC(=O)C₂₋₄alkynyl, -(CH₂)₀₋₁C(=O)OH, -C(=O)OC₁₋₄alkyl, -NHC(=O)OC₁₋₄alkyl, -NHC(=O)O(CH₂)₂OC₁₋₄alkyl, -NHC(=O)OCH₂-cyclopropyl, -NHC(=O)NH₂, C(=O)NHC₁₋₄alkyl, CONH(CH₂)₁₋₂C(=O)OH, -(CH₂)₀₋₁C(=O)NH₂, -(CH₂)₀₋₁C(=O)NHC₁₋₄alkyl, C(=O)NH-pyridine, -C(=O)NH(CH₂)₂N(C₁₋₄alkyl)₂, - C(=O)NH(CH₂)₂OH, -C(=O)NH(CH₂)₂S(O)₂C₁₋₄alkyl, and -OC(=O)C₁₋₄alkyl,

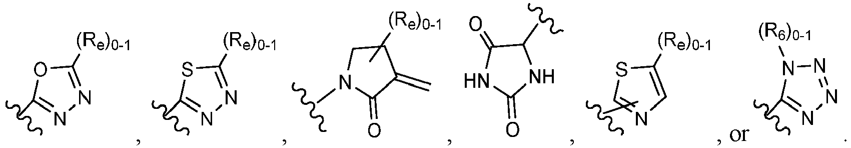

In some embodiments, Ring A is 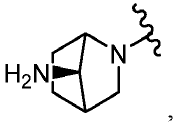 R₁ is -CH₃, R₂ is cyclopropylmethyl, R₃ is H, F, or -OCH₃, R₄ is 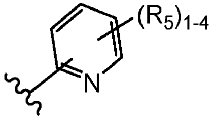 or 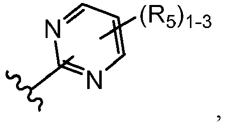 and R₅ is H, F, Cl, CN, C₁-₄alkyl, C₁-₄alkyl substituted with OH, NH₂, and COOH, SC₁₋₄alkyl, S(O)₂C₁₋₄alkyl, S(O)₂NH-cyclopropyl, -(CH₂)₀₋₁NHS(O)₂C₁₋₄alkyl, N(R_{d})S(O)₂C₂₋₄alkenyl, -(CH₂)₀₋₁OH, OC₁₋₄alkyl, -(CH₂)₀₋₁NH₂, - (CH₂)₀₋₁NHC(=O)C₁₋₄alkyl, -NR_{d}C(=O)C₂₋₄alkenyl, -NHC(=O)C₂₋₄alkynyl, -(CH₂)₀₋₁C(=O)OH, -C(=O)OC₁₋₄alkyl, -NHC(=O)OC₁₋₄alkyl, -NHC(=O)O(CH₂)₂OC₁₋₄alkyl, - NHC(=O)OCH₂-cyclopropyl, -NHC(=O)NH₂, C(=O)NHC₁₋₄alkyl, CONH(CH₂)₁₋₂C(=O)OH, -(CH₂)₀₋₁C(=O)NH₂, -(CH₂)₀₋₁C(=O)NHC₁₋₄alkyl, C(=O)NH-pyridine, - C(=O)NH(CH₂)₂N(C₁₋₄alkyl)₂, -C(=O)NH(CH₂)₂OH, -C(=O)NH(CH₂)₂S(O)₂C₁₋₄alkyl, and -OC(=O)C₁₋₄alkyl,

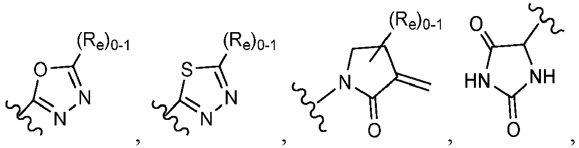

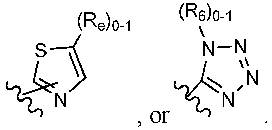

In some embodiments, Ring A is 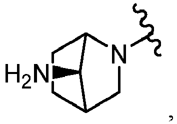 R₁ is -CH₃, R₂ is cyclopropylmethyl, R₃ is H, F or -OCH₃, R₄ is

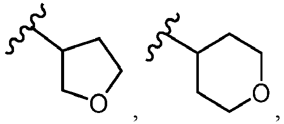

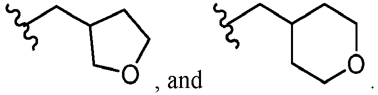

In some embodiments, Ring A is 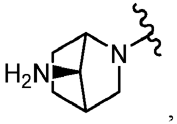 R₁ is -CH₃, R₂ is cyclopropylmethyl, R₃ is H, F or -OCH₃, R₄ is 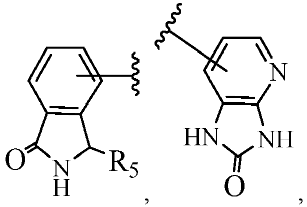 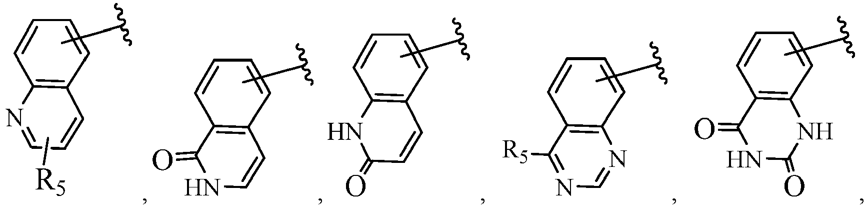 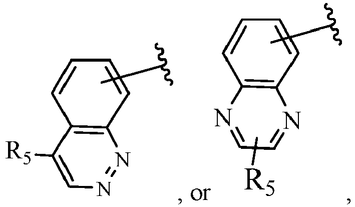 and R₅ H, F, R₅ C₁-₄alkyl, -OH, -OC₁₋₃alkyl and is -NHS(O)₂C₂₋₄alkenyl.

In some embodiments, the compound of Formula (I) is selected from examples depicted below. In certain embodiments, the present invention provides any compound described above and herein, or a pharmaceutically acceptable salt thereof. In some embodiments, the present invention provides any compound described above and herein in isolated form.

### 4. Uses, Formulation and Administration

### Pharmaceutically acceptable compositions

According to another embodiment, the invention provides a composition comprising a compound of this invention or a pharmaceutically acceptable derivative thereof and a pharmaceutically acceptable carrier, adjuvant, or vehicle. The amount of compound in compositions of this invention is such that is effective to measurably inhibit PAD4, in a biological sample or in a patient. A composition of this invention may be formulated for administration to a patient in need of such composition. A composition of this invention may be formulated for oral administration to a patient.

The term "subject," as used herein, is used interchangeably with the term "patient" and means an animal, preferably a mammal. A subject or patient may be a human. A subject (or patient) may be a veterinary subject (or patient). A veterinary subject (or patient) may be a canine, a feline, or an equine subject.

The term "pharmaceutically acceptable carrier, adjuvant, or vehicle" refers to a non-toxic carrier, adjuvant, or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a nontoxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

Pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, provided pharmaceutically acceptable compositions may be formulated in a suitable ointment containing the active component suspended or dissolved in one or more carriers. Carriers for topical administration of compounds of this invention include, but are not limited to, mineral oil, liquid petrolatum, white petrolatum, propylene glycol, polyoxyethylene, polyoxypropylene compound, emulsifying wax and water. Alternatively, provided pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, provided pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

Pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, pharmaceutically acceptable compositions of this invention are formulated for oral administration. Such formulations may be administered with or without food. Pharmaceutically acceptable compositions of this invention can be administered without food. Pharmaceutically acceptable compositions of this invention can be administered with food.

Pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. The compounds of the invention may be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of inj ectables.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

The amount of compounds of the present invention that may be combined with the carrier materials to produce a composition in a single dosage form will vary depending upon the host treated, the particular mode of administration. Preferably, provided compositions should be formulated so that a dosage of between 0.01 - 100 mg/kg body weight/day of the inhibitor can be administered to a patient receiving these compositions.

A compound of the current invention can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds. Exemplary of such other therapeutic agents include corticosteroids, rolipram, calphostin, cytokine-suppressive anti-inflammatory drugs (CSAIDs), Interleukin-10, glucocorticoids, salicylates, nitric oxide, and other immunosuppressants; nuclear translocation inhibitors, such as deoxyspergualin (DSG); non-steroidal antiinflammatory drugs (NSAIDs) such as ibuprofen, celecoxib and rofecoxib; steroids such as prednisone or dexamethasone; antiviral agents such as abacavir; antiproliferative agents such as methotrexate, leflunomide, FK506 (tacrolimus, Prograf); cytotoxic drugs such as azathiprine and cyclophosphamide; TNF-α inhibitors such as tenidap, anti-TNF antibodies or soluble TNF receptor, and rapamycin (sirolimus or Rapamune) or derivatives thereof. A compound of the current invention can besides or in addition be administered especially for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

Those additional agents may be administered separately from an inventive compound-containing composition, as part of a multiple dosage regimen. Alternatively, those agents may be part of a single dosage form, mixed together with a compound of this invention in a single composition. If administered as part of a multiple dosage regime, the two active agents may be submitted simultaneously, sequentially or within a period of time from one another normally within five hours from one another.

As used herein, the term "combination," "combined," and related terms refers to the simultaneous or sequential administration of therapeutic agents in accordance with this invention. For example, a compound of the present invention may be administered with another therapeutic agent simultaneously or sequentially in separate unit dosage forms or together in a single unit dosage form. Accordingly, the present invention may provide a single unit dosage form comprising a compound of the current invention, an additional therapeutic agent, and a pharmaceutically acceptable carrier, adjuvant, or vehicle.

The amount of both an inventive compound and additional therapeutic agent (in those compositions which comprise an additional therapeutic agent as described above) that may be combined with the carrier materials to produce a single dosage form will vary depending upon the host treated and the particular mode of administration. Preferably, compositions of this invention should be formulated so that a dosage of between 0.01 - 100 mg/kg body weight/day of an inventive compound can be administered.

In those compositions which comprise an additional therapeutic agent, that additional therapeutic agent and the compound of this invention may act synergistically. Therefore, the amount of additional therapeutic agent in such compositions will be less than that required in a monotherapy utilizing only that therapeutic agent.

The amount of additional therapeutic agent present in the compositions of this invention may be no more than the amount that would normally be administered in a composition comprising that therapeutic agent as the only active agent. The amount of additional therapeutic agent in the presently disclosed compositions may range from about 50% to 100% of the amount normally present in a composition comprising that agent as the only therapeutically active agent.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

### Uses of Compounds and Pharmaceutically Acceptable Compositions

Compounds and compositions described herein are generally useful for the inhibition of PALM.

The activity of a compound utilized in this invention as an inhibitor of PAD4, may be assayed *in vitro*, *in vivo* or in a cell line. *In vitro* assays include assays that determine the inhibition of PALM. Detailed conditions for assaying a compound utilized in this invention as an inhibitor of PAD4 are set forth in the Examples below. A provided compound may inhibit PAD4 selectively as compared to PAD2.

As used herein, the terms "treatment," "treat," and "treating" refer to reversing, alleviating, delaying the onset of, or inhibiting the progress of a disease or disorder, or one or more symptoms thereof, as described herein. In some embodiments, treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence.

Provided compounds are inhibitors of PAD4 and are therefore useful for treating one or more disorders associated with activity of PAD4. Thus, the compounds of the present invention may be used in a method for treating a PAD4-mediated disorder comprising the step of administering to a patient in need thereof a compound of the present invention, or pharmaceutically acceptable composition thereof.

A PAD4-mediated disorder may be a disease, condition, or disorder mediated by inappropriate PAD4 activity. A PAD4-mediated disorder may be selected from the group consisting of rheumatoid arthritis, vasculitis, systemic lupus erythematosus, ulcerative colitis, cancer, cystic fibrosis, asthma, cutaneous lupus erythematosus, and psoriasis. The disorder mediated by inappropriate PAD4 activity may be rheumatoid arthritis. The disorder mediated by inappropriate PAD4 activity may be systemic lupus. The disorder mediated by inappropriate PAD4 activity may be vasculitis. The disorder mediated by inappropriate PAD4 activity may be cutaneous lupus erythematosus. The disorder mediated by inappropriate PAD4 activity may be psoriasis.

Compounds of the present invention may be used in a method of treatment of rheumatoid arthritis, vasculitis, systemic lupus erythematosus, ulcerative colitis, cancer, cystic fibrosis, asthma, cutaneous lupus erythematosus, or psoriasis, which method comprises administering to a human subject in need thereof, a therapeutically effective amount of a provided compound or a pharmaceutically acceptable salt thereof.

A compound of the present inventio may be used in a method of treatment of rheumatoid arthritis, which method comprises administering to a human subject in need thereof, a therapeutically effective amount of a provided compound, or a pharmaceutically acceptable salt thereof. A compound of the present invention may be used in a method of treatment of systemic lupus, which method comprises administering to a human subject in need thereof, a therapeutically effective amount of a provided compound, or a pharmaceutically acceptable salt thereof. A compound of the present invention may be used in a method of treatment of vasculitis, which method comprises administering to a human subject in need thereof, a therapeutically effective amount of a provided compound, or a pharmaceutically acceptable salt thereof. A compound of the present invention may be used in a method of treatment of cutaneous lupus erythematosus, which method comprises administering to a human subject in need thereof, a therapeutically effective amount of a provided compound, or a pharmaceutically acceptable salt thereof. A compound of the present invention may be used in a method of treatment of psoriasis, which method comprises administering to a human subject in need thereof, a therapeutically effective amount of a provided compound, or a pharmaceutically acceptable salt thereof.

A PAD4-mediated disorder may be selected from the group consisting of acidinduced lung injury, acne (PAPA), acute lymphocytic leukemia, acute, respiratory distress syndrome, Addison's disease, adrenal hyperplasia, adrenocortical insufficiency, ageing, AIDS, alcoholic hepatitis, alcoholic hepatitis, alcoholic liver disease, allergen induced asthma, allergic bronchopulmonary, aspergillosis, allergic conjunctivitis, alopecia, Alzheimer's disease, amyloidosis, amyotropic lateral sclerosis, and weight loss, angina pectoris, angioedema, anhidrotic ecodermal dysplasia-ID, ankylosing spondylitis, anterior segment, inflammation, antiphospholipid syndrome, aphthous stomatitis, appendicitis, arthritis, asthma, atherosclerosis, atopic dermatitis, autoimmune diseases, autoimmune hepatitis, bee sting-induced inflammation, behcet's disease, Behcet's syndrome, Bells Palsey, berylliosis, Blau syndrome, bone pain, bronchiolitis, burns, bursitis, cancer, cardiac hypertrophy, carpal tunnel syndrome, catabolic disorders, cataracts, cerebral aneurysm, chemical irritant-induced inflammation, chorioretinitis, chronic heart failure, chronic lung disease of prematurity, chronic lymphocytic leukemia, chronic obstructive pulmonary disease, colitis, complex regional pain syndrome, connective tissue disease, corneal ulcer, crohn's disease, cryopyrin-associated periodic syndromes, cyrptococcosis, cystic fibrosis, deficiency of the interleukin-1-receptor antagonist (DIRA), dermatitis, dermatitis endotoxemia, dermatomyositis, diffuse intrinsic pontine glioma, endometriosis, endotoxemia, epicondylitis, erythroblastopenia, familial amyloidotic polyneuropathy, familial cold urticarial, familial Mediterranean fever, fetal growth retardation, glaucoma, glomerular disease, glomerular nephritis, gout, gouty arthritis, graft-versus-host disease, gut diseases, head injury, headache, hearing loss, heart disease, hemolytic anemia, Henoch-Scholein purpura, hepatitis, hereditary periodic fever syndrome, herpes zoster and simplex, HIV-1, Hodgkin's disease, Huntington's disease, hyaline membrane disease, hyperammonemia, hypercalcemia, hypercholesterolemia, hyperimmunoglobulinemia D with recurrent fever (HIDS), hypoplastic and other anemias, hypoplastic anemia, idiopathic thrombocytopenic purpura, incontinentia pigmenti, infectious mononucleosis, inflammatory bowel disease, inflammatory lung disease, inflammatory neuropathy, inflammatory pain, insect bite-induced inflammation, iritis, irritant-induced inflammation, ischemia/reperfusion, juvenile rheumatoid arthritis, keratitis, kidney disease, kidney injury caused by parasitic infections, kidney injury caused by parasitic infections, kidney transplant rejection prophylaxis, leptospiriosis, leukemia, Loeffler's syndrome, lung injury, lung injury, lupus, lupus, lupus nephritis, lymphoma, meningitis, mesothelioma, mixed connective tissue disease, Muckle-Wells syndrome (urticaria deafness amyloidosis), multiple sclerosis, muscle wasting, muscular dystrophy, myasthenia gravis, myocarditis, mycosis fungiodes, mycosis fungoides, myelodysplastic syndrome, myositis, nasal sinusitis, necrotizing enterocolitis, neonatal onset multisystem inflammatory disease (NOMID), nephrotic syndrome, neuritis, neuropathological diseases, non-allergen induced asthma, obesity, ocular allergy, optic neuritis, organ transplant, osterarthritis, otitis media, paget's disease, pain, pancreatitis, Parkinson's disease, pemphigus, pericarditis, periodic fever, periodontitis, peritoneal endometriosis, pertussis, pharyngitis and adenitis (PFAPA syndrome), plant irritant-induced inflammation, pneumonia, pneumonitis, pneumosysts infection, poison ivy/ urushiol oil-induced inflammation, polyarteritis nodosa, polychondritis, polycystic kidney disease, polymyositis, psoriasis, psoriasis, psoriasis, psoriasis, psychosocial stress diseases, pulmonary disease, pulmonary hypertension, pulmonayr fibrosis, pyoderma gangrenosum, pyogenic sterile arthritis, renal disease, retinal disease, rheumatic carditis, rheumatic disease, rheumatoid arthritis, sarcoidosis, seborrhea, sepsis, severe pain, sickle cell, sickle cell anemia, silica-induced disease, Sjogren's syndrome, skin diseases, sleep apnea, solid tumors, spinal cord injury, Stevens-Johnson syndrome, stroke, subarachnoid hemorrhage, sunburn, temporal arteritis, tenosynovitis, thrombocytopenia, thyroiditis, tissue transplant, TNF receptor associated periodic syndrome (TRAPS), toxoplasmosis, transplant, traumatic brain injury, tuberculosis, type 1 diabetes, type 2 diabetes, ulcerative colitis, urticarial, uveitis, and Wegener's granulomatosis.

In one embodiment, the invention provides a provided compound, or a pharmaceutically acceptable salt thereof, for use in therapy. A compound of the present invention, or a pharmaceutically acceptable salt thereof, may be used in a method of treatment of a disorder mediated by inappropriate PAD4 activity. A compound of the present invention, or a pharmaceutically acceptable salt thereof, may be used in a method of treatment of rheumatoid arthritis, vasculitis, systemic lupus erythematosus, ulcerative colitis, cancer, cystic fibrosis, asthma, cutaneous lupus erythematosus, or psoriasis. A compound of the present invention, or a pharmaceutically acceptable salt thereof, may be used in a method of treatment of rheumatoid arthritis. A compound of the present invention, or a pharmaceutically acceptable salt thereof, may be used in a method of treatment of systemic lupus. A compound of the present invention, or a pharmaceutically acceptable salt thereof, may be used in a method of treatment of vasculitis. A compound of the present invention, or a pharmaceutically acceptable salt thereof, may be used in a method of treatment of cutaneous lupus erythematosus. A compound of the present invention, or a pharmaceutically acceptable salt thereof, may be used in a method of treatment of psoriasis. A compound of the present invention, or a pharmaceutically acceptable salt thereof, may be used in the manufacture of a medicament for use in the treatment of a disorder mediated by inappropriate PAD4 activity. A compound of the present invention, or a pharmaceutically acceptable salt thereof, may be used in the manufacture of a medicament for use in the treatment of rheumatoid arthritis, vasculitis, systemic lupus erythematosus, ulcerative colitis, cancer, cystic fibrosis, asthma, cutaneous lupus erythematosus, or psoriasis. A compound of the present invention, or a pharmaceutically acceptable salt thereof, may be used in the manufacture of a medicament for use in the treatment of rheumatoid arthritis. A compound of the present invention, or a pharmaceutically acceptable salt thereof, may be used in the manufacture of a medicament for use in the treatment of systemic lupus. A compound of the present invention, or a pharmaceutically acceptable salt thereof, may be used in the manufacture of a medicament for use in the treatment of vasculitis. A compound of the present invention, or a pharmaceutically acceptable salt thereof, may be used in the manufacture of a medicament for use in the treatment of cutaneous lupus erythematosus. A compound of the present invention, or a pharmaceutically acceptable salt thereof, may be used in the manufacture of a medicament for use in the treatment of psoriasis. A pharmaceutical composition of the present invention may be used for the treatment or prophylaxis of a disorder mediated by inappropriate PAD4 activity comprising a provided compound, or a pharmaceutically acceptable salt thereof. A pharmaceutical composition of the present invention may be used for the treatment or prophylaxis of rheumatoid arthritis, vasculitis, systemic lupus erythematosus, ulcerative colitis, cancer, cystic fibrosis, asthma, cutaneous lupus erythematosus, or psoriasis, comprising a provided compound, or a pharmaceutically acceptable salt thereof. A pharmaceutical composition of the present invention may be used for the treatment or prophylaxis of rheumatoid arthritis comprising a provided compound, or a pharmaceutically acceptable salt thereof. A pharmaceutical composition of the present invention may be used for the treatment or prophylaxis of systemic lupus comprising a provided compound, or a pharmaceutically acceptable salt thereof. A pharmaceutical composition of the present invention may be used for the treatment or prophylaxis of vasculitis comprising a provided compound, or a pharmaceutically acceptable salt thereof. A pharmaceutical composition of the present invention may be used for the treatment or prophylaxis of cutaneous lupus erythematosus comprising a provided compound, or a pharmaceutically acceptable salt thereof. A pharmaceutical composition of the present invention may be used for the treatment or prophylaxis of psoriasis comprising a provided compound, or a pharmaceutically acceptable salt thereof.

All features of each of the aspects of the invention apply to all other aspects mutatis mutandis.
In order that the invention described herein may be more fully understood, the following examples are set forth. It should be understood that these examples are for illustrative purposes only and are not to be construed as limiting this invention in any manner.

As depicted in the Examples below, in certain exemplary embodiments, compounds are prepared according to the following general procedures. It will be appreciated that, although the general methods depict the synthesis of certain compounds of the present invention, the following general methods, and other methods known to one of ordinary skill in the art, can be applied to all compounds and subclasses and species of each of these compounds, as described herein.

### Synthetic Schemes

The following schemes elucidate the synthesis of example compounds embodied in this application.

For the synthesis of compounds involving photoredox chemistry, see Scheme 6.

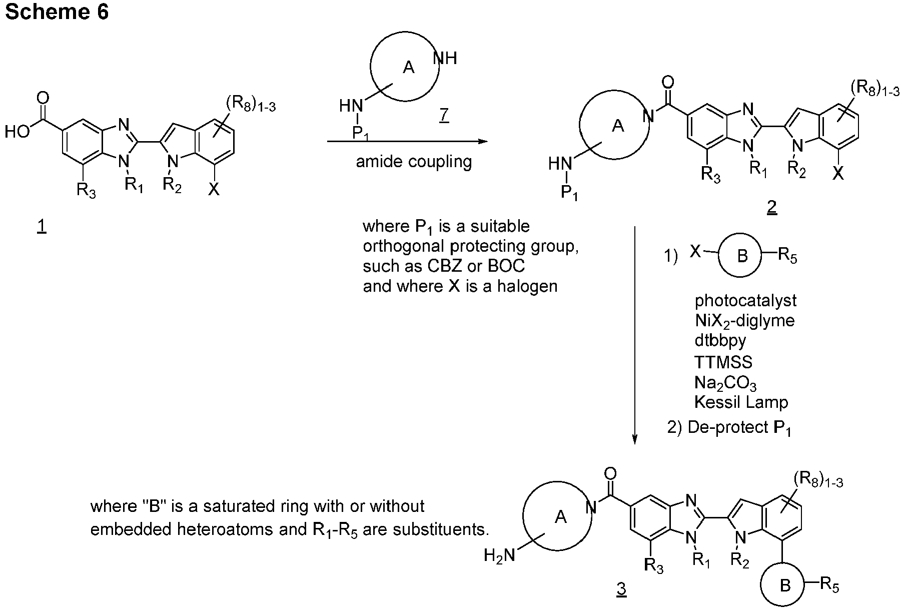

For the synthesis of compounds involving photoredox chemistry of cyclic, bicyclic, or tricyclic protected amines followed by deprotection and subsequent coupling see Scheme 7. Note that the cyclic amine used in the photoredox step can also be in fully elaborated form requiring no subsequent deprotection and coupling steps.

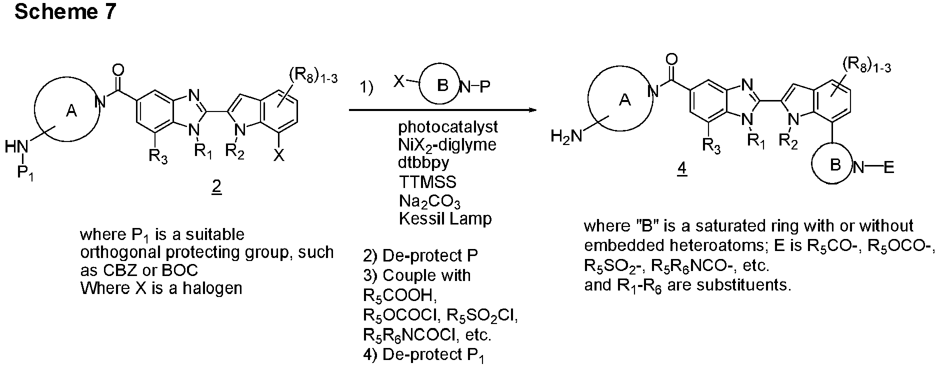

For the synthesis of compounds involving photoredox chemistry of cyclic, bicyclic, or tricyclic protected carboxylic acids (for example, esters) followed by deprotection and subsequent coupling see Scheme 8. Note that the cyclic carboxylic acid used in the photoredox step can also be in fully derivatized form requiring no subsequent deprotection and coupling steps.

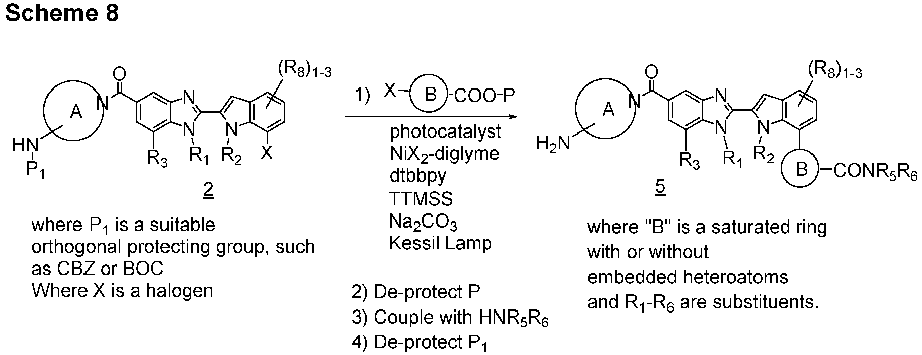

For the synthesis of compounds involving the Suzuki, Stille or other aromatic cross-coupling reactions, see Scheme 9. 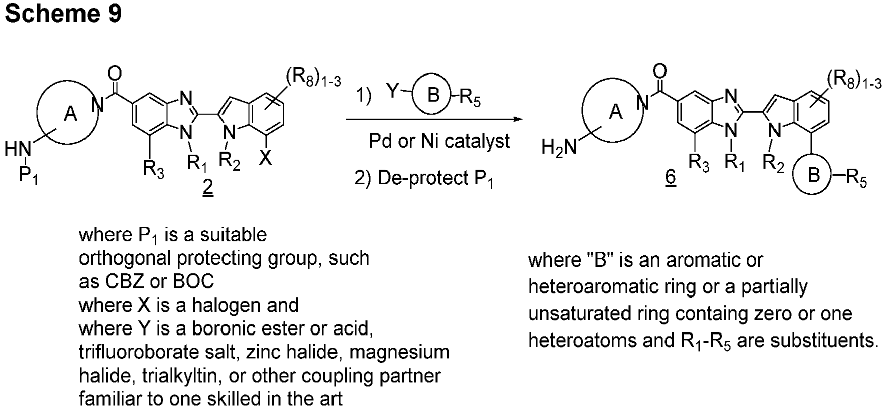 For the synthesis of 1,2,3-triazoles, see Scheme 10.

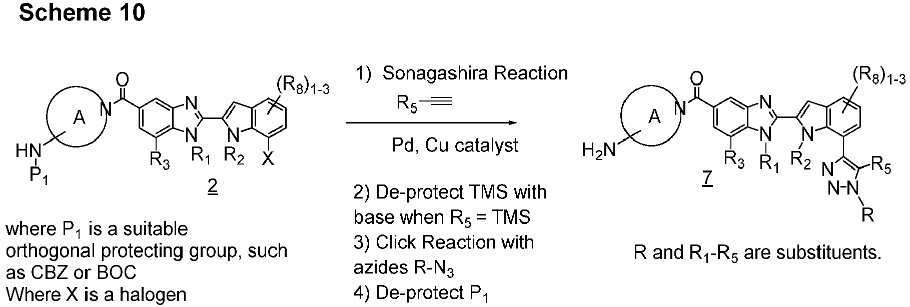

For the synthesis of Buchwald reaction-type coupled products, see Scheme 11.

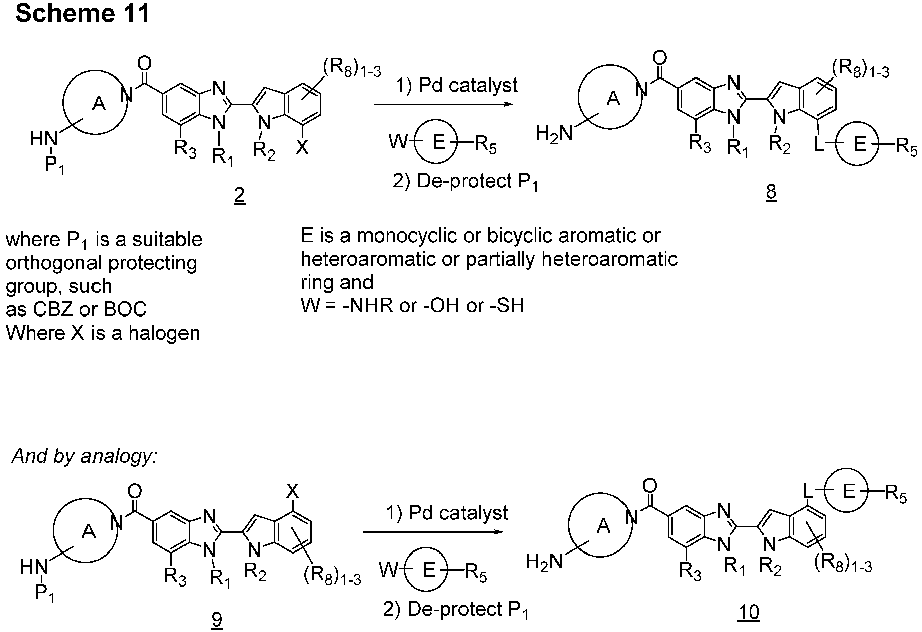

For the synthesis of nitrile coupled products, see Scheme 12 (reference). The nitrile can subsequently be converted into many other different functional groups familiar to one skilled in the art.

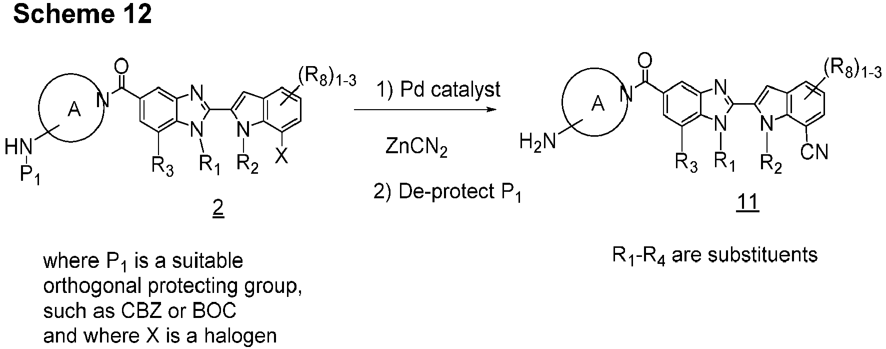

### VI. EXAMPLES

The following Examples are offered as illustrative, as a partial scope and particular embodiments of the invention and are not meant to be limiting of the scope of the invention. Abbreviations and chemical symbols have their usual and customary meanings unless otherwise indicated. Unless otherwise indicated, the compounds described herein have been prepared, isolated and characterized using the schemes and other methods disclosed herein or may be prepared using the same.
- AcOH or HOAc: acetic acid
- ACN: acetonitrile
- Alk: Alkyl
- AlMe₃: Trimethylaluminum
- BBr₃: boron tribromide
- Bn: benzyl
- Boc: *tert*-butyloxycarbonyl
- BOP reagent: benzotriazol-1-yloxytris(dimethylamino)phosphonium hexafluorophosphate
- Bu: butyl
- i-Bu: isobutyl
- *t*-Bu: *tert*-butyl
- *t*-BuOH: *tert*-butanol
- Cbz: carbobenzyloxy
- CDCl₃: deutero-chloroform
- CD₃OD: deutero-methanol
- CH₂Cl₂: dichloromethane
- CH₃CN: acetonitrile
- CHCl₃: chloroform
- DCM: dichloromethane
- DIEA, DIPEA or Hunig's base: diisopropylethylamine
- DMF: dimethyl formamide
- DMSO: dimethyl sulfoxide
- Et: ethyl
- Et₃N or TEA: triethylamine
- Et₂O: diethyl ether
- EtOAc: ethyl acetate
- EtOH: Ethanol
- HATU: 1-[Bis(dimethylamino)methylene]-1H-1,2,3-triazolo[4,5-b]pyridinium 3-oxid hexafluorophosphate
- HCl: hydrochloric acid
- HPLC: high-performance liquid chromatography
- Ir(dF(CF3)ppy)2(dtbbpy)PF6: (4,4'-Di-t-butyl-2,2'-bipyridine)bis[3,5-difluoro-2-(5-trifluoromethyl-2-pyridinyl-kN)phenyl-kC]iridum(III) hexafluorophosphate
- K₂CO₃: potassium carbonate
- K₂HPO₄: potassium hydrogenphosphate
- K₃PO₄: potassium phosphate, tribasic
- LCMS: liquid chromatography mass spectrometry
- LiHMDS: lithium bis(trimethylsilyl)amide
- LG: leaving group
- Me: methyl
- MeOH: methanol
- MgSO₄: magnesium sulfate
- MsOH or MSA: methylsulfonic acid
- NaCl: sodium chloride
- Na₂CO₃: sodium carbonate
- NaHCO₃: sodium bicarbonate
- NaOH: sodium hydroxide
- Na₂SO₄: sodium sulfate
- NH₃: ammonia
- NH₄Cl: ammonium chloride
- NH₄OAc: ammonium acetate
- Pd(OAc)₂: palladium(II) acetate
- Pd(dppf)Cl₂: [1,1'-Bis(diphenylphosphino)ferrocene]palladium(II) dichloride
- Pd(PPh₃)₄: tetrakis(triphenylphosphine)palladium(0)
- PG: protecting group
- Ph: phenyl
- Pr: propyl
- *i*-Pr: isopropyl
- *i*-PrOH or IPA: isopropanol
- Rt: retention time
- SiO₂: silica oxide
- Si-pyridine: SiliaBond^{©} Pyridine
- SFC: supercritical fluid chromatography
- TBAI: Tetrabutylammonium iodide
- TEA: triethylamine
- TFA: trifluoroacetic acid
- TFAA: Trifluoroacetic anhydride
- THF: tetrahydrofuran
- TiCl₄: titanium tetrachloride
- T3P: 1-propanephosphonic acid cyclic anhydride

### Description of analytical LCMS methods:

Method 1: Column: Waters XBridge C18, 2.1 mm x 50 mm, 1.7 µm particles; Mobile Phase A: 5:95 acetonitrile:water with 10 mM ammonium acetate; Mobile Phase B: 95:5 acetonitrile:water with 10 mM ammonium acetate; Temperature: 50 °C; Gradient: 0 %B to 100 %B over 3 min, then a 0.75 min hold at 100 %B; Flow: 1 mL/min; Detection: MS and UV (220 nm).

Method 2: Column: Waters XBridge C18, 2.1 mm x 50 mm, 1.7 µm particles; Mobile Phase A: 5:95 acetonitrile:water with 0.1 % trifluoroacetic acid; Mobile Phase B: 95:5 acetonitrile:water with 0.1 % trifluoroacetic acid; Temperature: 50 °C; Gradient: 0 %B to 100 %B over 3 min, then a 0.75 min hold at 100 %B; Flow: 1 mL/min; Detection: MS and UV (220 nm).

Method A: Column: Waters Acquity UPLC BEH C18, 2.1 x 50 mm, 1.7 µm particles; Mobile Phase A: 5:95 ACN:water with 10 mM NH₄OAc; Mobile Phase B: 95:5 ACN:water with 10 mM NH₄OAc; Temperature: 50 °C; Gradient: 0-100% B over 3 minutes, then a 0.75 minute hold at 100% B; Flow: 1.11 mL/min; Detection: UV at 220 nm.

Method B: Column: Waters Acquity UPLC BEH C18, 2.1 x 50 mm, 1.7 µm particles; mobile Phase A: 5:95 ACN:water with 0.1% TFA; Mobile Phase B: 95:5 ACN:water with 0.1% TFA; Temperature: 50 °C; Gradient: 0-100% B over 3 minutes, then a 0.75 minute hold at 100% B; Flow: 1.11 mL/min; Detection: UV at 220 nm.

Method C: Column: PHENOMENEX^{®} Luna 3 µm C18 (2.0 x 30 mm); mobile Phase A: 10:90 MeOH:water with 0.1% TFA; Mobile Phase B: 90:10 MeOH:water with 0.1% TFA; Gradient: 0-100% B over 2 minutes, then a 1 minute hold at 100% B; Flow: 1 mL/min; Detection: UV at 220 nm.

Method D: Waters Acquity UPLC BEH C18, 2.1 x 50 mm, 1.7 µm particles; Mobile Phase A: water with 0.05% TFA; Mobile Phase B: ACN with 0.05% TFA; Gradient: 2-98% B over 1 minute, then a 0.5 minute hold at 98% B; Flow: 0.8 mL/min; Detection: UV at 220 or 254 nm.

### Example 1 (reference)

### ((1R,4R,7R)-7-amino-2-azabicyclo[2.2.1]heptan-2-yl)(2-(7-bromo-1-(cyclopropylmethyl)-1H-indol-2-yl)-7-methoxy-1-methyl-1H-benzo[d]imidazol-5-yl)methanone

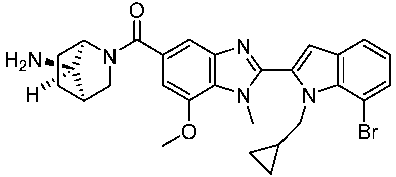

### Intermediate 1A: ethyl 7-bromo-1-(cyclopropylmethyl)-1H-indole-2-carboxylate

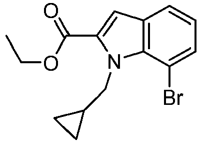

A stirring mixture of ethyl 7-bromo-1H-indole-2-carboxylate (2.8 g, 10.44 mmol) and potassium carbonate (4.33 g, 31.3 mmol) in DMF (20 mL) was treated with (bromomethyl)cyclopropane (2.026 mL, 20.89 mmol). The reaction was heated to 60 °C and stirred under a nitrogen atmosphere for 4 hours, at which point it was judged to be complete by LCMS. The reaction mixture poured into ethyl acetate (200 mL), and the turbid solution was washed 3X with 10% lithium chloride and once with brine, then dried over sodium sulfate and concentrated in vacuo. The residue was dried under high vacuum to yield ethyl 7-bromo-1-(cyclopropylmethyl)-1H-indole-2-carboxylate (3.36g, 10.43 mmol, 100 % yield). The material was used in the next step without further purification. ¹H NMR (499 MHz, chloroform-d) δ 7.65 (dd, *J*=7.9, 1.0 Hz, 1H), 7.54 (dd, *J*=7.6, 1.0 Hz, 1H), 7.35 (s, 1H), 7.00 (t, *J*=7.7 Hz, 1H), 5.12 (d, *J*=6.9 Hz, 2H), 4.39 (q, *J*=7.2 Hz, 2H), 1.43 (t, *J*=7.2 Hz, 3H), 1.38 - 1.26 (m, 1H), 0.42 (d, *J*=6.6 Hz, 4H). MS ESI m/z =322 (M+H).

### Intermediate 1B: 7-bromo-1-(cyclopropylmethyl)-1H-indole-2-carboxylic acid

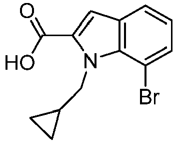

A stirring solution of ethyl 7-bromo-1-(cyclopropylmethyl)-1H-indole-2-carboxylate (5.2 g, 16.14 mmol) in methanol/THF (2:1) (75mL) was treated with 1M sodium hydroxide (48.4 mL, 48.4 mmol). The reaction was stirred at 50 °C for 18 hours, at which point it was judged to be complete by LCMS. The organic solvents were evaporated, and the remaining aqueous solution was washed twice with diethyl ether, then treated with 1M HCl (50 mL). The turbid mixture was extracted 4X with ethyl acetate, then the combined organic phases were washed once with brine, dried over sodium sulfate, and concentrated in vacuo to yield 7-bromo-1-(cyclopropylmethyl)-1H-indole-2-carboxylic acid (4.64g, 15.77 mmol, 98 % yield) as a colorless solid, which was used in the next step without further purification. ¹H NMR (499 MHz, DMSO-d₆) δ 13.17 (br s, 1H), 7.74 (dd, *J*=7.9, 1.0 Hz, 1H), 7.57 (dd, *J*=7.5, 1.0 Hz, 1H), 7.35 (s, 1H), 7.05 (t, *J*=7.7 Hz, 1H), 5.03 (d, *J*=7.0 Hz, 2H), 1.29 - 1.11 (m, 1H), 0.45 - 0.35 (m, 2H), 0.34 - 0.30 (m, 2H). MS ESI m/z =294 (M+H).

### Intermediate 1C: methyl 2-(7-bromo-1-(cyclopropylmethyl)-1H-indol-2-yl)-7-methoxy-1-methyl-1H-benzo[d]imidazole-5-carboxylate

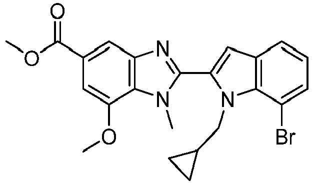

A stirring solution of methyl 3-amino-5-methoxy-4-(methylamino)benzoate hydrochloride (4.0 g, 16.21 mmol), 7-bromo-1-(cyclopropylmethyl)-1H-indole-2-carboxylic acid (4.64 g, 15.77 mmol), and Hunig's Base (6.89 mL, 39.4 mmol) in DMF (20 mL) was treated with HATU (7.20 g, 18.93 mmol). The reaction was stirred under a nitrogen atmosphere at room temperature for 18 hours, at which point it was judged to be complete by LCMS based on the disappearance of starting material, and the formation of a single product peak with m/z = 487 (M+H for intermediate carboximide). The reaction mixture was concentrated in vacuo, and the residue was taken up in ethyl acetate (350 mL) and water (100 mL). The turbid mixture was stirred for 15 minutes, and the resulting solids were collected by filtration, rinsed thoroughly with ethyl acetate and water, and dried under vacuum to yield 2.7 g of a colorless solid. LCMS of this material detects a single peak with m/z = 487. The combined filtrate and rinsings were transferred to a separatory funnel, the layers were separated, and the ethyl acetate phase was washed twice with 10% lithium chloride and once with brine, then dried over sodium sulfate and concentrated in vacuo. The residue and solids from the filtration were suspended in acetic acid (30 mL). The mixture was heated to 75 °C, resulting in a homogeneous solution, and the reaction was stirred for 90 minutes, at which point it was judged to be complete by LCMS. The mixture was concentrated in vacuo, and the residue was taken up in ethyl acetate (200 mL) and water (50 mL). A magnetic stir bar was added to the flask, and the stirring mixture was carefully treated with half-saturated sodium carbonate until gas evolution ceased. The mixture was stirred for another 10 minutes, during which time a colorless solid precipitated. The solids were collected by filtration, rinsed thoroughly with water, ethyl acetate, and methanol, and then dried under vacuum to yield 4.42 g of the title compound as a colorless solid. The combined filtrate and rinsings were transferred to a separatory funnel, and the layers were separated. The aqueous phase was extracted twice with ethyl acetate (75 mL), then the organic phases were combined, washed once with saturated sodium carbonate, once with water, and once with brine, then dried over sodium sulfate, and concentrated in vacuo. The residue was taken up in boiling methanol (100 mL), and the mixture was stirred for 10 minutes, then allowed to cool to room temperature. The resulting solids were collected by filtration, rinsed 3X with methanol, and dried under vacuum to yield 1.50 g of the title compound as a slightly amber solid. Combined, the two crops yielded methyl 2-(7-bromo-1-(cyclopropylmethyl)-1H-indol-2-yl)-7-methoxy-1-methyl-1H-benzo[d]imidazole-5-carboxylate (5.9g, 12.60 mmol, 80 % yield). ¹H NMR (499 MHz, DMSO-d₆) δ 7.98 (d, *J*=1.2 Hz, 1H), 7.75 (dd, *J*=7.9, 0.8 Hz, 1H), 7.55 (dd, *J*=7.5, 1.0 Hz, 1H), 7.41 (d, *J*=1.2 Hz, 1H), 7.17 (s, 1H), 7.09 (t, *J*=7.7 Hz, 1H), 4.76 (d, J=7.0 Hz, 2H), 4.09 (s, 3H), 4.04 (s, 3H), 3.90 (s, 3H), 1.09 - 0.99 (m, 1H), 0.30 - 0.22 (m, 2H), -0.14 - -0.21 (m, 2H). MS ESI m/z = 468 (M+H).

### Intermediate 1D: 2-(7-bromo-1-(cyclopropylmethyl)-1H-indol-2-yl)-7-methoxy-1-methyl-1H-benzo[d]imidazole-5-carboxylic acid

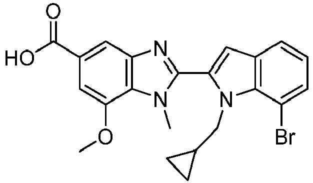

A stirring solution of methyl 2-(7-bromo-1-(cyclopropylmethyl)-1H-indol-2-yl)-7-methoxy-1-methyl-1H-benzo[d]imidazole-5-carboxylate (1.47g, 3.14 mmol) in 1:1 methanol/THF (10 mL) was treated with 1 M sodium hydroxide (9.42 mL, 9.42 mmol). The reaction was stirred at 50 °C for three hours, at which point it was judged to be complete by LCMS. The organic solvents were removed on the rotary evaporator, and the remaining aqueous suspension was adjusted to pH 5 with 1M HCl. The heterogeneous mixture was vigorously stirred for 20 minutes, then extracted 3X with ethyl acetate (100 mL). The combined organic phases were washed once with brine, dried over sodium sulfate and concentrated in vacuo to yield 2-(7-bromo-1-(cyclopropylmethyl)-1H-indol-2-yl)-7-methoxy-1-methyl-1H-benzo[d]imidazole-5-carboxylic acid (1.34g, 2.95 mmol, 94 % yield) as an off-white solid, which was used in the next step without further purification. ¹H NMR (499 MHz, CHLOROFORM-d) δ 7.94 (d, *J*=1.2 Hz, 1H), 7.74 (dd, *J*=7.9, 0.8 Hz, 1H), 7.54 (dd, *J*=7.6, 0.9 Hz, 1H), 7.40 (d, *J*=1.1 Hz, 1H), 7.15 (s, 1H), 7.08 (t, *J*=7.7 Hz, 1H), 4.75 (d, *J*=6.9 Hz, 2H), 4.08 (s, 3H), 4.02 (s, 3H), 1.07 - 0.98 (m, 1H), 0.29 - 0.21 (m, 2H), -0.16 - -0.22 (m, 2H). MS ESI m/z = 454 (M+H).

### Intermediate 1E: tert-butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate

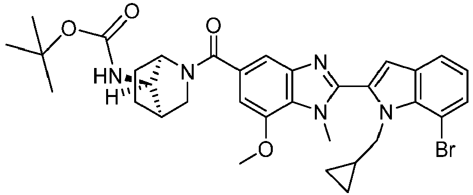

A stirring solution of *tert*-butyl ((1R,4R,7R)-2-azabicyclo[2.2.1]heptan-7-yl)carbamate (0.689 g, 3.24 mmol), 2-(7-bromo-1-(cyclopropylmethyl)-1H-indol-2-yl)-7-methoxy-1-methyl-1H-benzo[d]imidazole-5-carboxylic acid (1.34g, 2.95 mmol), and triethylamine (1.233 mL, 8.85 mmol) in DMF (20 mL) was treated with BOP (1.370 g, 3.10 mmol). The reaction was stirred at room temperature for 4 hours, at which point it was judged to be complete by LCMS. The mixture was concentrated in vacuo, and the residue was taken up in ethyl acetate (150 mL). The turbid solution was washed 3X with water, 3X with 1 M sodium hydroxide, and once with brine, then dried over sodium sulfate and concentrated in vacuo. The residue was chromatographed via MPLC over a 120 g silica gel column, eluting at 85 mL/min with a 0% to 10% methanol/dichloromethane gradient over 15 column volumes, holding at 4% methanol as the product eluted. Fractions containing the desired product were pooled and concentrated in vacuo to yield *tert*-butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (1.86g, 2.87 mmol, 97 % yield) as a white solid. ¹H NMR (499 MHz, chloroform-d) δ 7.70 - 7.62 (m, 1H), 7.57 - 7.47 (m, 2H), 7.08 - 6.99 (m, 2H), 6.88 - 6.80 (m, 1H), 4.87 - 4.70 (m, 2H), 4.68 - 4.46 (m, 1H), 4.36 (br s, 1H), 4.16 - 4.09 (m, 3H), 4.04 (s, 3H), 3.90 - 3.72 (m, 2H), 3.31 - 3.20 (m, 1H), 2.54 (br s, 1H), 2.11 - 1.83 (m, 3H), 1.77 - 1.68 (m, 1H), 1.52 - 1.35 (m, 9H), 1.20 - 1.07 (m, 1H), 0.37 - 0.23 (m, 2H), -0.02 - -0.25 (m, 2H). MS ESI m/z = 648 (M+H).

### Example 1: ((1R,4R,7R)-7-amino-2-azabicyclo[2.2.1]heptan-2-yl)(2-(7-bromo-1-(cyclopropylmethyl)-1H-indol-2-yl)-7-methoxy-1-methyl-1H-benzo[d]imidazol-5-yl)methanone

A stirring solution of *tert*-butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (1.6 g, 2.467 mmol) in dichloromethane (10 mL) was treated with 4M HCl in dioxane (10 mL). The reaction was stirred at room temperature for 1 hour, at which point it was judged to be complete by LCMS. The mixture was concentrated in vacuo, and the residue was taken up in water (15 mL). The solution was treated carefully with saturated sodium carbonate until further addition failed to produce gas evolution. The turbid solution was extracted 3X with ethyl acetate, then the combined organic phases were washed once with brine, dried over sodium sulfate, and concentrated in vacuo to yield ((1R,4R,7R)-7-amino-2-azabicyclo[2.2.1]heptan-2-yl)(2-(7-bromo-1-(cyclopropylmethyl)-1H-indol-2-yl)-7-methoxy-1-methyl-1H-benzo[d]imidazol-5-yl)methanone (1.23g, 2.243 mmol, 91 % yield) as a colorless solid. 1H NMR (499 MHz, chloroform-d) δ 7.70 - 7.62 (m, 1H), 7.58 - 7.49 (m, 2H), 7.08 - 7.00 (m, 2H), 6.88 - 6.80 (m, 1H), 4.87 - 4.71 (m, 2H), 4.17 - 4.10 (m, 3H), 4.08 - 4.00 (m, 4H), 3.81 - 3.67 (m, 1H), 3.52 - 3.34 (m, 1H), 3.33 - 3.17 (m, 1H), 2.39 - 2.24 (m, 1H), 2.16 - 1.90 (m, 3H), 1.69 - 1.59 (m, 1H), 1.20 - 1.09 (m, 1H), 0.35 - 0.24 (m, 2H), -0.08 - -0.21 (m, 2H). MS ESI m/z 548 (M+H). Anal. HPLC Retention time: 0.82 minutes, Method D.

### Example 2 (reference)

### ((1R,4R,7R)-7-amino-2-azabicyclo[2.2.1]heptan-2-yl)(2-(1-(cyclopropylmethyl)-7-(3-hydroxypropyvl)-1H-indol-2-yl)-7-methoxy-1-methyl-1H-benzo[d]imidazol-5-yl)methanone

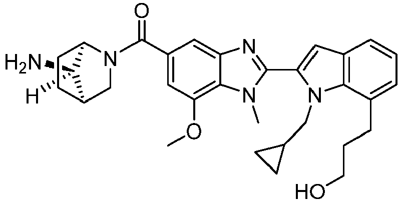

In a 2-dram vial, a stirring mixture of 3-bromo-1-propanol (10.05 µl, 0.116 mmol), *tert*-butyl (2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (50 mg, 0.077 mmol), tris(trimethylsilyl)silane (0.036 mL, 0.116 mmol), Ir(dF(CF3)ppy)2(dtbbpy)PF6 (4.32 mg, 3.85 µmol), and sodium carbonate (32.7 mg, 0.308 mmol) in 1,4-Dioxane (2 mL) was degassed with bubbling nitrogen for 10 minutes. In a second vial, a stirring mixture of nickel(II) chloride ethylene glycol dimethyl ether complex (4.23 mg, 0.019 mmol), and 4,4'-di-*tert*-butyl-2,2'-bipyridine (6.21 mg, 0.023 mmol) in 1,4-Dioxane (1 mL) was degassed with nitrogen for 20 minutes. The nickel complex was transferred via syringe to the first vial, and the mixture was degassed with bubbling nitrogen for an additional 5 minutes. The vial was sealed, and the reaction was stirred at room temperature under two blue Kessil lamps for 18 hours, at which point it was judged to be complete by LCMS based on the disappearance of starting material. The reaction mixture was diluted with dichloromethane (3 mL). Solids were removed by filtration and rinsed with dichloromethane, and the combined filtrates and rinsings were treated with 4 M HCl in dioxane (2 mL). The reaction was stirred at room temperature for 1 hour, at which point it was judged to be complete by LCMS. The mixture was concentrated in vacuo. The crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 20% B, 20-60% B over 20 minutes, then a 4-minute hold at 100% B; Flow Rate: 20 mL/min; Column Temperature: 25 C. Fraction collection was triggered by MS and UV signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield ((1R,4R,7R)-7-amino-2-azabicyclo [2.2.1]heptan-2-yl)(2-(1-(cyclopropylmethyl)-7-(3-hydroxypropyl)-1H-indol-2-yl)-7-methoxy-1-methyl-1H-benzo[d]imidazol-5-yl)methanone (19.9 mg, 0.036 mmol, 47.0 % yield). 1H NMR (500 MHz, DMSO-d6) δ 7.34 (br d, J=7.0 Hz, 1H), 7.26 - 7.12 (m, 1H), 6.92 - 6.85 (m, 2H), 6.82 (s, 1H), 6.77 - 6.68 (m, 1H), 4.26 (br d, J=5.8 Hz, 2H), 3.92 - 3.84 (m, 3H), 3.78 (s, 3H), 3.59 - 3.45 (m, 3H), 3.34 (br t, J=6.0 Hz, 2H), 2.94 - 2.82 (m, 3H), 2.15 - 1.97 (m, 1H), 1.87 - 1.44 (m, 6H), 1.39 - 1.14 (m, 1H), 0.71 - 0.52 (m, 1H), 0.01 (br d, J=7.6 Hz, 2H), -0.43 - -0.69 (m, 2H). MS ESI m/z 528 (M+H). Anal. HPLC Retention time: 1.56 minutes, Method 1.

The following compounds in Table 1 can be made by the procedures described in Example 2, substituting the appropriate alkyl halide for 3-bromo-1-propanol. For examples where the substituent at the indole 7-position contains a basic amine, the appropriate Boc-protected aminoalkyl halide was used, and the Boc-group was cleaved during the final deprotection step.

**Table 1**

| Ex # | Structure | Name | LC/MS Rt (min) | LC/ MS Meth od | M+H (obs ion) |
|---|---|---|---|---|---|
| 4 | 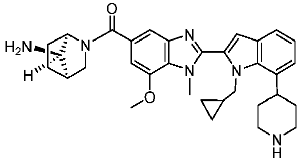 | (1R,4R,7R)-2-{2-[1-(cyclopropylmethyl)-7-(piperidin-4-yl)-1H-indol-2-yl]-7-methoxy-1-methyl-1H-1,3-benzodiazole-5-carbonyl }-2-azabicyclo[2.2.1]heptan-7-amine | 1.03 | 2 | 553.2 |
| 8 | 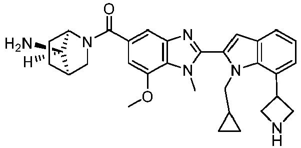 | (1R,4R,7R)-2-{2-[7-(azetidin-3-yl)-1-(cyclopropylmethyl)-1H-indol-2-yl]-7-methoxy-1-methyl-1H-1,3-benzodiazole-5-carbonyl }-2-azabicyclo[2.2. 1]heptan-7-amine | 1.25 | 1 | 525.0 |
| 9 | 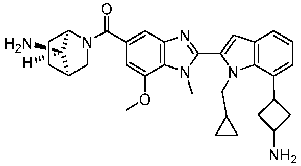 | (1R,4R,7R)-2-{2-[7-(3-aminocyclobutyl)-1-(cyclopropylmethyl)-1H-indol-2-yl]-7-methoxy-1-methyl-1H-1,3-benzodiazole-5-carbonyl }-2-azabicyclo[2.2. 1]heptan-7-amine | 1.13 | 1 | 539.4 |
| 10 | 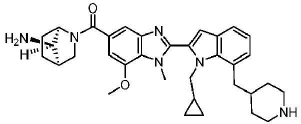 | (1R,4R,7R)-2-{2-[1-(cyclopropylmethyl)-7-[(piperidin-4-yl)methyl]-1H-indol-2-yl]-7-methoxy-1-methyl-1H-1,3-benzodiazole-5-carbonyl }-2-azabicyclo[2.2. 1]heptan-7-amine | 1.32 | 1 | 567.4 |
| 12 | 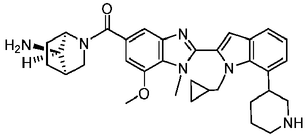 | (1R,4R,7R)-2-{2-[1-(cyclopropylmethyl)-7-(piperidin-3-yl)-1H-indol-2-yl]-7-methoxy-1-methyl-1H-1,3-benzodiazole-5-carbonyl}-2-azabicyclo[2.2.1]heptan-7-amine | 1.07 | 2 | 553.4 |
| 13 | 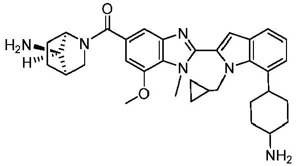 | (1R,4R,7R)-2-{2-[7-(4-aminocyclohexyl)-1-(cyclopropylmethyl)-1H-indol-2-yl]-7-methoxy-1-methyl-1H-1,3-benzodiazole-5-carbonyl }-2-azabicyclo[2.2. 1]heptan-7-amine | 1.09 | 2 | 567.4 |
| 14 | 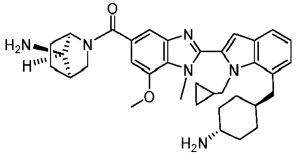 | ((1R,4R,7R)-7-amino-2-azabicyclo[2.2.1]heptan-2-*yl)(2-(7-(((trans)-4-*aminocyclohexyl)methyl)-1-(cyclopropylmethyl)-1H-indol-2-yl)-7-methoxy-1-methyl-1H-benzo[d]imidazol-5-yl)methanone | 1.46 | 1 | 581.5 |
| 15 | 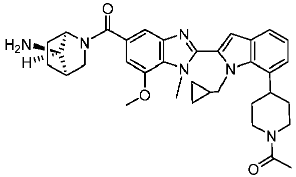 | 1-[4-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)piperidin-1-yl]ethan-1-one | 1.32 | 2 | 594.9 |
| 18 | 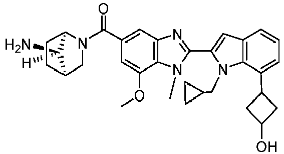 | *cis-* and *trans*-3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobutan-1-ol | 1.59/1. 63 | 1 | 539.9 |
| 22 | 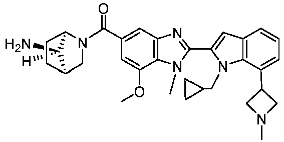 | (1R,4R,7R)-2-{2-[1-(cyclopropylmethyl)-7-(1-methylazetidin-3-yl)-1H-indol-2-yl]-7-methoxy-1-methyl-1H-1,3-benzodiazole-5-carbonyl}-2-azabicyclo[2.2. 1]heptan-7-amine | 1.17 | 1 | 539.5 |
| 23 | 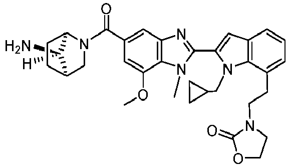 | 3-[2-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)ethyl]-1,3-oxazolidin-2-one | 1.41 | 1 | 583.5 |
| 24 | 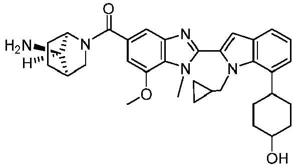 | 4-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclohexan-1-ol | 1.67 | 1 | 568.4 |
| 25 | 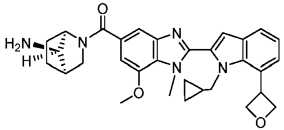 | (1R,4R,7R)-2-{2-[1-(cyclopropylmethyl)-7-(oxetan-3-yl)-1H-indol-2-yl]-7-methoxy-1-methyl-1H-1,3-benzodiazole-5-carbonyl }-2-azabicyclo[2.2.1]heptan-7-amine | 1.68 | 1 | 526.1 |
| 26 | 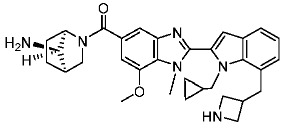 | (1R,4R,7R)-2-(2-{7-[(azetidin-3-yl)methyl]-1-(cyclopropylmethyl)-1H-indol-2-yl}-7-methoxy-1-methyl-1H-1,3-benzodiazole-5-carbonyl)-2-azabicyclo[2.2. 1]heptan-7-amine | 1.44 | 1 | 538.9 |
| 28 | 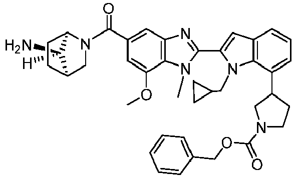 | benzyl 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)pyrrolidine-1 - carboxylate | 1.85 | 2 | 673.0 |
| 29 | 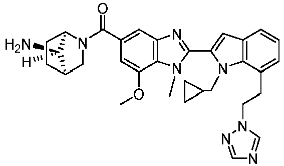 | (1R,4R,7R)-2-{2-[1-(cyclopropylmethyl)-7-[2-(1H-1,2,4-triazol-1-yl)ethyl]-1H-indol-2-yl]-7-methoxy-1-methyl-1H-1,3-benzodiazole-5-carbonyl }-2-azabicyclo[2.2. 1]heptan-7-amine | 1.48 | 1 | 565.0 |
| 30 | 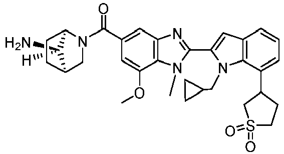 | ((1R,4R,7R)-7-amino-2-azabicyclo[2.2.1]heptan-2-yl)(2-(1-(cyclopropylmethyl)-7-dioxidotetrahydrothiophen-3-yl)-1H-indol-2-yl)-7-methoxy-1-methyl-1H-benzo[d]imidazol-5-yl)methanone | 1.47 | 1 | 588.4 |
| 31 | 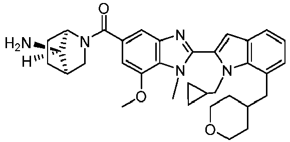 | (1R,4R,7R)-2-{2-[1-(cyclopropylmethyl)-7-[(oxan-4-yl)methyl]-1H-indol-2-yl]-7-methoxy-1-methyl-1H-1,3-benzodiazole-5-carbonyl }-2-azabicyclo[2.2. 1]heptan-7-amine | 1.55 | 2 | 568.2 |
| 32 | 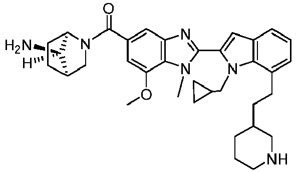 | (1R,4R,7R)-2-{2-[1-(cyclopropylmethyl)-7-[2-(piperidin-3-yl)ethyl]-1H-indol-2-yl]-7-methoxy-1-methyl-1H-1,3-benzodiazole-5-carbonyl }-2-azabicyclo[2.2. 1]heptan-7-amine | 1.38 | 1 | 581.3 |
| 33 | 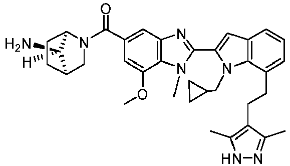 | (1R,4R,7R)-2-{2-[1-(cyclopropylmethyl)-7-[2-(3,5-dimethyl-1H-pyrazol-4-yl)ethyl]-1H-indol-2-yl]-7-methoxy-1-methyl-1H-1,3-benzodiazole-5-carbonyl }-2-azabicyclo[2.2.1]heptan-7-amine | 1.66 | 1 | 592.5 |
| 36 | 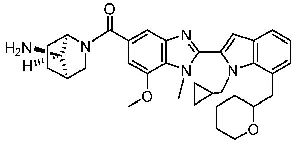 | (1R,4R,7R)-2-{2-[1-(cyclopropylmethyl)-7-[(oxan-2-yl)methyl]-1H-indol-2-yl]-7-methoxy-1-methyl-1H-1,3-benzodiazole-5-carbonyl }-2-azabicyclo[2.2. 1]heptan-7-amine | 2.05 | 1 | 568.3 |

### Example 37

### 1-(3-(2-(5-((1R,4R,7R)-7-amino-2-azabicvclo[2.2.1]heptane-2-carbonyl)-7-methoxy-1-methyl-1H-benzo[d]imidazol-2-yl)-1-(cyclopropylmethyl)-1H-indol-7-yl)azetidin-1-yl)ethan-1-one

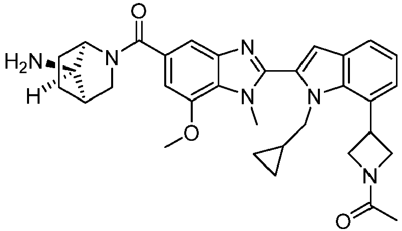

### Intermediate 37A: methyl 2-(7-(1-(tert-butoxycarbonyl)azetidin-3-yl)-1-(cyclopropylmethyl)-1H-indol-2-yl)-7-methoxy-1-methyl-1H-benzo[d]imidazole-5-carboxylate

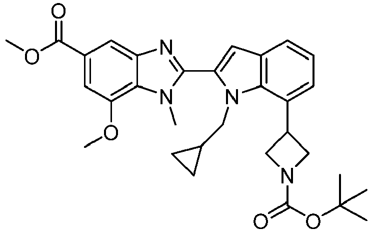

In a 1-dram vial, a stirring mixture of nickel(II) chloride ethylene glycol dimethyl ether complex (10.31 mg, 0.047 mmol) and 4,4'-di-*tert*-butyl-2,2'-bipyridine (15.11 mg, 0.056 mmol) in 1,4-Dioxane (2 mL) was degassed with bubbling nitrogen for 20 minutes. In a separate 20 mL scintillation vial, a stirring mixture of methyl 2-(7-bromo-1-(cyclopropylmethyl)-1H-indol-2-yl)-7-methoxy-1-methyl-1H-benzo[d]imidazole-5-carboxylate (293 mg, 0.626 mmol), *tert*-butyl 3-iodoazetidine-1-carboxylate (354 mg, 1.251 mmol), Ir(dF(CF3)ppy)2(dtbbpy)PF6 (10.53 mg, 9.38 µmol), tris(trimethylsilyl)silane (0.290 mL, 0.938 mmol), and sodium carbonate (265 mg, 2.502 mmol) in 1,4-Dioxane (10 mL) was degassed with nitrogen for 10 minutes. The nickel complex was transferred to the vial containing the reaction mixture, the vial was sealed, and the reaction was stirred at room temperature under a blue Kessil lamp for 60 hours, at which point it was judged to be complete by LCMS based on the disappearance of starting material. The reaction mixture was diluted with dichloromethane (20 mL). Solids were removed by filtration and rinsed with dichloromethane, and the combined filtrates and rinsings were concentrated in vacuo. The residue was chromatographed via MPLC over a 40 g silica gel column, eluting at 40 mL/min with a 0% to 5% methanol/dichloromethane gradient over 20 column volumes. Fractions containing the major peak were pooled and concentrated in vacuo to yield methyl 2-(7-(1-(*tert-*butoxycarbonyl)azetidin-3-yl)-1-(cyclopropylmethyl)-1H-indol-2-yl)-7-methoxy-1-methyl-1H-benzo[d]imidazole-5-carboxylate (278 mg, 0.510 mmol, 82 % yield) as a colorless solid. ¹H NMR (499 MHz, chloroform-d) δ 8.18 (d, *J*=1.2 Hz, 1H), 7.65 (dd, *J*=7.8, 0.8 Hz, 1H), 7.51 - 7.43 (m, 2H), 7.27 (t, *J*=7.7 Hz, 1H), 6.90 (s, 1H), 4.68 - 4.56 (m, 1H), 4.50 - 4.41 (m, 4H), 4.27 (br s, 2H), 4.17 (s, 3H), 4.07 (s, 3H), 3.98 (s, 3H), 3.73 (s, 2H), 1.50 (s, 9H), 1.47 - 1.43 (m, 2H), 0.88 - 0.79 (m, 1H), 0.30 - 0.24 (m, 2H), -0.26 (br s, 2H). MS ESI m/z = 545 (M+H).

### Intermediate 37B: methyl 2-(7-(1-acetylazetidin-3-yl)-1-(cyclopropylmethyl)-1H-indol-2-yl)-7-methoxy-1-methyl-1H-benzo[d]imidazole-5-carboxylate

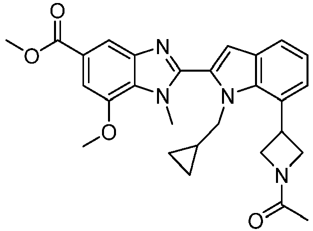

A stirring solution of methyl 2-(7-(1-(*tert*-butoxycarbonyl)azetidin-3-yl)-1-(cyclopropylmethyl)-1H-indol-2-yl)-7-methoxy-1-methyl-1H-benzo[d]imidazole-5-carboxylate (0.39 g, 0.716 mmol) in dichloromethane (4 mL) was treated with TFA (0.4 mL, 5.19 mmol). The reaction was stirred at room temperature for 1 hour, at which point it was judged to be complete by LCMS. The mixture was concentrated in vacuo, and the residue was taken up in dichloromethane. The mixture was treated with triethylamine (0.399 mL, 2.86 mmol) and stirred for 5 minutes, then treated with acetic anhydride (0.068 mL, 0.716 mmol). The reaction was stirred at room temperature for 1 hours, at which point it was judged to be complete by LCMS. The mixture was treated with methanol and concentrated in vacuo, and residue was chromatographed via MPLC over a 40 g silica gel column, eluting at 40 mL/min with a 0% to 2% methanol/dichloromethane gradient over 5 column volumes, then 2% methanol/dichloromethane for 7 column volumes as impurities eluted, then a 2 - 3.5% methanol/dichloromethane gradient over 3 column volumes, then 3.5% methanol/dichloromethane to fully elute the desired product. Fractions containing the desired product were pooled and concentrated in vacuo to yield methyl 2-(7-(1-acetylazetidin-3-yl)-1-(cyclopropylmethyl)-1H-indol-2-yl)-7-methoxy-1-methyl-1H-benzo[d]imidazole-5-carboxylate (270 mg, 0.555 mmol, 77 % yield) as a colorless solid. ¹H NMR (499 MHz, chloroform-d) δ 8.18 (d, *J*=1.2 Hz, 1H), 7.67 (dd, *J*=7.8, 0.8 Hz, 1H), 7.48 (d, *J*=1.2 Hz, 1H), 7.44 (d, *J*=7.3 Hz, 1H), 7.28 - 7.26 (m, 1H), 6.91 (s, 1H), 4.74 - 4.63 (m, 2H), 4.61 - 4.31 (m, 5H), 4.18 (s, 3H), 4.07 (s, 3H), 3.99 (s, 3H), 1.97 (s, 3H), 0.88 - 0.79 (m, 1H), 0.36 - 0.21 (m, 2H), -0.16 (dq, *J*=9.7, 4.9 Hz, 1H), -0.35 (dq, *J*=9.7, 4.9 Hz, 1H). MS ESI m/z = 487 (M+H).

### Intermediate 37C: 2-(7-(1-acetylazetidin-3-yl)-1-(cyclopropylmethyl)-1H-indol-2-yl)-7-methoxy-1-methyl-1H-benzo[d]imidazole-5-carboxylic acid

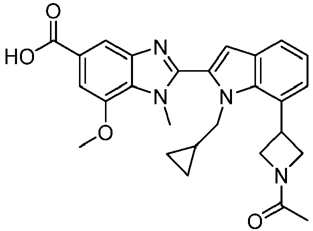

A stirring solution of methyl 2-(7-(1-acetylazetidin-3-yl)-1-(cyclopropylmethyl)-1H-indol-2-yl)-7-methoxy-1-methyl-1H-benzo[d]imidazole-5-carboxylate (105 mg, 0.216 mmol) in methanol (2 mL) was treated with 1M sodium hydroxide (0.259 mL, 0.259 mmol), and the reaction was stirred at room temperature for 18 hours, at which point it was judged to be complete by LCMS. The methanol was evaporated, and the remaining aqueous mixture was diluted with water (4 mL) and treated with 1 M HCl (0.26 mL). The heterogeneous mixture was extracted 5X with dichloromethane, and the combined organic phases were dried over sodium sulfate and concentrated in vacuo to yield 2-(7-(1-acetylazetidin-3-yl)-1-(cyclopropylmethyl)-1H-indol-2-yl)-7-methoxy-1-methyl-1H-benzo[d]imidazole-5-carboxylic acid (70 mg, 0.148 mmol, 68.6 % yield) as a colorless solid. ¹H NMR (499 MHz, DMSO-d₆) δ 13.12 - 12.21 (m, 1H), 7.95 - 7.92 (m, 1H), 7.66 - 7.61 (m, 1H), 7.48 (d, *J*=7.0 Hz, 1H), 7.41 (d, *J*=1.2 Hz, 1H), 7.22 (t, *J*=7.6 Hz, 1H), 7.11 (s, 1H), 4.72 - 4.60 (m, 2H), 4.50 - 4.33 (m, 4H), 4.15 - 4.06 (m, 4H), 4.03 (s, 3H), 1.85 (s, 3H), 0.86 - 0.73 (m, 1H), 0.28 - 0.16 (m, 2H), - 0.24 - -0.41 (m, 2H). MS ESI m/z = 473 (M+H).

### Intermediate 37D: tert-butyl ((1R,4R,7R)-2-(2-(7-(1-acetylazetidin-3-yl)-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate

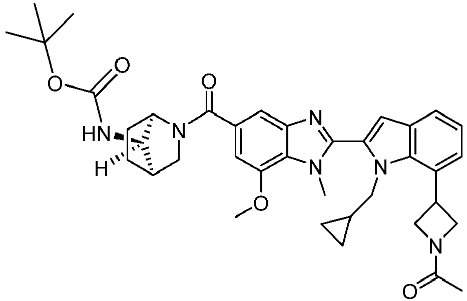

A stirring solution of *tert*-butyl ((1R,4R,7R)-2-azabicyclo[2.2.1]heptan-7-yl)carbamate (47.2 mg, 0.222 mmol), 2-(7-(1-acetylazetidin-3-yl)-1-(cyclopropylmethyl)-1H-indol-2-yl)-7-methoxy-1-methyl-1H-benzo[d]imidazole-5-carboxylic acid (70 mg, 0.148 mmol), and triethylamine (0.062 mL, 0.444 mmol) in dichloromethane (2 mL) was treated with BOP (79 mg, 0.178 mmol). The reaction was stirred at room temperature for 18 hours, at which point it was judged to be complete by LCMS. The mixture was diluted with dichloromethane (10 mL), and the solution was washed twice with 1M HCl, twice with 1M NaOH, and once with brine, then dried over sodium sulfate and concentrated in vacuo. The residue was chromatographed via MPLC over a 24 g silica gel column, eluting at 40 mL/min with a 0% to 7% methanol/dichloromethane gradient over 13 column volumes. Fractions containing the desired product were pooled and concentrated in vacuo to yield *tert*-butyl ((1R,4R,7R)-2-(2-(7-(1-acetylazetidin-3-yl)-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate. ¹H NMR (499 MHz, chloroform-d) δ 7.67 (d, *J*=7.7 Hz, 1H), 7.54 - 7.47 (m, 1H), 7.44 (d, *J*=7.4 Hz, 1H), 7.28 - 7.23 (m, 1H), 7.06 - 6.98 (m, 1H), 6.93 - 6.87 (m, 1H), 4.75 - 4.62 (m, 2H), 4.59 - 4.29 (m, 7H), 4.16 (s, 3H), 4.04 (d, *J*=1.0 Hz, 3H), 3.92 - 3.72 (m, 2H), 3.28 (dd, *J*=11.3*,* 1.9 Hz, 1H), 2.54 (br s, 1H), 1.97 (d, *J*=2.6 Hz, 5H), 1.61 - 1.35 (m, 10H), 0.96 - 0.76 (m, 2H), 0.38 - 0.18 (m, 2H), -0.06 - -0.46 (m, 2H). MS ESI m/z = 667 (M+H).

### Example 37: 1-(3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)azetidin-1-yl)ethan-1-one

A stirring solution of *tert*-butyl ((1R,4R,7R)-2-(2-(7-(1-acetylazetidin-3-yl)-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (79 mg, 0.118 mmol) in dichloromethane (2 mL) was treated with TFA (0.5 mL). The reaction was stirred at room temperature for 3 hours, at which point it was judged to be complete by LCMS. The mixture was concentrated in vacuo, and the residue was taken up in water (3 mL). The solution was treated with saturated sodium bicarbonate (5 mL), and the homogeneous solution was stirred for 10 minutes. The mixture was extracted 4X with ethyl acetate (5 mL) (failed to fully extract the product), then 3X with 9:1 dichloromethane/methanol (completely extracted the product). All organic phases were combined and dried over sodium sulfate, then concentrated in vacuo. The residue was chromatographed via MPLC over a 24 g silica gel column, eluting at 40 mL/min with a 3% to 10% methanol/dichloromethane gradient over 10 column volumes, then with 10% methanol/dichloromethane to completely elute the product. Fractions containing the desired product were pooled and concentrated in vacuo. The residue was taken up in 5:1 dichloromethane/methanol, and treated with Si-pyridine resin. The mixture was shaken for 4 hours so the resin could remove any trace metal contaminants, then the resin was removed by filtration and rinsed with 5:1 dichloromethane/methanol. The combined filtrate and rinsings were concentrated in vacuo, and the residue was taken up in 2:1 acetonitrile/water. The solution was freeze dried to yield 1-(3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)azetidin-1-yl)ethan-1-one (51 mg, 0.088 mmol, 74.4 % yield) as a colorless solid. ¹H NMR (499 MHz, chloroform-d) δ 7.67 (d, *J*=7.7 Hz, 1H), 7.54 - 7.50 (m, 1H), 7.44 (d, *J*=7.4 Hz, 1H), 7.28 - 7.25 (m, 1H), 7.04 - 7.00 (m, 1H), 6.92 - 6.87 (m, 1H), 4.74 - 4.62 (m, 2H), 4.54 (br t, *J*=8.8 Hz, 1H), 4.50 - 4.33 (m, 4H), 4.19 - 4.15 (m, 3H), 4.04 (s, 4H), 3.79 - 3.66 (m, 1H), 3.52 - 3.34 (m, 1H), 3.31 - 3.16 (m, 1H), 2.41 - 2.25 (m, 1H), 2.14 - 1.89 (m, 6H), 1.76 - 1.62 (m, 2H), 1.31 - 0.76 (m, 2H), 0.37 - 0.20 (m, 2H), -0.11 - -0.24 (m, 1H), -0.28 - -0.44 (m, 1H). MS ESI m/z = 567.2 (M+H). HPLC retention time 0.66 minutes, Method D.

The following compounds in Table 2 can be made by the procedures described in Example 37, substituting *tert*-butyl 3-iodopiperidine-1-carboxylate for *tert*-butyl 3-iodoazetidine-1-carboxylate in step 1, and the appropriate anhydride or isocyanate for acetic anhydride in step 2.

**Table 2**

| Ex # | Structure | Name | LC/MS Rt (min) | LC/ MS Met hod | M+H (obs ion) |
|---|---|---|---|---|---|
| 38 | 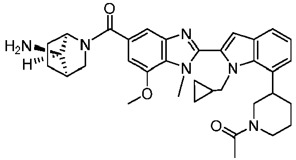 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)piperidin-1-yl]ethan-1-one | 1.71 | 1 | 595.2 |
| 39 | 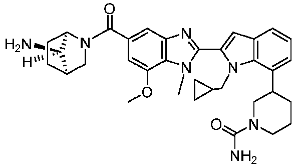 | 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)piperidine-1-carboxamide | 1.49 | 1 | 596.3 |

### Example 40

### Methyl 3-(2-(5-((1R,4R,7R)-7-amino-2-azabicyclo[2.2.1]heptane-2-carbonyl)-7-methoxy-1-methyl-1H-benzo[d1imidazol-2-yl)-1-(cyclopropylmethyl)-1H-indol-7-yl)azetidine-1-carboxylate

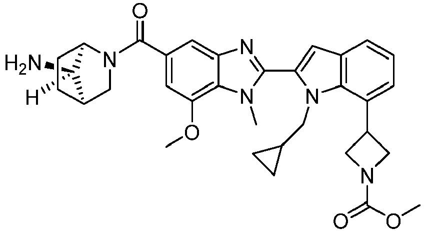

The title compound was prepared via the same procedures used to prepare Example 37, substituting methyl chloroformate for acetic anhydride in step 2. ¹H NMR (499 MHz, chloroform-d) δ 7.65 (d, *J*=7.7 Hz, 1H), 7.54 - 7.50 (m, 1H), 7.46 (d, *J*=7.4 Hz, 1H), 7.27 - 7.24 (m, 1H), 7.05 - 6.99 (m, 1H), 6.90 - 6.85 (m, 1H), 4.68 (quin, *J*=7.4 Hz, 1H), 4.52 (t, *J*=8.5 Hz, 2H), 4.46 - 4.40 (m, 2H), 4.32 (br d, *J*=4.4 Hz, 2H), 4.18 - 4.13 (m, 3H), 4.06 - 3.98 (m, 4H), 3.79 - 3.65 (m, 4H), 3.50 - 3.34 (m, 1H), 3.31 - 3.16 (m, 1H), 2.40 - 2.23 (m, 1H), 2.14 - 1.88 (m, 3H), 1.70 - 1.60 (m, 2H), 0.92 - 0.77 (m, 1H), 0.33 - 0.22 (m, 2H), -0.17 - -0.37 (m, 2H). MS ESI m/z = 583.6 (M+H). HPLC retention time 0.80 minutes, Method D.

### Example 41

### 1-[3-(2-{5-[(3R,5R)-3-amino-5-fluoropiperidine-1-carbonyl]-7-methoxy-1-methyl-1H-1,3-benzodiazol-2-yl}-1-(cyclopropylmethyl)-1H-indol-7-yl)azetidin-1-yl]ethan-1-one

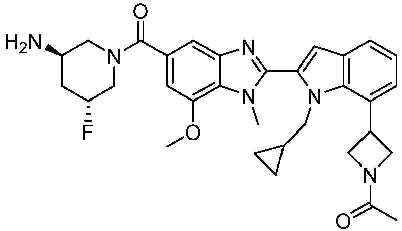

The title compound was prepared via the same procedures used to prepare Example 37, substituting *tert*-butyl ((3R, 5R)-5-fluoropiperidin-3-yl)carbamate for ((1R,4R,7R)-2-azabicyclo[2.2.1]heptan-7-yl)carbamate in step 4. ¹H NMR (500 MHz, DMSO-d₆) δ 7.39 (d, *J*=7.7 Hz, 1H), 7.21 (d, *J*=7.3 Hz, 1H), 7.07 (s, 1H), 6.98 (t, *J*=7.6 Hz, 1H), 6.81 (s, 1H), 6.61 (s, 1H), 4.77 - 4.57 (m, 1H), 4.49 - 4.35 (m, 2H), 4.23 - 4.06 (m, 4H), 3.84 (s, 4H), 3.74 (s, 3H), 3.17 (br s, 6H), 2.80 (br t, *J*=10.8 Hz, 1H), 1.95 (br t, *J*=11.1 Hz, 1H), 1.61 (s, 3H), 1.42 - 1.21 (m, 1H), 0.62 - 0.50 (m, 1H), -0.01 (brd, *J*=8.1 Hz, 2H), -0.51 (br dd, *J*=8.8, 4.7 Hz, 2H). MS ESI m/z = 572.9 (M+H). HPLC retention time 1.48 minutes, Method 1.

### Example 42

### 1-(3-(2-(5-((2S,5R)-5-amino-2-methylpiperidine-1-carbonyl)-7-methoxy-1-methyl-1H-benzo[d]imidazol-2-yl)-1-(cyclopropylmethyl)-1H-indol-7-yl)azetidin-1-yl)ethan-1-one

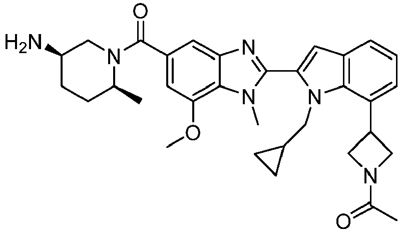

The title compound was prepared via the same procedures used to prepare Example 37, substituting *tert*-butyl ((3R,6S)-6-methylpiperidin-3-yl)carbamate for ((1R,4R,7R)-2-azabicyclo[2.2.1]heptan-7-yl)carbamate in step 4. ¹H NMR (500 MHz, DMSO-d₆) δ 7.43 (d, *J*=7.6 Hz, 1H), 7.25 (br d, *J*=7.6 Hz, 1H), 7.06 (s, 1H), 7.01 (t, *J*=7.6 Hz, 1H), 6.86 (s, 1H), 6.61 (s, 1H), 4.50 - 4.38 (m, 2H), 4.25 - 4.10 (m, 4H), 3.87 (s, 4H), 3.78 (s, 3H), 3.37 - 3.21 (m, 1H), 2.63 - 2.43 (m, 2H), 1.68 (s, 3H), 1.64 (s, 3H), 1.58 - 1.41 (m, 2H), 1.32 (br d, *J*=8.2 Hz, 2H), 1.00 (br d, *J*=6.7 Hz, 3H), 0.57 (br d, *J*=5.8 Hz, 1H), 0.01 (br d, *J*=7.9 Hz, 2H), -0.44 - -0.59 (m, 2H). MS ESI m/z = 569.1 (M+H). HPLC retention time 1.49 minutes, Method 1.

### Examples 43 and 44

### 3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-methylcyclobutane-l-carboxamide, ISOMER 1, and 3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 2.

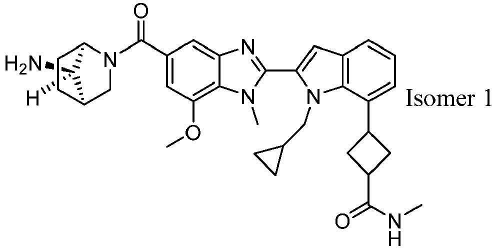

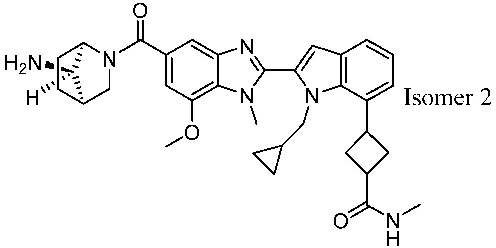

### Intermediate 43/44A: methyl 3-(tosyloxy)cyclobutane-1-carboxylate

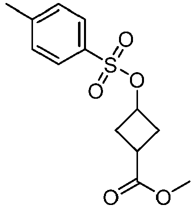

In a 40 mL scintillation vial, a stirring solution of methyl 3-hydroxycyclobutane-1-carboxylate (834 mg, 6.41 mmol), Ts-Cl (1833 mg, 9.61 mmol), and triethylamine (1.786 mL, 12.82 mmol) in dichloromethane (10 mL) was treated with DMAP (78 mg, 0.641 mmol). The vial was sealed, and the reaction was stirred at room temperature for 18 hours. TLC (50% EtOAc/hexane, UV, KMnO₄) indicated that the reaction was complete. The reaction mixture was concentrated onto celite and chromatographed via MPLC over an 80 g silica gel column, eluting at 60 mL/min with a 0% to 50% acetone/hexanes gradient over 15 column volumes. Fractions containing the desired product were pooled and concentrated in vacuo to yield methyl 3-(tosyloxy) cyclobutane-1-carboxylate (1.55g, 5.45 mmol, 85 % yield) as a colorless oil. ¹H NMR (499 MHz, chloroform-d) δ 7.83 - 7.75 (m, 2H), 7.36 (d, *J*=8.0 Hz, 2H), 4.81 - 4.72 (m, 1H), 3.68 (s, 3H), 2.69 - 2.59 (m, 1H), 2.56 - 2.39 (m, 7H).

### Intermediate 43/44B: methyl 3-iodocyclobutane-1-carboxylate

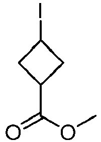

A mixture of methyl 3-(tosyloxy)cyclobutane-1-carboxylate (1.1g, 3.87 mmol) and sodium iodide (2.320 g, 15.48 mmol) in 2-butanone (4 mL) was stirred at 100 °C for 18 hours. The mixture was allowed to come to room temperature and diluted with dichloromethane (25 mL), and the resulting solids were removed by filtration. The residue was chromatographed via MPLC over a 24 g silica gel column, eluting at 40 mL/min with a 0% to 70% ethyl acetate/hexanes gradient over 15 column volumes. The *cis*-and *trans*-isomers were partially separated on the column, but all fractions were combined to yield methyl 3-iodocyclobutane-1-carboxylate (805 mg, 3.35 mmol, 87 % yield) as a colorless oil. ¹H NMR (499 MHz, chloroform -d) δ 4.73 - 4.34 (m, 1H), 3.76 - 3.65 (m, 3H), 3.49 - 3.10 (m, 1H), 3.02 - 2.89 (m, 3H), 2.89 - 2.73 (m, 1H).

### Intermediate 43/44C: methyl 3-(2-(5-((lR,4R,7R)-7-((tert-butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutane-1-carboxylate

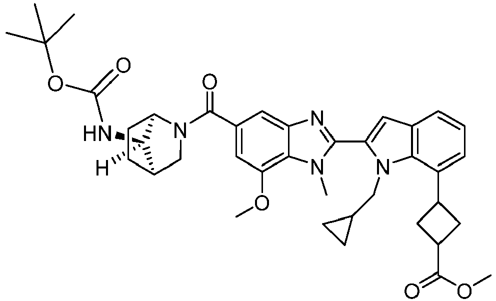

The title compound was prepared from methyl 3-iodocyclobutane-1-carboxylate and *tert-*butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate using the procedure described in Example 37, step 1. MS ESI m/z = 682.3 (M+H). HPLC retention time 0.94 and 0.95 minutes, Method D.

### Intermediate 43/44D: 3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutane-1-carboxylic acid

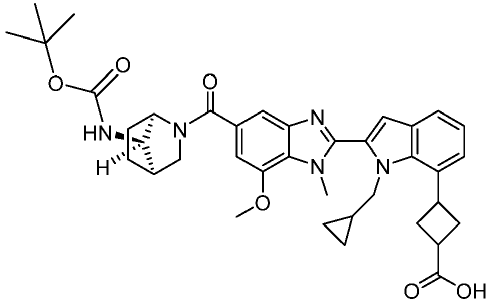

In a 2-dram vial, a solution of methyl 3-(2-(5-((1R,4R,7R)-7-((*tert-*butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutane-1-carboxylate (213 mg, 0.312 mmol) was treated with 1 M sodium hydroxide (0.937 mL, 0.937 mmol). The vial was sealed, and the reaction was stirred at 60 °C for 18 hours, at which point it was judged to be complete by LCMS. The mixture was diluted with water (3 mL), and the methanol was evaporated. The remaining, cloudy solution was washed 3X with ethyl acetate (the layers separated very slowly, so this took two days). LCMS of the combined washings detected some of the desired product. The washings were extracted 3X with 1M NaOH (2 mL) (a yellow, amorphous material settled to the bottom of the vial during the first extraction- this was removed and combined with the combined aqueous phases from the initial workup and these combined sodium hydroxide extractions. All aqueous phases were combined and the mixture was acidified to pH 3 with 1M HCl. The mixture was treated with pH 7 buffer (5 mL), and extracted 5X with ethyl acetate. The combined organic phases were dried over sodium sulfate and concentrated in vacuo to yield 3-(2-(5-((1R,4R,7R)-7-((*tert*-butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutane-1-carboxylic acid (103 mg, 0.154 mmol, 49.4 % yield) as an amber solid, which was used in the next step without further purification. MS ESI m/z = 668.3 (M+H). HPLC retention time 0.86 and 0.87 minutes, Method D.

### Examples 43 and 44: 3-(2-(5-((1R,4R,7R)-7-amino-2-azabicyclo[2.2.1]heptane-2-carbonyl)-7-methoxy-1-methyl-1H-benzo[dlimidazol-2-yl)-1-(cyclopropylmethyl)-1H-indol-7-yl)-N-methylcyclobutane-1-carboxamide, ISOMER 1, and 3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 2.

A stirring mixture of 3-(2-(5-((1R,4R,7R)-7-((*tert*-butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutane-1-carboxylic acid (30 mg, 0.045 mmol), methanamine hydrochloride (6.07 mg, 0.090 mmol), and triethylamine (0.025 mL, 0.180 mmol) in DMF (2 mL) was treated with BOP (23.84 mg, 0.054 mmol). The reaction was stirred at room temperature for 18 hours, at which point it was judged to be complete by LCMS. The mixture was concentrated in vacuo, and the residue was taken up in dichloromethane (2 mL). The mixture was treated with 4M HCl in dioxane (2 mL), and the reaction was stirred at room temperature for 1 hour, at which point it was judged to be complete by LCMS. The mixture was concentrated in vacuo, and the crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 19% B, 19-45% B over 35 minutes, then a 4-minute hold at 100% B; Flow Rate: 20 mL/min; Column Temperature: 25 C. Fraction collection was triggered by MS and UV signals. Two isomers were separated, and each was handled separately for the remainder of the process. Fractions were dried via centrifugal evaporation. The purified material was then diluted with DMF, treated with Si-Pyridine and shaken for a minimum of 2 h. The resulting mixture was filtered and dried via centrifugal evaporation to yield:

First Eluting: 3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 1 (8.2 mg, 0.014 mmol, 31.4 % yield). ¹H NMR (500 MHz, DMSO-d₆) δ 7.60 (br d, *J*=4.3 Hz, 1H), 7.37 (d, *J*=7.6 Hz, 1H), 7.29 - 7.13 (m, 1H), 7.07 (d, *J*=7.3 Hz, 1H), 7.02 - 6.94 (m, 1H), 6.86 (s, 1H), 6.79 - 6.71 (m, 1H), 4.29 (br d, *J*=6.4 Hz, 2H), 3.97 - 3.55 (m, 7H), 3.31 - 2.95 (m, 1H), 2.93 - 2.79 (m, 2H), 2.41 (d, *J*=4.3 Hz, 3H), 2.36 (s, 4H), 2.27 - 2.16 (m, 2H), 2.06 - 1.92 (m, 1H), 1.86 - 1.72 (m, 2H), 1.60 - 1.44 (m, 1H), 1.30 - 1.11 (m, 1H), 0.65 - 0.50 (m, 1H), 0.01 (br d, *J*=7.6 Hz, 2H), -0.48 - -0.63 (m, 2H). MS ESI m/z = 581.1 (M+H). HPLC retention time 1.31 minutes, Method 2.

Second Eluting: 3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 2 (5.9 mg, 9.84 µmol, 21.91 % yield). ¹H NMR (500 MHz, DMSO-d₆) δ 7.63 - 7.53 (m, 1H), 7.39 (d, *J*=7.6 Hz, 1H), 7.30 - 7.14 (m, 2H), 6.99 (t, *J*=7.6 Hz, 1H), 6.87 (s, 1H), 6.81 - 6.72 (m, 1H), 4.23 (br d, *J*=6.7 Hz, 2H), 4.16 - 4.06 (m, 1H), 3.96 - 3.87 (m, 3H), 3.83 - 3.69 (m, 3H), 3.62 - 3.31 (m, 1H), 3.04 - 2.82 (m, 2H), 2.51 - 2.41 (m, 4H), 2.36 (s, 3H), 2.29 - 2.17 (m, 2H), 2.08 - 1.93 (m, 1H), 1.87 - 1.73 (m, 2H), 1.65 - 1.51 (m, 1H), 1.33 - 1.14 (m, 1H), 0.64 - 0.47 (m, 1H), 0.01 (br d, *J*=7.6 Hz, 2H), -0.45 - -0.59 (m, 2H). MS ESI m/z = 581.0 (M+H). HPLC retention time 1.34 minutes, Method 2.

The following compounds in Table 3 can be made by the procedures described in Example 43, substituting the appropriate amine for methanamine hydrochloride in step 2. Examples 55 and 56 resulted from unreacted starting material from step 5 in the reaction which gave Examples 45 and 46 respectively carried through the subsequent deprotection step.

**Table 3**

| Ex # | Structure | Name | LC/ MS Rt (mi n) | LC/ MS Met hod | M+H (obs ion) |
|---|---|---|---|---|---|
| 45 | 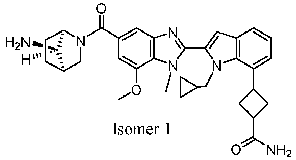 | 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobutane-1-carboxamide, ISOMER 1 | 1.2 8 | 1 | 567.4 |
| 46 | 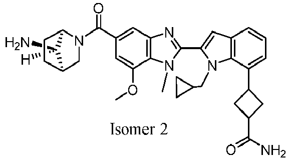 | 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobutane-1-carboxamide, ISOMER 2 | 1.3 9 | 1 | 567.2 |
| 47 | 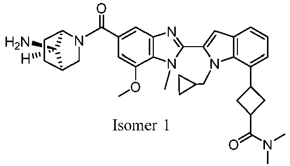 | 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-N,N-dimethylcyclobutane-1-carboxamide, ISOMER 1 | 1.4 0 | 2 | 595.0 |
| 48 | 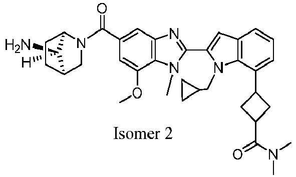 | 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-N,N-dimethylcyclobutane-1-carboxamide, ISOMER 2 | 2.5 5 | 1 | 595.4 |
| 49 | 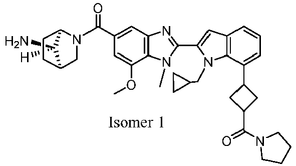 | (3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)(pyrrolidin-1- yl)methanone, ISOMER 1 | 1.4 0 | 2 | 621.3 |
| 50 | 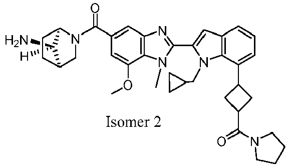 | (3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)(pyrrolidin-1 - yl)methanone, ISOMER 2 | 1.4 7 | 2 | 621.3 |
| 51 | 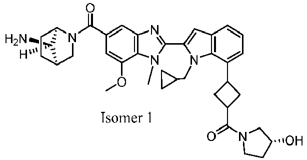 | (3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)((R)-3-hydroxypyrrolidin-1-yl)methanone, ISOMER 1 | 1.1 8 | 2 | 637.1 |
| 52 | 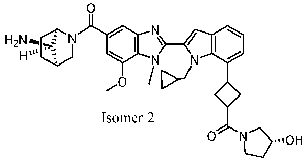 | (3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)((R)-3-hydroxypyrrolidin-1-yl)methanone, ISOMER 2 | 1.2 5 | 2 | 637.1 |
| 53 | 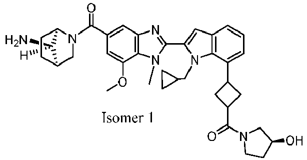 | (3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)((S)-3-hydroxypyrrolidin-1-yl)methanone, ISOMER 2 | 1.1 9 | 2 | 637.3 |
| 54 | 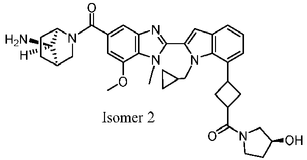 | (3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)((S)-3-hydroxypyrrolidin-1-yl)methanone, ISOMER 2 | 1.2 5 | 2 | 637.1 |
| 55 | 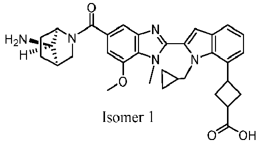 | 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobutane-1-carboxylic acid, ISOMER 1 | 1.18 | 1 | 568.3 |
| 56 | 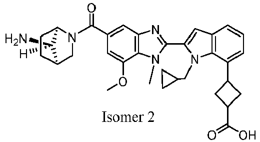 | 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobutane-1-carboxylic acid, ISOMER 2 | 1.2 5 | 1 | 568.3 |

### Examples 57. 58, and 59

### N-[3-(2-(5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobulyllacetamide (cis/trans mixture, ISOMER 1, ISOMER 2)

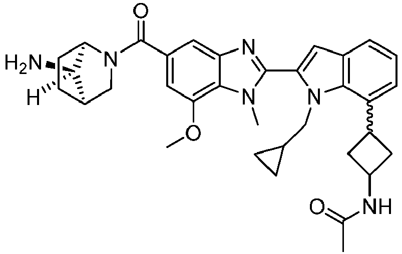

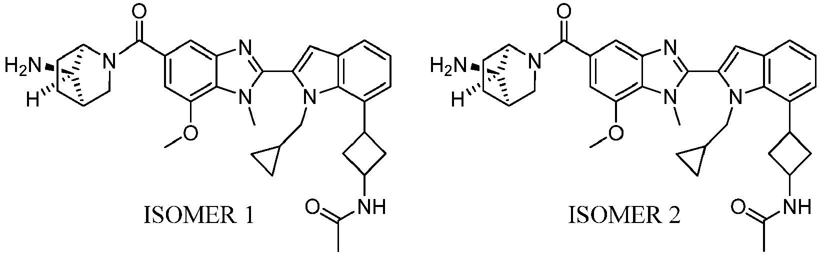

### Step 1: 3-acetamidocyclobutyl 4-methylbenzenesulfonate

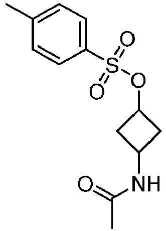

A stirring mixture of 3-aminocyclobutan-1-ol (170 mg, 1.951 mmol) and triethylamine (0.816 mL, 5.85 mmol) in dichloromethane (5 mL) was cooled to 0 °C and treated with acetic anhydride (0.184 mL, 1.951 mmol). The reaction was allowed to come to room temperature and stirred for 7 days. The mixture was concentrated in vacuo, and the residue was taken up in dichloromethane (5 mL). The turbid solution was treated with tosyl-Cl (409 mg, 2.145 mmol), and triethylamine (0.544 mL, 3.90 mmol), followed by DMAP (11.91 mg, 0.098 mmol). The reaction was stirred at room temperature for 18 hours. The reaction mixture was injected onto a 24 g silica gel column, and chromatographed via MPLC eluting at 40 mL/min with a 0% to 10% methanol/dichloromethane gradient over 14 column volumes. Fractions containing the desired product were pooled and concentrated in vacuo to yield 3-acetamidocyclobutyl 4-methylbenzenesulfonate (226 mg, 0.798 mmol, 40.9 % yield) as a colorless solid, which was used in the next step without further purification. ¹H NMR (499 MHz, chloroform-d) δ 7.83 - 7.76 (m, 2H), 7.39 - 7.34 (m, 2H), 5.71 (br d, J=4.9 Hz, 1H), 4.51 (quin, J=7.2 Hz, 1H), 4.07 - 3.97 (m, 1H), 2.81 - 2.69 (m, 2H), 2.47 (s, 3H), 2.12 - 2.02 (m, 2H), 1.95 (s, 3H). MS ESI m/z = 284.0 (M+H). HPLC retention time 0.74 minutes, Method D.

### Step 2: N-(3-iodocyclobutyl)acetamide

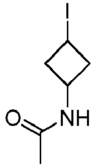

The title compound was prepared via the procedure used in Example 43, step 2, substituting 3-acetamidocyclobutyl 4-methylbenzenesulfonate for methyl 3-(tosyloxy)cyclobutane-1-carboxylate. ¹H NMR (500 MHz, CHLOROFORM-d) (NMR indicates a mixture of isomers) δ 6.00 - 5.77 (m, 0.6H), 5.76 - 5.59 (m, 0.4H), 4.87 - 4.76 (m, 0.3H), 4.56 - 4.32 (m, 1H), 4.11 (tt, *J*=9.2, 7.3 Hz, 0.7H), 3.27 - 3.07 (m, 1.3H), 2.94 - 2.75 (m, 0.7H), 2.71 - 2.58 (m, 0.7H), 2.56 - 2.40 (m, 1.3H), 1.99 - 1.97 (m, 3H).

### Example 57: N-[3-(2-{5-[(1R,4R,7R)-7-amino-2-azabicyclo[2.2.1]heptane-2-carbonyl]-7-methoxy-1-methyl-1H-1,3-benzodiazol-2-yl}1-1-(cyclopropylmethyl)-1H-indol-7-vDcyclobutyllacetamide, cis/trans mixture

The title compound was prepared from N-(3-iodocyclobutyl)acetamide and ((1R,4R,7R)-7-amino-2-azabicyclo[2.2.1]heptan-2-yl)(2-(7-bromo-1-(cyclopropylmethyl)-1H-indol-2-yl)-7-methoxy-1-methyl-1H-benzo[d]imidazol-5-yl)methanone via the procedure described in Example 2. ¹H NMR (500 MHz, DMSO-d₆) (Proton count is low due to the water suppression algorithm used during data processing) δ 8.11 - 7.86 (m, 1H), 7.35 (t, *J*=8.0 Hz, 1H), 7.18 - 7.12 (m, 1H), 7.08 - 6.92 (m, 1H), 6.81 (d, *J*=5.0 Hz, 1H), 6.71 (br s, 1H), 4.26 (br d, *J*=6.3 Hz, 1H), 4.18 (br d, *J*=6.1 Hz, 1H), 4.15 - 4.05 (m, 1H), 3.96 (br dd, *J*=8.8, 4.1 Hz, 1H), 3.86 (s, 3H), 3.78 (s, 2H), 3.63 - 3.51 (m, 1H), 3.44 - 3.27 (m, 2H), 2.77 (br d, *J*=6.3 Hz, 1H), 2.54 (br d, *J*=7.9 Hz, 1H), 2.36 (br d, *J*=6.1 Hz, 1H), 2.05 - 1.90 (m, 2H), 1.85 - 1.71 (m, 2H), 1.65 - 1.48 (m, 4H), 1.30 - 1.10 (m, 1H), 0.62 - 0.44 (m, 1H), 0.06 - -0.09 (m, 2H), -0.46 - -0.62 (m, 2H). MS ESI m/z = 581.2 (M+H). HPLC retention time 1.42 minutes, Method 2.

### Examples 58 and 59: The two isomers of Example 57 were resolved using the following conditions:

| | |
|---|---|
| **Preparative Chromatographic Conditions:** | Waters 100 Prep SFC |
| Instrument: | |
| Column: | Chiral OD, 30 x 250 mm. 5 micron |
| Mobile Phase: | 75% CO2/ 25% MeOH w/0.1%DEA |
| Flow Conditions: | 100 mL/min |
| Detector Wavelength: | 220 nm |
| Injection Details: | 1300 µL 13.5 mg dissolved in 2 mL MeOH |

Isomer 1 (first-eluting): ¹H NMR (500 MHz, DMSO-d₆) δ 8.00 (br d, *J*=7.6 Hz, 1H), 7.37 (br d, *J*=7.6 Hz, 1H), 7.30 - 7.15 (m, 1H), 7.06 (br d, *J*=7.3 Hz, 1H), 6.98 (br t, *J*=7.3 Hz, 1H), 6.86 (s, 1H), 6.80 - 6.72 (m, 1H), 4.31 (br d, *J*=6.1 Hz, 2H), 4.23 - 4.10 (m, 1H), 3.90 (br s, 3H), 3.81 (s, 3H), 3.67 - 3.57 (m, 1H), 3.53 - 3.15 (m, 1H), 3.04 - 2.85 (m, 1H), 2.81 (s, 1H), 2.55 (br d, *J*=6.7 Hz, 2H), 2.12 - 1.92 (m, 3H), 1.87 - 1.74 (m, 2H), 1.62 (s, 3H), 1.28 (br s, 1H), 1.05 (br s, 3H), 0.72 - 0.51 (m, 2H), 0.01 (br d, *J*=7.6 Hz, 2H), -0.42 - -0.70 (m, 2H). MS ESI m/z = 581.2 (M+H). HPLC retention time 1.41 minutes, Method 2.

Isomer 2 (second-eluting): ¹H NMR (500 MHz, DMSO-d₆) δ 8.15 (br d, J=7.0 Hz, 1H), 7.40 (br d, *J*=7.6 Hz, 1H), 7.30 - 7.15 (m, 2H), 6.99 (br t, *J*=7.5 Hz, 1H), 6.88 (s, 1H), 6.81 - 6.72 (m, 1H), 4.22 (br d, *J*=6.1 Hz, 2H), 4.17 - 4.09 (m, 1H), 4.07 - 3.95 (m, 1H), 3.91 (br s, 3H), 3.81 (s, 3H), 3.69 - 3.48 (m, 1H), 3.42 - 3.31 (m, 1H), 2.90 (br d, *J*=10.7 Hz, 1H), 2.82 (s, 1H), 2.37 - 2.24 (m, 1H), 2.14 - 2.04 (m, 1H), 1.89 - 1.74 (m, 2H), 1.68 - 1.54 (m, 4H), 1.29 (br d, *J*=7.6 Hz, 1H), 1.06 (br s, 4H), 0.73 - 0.52 (m, 2H), 0.01 (br d, *J*=7.6 Hz, 2H), -0.56 (br d, *J*=4.0 Hz, 2H). MS ESI m/z = 581.2 (M+H). HPLC retention time 1.41 minutes, Method 2.

### Example 61

### (1R,4R,7R)-2-[2-(7-{2-azaspiro[3.3]heptan-6-yl}-1-(cyclopropylmethyl)-1H-indol-2-yl)-7-methoxy-1-methyl-1H-1,3-benzodiazole-5-carbonyl]-2-azabicyclo[2.2.1]heptan-7-amine

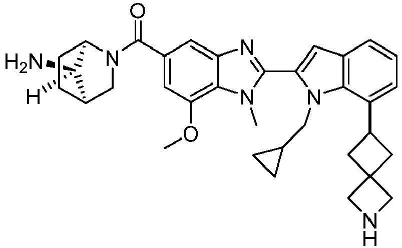

### Intermediate 61A: tert-butyl 6-iodo-2-azaspiro[3.3]heptane-2-carboxylate

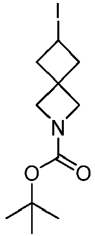

The title compound was prepared from *tert*-butyl 6-hydroxy-2-azaspiro[3.3]heptane-2-carboxylate using the procedures described in Example 43, steps 1 and 2. ¹H NMR (499 MHz, chloroform-d) δ 4.31 (quin, *J*=7.8 Hz, 1H), 3.96 (d, *J*=15.1 Hz, 4H), 2.99 - 2.89 (m, 2H), 2.77 - 2.68 (m, 2H), 1.45 (s, 9H).

### Intermediate 61B: tert-butyl 6-(2-(5-((1R,4R,7R)-7-((tert-butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-azaspiro[3.3]heptane-2-carboxylate

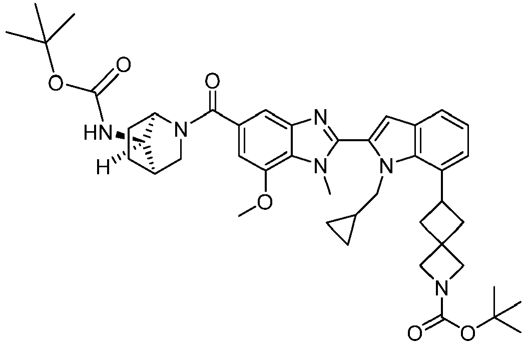

The title compound was prepared from *tert*-butyl 6-iodo-2-azaspiro[3.3]heptane-2-carboxylate and *tert*-butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate using the procedure described in Example 37, step 1.

### Example 61: (1R,4R,7R)-2-[2-(7-{2-azaspiro[3.3]heptan-6-yl}-1-(cyclopropylmethyl)-1H-indol-2-yl)-7-methoxy-1-methyl-1H-1,3-benzodiazole-5-carbonyl]-2-azabicyclo[2.2.1]heptan-7-amine

*tert*-butyl 6-(2-(5-((1R,4R,7R)-7-((*tert*-butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-azaspiro[3.3]heptane-2-carboxylate (27 mg, 0.035 mmol) was dissolved in 10% TFA/dichloromethane (1mL), and the reaction was stirred at room temperature for 3 hours, at which point it was judged to be complete by LCMS. The mixture was concentrated in vacuo, and the residue was concentrated 3X from dichloromethane to remove residual TFA. The crude material was purified via preparative LC/MS with the following conditions: Column: XBridge C18, 200 mm x 19 mm, 5-µm particles; Mobile Phase A: 5:95 acetonitrile: water with 0.1% trifluoroacetic acid; Mobile Phase B: 95:5 acetonitrile: water with 0.1% trifluoroacetic acid; Gradient: a 0-minute hold at 7% B, 7-47% B over 20 minutes, then a 4-minute hold at 100% B; Flow Rate: 20 mL/min; Column Temperature: 25 C. Fraction collection was triggered by MS signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound, (19.3 mg, 0.034 mmol, 97 % yield). ¹H NMR (500 MHz, DMSO-d₆) δ 7.35 (d, *J*=7.7 Hz, 1H), 7.31 - 7.15 (m, 1H), 7.04 (d, *J*=7.3 Hz, 1H), 6.98 - 6.91 (m, 1H), 6.83 (s, 1H), 6.76 (br s, 1H), 4.23 (br d, *J*=6.4 Hz, 2H), 3.99 (s, 2H), 3.88 (s, 3H), 3.87 - 3.82 (m, 1H), 3.80 (s, 3H), 3.75 (s, 2H), 3.54 - 3.23 (m, 2H), 2.59 (br t, *J*=10.4 Hz, 2H), 2.53 - 2.38 (m, 1H), 1.74 (br s, 3H), 1.45 (br s, 1H), 1.15 - 0.68 (m, 1H), 0.61 - 0.46 (m, 1H), -0.01 (br d, *J*=7.9 Hz, 2H), -0.52 (br s, 2H). (Proton count is low due to the water suppression algorithm used during data processing). MS ESI m/z = 565.4 (M+H). HPLC retention time 1.02 minutes, Method 2.

### Example 62

### 1-[6-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-2-azaspiro[3.3]heptan-2-yl]ethan-1-one

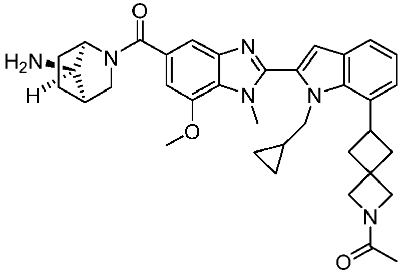

*tert*-Butyl 6-(2-(5-((1R,4R,7R)-7-((*tert*-butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-azaspiro[3.3]heptane-2-carboxylate (54 mg, 0.071 mmol) was dissolved in 10% TFA/dichloromethane (1mL), and the reaction was stirred at room temperature for 3 hours, at which point it was judged to be complete by LCMS. The mixture was diluted with dichloromethane (20 mL) and concentrated in vacuo, and the residue was taken up in dichloromethane (3 mL). The mixture was treated with 1.5 M potassium phosphate (dibasic) (3 mL), and vigorously shaken for 10 minutes. The layers were separated, and the aqueous phase was extracted 3X with dichloromethane (2 mL). The combined organic phases were dried over sodium sulfate and concentrated in vacuo, and the residue was taken up in dichloromethane (2 mL). Triethylamine (0.013 mL, 0.096 mmol) was added, and the solution was cooled to 0 °C and treated with acetic anhydride (3.38 µl, 0.036 mmol). The reaction was allowed to slowly come to room temperature and stirred for 1 hour, at which point it was judged to be complete by LCMS. The mixture was diluted with methanol to quench any residual acetic anhydride, then concentrated in vacuo. The crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 18% B, 18-58% B over 20 minutes, then a 4-minute hold at 100% B; Flow Rate: 20 mL/min; Column Temperature: 25 C. Fraction collection was triggered by MS and UV signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound, (22.9 mg, 0.038 mmol, 79 % yield). ¹H NMR (500 MHz, DMSO-d₆) δ 7.38 (br d, *J*=7.9 Hz, 1H), 7.28 - 7.14 (m, 1H), 7.07 (br d, *J*=7.3 Hz, 1H), 6.96 (br t, *J*=7.5 Hz, 1H), 6.87 (br s, 1H), 6.79 - 6.71 (m, 1H), 4.27 (br d, *J*=4.9 Hz, 2H), 4.14 (s, 1H), 3.95 - 3.77 (m, 9H), 3.69 - 3.52 (m, 2H), 3.52 - 3.30 (m, 1H), 3.02 - 2.80 (m, 1H), 2.53 (br d, *J*=8.9 Hz, 2H), 2.05 - 1.91 (m, 1H), 1.87 - 1.51 (m, 6H), 1.30 - 1.12 (m, 1H), 0.56 (br s, 1H), 0.01 (br d, *J*=7.9 Hz, 2H), -0.54 (br d, *J*=3.7 Hz, 2H). (Proton count is low due to the water suppression algorithm used during data processing). MS ESI m/z = 607.0 (M+H). HPLC retention time 1.69 minutes, Method 1.

### Example 63

### 1-{3-[(2-{5-[(1R,4R,7R)-7-amino-2-azabicyclo[2.2,1]heptane-2-carbonyl]-7-methoxy-1-methyl-1H-1,3-benzodiazol-2-yl}-1-(cyclopropylmethyl)-1H-indol-7-yl)methyl]azetidin-1-yl}ethan-1-one

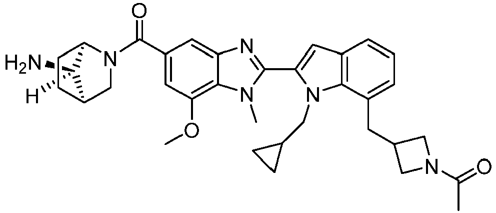

A stirring solution of ((1R,4R,7R)-7-amino-2-azabicyclo[2.2.1]heptan-2-yl)(2-(7-(azetidin-3-ylmethyl)-1-(cyclopropylmethyl)-1H-indol-2-yl)-7-methoxy-1-methyl-1H-benzo[d]imidazol-5-yl)methanone (Example 26) (68 mg, 0.126 mmol) and triethylamine (0.070 mL, 0.505 mmol) in dichloromethane (2 mL) was cooled to 0 °C and treated with acetic anhydride (9.53 µl, 0.101 mmol). The reaction was allowed to slowly come to room temperature and stirred for 1 hour, at which point it was judged to be complete by LCMS. The mixture was diluted with methanol to quench any residual acetic anhydride, then concentrated in vacuo. The crude material was purified via preparative LC/MS with the following conditions: Column: XBridge C18, 200 mm x 19 mm, 5-µm particles; Mobile Phase A: 5:95 acetonitrile: water with 0.1% trifluoroacetic acid; Mobile Phase B: 95:5 acetonitrile: water with 0.1% trifluoroacetic acid; Gradient: a 0-minute hold at 12% B, 12-52% B over 20 minutes, then a 4-minute hold at 100% B; Flow Rate: 20 mL/min; Column Temperature: 25 C. Fraction collection was triggered by MS and UV signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound, (43 mg, 0.072 mmol, 57.3 % yield). ¹H NMR (500 MHz, DMSO-d₆) δ 7.34 (br d, *J*=7.8 Hz, 1H), 7.26 - 7.11 (m, 1H), 6.85 (br d, *J*=7.6 Hz, 1H), 6.81 (br d, *J*=7.0 Hz, 1H), 6.78 (s, 1H), 6.73 (br s, 1H), 4.23 (br d, *J*=6.3 Hz, 2H), 4.00 (br t, *J*=7.6 Hz, 2H), 3.84 (s, 3H), 3.81 - 3.61 (m, 5H), 3.40 (br dd, *J*=9.0, 5.8 Hz, 1H), 3.27 - 3.07 (m, 2H), 2.99 - 2.91 (m, 1H), 2.89 - 2.76 (m, 1H), 2.52 - 2.38 (m, 1H), 1.70 (br s, 3H), 1.51 (s, 3H), 1.46 - 1.37 (m, 1H), 0.63 (br d, *J*=5.0 Hz, 1H), -0.01 (br d, *J*=7.9 Hz, 2H), -0.53 (br s, 2H). (Proton count is low due to the water suppression algorithm used during data processing). MS ESI m/z = 581.0 (M+H). HPLC retention time 1.29 minutes, Method 2.

### Example 64

### (1R,4R,7R)-2-{2-[1-(cyclopropylmethyl)-7-(1-methanesulfonylazetidin-3-yl)-1H-indol-2-yl]-7-methoxy-1-methyl-1H-1,3-benzodiazole-5-carbonyl}-2-azabicyclo[2.2.1]heptan-7-amine

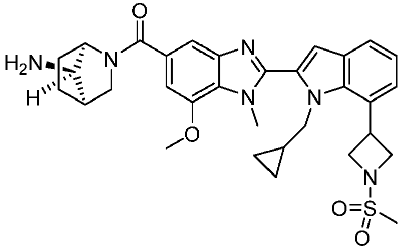

Intermediate 64A: benzyl 3-(2-(5-((1R,4R,7R)-7-((*tert-*butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)azetidine-1-carboxylate

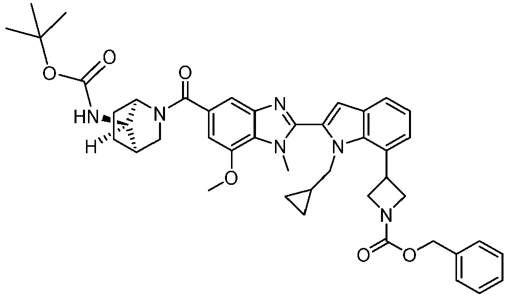

In a 40 mL scintillation vial, a stirring mixture of benzyl 3-iodoazetidine-1-carboxylate (0.782 g, 2.467 mmol), *tert*-butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (0.8 g, 1.233 mmol), tris(trimethylsilyl)silane (0.571 mL, 1.850 mmol), Ir(dF(CF3)ppy)2(dtbbpy)PF6 (0.042 g, 0.037 mmol), and sodium carbonate (0.523 g, 4.93 mmol) in 1,4-Dioxane (15 mL) was degassed with bubbling nitrogen for 10 minutes. In a separate 2-dram vial, a stirring mixture of nickel(II) chloride ethylene glycol dimethyl ether complex (0.041 g, 0.185 mmol), and 4,4'-di-*tert*-butyl-2,2'-bipyridine (0.056 g, 0.210 mmol) in 1,4-dioxane (5 mL) was degassed with nitrogen for 20 minutes. The nickel complex was transferred to the containing the other mixture, the vial was sealed, and the reaction was stirred at room temperature under a blue Kessil lamp for 18 hours, at which point it was judged to be complete by LCMS. The mixture was diluted with ethyl acetate (15 mL) and solids were removed by filtration and rinsed thoroughly with ethyl acetate. The combined filtrate and rinsings were concentrated in vacuo, and the residue was concentrated 3X from methanol to completely remove the other solvents. The crude material was used in the next step without further purification. MS ESI m/z = 759.5 (M+H). HPLC retention time 0.98 minutes, Method D.

### Intermediate 64B: tert-butyl ((1R,4R,7R)-2-(2-(7-(azetidin-3-yl)-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate

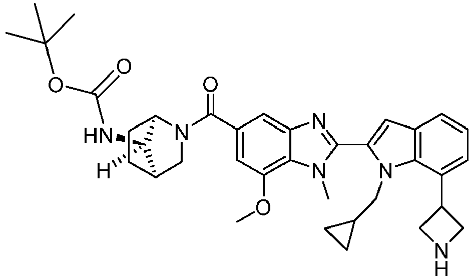

A stirring mixture of benzyl 3-(2-(5-((1R,4R,7R)-7-((*tert-*butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)azetidine-1-carboxylate (0.936 g, 1.233 mmol) and 10% Pd-C, Degussa type (1g, 0.940 mmol) in methanol (15 mL) was degassed 3X with nitrogen/vacuum. The reaction was hydrogenated at atmospheric pressure for 48 hours, at which point it was judged to be complete by LCMS. The catalyst was removed by filtration through 3 layers of Whatman GF/A filter paper and rinsed thoroughly with methanol and ethyl acetate, and the combined filtrate and rinsings were concentrated in vacuo. The residue was chromatographed via MPLC over a 40 g silica gel column, eluting at 40 mL/min with a 0% to 10% (7 M ammonia in methanol)/dichloromethane gradient over 15 column volumes. Fractions containing the desired product were pooled and concentrated in vacuo to yield the title compound (407 mg, 0.651 mmol, 52.8 % yield) as a yellow solid. ¹H NMR (499 MHz, chloroform-d) δ 7.62 (d, *J*=7.7 Hz, 1H), 7.55 - 7.47 (m, 1H), 7.35 (d, *J*=7.4 Hz, 1H), 7.23 (t, *J*=7.6 Hz, 1H), 7.06 - 6.97 (m, 1H), 6.91 - 6.84 (m, 1H), 4.77 (quin, J=8.0 Hz, 1H), 4.60 - 4.42 (m, 3H), 4.35 (br s, 1H), 4.19 - 4.05 (m, 5H), 4.05 - 3.97 (m, 5H), 3.90 - 3.72 (m, 2H), 3.32 - 3.19 (m, 1H), 2.54 (br s, 1H), 2.12 - 1.85 (m, 3H), 1.75 - 1.67 (m, 1H), 1.60 - 1.31 (m, 10H), 0.89 - 0.78 (m, 1H), 0.32 - 0.18 (m, 2H), -0.17 - -0.41 (m, 2H). MS ESI m/z = 625.3 (M+H). HPLC retention time 0.75 minutes, Method D.

### Example 64: (1R,4R,7R)-2-(2-[1-(cyclopropylmethyl)-7-(1-methanesulfonylazetidin-3-yl)-1H-indol-2-yl]-7-methoxy-1-methyl-1H-1,3-benzodiazole-5-carbonyl}-2-azabicyclo[2.2.1]heptan-7-amine

In a 2-dram vial, a stirring solution of *tert*-butyl ((1R,4R,7R)-2-(2-(7-(azetidin-3-yl)-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (15 mg, 0.024 mmol) and triethylamine (5.02 µl, 0.036 mmol) in dichloromethane (1 mL) was treated with methanesulfonyl chloride (1.777 µl, 0.023 mmol). The vial was sealed, and the reaction was stirred at room temperature for 2 hours, at which point it was judged to be complete by LCMS. The mixture was treated with TFA (100 µl, 1.298 mmol), and the reaction was stirred at room temperature for 2 hours, at which point it was judged to be complete by LCMS. The mixture was diluted with dichloromethane (20 mL), and concentrated in vacuo, and the residue was concentrated twice from DCM to remove residual TFA. The crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 17% B, 17-57% B over 20 minutes, then a 4-minute hold at 100% B; Flow Rate: 20 mL/min; Column Temperature: 25 C. Fraction collection was triggered by MS and UV signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound (7.2 mg, 0.011 mmol, 47.3 % yield). ¹H NMR (500 MHz, DMSO-d₆) δ 7.45 (br d, *J*=7.6 Hz, 1H), 7.28 (br d, *J*=7.3 Hz, 1H), 7.25 - 7.12 (m, 1H), 7.03 (br t, *J*=7.5 Hz, 1H), 6.87 (br s, 1H), 6.77 - 6.68 (m, 1H), 4.48 - 4.37 (m, 1H), 4.21 - 4.11 (m, 4H), 3.94 (br s, 2H), 3.87 (br s, 3H), 3.78 (br s, 3H), 3.61 - 3.40 (m, 2H), 3.00 - 2.83 (m, 5H), 2.72 - 2.48 (m, 1H), 2.10 - 1.94 (m, 1H), 1.87 - 1.67 (m, 2H), 1.61 - 1.47 (m, 1H), 1.33 - 1.16 (m, 1H), 1.02 (br s, 1H), 0.57 (br s, 1H), 0.01 (br d, *J*=7.6 Hz, 2H), -0.56 (br d, *J*=4.0 Hz, 2H). MS ESI m/z = 603.1 (M+H). HPLC retention time 1.44 minutes, Method 2.

### Example 65

### 2-amino-1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]ethan-1-one

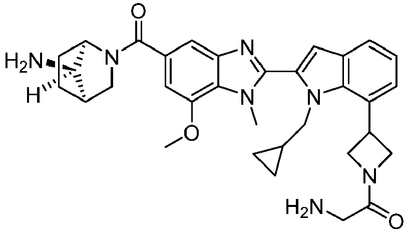

In a 2-dram vial, a stirring solution of *tert*-butyl ((1R,4R,7R)-2-(2-(7-(azetidin-3-yl)-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (15 mg, 0.024 mmol), (*tert*-butoxycarbonyl)glycine (4.63 mg, 0.026 mmol), and triethylamine (8.37 µl, 0.060 mmol) in dichloromethane (1 mL) was treated with BOP (13 mg, 0.030 mmol). The vial was sealed, and the reaction was stirred at room temperature for 2 hours, at which point it was judged to be complete by LCMS. The mixture was treated with TFA (100 µl, 1.298 mmol), and the reaction was stirred at room temperature for 2 hours, at which point it was judged to be complete by LCMS. The mixture was diluted with dichloromethane (20 mL), and concentrated in vacuo, and the residue was concentrated twice from DCM to remove residual TFA. The crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 7% B, 7-47% B over 20 minutes, then a 4-minute hold at 100% B; Flow Rate: 20 mL/min; Column Temperature: 25 C. Fraction collection was triggered by MS signals. Fractions containing the desired product were combined and dried via centrifugal evaporation. The material was further purified via preparative LC/MS with the following conditions: Column: XBridge C18, 150 mm x 30 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 8% B, 8-48% B over 20 minutes, then a 2-minute hold at 100% B; Flow Rate: 40 mL/min; Column Temperature: 25 C. Fraction collection was triggered by MS and UV signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound (3.8 mg, 5.25 µmol, 21.85 % yield). ¹H NMR (500 MHz, DMSO-d₆) δ 7.40 (br d, *J*=7.8 Hz, 1H), 7.28 - 7.18 (m, 1H), 7.12 (br s, 1H), 6.98 (br t, *J*=7.6 Hz, 1H), 6.81 (s, 1H), 6.70 (br s, 1H), 4.45 (br s, 2H), 4.30 - 4.07 (m, 3H), 3.95 - 3.81 (m, 3H), 3.78 - 3.48 (m, 3H), 3.36 - 2.91 (m, 1H), 2.09 - 1.87 (m, 1H), 1.83 - 1.58 (m, 5H), 1.56 - 1.39 (m, 1H), 1.30 - 1.15 (m, 1H), 1.10 - 0.95 (m, 2H), 0.89 - 0.69 (m, 1H), 0.56 (br s, 1H), -0.01 (br d, *J*=7.6 Hz, 2H), -0.12 - -0.35 (m, 1H), -0.51 (br s, 2H). (Proton count is low due to the water suppression algorithm used during data processing). MS ESI m/z = 582.6 (M+H). HPLC retention time 1.10 minutes, Method 1.

The following compounds in Table 4 can be made using the procedures described in Examples 64 and 65, substituting the appropriate acid chloride, isocyanate, anhydride, or sulfonyl chloride for methanesulfonyl chloride in Example 64 step 2, or the appropriate carboxylic acid for (tert-butoxycarbonyl)glycine in Example 65.

**Table 4**

| Ex # | Structure | Name | LC/ MS Rt (min ) | LC/ MS Met hod | M+H (obs ion) |
|---|---|---|---|---|---|
| 66 | 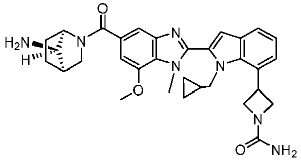 | 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidine-1-carboxamide | 1.27 | 1 | 568.4 |
| 67 | 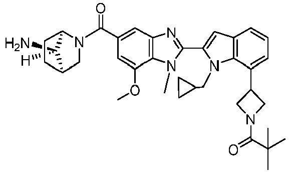 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2,2-dimethylpropan-1-one | 1.86 | 1 | 609.3 |
| 68 | 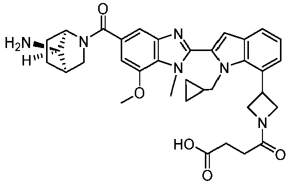 | 4-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-4-oxobutanoic acid | 1.29 | 2 | 625.2 |
| 69 | 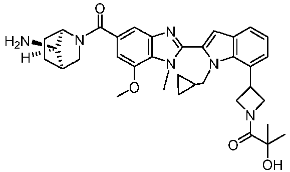 | 1-[3-(2-{5-[(1R,4R,7R)-7-amino-2-azabicyclo[2.2.1]heptane-2-carbonyl]-7-methoxy-1-methyl-1H-1,3-bezodiazol-2yl}-1-(cyclopropylmethyl)-1H-indol-7-yl)azetidin-1-yl]-2-hydroxy-2-methylpropan-1-one | 1.34 | 1 | 611.5 |
| 70 | 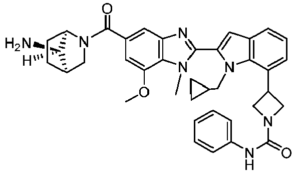 | 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-N-phenylazetidine-1-carboxamide | 1.48 | 2 | 644.1 |
| 71 | 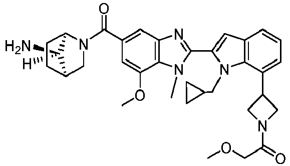 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-methoxyethan-1-one | 1.49 | 1 | 597.4 |
| 72 | 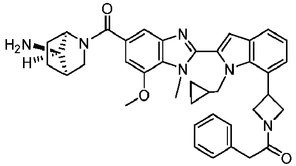 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-phenylethan-1-one | 1.56 | 2 | 643.2 |
| 73 | 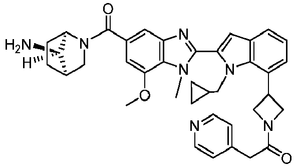 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(pyridin-4-yl)ethan-1-one | 1.43 | 1 | 644.6 |
| 74 | 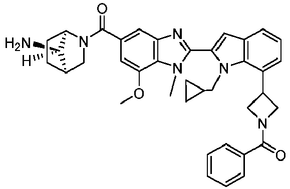 | (lR,4R, 7R)-2-{ 2-[7 -(1-benzoylazetidin-3-yl)-1-(cyclopropylmethyl)-1H-indol-2-yl]-7-methoxy-1-methyl-1H-1,3-benzodiazole-5-carbonyl}-2-azabicyclo[2.2.1]heptan-7-amine | 1.38 | 2 | 629.3 |
| 75 | 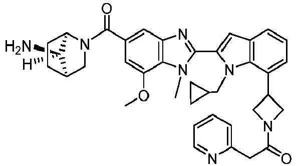 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(pyridin-2-yl)ethan-1-one | 1.23 | 2 | 644.4 |
| 76 | 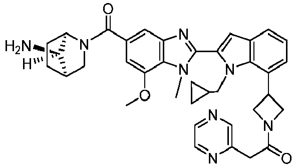 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(pyrazin-2-yl)ethan-1-one | 1.46 | 1 | 645.1 |
| 77 | 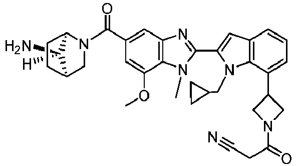 | 3-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-3-oxopropanenitrile | 1.52 | 1 | 592.0 |
| 78 | 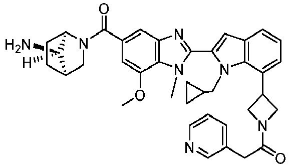 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(pyridin-3-yl)ethan-1-one | 1.18 | 2 | 643.9 |
| 79 | 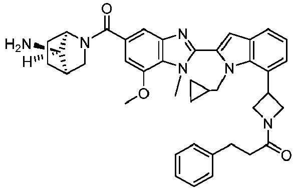 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-3-phenylpropan-1-one | 1.45 | 2 | 657.2 |
| 80 | 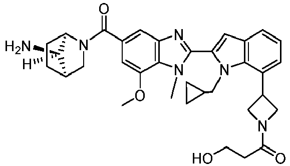 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-3-hydroxypropan-1-one | 1.25 | 2 | 597.1 |
| 81 | 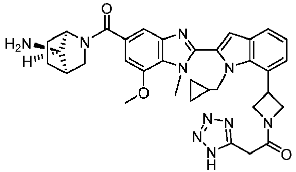 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(1H-1,2,3,4-tetrazol-5-yl)ethan-1-one | 1.26 | 2 | 635.3 |
| 82 | 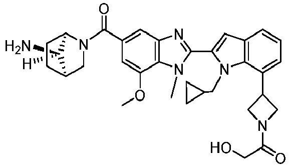 | 1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-hydroxyethan-1-one | 1.24 | 2 | 583.0 |
| 83 | 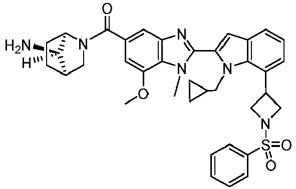 | (1R,4R,7R)-2-(2-{7-[1-(benzenesulfonyl)azetidin-3-yl]-1-(cyclopropylmethyl)-1H-indol-2-yl}-7-methoxy-1-methyl-1H-1,3-benzodiazole-5-carbonyl)-2-azabicyclo[2.2.1]heptan-7-amine | 1.79 | 1 | 665.0 |

### Example 84

### (1R,4R,7R)-2-{2-[1-(cyclopropylmethyl)-7-(1,2,3,6-tetrahydropyridin-4-yl)-1H-indol-2-yl]-7-methoxy-1-methyl-1H-1,3-benzodiazole-5-carbonyl}-2-azabicyclo[2.2.1]heptan-7-amine

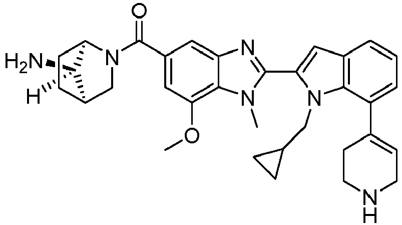

In a 2-dram vial, a stirring mixture of tert-butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (50 mg, 0.077 mmol), tert-butyl 4-(4,4, 5, 5-tetramethyl-1,3,2-dioxaborolan-2-yl)-3,6-dihydropyridine-1 (2H)-carboxylate (28.6 mg, 0.093 mmol), and 2 M potassium phosphate, tribasic (0.116 mL, 0.231 mmol) in dioxane (2 mL) was degassed with bubbling nitrogen for 5 minutes. The mixture was treated with 2nd generation XPhos precatalyst (2.96 mg, 3.85 µmol) and degassed for another five minutes, then the vial was sealed. The reaction was heated at 50 °C for 4 hours, at which point it was judged to be complete by LCMS. Most of the dioxane was evaporated, and the residue was taken up in ethyl acetate (5 mL). The turbid solution was washed twice with water and once with brine, then dried over sodium sulfate and concentrated in vacuo. The residue was chromatographed via MPLC over a 12 g silica gel column, eluting at 30 mL/min with a 0% to 6% methanol/dichloromethane gradient over 40 column volumes. Fractions containing the desired product were pooled and concentrated in vacuo to yield *tert*-butyl 4-(2-(5-((1R,4R,7R)-7-((*tert-*butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)-3,6-dihydropyridine-1(2H)-carboxylate (46 mg, 0.061 mmol, 79 % yield) as a colorless solid. MS ESI m/z = 751.5 (M+H). HPLC retention time 1.03 minutes, Method D. The material was taken up in dichloromethane (2 mL) and treated with 4M HCl in dioxane (2 ml, 8.00 mmol). The reaction was stirred at room temperature for 2 hours, at which point it was judged to be complete by LCMS. The mixture was concentrated in vacuo, and the crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 10% B, 10-50% B over 20 minutes, then a 4-minute hold at 100% B; Flow Rate: 20 mL/min; Column Temperature: 25 C. Fraction collection was triggered by MS signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound,

(3.7 mg, 5.85 µmol, 24.39 % yield). ¹H NMR (500 MHz, DMSO-d₆) δ 7.59 - 7.46 (m, 1H), 7.36 - 7.20 (m, 1H), 7.10 - 6.92 (m, 3H), 6.90 - 6.74 (m, 2H), 5.80 - 5.15 (m, 1H), 3.99 (br d, *J*=6.7 Hz, 2H), 3.87 (br d, *J*=3.4 Hz, 2H), 3.29 - 2.83 (m, 4H), 2.80 - 2.55 (m, 3H), 2.22 - 2.05 (m, 1H), 1.99 - 1.80 (m, 2H), 1.71 - 1.29 (m, 2H), 1.24 - 1.08 (m, 3H), 1.03 - 0.71 (m, 2H), 0.49 (br s, 1H), 0.11 - -0.22 (m, 4H), -0.32 - -0.60 (m, 1H). (Proton count is low due to the water suppression algorithm used during data processing). MS ESI m/z = 551.4 (M+H). HPLC retention time 1.23 minutes, Method 1.

### Examples 85 (Isomer 1) and 86 (Isomer2)

### 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclohexane-1-carboxamide, ISOMERS 1 and 2

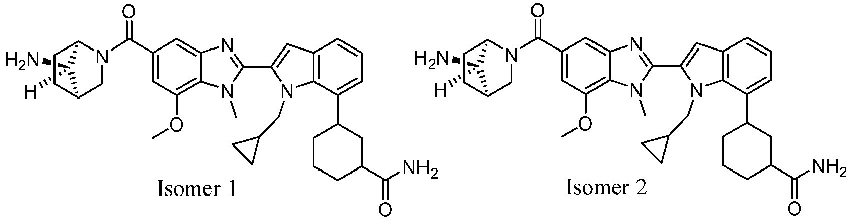

A stirring solution of *tert*-butyl ((1R,4R,7R)-2-(2-(7-(3-cyanocyclohexyl)-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (33 mg, 0.049 mmol) (prepared from 3-iodocyclohexane-1-carbonitrile and *tert*-butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate using the conditions described in Example 37, step 1) in 1:1 DMSO/water (1 mL) was treated with potassium carbonate (13.48 mg, 0.098 mmol) and hydrogen peroxide (4.98 µl, 0.049 mmol). The reaction was stirred at room temperature for 18 hours. LCMS indicated that the reaction had not gone to completion. The mixture was treated with potassium carbonate (13.48 mg, 0.098 mmol) and hydrogen peroxide (4.98 µl, 0.049 mmol), and the reaction was stirred at 50 °C for 18 hours. The above additions/heating at 50 °C was repeated each day for five days, until the reaction was judged to be essentially complete by LCMS. The mixture was diluted with ethyl acetate (15 mL), and the turbid solution was washed 3X with water and once with brine. The organic phase was dried over sodium sulfate and concentrated in vacuo. The residue was taken up in dichloromethane (2 mL), and the solution was treated with 4 M HCl (2 mL). The reaction was stirred for 1 hour, at which point it was judged to be complete by LCMS. The mixture was concentrated in vacuo, and the crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 30% B, 30-60% B over 30 minutes, then a 4-minute hold at 100% B; Flow Rate: 20 mL/min; Column Temperature: 25 C. Fraction collection was triggered by MS signals. The *cis-* and *trans*-isomers were resolved. The two isomers were handled separately for the remainder of the process. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation.

Example 85, ISOMER 1 (First eluting), (2.0 mg, 3.34 µmol, 6.85 % yield). ¹H NMR (500 MHz, DMSO-d₆) δ 7.29 (d, *J*=7.7 Hz, 1H), 7.14 (br s, 1H), 7.01 (d, *J*=7.4 Hz, 1H), 6.90 - 6.86 (m, 1H), 6.76 - 6.67 (m, 2H), 4.61 - 4.49 (m, 1H), 4.20 - 4.11 (m, 1H), 3.89 - 3.80 (m, 3H), 3.77 (s, 3H), 3.70 - 3.55 (m, 2H), 3.39 - 3.06 (m, 4H), 2.95 - 2.76 (m, 1H), 2.47 (br s, 1H), 2.03 (br d, *J*=10.4 Hz, 2H), 1.88 - 1.69 (m, 5H), 1.60 - 1.20 (m, 8H), 0.70 - 0.60 (m, 2H), 0.04 - -0.06 (m, 2H), -0.36 - -0.55 (m, 2H). MS ESI m/z = 595.0 (M+H). HPLC retention time 1.75 minutes, Method 1.

Example 86, ISOMER 2 (Second eluting), (4.3 mg, 6.15 µmol, 12.60 % yield). ¹H NMR (500 MHz, DMSO-d₆) δ 7.75 (d, *J*=7.7 Hz, 1H), 7.58 (br s, 1H), 7.44 (br d, *J*=7.4 Hz, 1H), 7.39 - 7.31 (m, 2H), 7.24 - 7.18 (m, 1H), 7.15 (br s, 1H), 6.81 - 6.70 (m, 1H), 4.78 - 4.57 (m, 2H), 4.28 (s, 3H), 4.21 (s, 3H), 4.03 - 3.90 (m, 1H), 3.86 - 3.72 (m, 2H), 3.70 - 3.56 (m, 1H), 3.36 - 3.23 (m, 1H), 2.68 - 2.58 (m, 1H), 2.50 - 2.36 (m, 1H), 2.32 - 2.15 (m, 5H), 2.11 (br d, *J*=2.9 Hz, 1H), 2.01 - 1.61 (m, 6H), 1.53 - 1.43 (m, 2H), 1.19 - 1.10 (m, 1H), 0.50 (br d, *J*=8.1 Hz, 2H), -0.01 (br s, 2H). MS ESI m/z = 595.4 (M+H). HPLC retention time 1.37 minutes, Method 2.

### Example 87

### 2-(2-{5-[(1R.4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)phenol

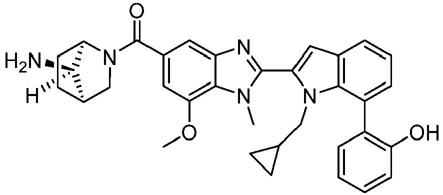

In a 2-dram vial, a stirring mixture of 2-(4,4,5,5-tetramethyl-1,3,2-dioxaborolan-2-yl)phenol (15 mg, 0.068 mmol), ((1R,4R,7R)-7-amino-2-azabicyclo[2.2.1]heptan-2-yl)(2-(7-bromo-1-(cyclopropylmethyl)-1H-indol-2-yl)-7-methoxy-1-methyl-1H-benzo[d]imidazol-5-yl)methanone (32 mg, 0.058 mmol), PdCl2(dppf) (4.27 mg, 5.83 µmol), and 2 M potassium phosphate, tribasic (0.117 mL, 0.233 mmol) (previously degassed) in 1,4-Dioxane (2 mL) was degassed with bubbling nitrogen for 10 minutes. The vial was sealed, and the reaction was stirred at 80 °C for 18 hours, at which point it was judged to be complete by LCMS. The solvents were evaporated, and the residue was taken up in DMF (2 mL). The crude material was purified via preparative LC/MS with the following conditions: Column: XBridge C18, 200 mm x 19 mm, 5-µm particles; Mobile Phase A: 5:95 acetonitrile: water with 0.1% trifluoroacetic acid; Mobile Phase B: 95:5 acetonitrile: water with 0.1% trifluoroacetic acid; Gradient: a 0-minute hold at 16% B, 16-56% B over 20 minutes, then a 4-minute hold at 100% B; Flow Rate: 20 mL/min; Column Temperature: 25 C. Fraction collection was triggered by MS and UV signals. Fractions containing the desired product were combined and dried via centrifugal evaporation. The material was further purified via preparative LC/MS with the following conditions: Column: XBridge Shield RP18, 200 mm x 19 mm, 5-µm particles; Mobile Phase A: 5:95 acetonitrile: water with 10-mM ammonium acetate; Mobile Phase B: 95:5 acetonitrile: water with 10- mM ammonium acetate; Gradient: a 0-minute hold at 35% B, 35-57% B over 25 minutes, then a 2-minute hold at 100% B; Flow Rate: 20 mL/min; Column Temperature: 25 C. Fraction collection was triggered by MS signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound (3.2 mg, 5.70 µmol, 9.76 % yield). ¹H NMR (500 MHz, DMSO-d₆) δ 8.03 (br s, 1H), 7.75 (br d, J=7.0 Hz, 1H), 7.51 - 7.21 (m, 4H), 7.16 (br s, 1H), 7.11 - 6.93 (m, 3H), 4.16 (br s, 3H), 4.10 - 3.92 (m, 4H), 3.85 - 3.43 (m, 2H), 3.28 - 3.05 (m, 1H), 2.32 - 2.15 (m, 1H), 2.13 - 1.92 (m, 3H), 1.87 - 1.69 (m, 1H), 1.51 (br d, J=9.8 Hz, 1H), 0.93 (br s, 1H), 0.52 (br s, 1H), 0.27 - 0.06 (m, 2H), -0.38 - -0.75 (m, 2H). MS ESI m/z = 562.2 (M+H). HPLC retention time 1.86 minutes, Method 1.

The following compounds in Table 5 can be made by the procedures described in Example 87, substituting the appropriate boronic acid or boronic ester for 2-(4,4,5,5-tetramethyl-1,3,2-dioxaborolan-2-yl)phenol.

**Table 5**

| Ex # | Structure | Name | LC/ MS Rt (min ) | LC/ MS Met hod | M+H (obs ion) |
|---|---|---|---|---|---|
| 88 | 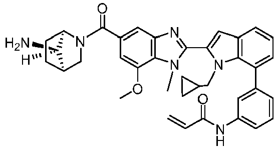 | N-[3-(2-{5-[(1R,4R,7R)-7-amino-2-azabicyclo[2.2. 1]heptan e-2-carbonyl]-7-methoxy-1-methyl-1H-1,3-benzodiazol-2-yl}-1-(cyclopropylmethyl)-1H-indol-7-yl)phenyl]prop-2-enamide | 1.78 | 1 | 615.2 |
| 89 | 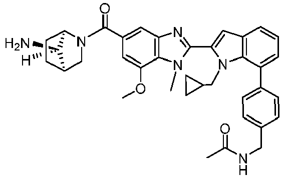 | N-{[4-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)phenyl]methyl}aceta mide | 1.60 | 1 | 617.0 |
| 90 | 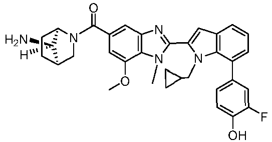 | 4-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-2-fluorophenol | 1.71 | 1 | 580.3 |
| 91 | 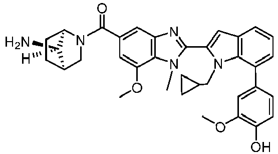 | 4-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-2-methoxyphenol | 1.49 | 2 | 592.3 |
| 92 | 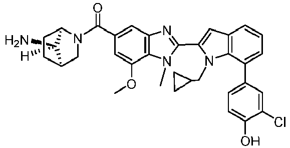 | 4-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-2-chlorophenol | 1.81 | 1 | 596.3 |
| 93 | 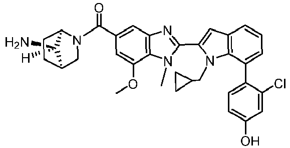 | 4-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-3-chlorophenol | 1.55 | 2 | 596.3 |
| 94 | 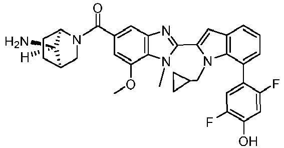 | 4-(2-{5-[(1R,4R,7R)-7-amino-2-azabicyclo[2.2. 1]heptan e-2-carbonyl]-7-methoxy-1-methyl-1H-1,3-benzodiazol-2-yl}- 1-(cyclopropylmethyl)-1H-indol-7-yl)-2, 5-difluorophenol | 1.50 | 2 | 598.0 |
| 95 | 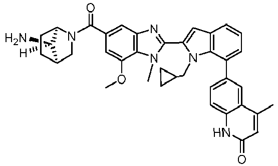 | 6-(2-{5-[(1R,4R,7R)-7-amino-2-azabicyclo[2.2. 1]heptan e-2-carbonyl]-7-methoxy-1-methyl-1H-1,3-benzodiazol-2-yl}-1-(cyclopropylmethyl)-1H-indol-7-yl)-4-methyl-1,2-dihydroquinolin-2-one | 1.51 | 2 | 627.3 |
| 96 | 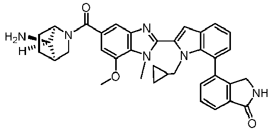 | 4-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-2,3-dihydro-1H-isoindol-1-one | 1.46 | 2 | 601.2 |
| 97 | 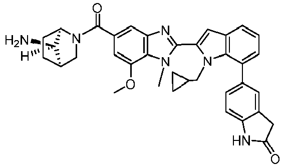 | 2'-{5-[(1R,4R,7R)-7-amino-2-azabicyclo[2.2. 1]heptan e-2-carbonyl]-7-methoxy-1-methyl-1H-1,3-benzodiazol-2-yl}-1'-(cyclopropylmethyl)-2,3-dihydro-1H,1'H-[5,7'-biindole]-2-one | 1.74 | 1 | 601.3 |
| 98 | 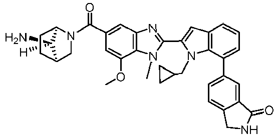 | 6-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-2,3-dihydro-1H-isoindol-1- one | 1.41 | 2 | 601.2 |
| 99 | 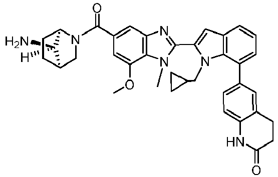 | 6-(2-{5-[(1R,4R,7R)-7-amino-2-azabicyclo[2.2. 1]heptan e-2-carbonyl]-7-methoxy-1-methyl-1H-1,3-benzodiazol-2-yl}- 1-(cyclopropylmethyl)-1H-indol-7-yl)-1,2,3,4-tetrahydroquinolin-2-one | 1.36 | 2 | 615.2 |
| 100 | 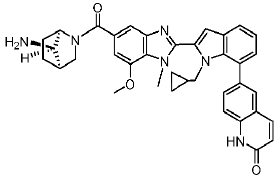 | 6-(2-{5-[(1R,4R,7R)-7-amino-2-azabicyclo[2.2. 1]heptan e-2-carbonyl]-7-methoxy-1-methyl-1H-1,3-benzodiazol-2-yl}-1-(cyclopropylmethyl)-1H-indol-7-yl)-1,2-dihydroquinolin-2-one | 1.45 | 2 | 613.1 |
| 101 | 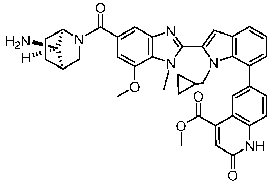 | methyl 6-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-2-oxo-1,2-dihydroquinoline-4-carboxylate | 1.37 | 2 | 671.3 |
| 102 | 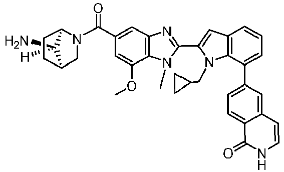 | 6-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-1,2-dihydroisoquinolin-1-one | 1.35 | 2 | 613.4 |
| 103 | 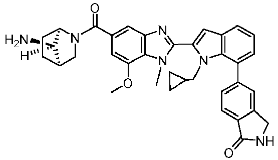 | 5-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-2,3-dihydro-1H-isoindol-1-one | 1.28 | 2 | 601.3 |
| 104 | 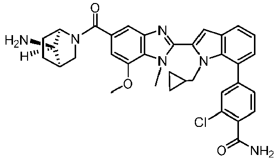 | 4-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)-2-chlorobenzamide | 1.68 | 1 | 623.4 |
| 105 | 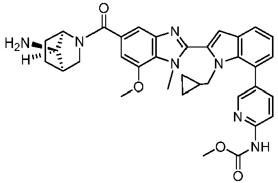 | methyl N-[5-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)pyridin-2-yl]carbamate | 1.68 | 1 | 620.2 |
| 106 | 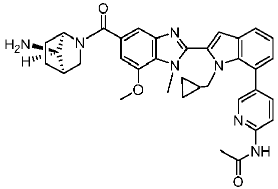 | N-[5-(2-{5-[(1R,4R,7R)-7-amino-2-azabicyclo[2.2.1]heptan e-2-carbonyl]-7-methoxy-1-methyl-1H-1,3-benzodiazol-2-yl}-1-(cyclopropylmethyl)-1H-indol-7-yl)pyridin-2-yl]acetamide | 1.64 | 1 | 604.1 |

### Example 107

### diethyl (2-{5-[(1R,4R,7R)-7-amino-2-azabicyclo[2.2.1]heptane-2-carbonyl]-7-methoxy-1-methyl-1H-1,3-benzodiazol-2-yl1-1-(cyclopropylmethyl)-1H-indol-7-yl)phosphonate

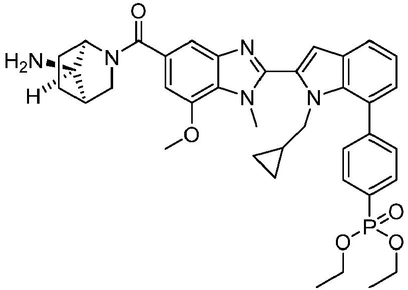

In a 40 mL vial, a mixture of *tert*-butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (100 mg, 0.154 mmol), diethyl phosphite (0.070 mL, 0.540 mmol), triethylamine (0.071 mL, 0.509 mmol), and PdCl2(dppf) (6 mg, 8.20 µmol) in toluene (2 mL) was placed in a sonicator and degassed with bubbling nitrogen for 1 minute. The vial was sealed, and the reaction was stirred at 100 °C for 18 hours. LCMS detected mostly starting material, but did detect a trace of the desired product. The mixture was treated with diethyl phosphite (0.070 mL, 0.540 mmol), triethylamine (0.071 mL, 0.509 mmol), and PdCl2(dppf) (6 mg, 8.20 µmol), and degassed with bubbling nitrogen for 10 minutes. The vial was sealed, and the reaction was stirred at 120 °C for 18 hours, at which point it was judged to be complete by LCMS. The mixture was filtered and concentrated in vacuo, and the residue was taken up in dichloromethane (2 mL). The solution was treated with TFA (1 mL), and the reaction was stirred at room temperature for 3 hours, at which point it was judged to be complete by LCMS. The mixture was concentrated in vacuo, and the crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 19% B, 19-59% B over 20 minutes, then a 4-minute hold at 100% B; Flow Rate: 20 mL/min; Column Temperature: 25 C. Fraction collection was triggered by MS and UV signals. Fractions containing the desired product were combined and dried via centrifugal evaporation. The material was further purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 25% B, 25-46% B over 25 minutes, then a 2-minute hold at 100% B; Flow Rate: 20 mL/min; Column Temperature: 25 C. Fraction collection was triggered by MS signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound, (12 mg, 0.019 mmol, 12.14 % yield). ¹H NMR (500 MHz, DMSO-d₆) δ 7.87 (br d, *J*=7.6 Hz, 1H), 7.72 (br dd, *J*=16.2, 7.3 Hz, 1H), 7.54 - 7.40 (m, 1H), 7.36 - 7.21 (m, 1H), 7.16 (br t, *J*=6.7 Hz, 1H), 7.10 (s, 1H), 6.88 - 6.77 (m, 1H), 4.76 - 4.59 (m, 2H), 4.02 (br t, *J*=6.7 Hz, 4H), 3.98 (br s, 3H), 3.87 (s, 3H), 3.69 - 3.35 (m, 1H), 3.19 (br s, 1H), 3.07 - 2.88 (m, 1H), 2.17 - 2.00 (m, 1H), 1.93 - 1.70 (m, 2H), 1.68 - 1.52 (m, 1H), 1.39 - 1.06 (m, 8H), 0.61 (br s, 1H), 0.01 (br d, *J*=7.3 Hz, 2H), -0.44 - -0.63 (m, 2H). MS ESI m/z = 606.0 (M+H). HPLC retention time 1.49 minutes, Method 2.

### Example 108

### 2-{4-[2-(5-{7-amino-2-azabicyclo[2.2.1]heptane-2-carbonyl}-7-methoxy-1-methyl-1H-1,3-benzodiazol-2-yl)-1-(cyclopropylmethyl)-1H-indol-7-yl]-1H-1,2,3-triazol-1-yl}ethan-1-ol

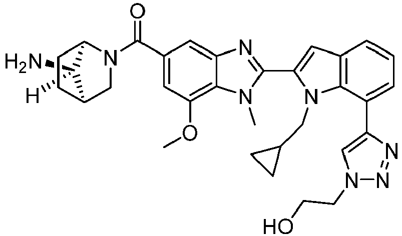

### Intermediate 108A: tert-butyl (2-(2-(1-(cyclopropylmethyl)-7-((trimethylsilyl)ethynyl)-1H-indol-2-yl)-7-methoxy-1-methyl-1H-benzo[d]imidazole-5-carbonyl)-2-azabicyclo[2.2.1]heptan-7-yl)carbamate

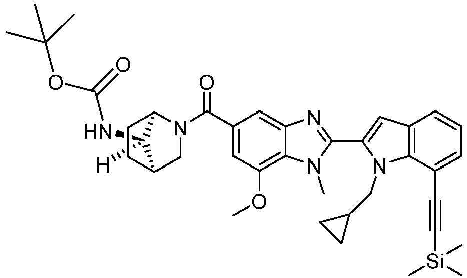

In a 2 mL microwave vial, a solution of *tert*-butyl (2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (265 mg, 0.409 mmol) and copper(I) iodide (9 mg, 0.047 mmol) in DMF (1.5 mL) was degassed with bubbling nitrogen for 10 minutes. The mixture was treated with bis(triphenylphosphine)palladium(II) dichloride (31.5 mg, 0.045 mmol) and triethylamine (1.139 mL, 8.17 mmol), and degassed for 5 minutes, trimethylsilylacetylene (0.113 mL, 0.817 mmol) was added, the vial was sealed, and the reaction was heated at 120 °C via microwave for 25 minutes, at which point it was judged to be complete by LCMS. The mixture was concentrated in vacuo, and the residue was taken up in ethyl acetate (35 mL). The solution was filtered, then washed twice with 10% lithium chloride and once with brine, dried over sodium sulfate, and concentrated in vacuo. The residue was chromatographed via MPLC over a 40 g silica gel column, eluting at 40 mL/min with a 20% to 100% ethyl acetate/hexanes gradient over 25 column volumes. Fractions containing the desired product were pooled and concentrated in vacuo to yield 256 mg of an amber solid, which was used without further purification in the next step. MS ESI m/z = 666.7 (M+H). HPLC retention time 1.27 minutes, Method D.

### Intermediate 108B: tert-butyl (2-(2-(1-(cyclopropylmethyl)-7-ethynyl-1H-indol-2-yl)-7-methoxy-1-methyl-1H-benzo[d]imidazole-5-carbonyl)-2-azabicyclo[2.2.1]heptan-7-yl)carbamate

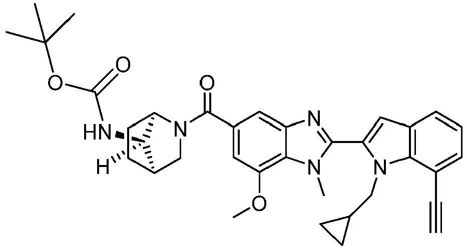

A stirring solution of *tert*-butyl (2-(2-(1-(cyclopropylmethyl)-7-((trimethylsilyl)ethynyl)-1H-indol-2-yl)-7-methoxy-1-methyl-1H-benzo[d]imidazole-5-carbonyl)-2-azabicyclo[2.2.1]heptan-7-yl)carbamate (256 mg, 0.384 mmol) in methanol (5 mL) was treated with potassium carbonate (26.6 mg, 0.192 mmol). The reaction was stirred at room temperature for 2 hours, at which point it was judged to be complete by LCMS. Most of the methanol was evaporated, and the residue was taken up in ethyl acetate. The turbid solution was washed 3X with water, dried over sodium sulfate, and concentrated in vacuo. The residue was chromatographed via MPLC over a 40 g silica gel column, eluting at 40 mL/min with a 0% to 5% methanol/dichloromethane gradient over 12 column volumes. Fractions containing the desired product were pooled and concentrated in vacuo to yield the title compound as an amber solid (80 mg, 0.135 mmol, 35.0 % yield).. MS ESI m/z = 594.6 (M+H). HPLC retention time 1.07 minutes, Method D.

### Example 108: 2-{4-[2-(5-{7-amino-2-azabicyclo[2.2.1]heptane-2-carbonyl}-7-methoxy-1-methyl-1H-1,3-benzodiazol-2-yl)-1-(cyclopropylmethyl)-1H-indol-7-yl]-1H-1,2,3-triazol-1-yl}ethan-1-ol

In a 2-dram vial, a mixture of *tert*-butyl (2-(2-(1-(cyclopropylmethyl)-7-ethynyl-1H-indol-2-yl)-7-methoxy-1-methyl-1H-benzo[d]imidazole-5-carbonyl)-2-azabicyclo[2.2.1]heptan-7-yl)carbamate (13 mg, 0.022 mmol), 2-azidoethan-1-ol (10% in ethanol) (38.1 mg, 0.044 mmol), and sodium ascorbate (0.51 M in water) (0.013 mL, 6.57 µmol) in 5:1 THF/water (0.2 mL) was treated with copper(II) sulfate (0.62 M in water) (3.53 µl, 2.190 µmol). The vial was sealed, and the reaction was stirred at 50 °C for 18 hours. The mixture was treated with 2-azidoethan-1-ol (10% in ethanol) (38.1 mg, 0.044 mmol), and the reaction was stirred at 50 °C for 18 hours, at which point it was judged to be essentially complete by LCMS. The mixture was diluted with ethyl acetate (5 mL) and filtered, and the filtrate was washed once with brine. The organic phase was dried over sodium sulfate and concentrated in vacuo. The residue was taken up in dichloromethane (3 mL), and the solution was treated with 4 M HCl in dioxane (2 mL, 8.00 mmol). The reaction was stirred at room temperature for 2 hours, at which point it was judged to be complete by LCMS. The reaction mixture was concentrated in vacuo, and the crude material was purified via preparative LC/MS with the following conditions: Column: XBridge C18, 19 x 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 10-50% B over 20 minutes, then a 4-minute hold at 100% B; Flow: 20 mL/min. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound, (5.5 mg, 8.90 µmol, 40.5 % yield). ¹H NMR (500 MHz, DMSO-d₆) δ 8.30 (s, 1H), 7.79 (br d, J=6.8 Hz, 1H), 7.45 - 7.31 (m, 1H), 7.26 - 7.19 (m, 2H), 7.11 (s, 1H), 6.98 - 6.88 (m, 1H), 4.53 (t, J=5.4 Hz, 2H), 4.14 - 4.04 (m, 5H), 3.98 (s, 3H), 3.91 - 3.85 (m, 2H), 3.15 - 2.97 (m, 1H), 2.94 - 2.66 (m, 1H), 2.28 - 2.12 (m, 1H), 2.05 - 1.84 (m, 5H), 1.79 - 1.64 (m, 1H), 1.55 - 1.34 (m, 1H), 1.09 - 0.78 (m, 1H), 0.42 (br s, 1H), -0.01 (br d, *J*=7.8 Hz, 2H), -0.50 (br s, 2H). MS ESI m/z = 581.4 (M+H). HPLC retention time 1.28 minutes, Method 1.

### Example 109

### (1R,4R,7R)-2-{2-[1-(cyclopropylmethyl)-7-14-[(1r,4r)-4-aminocyclohexyl]-1H-1,2,3-triazol-1-yl}-1H-indol-2-yl]1-7-methoxy-1-methyl-1H-1,3-benzodiazole-5-carbonyl}-2-azabicyclo[2.2.1]heptan-7-amine

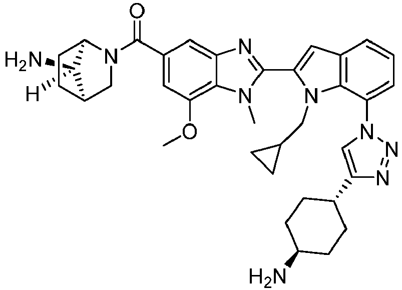

In a scintillation vial, a mixture of *tert*-butyl ((1r,4r)-4-ethynylcyclohexyl)carbamate (31.0 mg, 0.139 mmol), *tert*-butyl ((1r,4r)-4-ethynylcyclohexyl)carbamate (31.0 mg, 0.139 mmol), trans-n,n'-dimethylcyclohexane-1,2-diamine (0.036 mL, 0.231 mmol), and sodium azide (7.89 mg, 0.121 mmol) in DMSO (0.4 mL) and water (0.1 mL) was treated with copper(I) iodide (24.22 mg, 0.127 mmol) and sodium ascorbate (25.2 mg, 0.127 mmol). The vial was sealed, and the reaction was stirred at 70 °C for 18 hours. LCMS indicated that the reaction had not gone to completion. The mixture was allowed to come to room temperature and treated with *tert*-butyl ((1r,4r)-4-ethynylcyclohexyl)carbamate (31.0 mg, 0.139 mmol), sodium azide (7.89 mg, 0.121 mmol), copper(I) iodide (24.22 mg, 0.127 mmol), and sodium ascorbate (25.2 mg, 0.127 mmol). The vial was sealed, and the reaction was stirred at 70 °C for 7 hours, then at room temperature for 2 days. The mixture was cooled to room temperature and diluted with ethyl acetate. The turbid solution was washed 3X with water, then dried over sodium sulfate and concentrated in vacuo. The residue was taken up in dichloromethane (5 mL), and the solution was treated with 4M HCl in dioxane (7.05 µl, 0.232 mmol). The reaction was stirred at room temperature for 2 hours, at which point it was judged to be complete by LCMS. The reaction mixture was concentrated in vacuo, and the crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 7% B, 7-47% B over 20 minutes, then a 4-minute hold at 100% B; Flow Rate: 20 mL/min; Column Temperature: 25 C. Fraction collection was triggered by MS and UV signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound, (17.7 mg, 0.028 mmol, 24.07 % yield). ¹H NMR (500 MHz, DMSO-d₆) δ 8.35 (s, 1H), 7.95 - 7.87 (m, 1H), 7.41 - 7.24 (m, 3H), 7.18 (s, 1H), 6.94 - 6.84 (m, 1H), 4.03 (s, 3H), 3.94 (s, 3H), 3.47 (br d, *J*=10.9 Hz, 2H), 3.20 - 2.91 (m, 1H), 2.80 - 2.70 (m, 2H), 2.21 - 2.00 (m, 3H), 1.91 (br d, *J*=13.0 Hz, 4H), 1.72 - 1.60 (m, 1H), 1.56 - 1.42 (m, 2H), 1.40 - 1.16 (m, 3H), 0.41 (br d, *J*=5.4 Hz, 1H), -0.01 (br d, *J*=7.8 Hz, 2H), -0.57 (br s, 2H). (Proton count is low due to the water suppression algorithm used during data processing). MS ESI m/z = 634.2 (M+H). HPLC retention time 1.42 minutes, Method 1.

### Example 110

### (1R,4R,7R)-2-(2-{7-[4-(azetidin-3-yl)-1H-1,2,3-triazol-1-yl]-1-(cyclopropylmethyl)-1H-indol-2-yl}-7-methoxy-1-methyl-1H-1,3-benzodiazole-5-carbonyl)-2-azabicyclo[2.2.1]heptan-7-amine

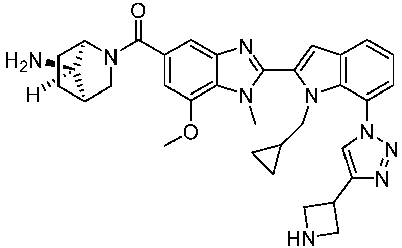

The title compound was prepared via the procedure described in Example 109. MS ESI m/z = 592.5 (M+H). HPLC retention time 1.10 minutes, Method 2.

### Examples 111 (Isomer 1) and 112 (Isomer 2) 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 1, and 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 2

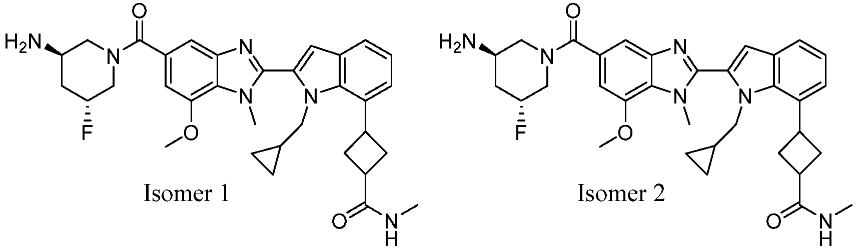

### Intermediate 111/112 A: tert-butyl ((3R,5R)-1-(2-(7-bromo-1-(cyclopropylmethyl)-1H-indol-2-yl)-7-methoxy-1-methyl-1H-benzo[d]imidazole-5-carbonyl)-5-fluoropiperidin-3-yl)carbamate

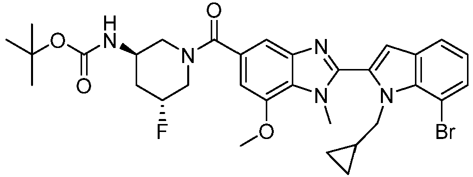

The title compound was prepared from 2-(7-bromo-1-(cyclopropylmethyl)-1H-indol-2-yl)-7-methoxy-1-methyl-1H-benzo[d]imidazole-5-carboxylic acid and *tert-*butyl ((3R, 5R)-5-fluoropiperidin-3-yl)carbamate using the conditions described in Example 1, Step 5. ¹H NMR (499 MHz, chloroform-d) δ 7.66 (dd, *J*=7.9, 1.0 Hz, 1H), 7.54 - 7.49 (m, 2H), 7.05 (t, *J*=7.7 Hz, 1H), 6.94 (d, *J*=1.0 Hz, 1H), 6.84 (s, 1H), 5.02 - 4.30 (m, 5H), 4.12 (s, 3H), 4.09 - 4.00 (m, 4H), 3.45 - 3.29 (m, 1H), 3.23 - 3.02 (m, 1H), 2.43 - 2.31 (m, 1H), 2.02 - 1.74 (m, 1H), 1.56 - 1.24 (m, 9H), 1.19 - 1.07 (m, 1H), 0.29 (br dd, *J*=8.0, 0.8 Hz, 2H), -0.14 (br d, *J*=4.6 Hz, 2H). MS ESI m/z = 654.4 (M+H). HPLC retention time 1.00 minutes, Method D.

### Intermediate 111/112 B: 3-(2-(5-((3R,5R)-3-((tert-butoxycarbonyl)amino)-5-fluoropiperidine-1-carbonyl)-7-methoxy-1-methyl-1H-benzo[d]imidazol-2-yl)-1-(cyclopropylmethyl)-1H-indol-7-yl)cyclobutane-1-carboxylic acid (cis/trans mixture)

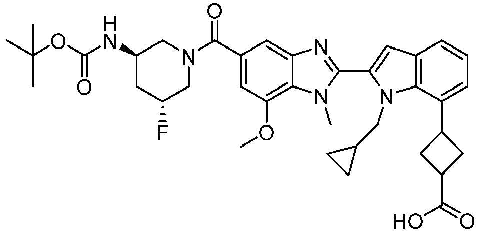

The title compound was prepared from *tert*-butyl ((3R,5R)-1-(2-(7-bromo-1-(cyclopropylmethyl)-1H-indol-2-yl)-7-methoxy-1-methyl-1H-benzo[d]imidazole-5-carbonyl)-5-fluoropiperidin-3-yl)carbamate and methyl 3-iodocyclobutane-1-carboxylate using the conditions described in Examples 43 and 44, Steps 3 and 4. MS ESI m/z = 674.6 (M+H). HPLC retention time 0.85, 0.86 minutes, Method D.

### Examples 111 and 112: 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 1, and 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 2

The title compounds were prepared from 3-(2-(5-((3R,SR)-3-((*tert-*butoxycarbonyl)amino)-5-fluoropiperidine-1-carbonyl)-7-methoxy-1-methyl-1H-benzo[d]imidazol-2-yl)-1-(cyclopropylmethyl)-1H-indol-7-yl)cyclobutane-1-carboxylic acid (*cis*/*trans* mixture) and methanamine hydrochloride using the procedure described in Examples 43 and 44, Step 5.
Example 111, First Eluting: 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 1. ¹H NMR (500 MHz, DMSO-d₆) δ 7.59 (br d, *J*=4.3 Hz, 1H), 7.35 (d, *J*=7.8 Hz, 1H), 7.09 (s, 1H), 7.05 (br d, *J*=7.0 Hz, 1H), 7.00 - 6.92 (m, 1H), 6.84 (s, 1H), 6.63 (s, 1H), 5.01 - 4.48 (m, 1H), 4.26 (br d, *J*=5.1 Hz, 2H), 3.88 (s, 3H), 3.85 - 3.79 (m, 1H), 3.79 - 3.74 (m, 3H), 3.31 (br d, *J*=1.6 Hz, 2H), 2.91 - 2.76 (m, 2H), 2.38 (d, *J*=4.5 Hz, 3H), 2.35 - 2.32 (m, 4H), 2.24 - 2.12 (m, 2H), 2.06 - 1.89 (m, 1H), 1.44 - 1.23 (m, 1H), 0.64 - 0.50 (m, 1H), -0.01 (br d, *J*=7.9 Hz, 2H), -0.49 - - 0.64 (m, 2H). MS ESI m/z = 587.3 (M+H). HPLC retention time 1.22 minutes, Method 2. Example 112, Second Eluting: 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N-methylcyclobutane-1-carboxamide, ISOMER 2. ¹H NMR (500 MHz, DMSO-d₆) δ 7.59 (br d, *J*=4.4 Hz, 1H), 7.37 (d, *J*=7.8 Hz, 1H), 7.15 (d, *J*=7.2 Hz, 1H), 7.10 (s, 1H), 6.97 (t, *J*=7.6 Hz, 1H), 6.85 (s, 1H), 6.64 (s, 1H), 4.99 - 4.51 (m, 1H), 4.20 (br d, *J*=6.4 Hz, 2H), 4.12 - 4.02 (m, 1H), 3.88 (s, 3H), 3.77 (s, 3H), 3.56 - 3.39 (m, 1H), 2.90 - 2.76 (m, 2H), 2.49 - 2.41 (m, 5H), 2.38 - 2.33 (m, 5H), 2.27 - 2.18 (m, 2H), 2.06 - 1.91 (m, 1H), 1.44 - 1.23 (m, 1H), 0.64 - 0.48 (m, 1H), -0.01 (br d, *J*=8.2 Hz, 2H), -0.55 (br d, *J*=2.6 Hz, 2H). MS ESI m/z = 587.3 (M+H). HPLC retention time 1.27 minutes, Method 2.

The following compounds in Table 6 can be made by the procedures described in Examples 111 and 112, substituting the appropriate amine for methanamine hydrochloride in step 3.

**Table 6**

| Ex # | Structure | Name | LC/ MS Rt (min ) | LC/ MS Met hod | M+H (obs ion) |
|---|---|---|---|---|---|
| 113 | 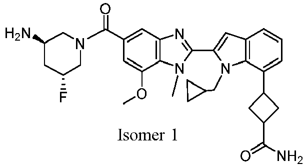 | 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)cyclobutane-1-carboxamide, ISOMER 1 | 1.17 | 2 | 573.2 |
| 114 | 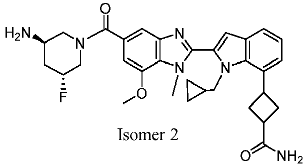 | 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)cyclobutane-1-carboxamide, ISOMER 2 | 1.22 | 2 | 573.3 |
| 115 | 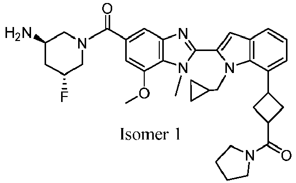 | ((3R,5R)-3-amino-5-fluoropiperidin-1-yl)(2-(1-(cyclopropylmethyl)-7-(3-(pyrrolidine-1- carbonyl)cyclobutyl)-1H-indol-2-yl)-7-methoxy-1-methyl-1H-benzo[d]imidazol-5-yl)methanone, ISOMER 1 | 1.41 | 2 | 627.4 |
| 116 | 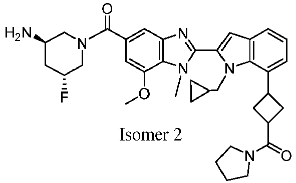 | ((3R,5R)-3-amino-5-fluoropiperidin-1-yl)(2-(1-(cyclopropylmethyl)-7-(3-(pyrrolidine-1- carbonyl)cyclobutyl)-1H-indol-2-yl)-7-methoxy-1-methyl-1H-benzo[d]imidazol-5-yl)methanone, ISOMER 2 | 1.50 | 2 | 627.3 |
| 117 | 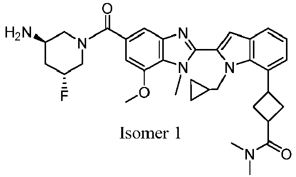 | 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N,N-dimethylcyclobutane-1-carboxamide, ISOMER 1 | 1.33 | 2 | 601.3 |
| 118 | 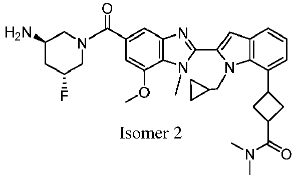 | 3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N,N-dimethylcyclobutane-1-carboxamide, ISOMER 2 | 1.41 | 2 | 601.4 |

### Example 119

### ((1R,4R,7R)-7-amino-2-azabicyclo[2.2.1]heptan-2-yl)(2-(1-(cyclopropylmethyl)-7-(4-hydroxy-3-(hydroxymethyl)phenyl)-1H-indol-2-yl)-7-methoxy-1-methyl-1H-benzo[d]imidazol-5-yl)methanone

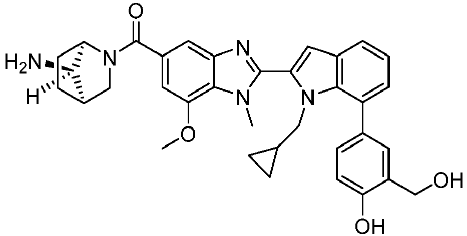

### Intermediate 119A: methyl 5-(2-(5-((lR,4R,7R)-7-((tert-butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzoate

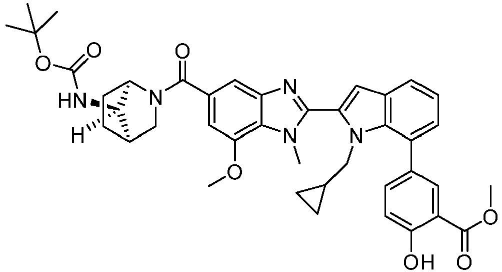

In a 2-dram vial, a stirring mixture of methyl 2-hydroxy-5-(4,4,5,5-tetramethyl-1,3,2-dioxaborolan-2-yl)benzoate (129 mg, 0.463 mmol), *tert*-butyl ((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (200 mg, 0.308 mmol), PdCl2(dppf) (22.56 mg, 0.031 mmol), and 2 M potassium phosphate, tribasic (0.694 mL, 1.388 mmol) (previously degassed) in DMF (2 mL) was degassed with bubbling nitrogen for 10 minutes. The vial was sealed, and the reaction was stirred at 80 °C for hour, at which point it was judged to be complete by LCMS. The solution was allowed to come to room temperature, diluted with ethyl acetate (10 mL) and water (5 mL), and filtered. The layers were separated, and the organic phase was washed twice with 10% lithium chloride and once with brine, then dried over sodium sulfate and concentrated in vacuo. The residue was chromatographed via MPLC over a 24 g silica gel column, eluting at 40 mL/min with a 0% to 100% ethyl acetate/hexanes gradient over 14 column volumes, then with ethyl acetate to completely elute the product. Fractions containing the desired product were pooled and concentrated in vacuo to yield the title compound, (77 mg, 0.107 mmol, 34.7 % yield). ¹H NMR (500 MHz, chloroform-d) δ 10.88 (s, 1H), 8.01 (d, *J*=2.1 Hz, 1H), 7.74 (dd, *J*=7.9, 1.0 Hz, 1H), 7.64 (dd, *J*=8.5, 2.3 Hz, 1H), 7.47 (s, 1H), 7.24 (t, *J*=7.5 Hz, 1H), 7.14 - 7.07 (m, 2H), 7.04 - 6.99 (m, 1H), 6.95 (s, 1H), 4.68 - 4.44 (m, 1H), 4.42 - 4.29 (m, 1H), 4.22 - 4.11 (m, 3H), 4.03 (s, 3H), 3.98 (s, 5H), 3.89 - 3.71 (m, 2H), 3.30 - 3.18 (m, 1H), 2.54 (br s, 1H), 2.06 - 1.82 (m, 3H), 1.76 - 1.59 (m, 4H), 1.46 - 1.27 (m, 9H), 0.51 - 0.39 (m, 1H), -0.37 - -0.73 (m, 2H). MS ESI m/z = 720.5 (M+H). HPLC retention time 1.03 minutes, Method D.

### Example 119: ((1R,4R,7R)-7-amino-2-azabicyclo[2.2.1]heptan-2-yl)(2-(1-(cyclopropylmethyl)-7-(4-hydroxy-3-(hydroxymethyl)phenyl)-1H-indol-2-yl)-7-methoxy-1-methyl-1H-benzor dlimidazol-5-yl)methanone

A stirring solution of methyl 5-(2-(5-((1R,4R,7R)-7-((*tert-*butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzoate (38 mg, 0.053 mmol) in anhydrous THF (1.5 mL) was cooled to 0 C and treated with lithium borohydride (2 M in THF) (0.053 mL, 0.106 mmol). The reaction was allowed to come to room temperature and stirred for 18 hours, at which point it was judged to be complete by LCMS. The reaction was quenched with methanol (2 mL) and stirred for 30 minutes, then the mixture was concentrated in vacuo. The residue was taken up in dichloromethane (2 mL), and the solution was treated with 4M HCl in dioxane (2 mL). The reaction was stirred at room temperature for 1 hour, at which point it was judged to be complete by LCMS. The mixture was concentrated in vacuo. The crude material was purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 17% B, 17-57% B over 20 minutes, then a 4-minute hold at 100% B; Flow Rate: 20 mL/min; Column Temperature: 25 C. Fraction collection was triggered by MS signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound. ¹H NMR (500 MHz, DMSO-d₆) δ 9.56 (br s, 1H), 7.71 - 7.63 (m, 1H), 7.44 - 7.32 (m, 2H), 7.23 - 7.17 (m, 2H), 7.15 - 7.11 (m, 1H), 7.07 - 7.00 (m, 1H), 6.97 - 6.88 (m, 2H), 5.05 (br s, 1H), 4.57 (br d, *J*=3.5 Hz, 2H), 4.18 - 4.08 (m, 3H), 3.99 (s, 3H), 3.95 (br d, *J*=6.9 Hz, 1H), 3.75 (s, 1H), 3.67 - 3.47 (m, 1H), 3.16 (s, 1H), 3.10 - 3.00 (m, 1H), 2.25 - 2.10 (m, 1H), 2.07 - 1.92 (m, 2H), 1.81 - 1.63 (m, 1H), 1.51 - 1.32 (m, 1H), 0.46 - 0.34 (m, 1H), -0.05 (br d, *J*=8.1 Hz, 2H), -0.45 - -0.73 (m, 2H). MS ESI m/z = 592.2 (M+H). HPLC retention time 1.30 minutes, Method 2.

### Examples 120 and 121

### 5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzamide and 5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzoic acid

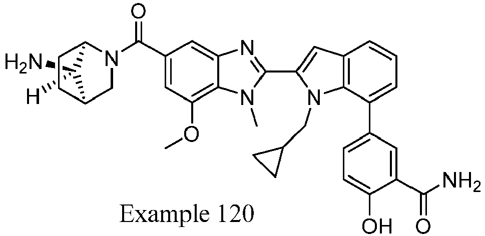

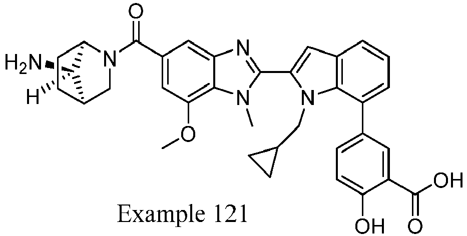

### Intermediate 120/121 A: 5-(2-(5-((1R,4R,7R)-7-((tert-butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzoic acid

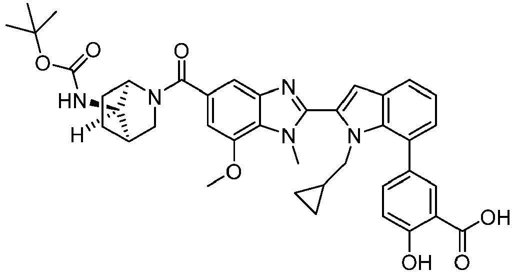

In a 2 dram vial, a stirring solution of methyl 5-(2-(5-((1R,4R,7R)-7-((*tert-*butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzoate (38 mg, 0.053 mmol) in methanol (1 mL) was treated with 1M sodium hydroxide (0.158 mL, 0.158 mmol). The vial was sealed, and the reaction was stirred at 70 C for 18 hours, at which point it was judge to be complete by LCMS. The mixture was diluted with water (1 mL) and the methanol was allowed to evaporate. The aqueous mixture was acidified to pH 5 with 1M HCl, and the product was extracted into dichloromethane. The combined organic phases were dried over sodium sulfate and concentrated in vacuo to yield the title compound, which was used without further purification in the next step. MS ESI m/z = 706.3 (M+H). HPLC retention time 0.91 minutes, Method D.

### Examples 120 and 121: 5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzamide and 5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzoic acid

A stirring mixture of 5-(2-(5-((1R,4R,7R)-7-((*tert*-butoxycarbonyl)amino)-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzoic acid (30 mg, 0.043 mmol), ammonium hydroxide (0.017 mL, 0.425 mmol), and triethylamine (0.024 mL, 0.170 mmol) in DMF (2 mL) was treated with BOP (22.56 mg, 0.051 mmol). The reaction was stirred at room temperature for 18 hours, at which point LCMS detected the desired product and unreacted starting carboxylic acid. The mixture was concentrated in vacuo, and the residue was taken up in dichloromethane (2 mL). The mixture was treated with 4M HCl in dioxane (2 mL), and the reaction was stirred at room temperature for 1 hour, at which point it was judged to be complete by LCMS. The mixture was concentrated in vacuo. The crude material was purified via preparative LC/MS with the following conditions: Column: XBridge C18, 200 mm x 19 mm, 5-µm particles; Mobile Phase A: 5:95 acetonitrile: water with 0.1% trifluoroacetic acid; Mobile Phase B: 95:5 acetonitrile: water with 0.1% trifluoroacetic acid; Gradient: a 0-minute hold at 17% B, 17-57% B over 24 minutes, then a 4-minute hold at 100% B; Flow Rate: 20 mL/min; Column Temperature: 25 C. Fraction collection was triggered by UV signals. Fractions containing the Example 120 were combined and dried via centrifugal evaporation. The purified material was then diluted with DMF, treated with Si-Pyridine and shaken for a minimum of 2 h. The resulting mixture was filtered and dried via centrifugal evaporation to yield the title compound Example 120, (9.1 mg, 0.014 mmol, 33.7 % yield). ¹H NMR (500 MHz, DMSO-d₆) δ 8.49 (br s, 1H), 8.11 (s, 1H), 7.98 (br s, 1H), 7.78 (d, *J*=7.6 Hz, 1H), 7.66 - 7.57 (m, 1H), 7.55 - 7.37 (m, 1H), 7.27 (br t, *J*=7.6 Hz, 1H), 7.20 (s, 1H), 7.14 (br d, *J*=7.3 Hz, 1H), 7.07 (d, *J*=8.5 Hz, 1H), 7.03 - 6.96 (m, 1H), 4.61 - 4.20 (m, 1H), 4.15 (br s, 3H), 4.03 (br s, 3H), 3.97 - 3.76 (m, 2H), 3.71 - 3.59 (m, 1H), 3.57 - 3.38 (m, 1H), 3.30 - 3.17 (m, 1H), 2.97 (br d, *J*=5.2 Hz, 4H), 2.08 - 1.85 (m, 3H), 1.79 - 1.58 (m, 1H), 1.40 - 1.28 (m, 1H), 0.60 - 0.40 (m, 1H), 0.07 - -0.09 (m, 2H), -0.57 (br d, *J*=0.9 Hz, 2H). MS ESI m/z = 605.3 (M+H). HPLC retention time 1.41 minutes, Method 2.

Fractions containing the Example 121 were combined and dried via centrifugal evaporation. The material was further purified via preparative LC/MS with the following conditions: Column: XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 11% B, 11-51% B over 25 minutes, then a 6-minute hold at 100% B; Flow Rate: 20 mL/min; Column Temperature: 25 C. Fraction collection was triggered by MS signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation to yield the title compound Example 121, (4.2 mg, 6.64 µmol, 15.63 % yield). ¹H NMR (500 MHz, DMSO-d₆) δ 7.83 (s, 1H), 7.72 (br d, *J*=7.5 Hz, 1H), 7.62 - 7.44 (m, 1H), 7.37 (br d, *J*=8.4 Hz, 1H), 7.25 (br t, *J*=7.6 Hz, 1H), 7.20 (s, 1H), 7.10 (br d, *J*=7.2 Hz, 1H), 7.04 - 6.96 (m, 1H), 6.84 (d, *J*=8.2 Hz, 1H), 4.24 - 4.13 (m, 3H), 4.10 - 3.98 (m, 4H), 3.81 - 3.52 (m, 3H), 3.29 - 3.11 (m, 3H), 2.17 - 1.83 (m, 4H), 1.80 - 1.44 (m, 2H), 1.40 - 1.20 (m, 1H), 0.58 - 0.35 (m, 1H), -0.01 (br d, *J*=7.6 Hz, 2H), -0.45 - -0.69 (m, 2H). MS ESI m/z = 606.1(M+H). HPLC retention time 1.48 minutes, Method 1.

### Example 122

### ((1R,4R,7R)-7-amino-2-azabicyclo[2.2.1]heptan-2-yl)(2-(1-(cyclopropylmethyl)-7-(1-methyl-1H-pyrazol-4-yl)-1H-indol-2-yl)-7-methoxy-1-methyl-1H-benzo[d]imidazol-5-yl)methanone

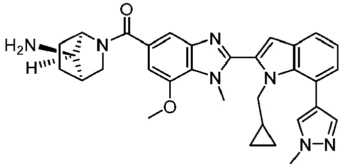

A vial was charged with tert-butyl-((1R,4R,7R)-2-(2-(7-bromo-1-(cyclopropylmethyl)-1H-indol-2-yl)-7-methoxy-1-methyl-1H-benzo[d]imidazole-5-carbonyl)-2-azabicyclo[2.2.1]heptan-7-yl)carbamate (30 mg, 0.046 mmol), 1-methyl-4-(4,4,5,5-tetramethyl-1,3,2-dioxaborolan-2-yl)-1H-pyrazole (9.62 mg, 0.046 mmol), Pd(dppf)Cl₂ (3.38 mg, 4.63 µmol) and dioxane (2 mL). The slurry was sparged with argon for 5 min. 2M K₂HPO₄ (0.060 mL, 0.120 mmol) was added and the vial was capped and heated at 85 °C for 4 hours. LC/MS detects (M+H)⁺ = 650.60 for titled compound. The reaction was filtered and concentrated. The residue was dissolved in dioxane (2 mL) and 4N HCl in dioxane (0.116 mL, 0.463 mmol) was then added thereto. The reaction was stirred for 4 h until LC/MS detected no starting material. The reaction was concentrated 3 times from methylene chloride to remove traces of HCl to yield an amber oil. The crude material was purified via preparative LC/MS with the following conditions: Column: XBridge C18, 19 x 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 16-56% B over 25 minutes, then a 4-minute hold at 100% B; Flow: 20 mL/min. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation. The yield of the titled compound Example 122 was 7.0 mg (26%), and its estimated purity by analytical LC/MS analysis was 97.3% (Method 1) and 98.3% (Method 2). ¹H NMR (500 MHz, DMSO-d6) δ 7.90 (s, 1H), 7.63 (d, J=7.6 Hz, 1H), 7.60 - 7.56 (m, 1H), 7.41 - 7.24 (m, 1H), 7.16 - 7.08 (m, 1H), 7.08 - 7.04 (m, 1H), 7.03 - 6.98 (m, 1H), 6.92 - 6.82 (m, 1H), 4.12 - 4.00 (m, 5H), 3.95 - 3.90 (m, 3H), 3.90 - 3.87 (m, 3H), 3.74 - 3.43 (m, 1H), 3.12 - 3.05 (m, 1H), 3.07 - 2.88 (m, 1H), 2.26 - 2.13 (m, 1H), 2.00 - 1.80 (m, 3H), 1.78 - 1.58 (m, 1H), 1.47 - 1.23 (m, 1H), 1.23 - 1.08 (m, 1H), 0.43 (br d, J=4.9 Hz, 1H), 0.02 - -0.13 (m, 2H), - 0.51 - -0.66 (m, 2H). MS ESI m/z 550.10 (M+H). Analytical LC/MS retention time: 1.51 min (Method 1).

The following compounds in Table 7 can be made by the procedures described in Example 122 using the appropriate starting materials.

**Table 7**

| Ex # | Structure | Name | LC/ MS Rt (min ) | LC/ MS Met hod | M+H (obs ion) |
|---|---|---|---|---|---|
| 123 | 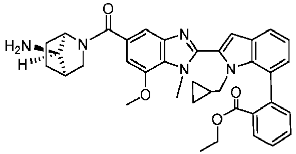 | ethyl 2-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)benzoate | 1.96 | 1 | 618.38 |
| 124 | 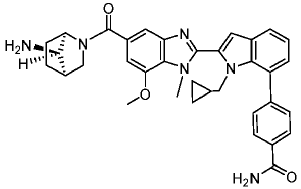 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)benzamide | 1.46 | 1 | 589.35 |
| 125 | 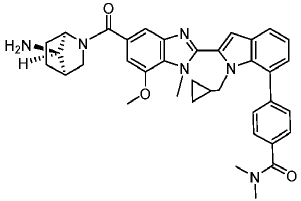 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N,N-dimethylbenzamide | 1.65 | 1 | 617.23 |
| 126 | 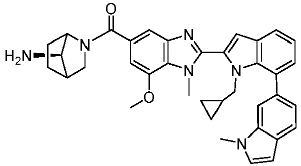 | ((7R)-7-amino-2-azabicyclo[2.2.1]hepta n-2-yl)(2-(1'-(cyclopropylmethyl)-1-methyl-1H,1'H-[6,7'-biindol]-2'-yl)-7-methoxy-1-methyl-1H-benzo[d]imidazol-5-yl)methanone | 2.06 | 1 | 599.47 |
| 127 | 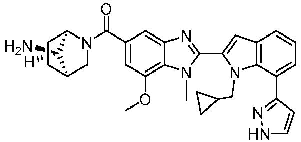 | ((1R,4R,7R)-7-amino-2-azabicyclo[2.2.1]hepta n-2-yl)(2-(1-(cyclopropylmethyl)-7-(1H-pyrazol-3-yl)-1H-indol-2-yl)-7-methoxy-1-methyl-1H-benzo[d]imidazol-5-yl)methanone | 1.49 | 1 | 536.41 |
| 128 | 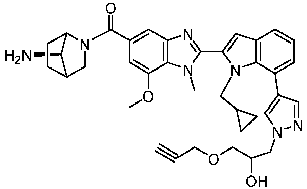 | ((7R)-7-amino-2-azabicyclo[2.2.1]hepta n-2-yl)(2-(1-(cyclopropylmethyl)-7-(1-(2-hydroxy-3-(prop-2-yn-1-yloxy)propyl)-1H-pyrazol-4-yl)-1H-indol-2-yl)-7-methoxy-1-methyl-1H-benzo[d]imidazol-5-yl)methanone | 1.49 | 1 | 648.27 |
| 129 | 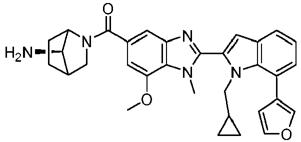 | ((7R)-7-amino-2-azabicyclo[2.2.1]hepta n-2-yl)(2-(1-(cyclopropylmethyl)-7-(furan-3-yl)-1H-indol-2-yl)-7-methoxy-1- methyl-1H-benzo[d]imidazol-5-yl)methanone | 1.87 | 1 | 536.15 |
| 130 | 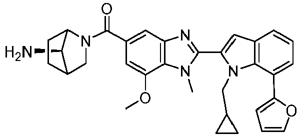 | ((7R)-7-amino-2-azabicyclo[2.2.1]hepta n-2-yl)(2-(1-(cyclopropylmethyl)-7-(furan-2-yl)-1H-indol-2-yl)-7-methoxy-1- methyl-1H-benzo[d]imidazol-5-yl)methanone | 1.91 | 1 | 536.33 |
| 131 | 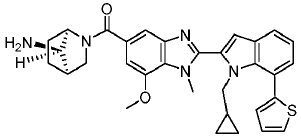 | ((1R,4R,7R)-7-amino-2-azabicyclo[2.2.1]hepta n-2-yl)(2-(1-(cyclopropylmethyl)-7-(thiophen-2-yl)-1H-indol-2-yl)-7-methoxy-1-methyl-1H-benzo[d]imidazol-5-yl)methanone | 2.00 | 1 | 552.35 |
| 132 | 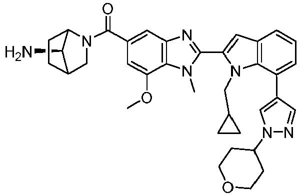 | ((7R)-7-amino-2-azabicyclo[2.2.1]hepta n-2-yl)(2-(1-(cyclopropylmethyl)-7-(1-(tetrahydro-2H-pyran-4-yl)-1H-pyrazol-4-yl)-1H-indol-2-yl)-7-methoxy-1-methyl-1H-benzo[d]imidazol-5-yl)methanone | 1.57 | 1 | 620.42 |
| 133 | 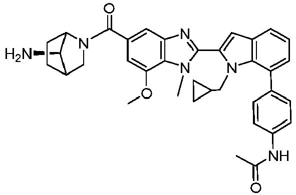 | N-(4-(2-(5-((7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)phenyl)acetamide | 1.71 | 1 | 603.44 |
| 134 | 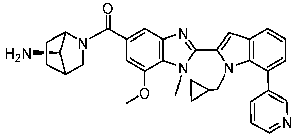 | ((7R)-7-amino-2-azabicyclo[2.2.1]hepta n-2-yl)(2-(1-(cyclopropylmethyl)-7-(pyridin-3-yl)-1H-indol-2-yl)-7-methoxy-1-methyl-1H-benzo[d]imidazol-5-yl)methanone | 1.54 | 1 | 547.21 |
| 135 | 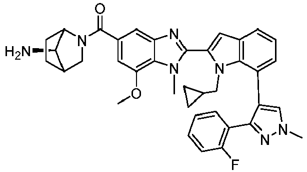 | ((7R)-7-amino-2-azabicyclo[2.2.1]hepta n-2-yl)(2-(1-(cyclopropylmethyl)-7-(3-(2-fluorophenyl)-1-methyl-1H-pyrazol-4-yl)-1H-indol-2-yl)-7-methoxy-1-methyl-1H-benzo[d]imidazol-5-yl)methanone | 1.74 | 1 | 644.40 |
| 136 | 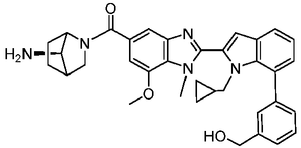 | ((7R)-7-amino-2-azabicyclo[2.2.1]hepta n-2-yl)(2-(1-(cyclopropylmethyl)-7-(3-(hydroxymethyl)phenyl )-1H-indol-2-yl)-7-methoxy-1-methyl-1H-benzo[d]imidazol-5-yl)methanone | 1.68 | 1 | 576.36 |
| 137 | 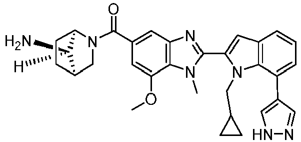 | ((1R,4R,7R)-7-amino-2-azabicyclo[2.2.1]hepta n-2-yl)(2-(1-(cyclopropylmethyl)-7-(1H-pyrazol-4-yl)-1H-indol-2-yl)-7-methoxy-1-methyl-1H-benzo[d]imidazol-5-yl)methanone | 1.43 | 1 | 536.37 |
| 138 | 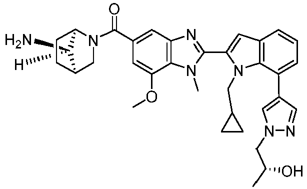 | ((1R,4R,7R)-7-amino-2-azabicyclo[2.2.1]hepta n-2-yl)(2-(1-(cyclopropylmethyl)-7-(1-((R)-2-hydroxypropyl)-1H-pyrazol-4-yl)-1H-indol-2-yl)-7-methoxy-1-methyl-1H-benzo[d]imidazol-5-yl)methanone | 1.51 | 1 | 593.98 |
| 139 | 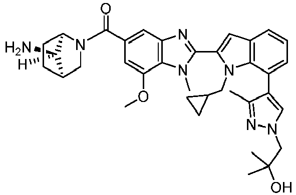 | ((1R,4R,7R)-7-amino-2-azabicyclo[2.2.1]hepta n-2-yl)(2-(1-(cyclopropylmethyl)-7-(1-(2-hydroxy-2-methylpropyl)-3-methyl-1H-pyrazol-4-yl)-1H-indol-2-yl)-7-methoxy-1-methyl-1H-benzo[d]imidazol-5-yl)methanone | 1.62 | 1 | 622.27 |
| 140 | 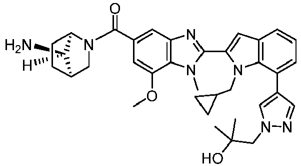 | ((1R,4R,7R)-7-amino-2-azabicyclo[2.2.1]hepta n-2-yl)(2-(1-(cyclopropylmethyl)-7-(1-(2-hydroxy-2-methylpropyl)-1H-pyrazol-4-yl)-1H-indol-2-yl)-7-methoxy-1-methyl-1H-benzo[d]imidazol-5-yl)methanone | 1.59 | 1 | 608.28 |
| 141 | 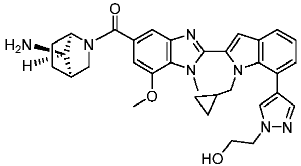 | ((1R,4R,7R)-7-amino-2-azabicyclo[2.2.1]hepta n-2-yl)(2-(1-(cyclopropylmethyl)-7-(1-(2-hydroxyethyl)-1H-pyrazol-4-yl)-1H-indol-2-yl)-7-methoxy-1-methyl-1H-benzo[d]imidazol-5-yl)methanone | 1.45 | 1 | 580.35 |
| 142 | 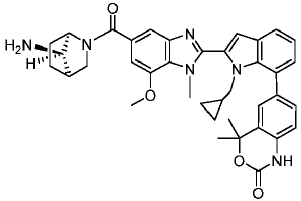 | 6-(2-(5-((1R,4S)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-4,4-dimethyl-1,4-dihydro-2H-benzo[d][1,3]oxazin-2-one | 1.55 | 2 | 645.46 |
| 143 | 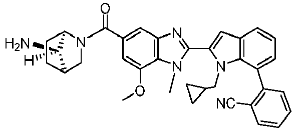 | 2-(2-(5-((1R,4S)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)benzonitrile | 1.75 | 1 | 571.29 |

The following compounds in Table 8 can be synthesized by the methods discussed in Examples 1 and/or 122 using the appropriate starting materials.

**Table 8**

| Ex # | Structure | Name | LC/ MS Rt (min ) | LC/ MS Met hod | M+H (obs ion) |
|---|---|---|---|---|---|
| 149 | 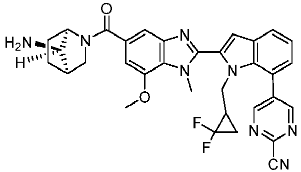 | 5-(2-(5-((1R,4R,7R)-7-amino-2-azabicyclo[2.2.1]hepta ne-2-carbonyl)-7-methoxy-1-methyl-1H-benzo[d]imidazol-2-yl)-1-((2,2-difluorocyclopropyl)me thyl)-1H-indol-7-yl)pyrimidine-2-carbonitrile | 1.64 | 1 | 609.12 |
| 150 | 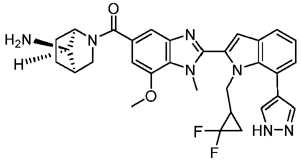 | ((1R,4R,7R)-7-amino-2-azabicyclo[2.2.1]hepta n-2-yl)(2-(1-((2,2-difluorocyclopropyl)me thyl)-7-(1H-pyrazol-4-yl)-1H-indol-2-yl)-7-methoxy-1-methyl-1H-benzo[d]imidazol-5-yl)methanone | 1.53 | 1 | 572.32 |
| 151 | 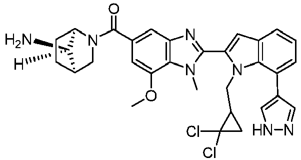 | ((1R,4R,7R)-7-amino-2-azabicyclo[2.2.1]hepta n-2-yl)(2-(1-((2,2-dichlorocyclopropyl)m ethyl)-7-(1H-pyrazol-4-yl)-1H-indol-2-yl)-7-methoxy-1-methyl-1H-benzo[d]imidazol-5-yl)methanone | 1.51 | 1 | 604.39 |
| 152 | 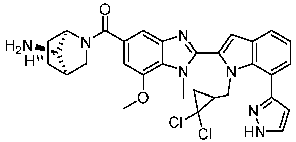 | ((1R,4R,7R)-7-amino-2-azabicyclo[2.2.1]hepta n-2-yl)(2-(1-((2,2-dichlorocyclopropyl)m ethyl)-7-(1H-pyrazol-3-yl)-1H-indol-2-yl)-7-methoxy-1-methyl-1H-benzo[d]imidazol-5-yl)methanone | 1.72 | 1 | 604.25 |
| 154 | 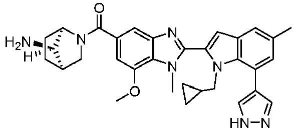 | ((1R,4R,7R)-7-amino-2-azabicyclo[2.2.1]hepta n-2-yl)(2-(1-(cyclopropylmethyl)-5-methyl-7-(1H-pyrazol-4-yl)-1H-indol-2-yl)-7-methoxy-1-methyl-1H-benzo[d]imidazol-5-yl)methanone | 1.76 | 1 | 550.21 |
| 155 | 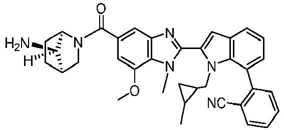 | 2-(2-(5-((1R,4R,7R)-7-amino-2-azabicyclo[2.2.1]hepta ne-2-carbonyl)-7-methoxy-1-methyl-1H-benzo[d]imidazol-2-yl)-1-((2-methylcyclopropyl)met hyl)-1H-indol-7-yl)benzonitrile | 1.95 | 1 | 585.19 |
| 156 | 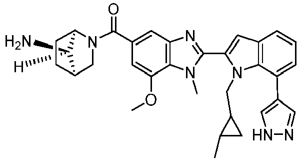 | ((1R,4R,7R)-7-amino-2-azabicyclo[2.2.1]hepta n-2-yl)(7-methoxy-1-methyl-2-(1-((2-methylcyclopropyl)methyl)-7-(1H-pyrazol-4-yl)-1H-indol-2-yl)-1H-benzo[d]imidazol-5-yl)methanone | 1.61 | 1 | 550.18 |
| 157 | 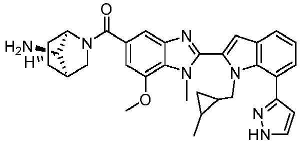 | ((1R,4R,7R)-7-amino-2-azabicyclo[2.2.1]hepta n-2-yl)(7-methoxy-1-methyl-2-(1-((2-methylcyclopropyl)met hyl)-7-(1H-pyrazol-3-yl)-1H-indol-2-yl)-1H-benzo[d]imidazol-5-yl)methanone | 1.70 | 1 | 550.12 |

### Example 181

### ((1R,4R,7R)-7-amino-2-azabicyclo[2.2.1]heptan-2-yl)(2-(1-(cyclopropylmethyl)-7-(2-(hydroxymethyl)phenyl)-1H-indol-2-yl)-7-methoxy-1-methyl-1H-benzo[d]imidazol-5-yl)methanone

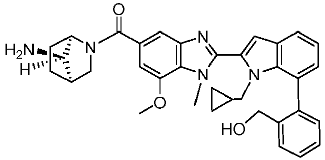

To a solution of ethyl 2-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)benzoate (20 mg, 0.032 mmol) in anhydrous THF (3 mL) at 0 °C was added 2N lithium borohydride in THF (0.016 mL, 0.032 mmol). The reaction was stirred for 16 hours at room temperature. LC/MS detects incomplete reaction and therefore more 2N lithium borohydride in THF (0.016 mL, 0.032 mmol) as above. After 1 hour, LC/MS showed reaction had progressed about 60%. At this point, the reaction was quenched under nitrogen with the slow addition of a few drops of saturated aqueous NH₄Cl solution. Workup entailed extracting the aqueous 3 times from methylene chloride, drying over Na₂SO₄ and evaporating to obtain an amber oil. The crude material was purified via preparative LC/MS with the following conditions: Column: XBridge C18, 19 x 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 24-64% B over 20 minutes, then a 4-minute hold at 100% B; Flow: 20 mL/min. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation. The yield of the product was 2.3 mg, and its estimated purity by LCMS analysis was 88%. The yield of the product was 2.3 mg (11%), and its estimated purity by analytical LC/MS analysis was 95.1% (Method 1) and 87.6 % (Method 2). ¹H NMR (500 MHz, DMSO-d6) δ 7.69 (br d, J=7.9 Hz, 1H), 7.60 (br d, J=7.6 Hz, 1H), 7.45 (br t, J=7.2 Hz, 1H), 7.35 - 7.25 (m, 3H), 7.17 (br t, J=7.5 Hz, 1H), 7.11 - 7.06 (m, 1H), 6.98 - 6.93 (m, 1H), 6.91 - 6.85 (m, 1H), 4.28 - 4.21 (m, 1H), 4.15 - 4.07 (m, 1H), 4.07 - 4.00 (m, 3H), 3.93 (s, 3H), 3.80 - 3.68 (m, 1H), 3.61 - 3.51 (m, 1H), 3.54 (br s, 1H), 3.06 - 2.94 (m, 1H), 2.87 - 2.65 (m, 2H), 2.23 - 2.10 (m, 1H), 1.96 - 1.84 (m, 2H), 1.75 - 1.56 (m, 1H), 1.47 - 1.35 (m, 1H), 1.02 - 0.86 (m, 1H), 0.83 - 0.75 (m, 1H), 0.33 (br s, 1H), -0.10 (br d, J=6.1 Hz, 3H), -0.55 - -0.69 (m, 1H), -0.90 (br s, 1H). MS ESI m/z 576.4 (M+H). Analytical LC/MS retention time: 1.91 (Method 1).

The following compounds in Table 12 can be synthesized by the methods discussed heretofore using the appropriate starting materials.

**Table 12**

| Ex # | Structure | Name | LC/ MS Rt (min ) | LC/ MS Met hod | M+H (obs ion) |
|---|---|---|---|---|---|
| 186 | 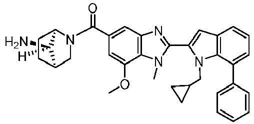 | ((1R,4R,7R)-7-amino-2-azabicyclo[2.2.1]heptan-2-yl)(2-(1-(cyclopropylmethyl)-7-phenyl-1H-indol-2-yl)-7-methoxy-1-methyl-1H-benzo[d]imidazol-5-yl)methanonemethyl-1H-benzo[d]imidazol-5-yl)methanone | 2.00 | 1 | 546.0 |

### Example 187

### 4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-phenylpiperidine-1-carboxamide

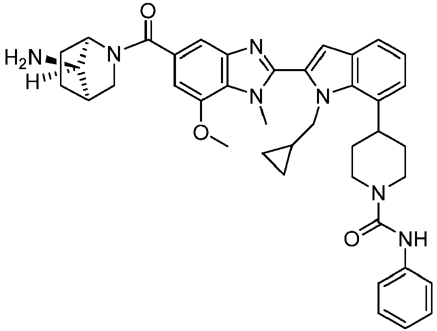

A stirring solution of tert-butyl ((7R)-2-(2-(1-(cyclopropylmethyl)-7-(piperidin-4-yl)-1H-indol-2-yl)-7-methoxy-1-methyl-1H-benzo[d]imidazole-5-carbonyl)-2-azabicyclo[2.2.1]heptan-7-yl)carbamate (15 mg, 0.023 mmol) in dichloromethane (1 mL) was treated with phenyl isocyanate (2.51 µl, 0.023 mmol). The reaction was stirred at room temperature for 2 hours, at which point it was judged to be complete by LCMS. The mixture was treated with 4M HCl in dioxane (1 mL), and the reaction was stirred at room temperature for 1 hour, at which point it was judged to be complete by LCMS. The mixture was conecntrated in vacuo, and the crude material was purified via preparative LC/MS with the following conditions: Column: XBridge C18, 200 mm x 19 mm, 5-µm particles; Mobile Phase A: 5:95 acetonitrile: water with 0.05% trifluoroacetic acid; Mobile Phase B: 95:5 acetonitrile: water with 0.05% trifluoroacetic acid; Gradient: a 0-minute hold at 20% B, 20-60% B over 23 minutes, then a 0-minute hold at 100% B; Flow Rate: 20 mL/min; Column Temperature: 25 C. Fraction collection was triggered by MS signals. Fractions containing the desired product were combined and dried via centrifugal evaporation. The purified material was then diluted with DMF, treated with Si-Pyridine and shaken for a minimum of 2 h. The resulting mixture was filtered and dried via centrifugal evaporation.

The yield of the product was 13.6 mg, and its estimated purity by LCMS analysis was 100%. Analytical LC/MS was used to determine the final purity.

Proton NMR was acquired in deuterated DMSO and shows multiple conformers. ¹H NMR (500 MHz, DMSO-δ6) δ 8.57 - 8.52 (m, 1H), 7.53 - 7.33 (m, 4H), 7.27 - 7.13 (m, 4H), 7.12 - 7.06 (m, 1H), 7.06 - 6.96 (m, 1H), 6.96 - 6.87 (m, 2H), 4.55 - 3.87 (m, 10H), 3.62 - 3.39 (m, 1H), 3.23 - 3.07 (m, 1H), 3.02 - 2.90 (m, 2H), 2.66 - 2.52 (m, 1H), 2.03 - 1.52 (m, 9H), 1.24 - 1.11 (m, 1H), 0.95 - 0.82 (m, 1H), 0.27 - 0.15 (m, 2H), -0.29 - -0.45 (m, 2H). MS ESI m/z = 670.2 (M+H). HPLC retention time 1.91 minutes, Method 1.

### Example 188

### 4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N,N-dimethylpiperidine-1-carboxamide

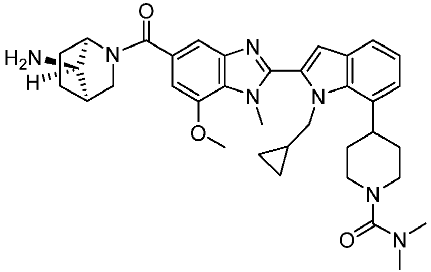

A stirring solution of tert-butyl ((7R)-2-(2-(1-(cyclopropylmethyl)-7-(piperidin-4-yl)-1H-indol-2-yl)-7-methoxy-1-methyl-1H-benzo[d]imidazole-5-carbonyl)-2-azabicyclo[2.2.1]heptan-7-yl)carbamate (20 mg, 0.031 mmol) in dichloromethane (1 mL) was treated with triethylamine (4.3 µL, 0.031 mmol) and dimethylcarbamic chloride (2.8 µL, 0.031 mmol) at rt. After 10 minutes, LCMS had detected product. 4N HCl in dioxane was added thereto (153 µL, 0.61 mmol) and the mixture stirred at rt. After 30 minutes, the reaction was judged to be complete by LCMS. The mixture was evaporated, and the crude material was purified via preparative LC/MS with the following conditions: Column: XBridge C18, 200 mm x 19 mm, 5-µm particles; Mobile Phase A: 5:95 acetonitrile: water with 0.05% trifluoroacetic acid; Mobile Phase B: 95:5 acetonitrile: water with 0.05% trifluoroacetic acid; Gradient: a 0-minute hold at 15% B, 15-55% B over 20 minutes, then a 0-minute hold at 100% B; Flow Rate: 20 mL/min; Column Temperature: 25 C. Fraction collection was triggered by MS and UV signals. Fractions containing the desired product were combined and dried via centrifugal evaporation.

The purified material was then diluted with DMF, treated with Si-Pyridine and shaken for a minimum of 2 h. The resulting mixture was filtered and dried via centrifugal evaporation.

The yield of the product was 12.1 mg, and its estimated purity by LCMS analysis was 95%. Analytical LC/MS was used to determine the final purity.

Proton NMR was acquired in deuterated DMSO and shows multiple conformers. ¹H NMR (500 MHz, DMSO-δ₆) δ 7.57 - 7.47 (m, 1H), 7.47 - 7.32 (m, 1H), 7.19 - 7.15 (m, 1H), 7.12 - 7.06 (m, 1H), 7.00 - 6.97 (m, 1H), 6.95 - 6.88 (m, 1H), 4.55 - 3.86 (m, 8H), 3.78 - 3.64 (m, 1H), 3.55 - 3.41 (m, 1H), 3.16 - 3.07 (m, 1H), 2.91 - 2.80 (m, 2H), 2.79 - 2.65 (m, 7H), 2.64 - 2.52 (m, 1H), 2.02 - 1.51 (m, 9H), 1.28 - 1.11 (m, 1H), 0.93 - 0.83 (m, 1H), 0.26 - 0.15 (m, 2H), -0.27 - -0.44 (m, 2H). MS ESI m/z = 624.0 (M+H). HPLC retention time 1.88 minutes, Method 1.

### Example 189

### 1-(4-(2-(5-((3R,5R)-3-amino-5-fluoropiperidine-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)piperidin-1-yl)ethan-1-one

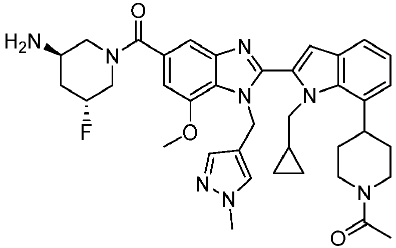

### Intermediate 189A: methyl 2-(7-bromo-1-(cyclopropylmethyl)-1H-indol-2-yl)-7-methoxy-1-((1-methyl-1H-pyrazol-4-yl)methyl)-1H-benzo[d]imidazole-5-carboxylate

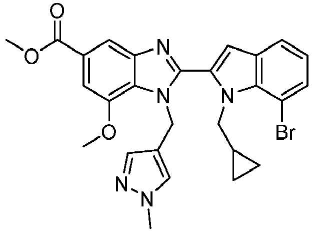

To a solution of 7-bromo-1-(cyclopropylmethyl)-1H-indole-2-carbaldehyde (350 mg, 1.258 mmol) and methyl 3-methoxy-4-(((1-methyl-1H-pyrazol-4-yl)methyl)amino)-5-nitrobenzoate (403 mg, 1.258 mmol) in EtOH (8.0 mL) was added a solution of sodium dithionite (657 mg, 3.77 mmol) in water (4.00 mL), the mixture was stirred at 80 °C for 6 hour. The mixture was diluted with EtOAc (25 mL) and was washed with a solution of aqueous saturated sodium bicarbonate (2 x 25 mL). The ethyl acetate layer was dried over sodium sulfate and concentrated. The crude product was subjected to ISCO flash chromatography (silica gel/hexane-EtOAc 100:0 to 0:100 gradient). Yield methyl 2-(7-bromo-1-(cyclopropylmethyl)-1H-indol-2-yl)-7-methoxy-1-((1-methyl-1H-pyrazol-4-yl)methyl)-1H-benzo[d]imidazole-5-carboxylate (409 mg, 0.708 mmol, 56.3 % yield) off-white foam. ¹H NMR (499 MHz, CHLOROFORM-d) δ 8.22 (d, *J*=1.2 Hz, 1H), 7.66 (dd, *J*=7.9, 1.0 Hz, 1H), 7.55 - 7.53 (m, 2H), 7.28 (s, 1H), 7.08 - 6.99 (m, 2H), 6.84 (s, 1H), 5.56 (s, 2H), 4.63 (d, *J*=6.9 Hz, 2H), 4.09 (s, 3H), 3.99 (s, 3H), 3.81 (s, 3H), 1.15 - 1.03 (m, 1H), 0.24 - 0.17 (m, 2H), -0.07 - -0.11 (m, 2H). LC/MS (M+H): 549; LC retention time: 1.07 min (analytical HPLC Method B).

### Intermediate 189B: methyl 2-(1-(cyclopropylmethyl)-7-(piperidin-4-yl)-1H-indol-2-yl)-7-methoxy-1-((1-methyl-1H-pyrazol-4-yl)methyl)-1H-benzo[d]imidazole-5-carboxylate

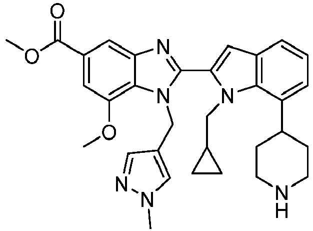

A stirring mixture of methyl 2-(7-bromo-1-(cyclopropylmethyl)-1H-indol-2-yl)-7-methoxy-1-((1-methyl-1H-pyrazol-4-yl)methyl)-1H-benzo[d]imidazole-5-carboxylate (49.0 mg, 0.089 mmol), tris(trimethylsilyl)silane (0.055 mL, 0.179 mmol), Ir(dF(CF3)ppy)2(dtbbpy)PF6 (3.01 mg, 2.68 µmol), tert-butyl 4-bromopiperidine-1-carboxylate (47.2 mg, 0.179 mmol) and sodium carbonate (37.9 mg, 0.357 mmol) in 1,4-dioxane (2 mL) was degassed with bubbling nitrogen for 10 minutes. In a separate vial, a stirring mixture of nickel (II) chloride ethylene glycol dimethyl ether complex (2.94 mg, 0.013 mmol), and 4,4'-di-tert-butyl-2,2'-bipyridine (4.08 mg, 0.015 mmol) in 1,4-dioxane (1 mL) was degassed with nitrogen for 15 minutes. The nickel complex was transferred to the vial containing the other mixture and the reaction was stirred at room temperature under a blue Kessil lamp for 18 hours. The mixture was diluted with EtOAc (10 mL) and was washed with a solution of aqueous saturated sodium bicarbonate (2 x 10 mL). The ethyl acetate layer was dried over sodium sulfate and concentrated to give crude methyl 2-(7-(1-(tert-butoxycarbonyl)piperidin-4-yl)-1-(cyclopropylmethyl)-1H-indol-2-yl)-7-methoxy-1-((1-methyl-1H-pyrazol-4-yl)methyl)-1H-benzo[d]imidazole-5-carboxylate. The crude methyl 2-(7-(1-(tert-butoxycarbonyl)piperidin-4-yl)-1-(cyclopropylmethyl)-1H-indol-2-yl)-7-methoxy-1-((1-methyl-1H-pyrazol-4-yl)methyl)-1H-benzo[d]imidazole-5-carboxylate in DCM (1.0 mL) and TFA (1.0 mL) was stirred at RT for 30 min. The mixture was concentrated. The crude product was purified by prep-HPLC (Phenomenex, Luna 5 micron 30 x 250 mm, flow rate = 30 ml/min., gradient = 20% A to 100%B in 30 min., A =H2O/ ACN/TFA(90:10:0.1), B = H2O/ ACN/TFA(10:90:0.1)). Yield methyl 2-(1-(cyclopropylmethyl)-7-(piperidin-4-yl)-1H-indol-2-yl)-7-methoxy-1-((1-methyl-1H-pyrazol-4-yl)methyl)-1H-benzo[d]imidazole-5-carboxylate (12 mg, 0.021 mmol, 23.09 % yield) as clear gum. LC/MS (M+H): 553; LC retention time: 0.74 min (analytical HPLC Method B).

### Intermediate 189C: 2-(7-(1-acetylpiperidin-4-yl)-1-(cyclopropylmethyl)-1H-indol-2-yl)-7-methoxy-1-((1-methyl-1H-pyrazol-4-yl)methyl)-1H-benzo[d]imidazole-5-carboxylic acid

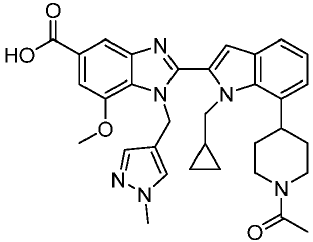

To a solution of methyl 2-(1-(cyclopropylmethyl)-7-(piperidin-4-yl)-1H-indol-2-yl)-7-methoxy-1-((1-methyl-1H-pyrazol-4-yl)methyl)-1H-benzo[d]imidazole-5-carboxylate (12 mg, 0.022 mmol) and TEA (15.13 µl, 0.109 mmol) in THF (1.0 mL) was added acetic anhydride (2.049 µl, 0.022 mmol), the mixture was stirred at RT for 1 hours. The mixture was diluted with EtOAc (5 mL) and was washed with a solution of aqueous saturated sodium bicarbonate (2 x 5 mL). The ethyl acetate layer was dried over sodium sulfate and concentrated to give crude methyl 2-(7-(1-acetylpiperidin-4-yl)-1-(cyclopropylmethyl)-1H-indol-2-yl)-7-methoxy-1-((1-methyl-1H-pyrazol-4-yl)methyl)-1H-benzo[d]imidazole-5-carboxylate.
A mixture of methyl 2-(7-(1-acetylpiperidin-4-yl)-1-(cyclopropylmethyl)-1H-indol-2-yl)-7-methoxy-1-((1-methyl-1H-pyrazol-4-yl)methyl)-1H-benzo[d]imidazole-5-carboxylate and 1.0 M aqueous sodium hydroxide (109 µl, 0.109 mmol) in MeOH (2.0 mL) was stirred at 50 °C for 3 hours. The mixture was cooled to RT. A solution of 1.0 M aqueous HCl (0.10 mL) was added and the mixture was concentrated to give crude 2-(7-(1-acetylpiperidin-4-yl)-1-(cyclopropylmethyl)-1H-indol-2-yl)-7-methoxy-1-((1-methyl-1H-pyrazol-4-yl)methyl)-1H-benzo[d]imidazole-5-carboxylic acid (14 mg, 0.019 mmol, 89 % yield) as white solid. LC/MS (M+H): 581; LC retention time: 0.78 min (analytical HPLC Method B).

### Example 189: 1-(4-(2-(5-((3R,5R)-3-amino-5-fluoropiperidine-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)piperidin-1-yl)ethan-1-one

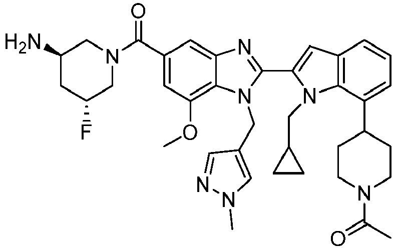

A mixture of 2-(7-(1-acetylpiperidin-4-yl)-1-(cyclopropylmethyl)-1H-indol-2-yl)-7-methoxy-1-((1-methyl-1H-pyrazol-4-yl)methyl)-1H-benzo[d]imidazole-5-carboxylic acid (14 mg, 0.024 mmol), tert-butyl ((3R,5R)-5-fluoropiperidin-3-yl)carbamate (5.26 mg, 0.024 mmol), BOP (10.66 mg, 0.024 mmol) and TEA (16.80 µl, 0.121 mmol) in DMF (1.0 mL) was stirred at RT for 2 hours. The mixture was diluted with EtOAc (5 mL) and was washed with a solution of aqueous saturated sodium bicarbonate (2 x 5 mL). The ethyl acetate layer was dried over sodium sulfate and concentrated to give tert-butyl ((3R,5R)-1-(2-(7-(1-acetylpiperidin-4-yl)-1-(cyclopropylmethyl)-1H-indol-2-yl)-7-methoxy-1-((1-methyl-1H-pyrazol-4-yl)methyl)-1H-benzo[d]imidazole-5-carbonyl)-5-fluoropiperidin-3-yl)carbamate. LC/MS (M+H): 781; LC retention time: 0.84 min (analytical HPLC Method B).
A mixture of tert-butyl ((3R,SR)-1-(2-(7-(1-acetylpiperidin-4-yl)-1-(cyclopropylmethyl)-1H-indol-2-yl)-7-methoxy-1-((1-methyl-1H-pyrazol-4-yl)methyl)-1H-benzo[d]imidazole-5-carbonyl)-5-fluoropiperidin-3-yl)carbamate in DCM (1.0 mL) and TFA (1.0 mL) was stirred at RT for 30 min. The mixture was concentrated. The crude product was purified by XBridge C18, 200 mm x 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 14% B, 14-54% B over 20 minutes, then a 4-minute hold at 100% B; Flow Rate: 20 mL/min; Column Temperature: 25 C. Yield 1-(4-(2-(5-((3R,SR)-3-amino-5-fluoropiperidine-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)piperidin-1-yl)ethan-1-one (6.10 mg, 8.84 µmol, 36.7 % yield). ¹H NMR (500 MHz, DMSO-d₆) δ 7.81 - 7.75 (m, 1H), 7.64 - 7.58 (m, 1H), 7.55 - 7.50 (m, 1H), 7.45 - 7.39 (m, 1H), 7.38 - 7.31 (m, 1H), 7.28 - 7.23 (m, 1H), 7.22 - 7.17 (m, 1H), 7.13 - 7.08 (m, 1H), 5.75 - 5.68 (m, 2H), 5.25 - 4.99 (m, 1H), 4.86 - 4.78 (m, 1H), 4.56 - 4.48 (m, 2H), 4.25 - 4.15 (m, 3H), 3.95 - 3.89 (m, 2H), 3.86 - 3.76 (m, 1H), 3.49 - 3.37 (m, 1H), 3.28 - 3.18 (m, 1H), 3.15 - 2.92 (m, 1H), 2.93 - 2.84 (m, 1H), 2.45 - 2.32 (m, 1H), 2.29 - 2.14 (m, 5H), 2.14 - 2.07 (m, 1H), 2.07 - 1.93 (m, 2H), 1.90 - 1.62 (m, 3H), 1.53 - 1.40 (m, 1H), 1.26 - 1.03 (m, 2H), 0.42 - 0.32 (m, 2H), 0.06 - -0.05 (m, 2H). LC/MS (M+H): 681; LC retention time: 1.19 min (analytical HPLC Method 2).

The following compounds in Table 13 can be synthesized by the methods discussed heretofore using the appropriate starting materials.

**Table 13**

| Ex # | Structure | Name | LC/ MS Rt (min ) | LC/ MS Met hod | M+H (obs ion) |
|---|---|---|---|---|---|
| 190 | 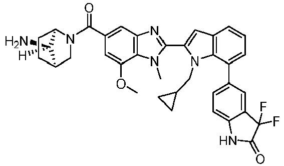 | 5-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-3,3-difluoroindolin-2-one | 1.85 | 1.55 | 637.3 |
| 191 | 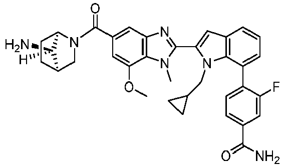 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmEthyl)-1H-indol-7-yl)-3-fluorobenzamide | 1.55 | 1.30 | 607.1 |
| 192 | 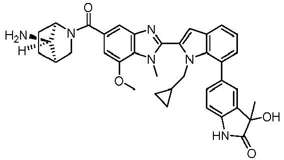 | 5-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-3-hydroxy-3-methylindolin-2-one | 1.47 | 1.32 | 631.2 |
| 193 | 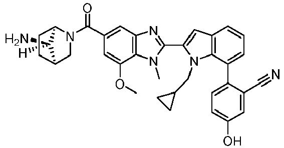 | 2-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-5-hydroxybenzonitrile | 1.62 | 1.42 | 587.4 |
| 194 | 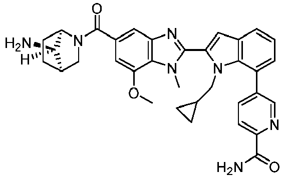 | 5-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)picolinamide | 1.51 | 1.26 | 590.3 |
| 195 | 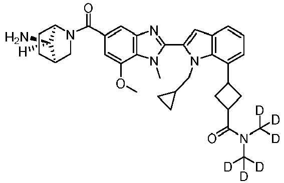 | 3-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N,N-bis(methyl-d3)cyclobutane-1-carboxamide, isomer 1 | 1.31 | 1.38 | 601.5 |
| 196 | 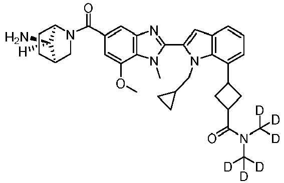 | 3-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N,N-bis(methyl-d3)cyclobutane-1-carboxamide, isomer 2 | 1.54 | 1.62 | 601.4 |
| 197 | 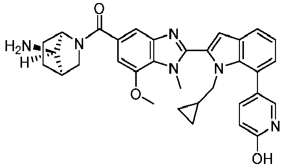 | ((1R,4R,7R)-7-amino-2-azabicyclo[2.2.1]hepta n-2-yl)(2-(1-(cyclopropylmethyl)-7-(6-hydroxypyridin-3-yl)-1H-indol-2-yl)-7-methoxy-1-methyl-1H-benzo[d]imidazol-5-yl)methanone | 1.34 | 1.12 | 563.4 |
| 198 | 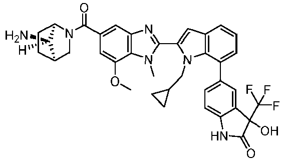 | 5-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-3-hydroxy-3-(trifluoromethyl)indoli n-2-one | 1.69 | 1.41 | 685.2 |
| 199 | 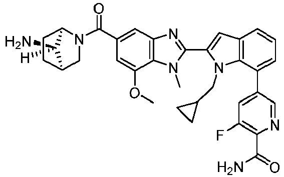 | 5-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-3-fluoropicolinamide | 1.58 | 1.40 | 608.2 |
| 200 | 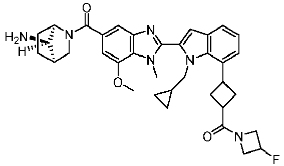 | (3-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)cyclobutyl)(3-fluoroazetidin-1-yl)methanone, Isomer 1 | 1.66 | 1.40 | 625.3 |
| 201 | 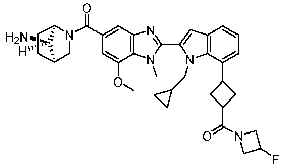 | (3-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)cyclobutyl)(3-fluoroazetidin-1-yl)methanone, Isomer 2 | 1.74 | 1.47 | 625.1 |
| 202 | 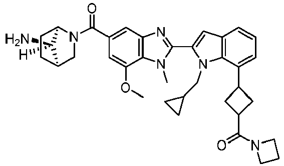 | (3-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)cyclobutyl)(azetidin -1-yl)methanone, Isomer 1 | 1.62 | 1.28 | 607.4 |
| 203 | 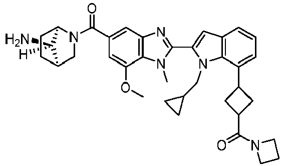 | (3-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)cyclobutyl)(azetidin -1-yl)methanone, Isomer 2 | 1.71 | 1.36 | 607.2 |
| 204 | 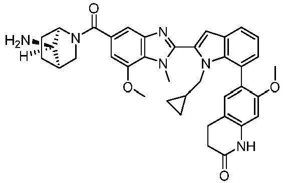 | 6-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-7-methoxy-3,4-dihydroquinolin-2(1H)-one | 1.62 | 1.39 | 645.4 |
| 205 | 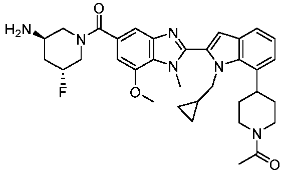 | 1-(4-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)piperidin-1-yl)ethan-1-one | 1.65 | 1.31 | 601.3 |
| 206 | 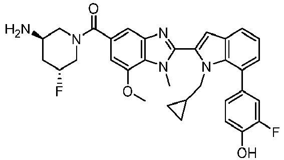 | ((3R,5R)-3-amino-5-fluoropiperidin-1-yl)(2-(1-(cyclopropylmethyl)-7-(3-fluoro-4-hydroxyphenyl)-1H-indol-2-yl)-7-methoxy-1-methyl-1H-benzo[d]imidazol-5-yl)methanone | 1.89 | 1.46 | 586.1 |
| 207 | 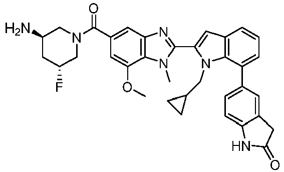 | 5-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)indolin-2-one | 1.69 | 1.35 | 607.2 |
| 208 | 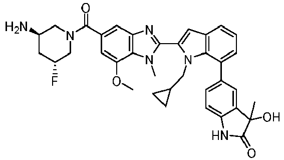 | 5-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-3-hydroxy-3-methylindolin-2-one | 1.61 | 1.28 | 637.2 |
| 209 | 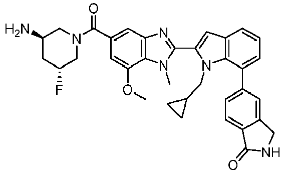 | 5-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)isoindolin-1-one | 1.62 | 1.42 | 607.0 |
| 210 | 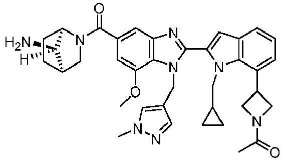 | 1-(3-(2-(5-((1R,4R,7R)-7-amino-2-azabicyclo[2.2.1]hepta ne-2-carbonyl)-7-methoxy-1-((1-methyl-1H-pyrazol-4-yl)methyl)-1H-benzo[d]imidazol-2-yl)-1-(cyclopropylmethyl)-1H-indol-7-yl)azetidin-1-yl)ethan-1-one | 1.37 | 1.10 | 647.2 |
| 211 | 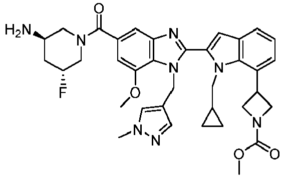 | methyl 3-(2-(5-((3R,5R)-3-amino-5-fluoropiperidine-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)azetidine-1-carboxylate | 1.49 | 1.35 | 669.5 |
| 212 | 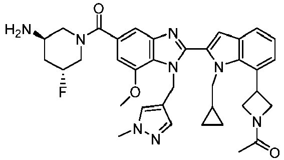 | 1-(3-(2-(5-((3R,5R)-3-amino-5-fluoropiperidine-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)azetidin-1-yl)ethan-1-one | 1.13 | 1.37 | 653.4 |
| 213 | 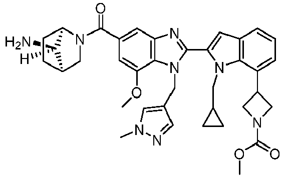 | methyl 3-(2-(5-((1R,4R,7R)-7-amino-2-azabicyclo[2.2.1]hepta ne-2-carbonyl)-7-methoxy-1-((1-methyl-1H-pyrazol-4-yl)methyl)-1H-benzo[d]imidazol-2-yl)-1-(cyclopropylmethyl)-1H-indol-7-yl)azetidine-1-carboxylate | 1.55 | 1.37 | 663.1 |
| 214 | 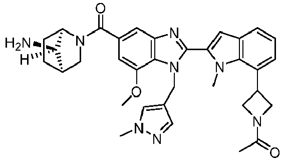 | 1-(3-(2-(5-((1R,4R,7R)-7-amino-2-azabicyclo[2.2.1]hepta ne-2-carbonyl)-7-methoxy-1-((1-methyl-1H-pyrazol-4-yl)methyl)-1H-benzo[d]imidazol-2-yl)-1-methyl-1H-indol-7-yl)azetidin-1-yl)ethan-1-one | 1.16 | 1.24 | 607.0 |
| 215 | 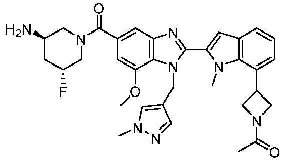 | 1-(3-(2-(5-((3R,5R)-3-amino-5-fluoropiperidine-1-carbonyl)-7-methoxy-1-((1-methyl-1H-pyrazol-4-yl)methyl)-1H-benzo[d]imidazol-2-yl)-1-methyl-1H-indol-7-yl)azetidin-1-yl)ethan-1-one | 1.24 | 1.17 | 613.2 |
| 216 | 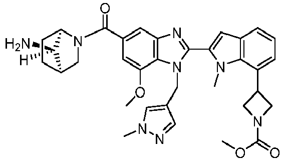 | methyl 3-(2-(5-((1R,4R,7R)-7-amino-2-azabicyclo[2.2.1]hepta ne-2-carbonyl)-7-methoxy-1-((1-methyl-1H-pyrazol-4-yl)methyl)-1H-benzo[d]imidazol-2-yl)-1-methyl-1H-indol-7-yl)azetidine-1- carboxylate | 1.45 | 1.32 | 623.2 |
| 217 | 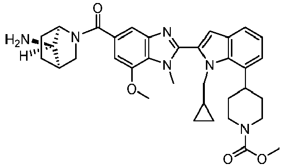 | methyl 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidine-1-carboxylate | 1.83 | 1.58 | 611.2 |
| 218 | 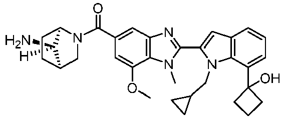 | ((1R,4R,7R)-7-amino-2-azabicyclo[2.2.1]hepta n-2-yl)(2-(1-(cyclopropylmethyl)-7-(1-hydroxycyclobutyl)-1H-indol-2-yl)-7-methoxy-1-methyl-1H-benzo[d]imidazol-5-yl)methanone | 1.74 | 1.40 | 540.2 |
| 220 | 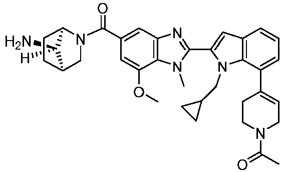 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-3,6-dihydropyridin-1(2H)-yl)ethan-1-one | 1.43 | 1.60 | 593.2 |
| 221 | 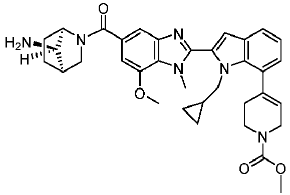 | methyl 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-3,6-dihydropyridine-1(2H)-carboxylate | 1.76 | 1.52 | 609.2 |
| 222 | 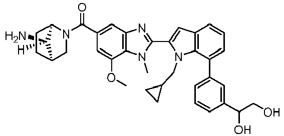 | ((1R,4R,7R)-7-amino-2-azabicyclo[2.2.1]hepta n-2-yl)(2-(1-(cyclopropylmethyl)-7-(3-(1,2-dihydroxyethyl)phenyl )-1H-indol-2-yl)-7-methoxy-1-methyl-1H-benzo[d]imidazol-5-yl)methanone | 1.71 | 1.44 | 606.2 |
| 223 | 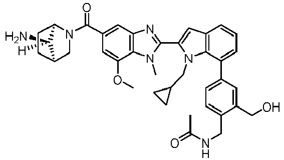 | N-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-2-(hydroxymethyl)benzy l)acetamide | 1.57 | 1.38 | 647.3 |
| 224 | 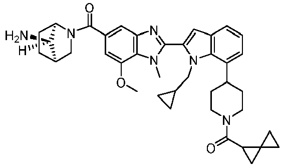 | (4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)(spiro[2.2]pentan-1-yl)methanone | 1.94 | 1.68 | 647.2 |
| 225 | 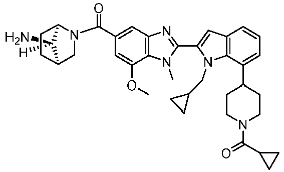 | (4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)(cyclopropyl)metha none | 1.77 | 1.51 | 621.3 |
| 226 | 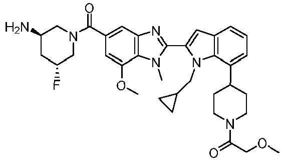 | 1-(4-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)piperidin-1-yl)-2-methoxyethan-1-one | 1.62 | 1.42 | 631.4 |
| 227 | 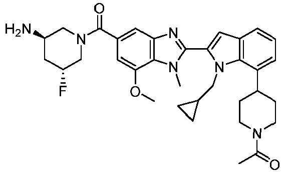 | 1-(4-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)piperidin-1-yl)ethan-1-one | 1.61 | 1.38 | 601.1 |
| 228 | 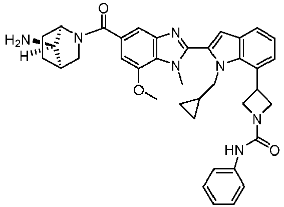 | 3-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-phenylazetidine-1-carboxamide | 1.73 | 1.56 | 644.0 |
| 229 | 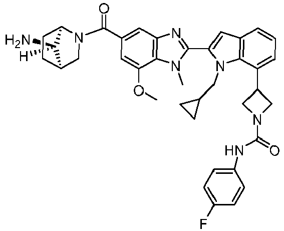 | 3-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-(4-fluorophenyl)azetidine -1-carboxamide | 1.78 | 1.54 | 662.4 |
| 230 | 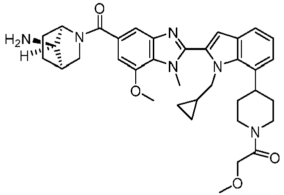 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-2-methoxyethan-1-one | 1.51 | 1.32 | 625.2 |
| 231 | 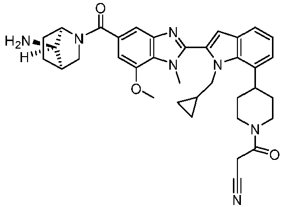 | 3-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-3-oxopropanenitrile | 1.53 | 1.33 | 620.2 |
| 232 | 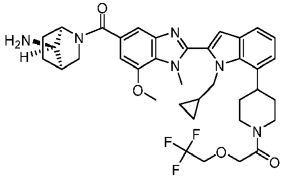 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-2-(2,2,2-trifluoroethoxy)ethan-1-one | 1.96 | 1.68 | 693.2 |
| 233 | 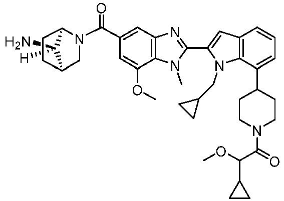 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-2-cyclopropyl-2-methoxyethan-1-one | 1.89 | 1.61 | 665.2 |
| 234 | 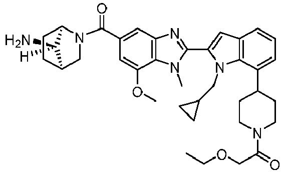 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-2-ethoxyethan-1-one | 1.65 | 1.48 | 639.1 |
| 235 | 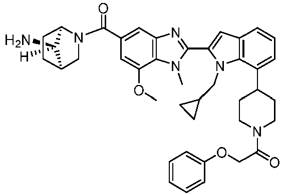 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-2-phenoxyethan-1-one | 1.97 | 1.72 | 687.0 |
| 236 | 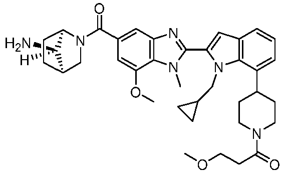 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-3-methoxypropan-1-one | 1.67 | 1.46 | 639.0 |
| 237 | 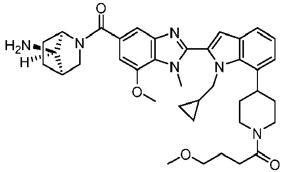 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-4-methoxybutan-1-one | 1.74 | 1.50 | 653.0 |
| 238 | 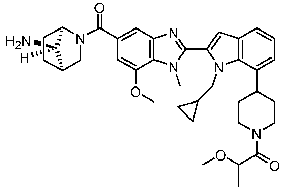 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-2-methoxypropan-1-one | 1.69 | 1.49 | 639.4 |
| 239 | 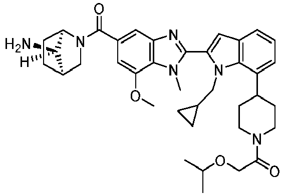 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-2-isopropoxyethan-1-one | 1.82 | 1.54 | 653.2 |
| 240 | 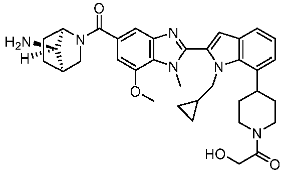 | 1-(4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidin-1-yl)-2-hydroxyethan-1-one | 1.35 | 1.50 | 611.3 |
| 241 | 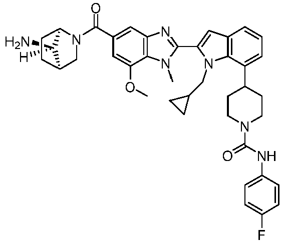 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-(4-fluorophenyl)piperidin e-1-carboxamide | 1.98 | 1.76 | 690.2 |
| 242 | 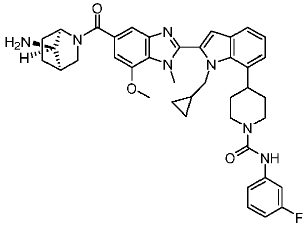 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-(3-fluorophenyl)piperidin e-1-carboxamide | 2.08 | 1.81 | 690.0 |
| 243 | 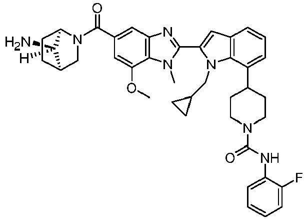 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-(2-fluorophenyl)piperidin e-1-carboxamide | 2.02 | 1.73 | 690.3 |
| 244 | 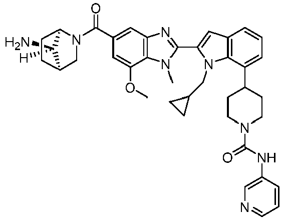 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-(pyridin-3-yl)piperidine-1-carboxamide | 1.80 | 1.40 | 689.2 |
| 245 | 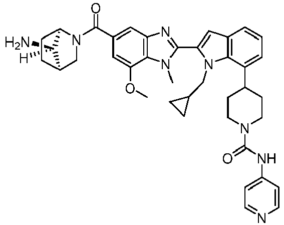 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-(pyridin-4-yl)piperidine-1-carboxamide | 1.74 | 1.37 | 673.3 |
| 246 | 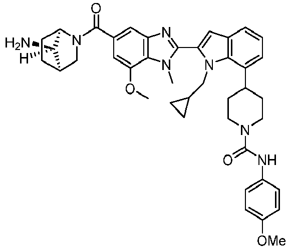 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-(4-methoxyphenyl)piperi dine-1-carboxamide | 1.96 | 1.70 | 702.0 |
| 247 | 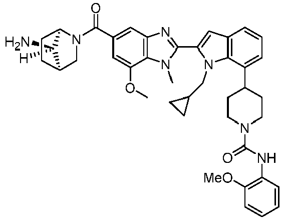 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-(2-methoxyphenyl)piperi dine-1-carboxamide | 2.05 | 1.77 | 702.2 |
| 248 | 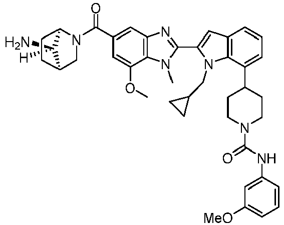 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-(3-methoxyphenyl)piperi dine-1-carboxamide | 1.91 | 1.73 | 702.1 |
| 249 | 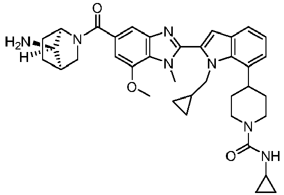 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-cyclopropylpiperidine-1-carboxamide | 1.76 | 1.52 | 636.0 |
| 250 | 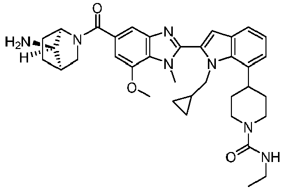 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-ethylpiperidine-1-carboxamide | 1.75 | 1.52 | 624.3 |
| 251 | 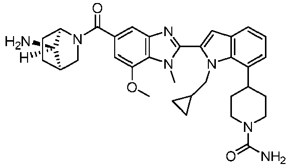 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)piperidine-1-carboxamide | 1.59 | 1.36 | 596.3 |
| 252 | 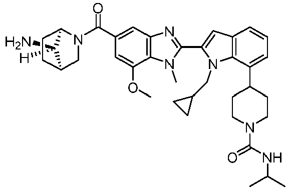 | 4-(2-(5-((1R,4R,7R)-7-amino-2-azabicyclo[2.2.1]hepta ne-2-carbonyl)-7-methoxy-1-methyl-1H-benzo[d]imidazol-2-yl)-1-(cyclopropylmethyl)-1H-indol-7-yl)-N-isopropylpiperidine-1-carboxamide | 1.85 | 1.60 | 638.0 |

### Biological Assays

Compounds of the present invention were assayed as inhibitors of PAD4 using the assay protocol described below.

### RFMS human PAD4 Functional Assay:

Compounds were solubilized in 100% DMSO to achieve a10 mM compound concentration. Compound stock solutions were stored at RT. A series of dilutions were prepared in DMSO and mixed 8 times with 20 µL mixing volume. Final top concentration of compound in the assay is 50 µM. Final assay conditions were as follows:
Reaction volume: 26 µl
Assay buffer: 25 mM hepes, pH 7.5, 5 mM NaCl, 1 mM DTT, 0.2 mg/ml BSA, 0.01% CHAPS, 50 µM Calcium, and 5 µM TPEN
Final concentrations: 5 nM hPAD4 enzyme, 250 µM BAEE, and 0.5% DMSO
Total incubation time: 30 mins compound and enzyme preincubation at 37 °C, 90 min enzyme/substrate reaction, 30 min reaction with phenyl glyoxal at 37 °C
Stop solution: 40 µl 5% TCA in ACN

0.13 µL of compound solution was added to 13 µL of 10 nM PAD4 in assay buffer. After 30 min 13 µl of 500 µM of BAEE was added in 25 mM hepes, pH 7.5, 5 mM NaCl, 1 mM DTT, 0.2 mg/ml BSA, 0.01% CHAPS, 50 µM Calcium, 5 µM TPEN was added and the reaction incubated for 90 min at 37 °C. The enzymatic reaction was quenched by addition of 15 µl of 6.1N TCA, 100% Final Concentration is 20%, 35 µl of 8.5 mM phenyl glyoxal (final concentration 4 mM) is then added and the reaction is incubated for 30 min at 37°C.

After 30 minutes the plates are spun down to remove all precipitate. The enzyme reaction was quenched with an equal volume of methanol containing internal standard (modified citrulline). Samples were loaded onto the Rapid Fire RF300 system (Agilent) wherein they were first sipped for 1000 ms and then directly loaded to a C18 separations cartridge using a mixture of acetonitrile containing 0.01% formic acid for 3000 ms desalting. The flow rate of the mobile phase was 1.5 ml/min. Once the samples were eluted from the cartridge, a mobile phase of acetonitrile containing 0.01% formic acid was used to move the samples into the mass spectrometer for 4000 ms at a flow rate of 1.25 ml/min/ Sciex API5500 triple quadrupole mass spectrometer (Applied Biosystems) equipped with ESI was used to analyze the peptidyl citrulline and internal standard ions.

MRM transition of product and internal standard were monitored at m/z 424.5 to 350.4 and m/z 293 to 247 respectively. The dwell time for each transition was set at 200 ms, and the ESI voltage was used at 5500 with a source temperature of 400°C. Extracted ion peaks for each transition were integrated using the Rapid Fire Integrator software. Peak area of analyte was normalized with internal standard.).

For a given compound example, the Table below shows the human PAD4 (hPAD4) IC₅₀ in the rapid-fire mass spectrum (RFMS) assay.

**Table 14**

| EX NO | hPAD4 RFMS IC₅₀ µM |
|---|---|
| 1 (reference) | 0.162 |
| 2 (reference) | 0.032 |
| 4 | 0.030 |
| 8 | 0.073 |
| 9 | 0.040 |
| 10 | 0.128 |
| 12 | 0.073 |
| 13 | 0.016 |
| 14 | 0.038 |
| 15 | 0.020 |
| 18 | 0.071 |
| 22 | 0.042 |
| 23 | 0.120 |
| 24 | 0.061 |
| 25 | 0.063 |
| 26 | 0.041 |
| 28 | 0.067 |
| 29 | 0.056 |
| 30 | 0.080 |
| 31 | 0.198 |
| 32 | 0.032 |
| 33 | 0.129 |
| 36 | 0.199 |
| 37 | 0.026 |
| 38 | 0.073 |
| 39 | 0.051 |
| 40 | 0.021 |
| 41 | 0.053 |
| 42 | 0.141 |
| 43 | 0.020 |
| 44 | 0.048 |
| 45 | 0.025 |
| 46 | 0.013 |
| 47 | 0.013 |
| 48 | 0.050 |
| 49 | 0.034 |
| 50 | 0.081 |
| 51 | 0.048 |
| 52 | 0.097 |
| 53 | 0.051 |
| 54 | 0.092 |
| 55 | 0.122 |
| 56 | 0.170 |
| 57 | 0.010 |
| 58 | 0.075 |
| 59 | 0.059 |
| 61 | 0.034 |
| 62 | 0.110 |
| 63 | 0.062 |
| 64 | 0.031 |
| 65 | 0.024 |
| 66 | 0.030 |
| 67 | 0.189 |
| 68 | 0.170 |
| 69 | 0.050 |
| 70 | 0.015 |
| 71 | 0.016 |
| 72 | 0.010 |
| 73 | 0.005 |
| 74 | 0.071 |
| 75 | 0.034 |
| 76 | 0.019 |
| 77 | 0.038 |
| 78 | 0.010 |
| 79 | 0.012 |
| 80 | 0.016 |
| 81 | 0.016 |
| 82 | 0.010 |
| 83 | 0.123 |
| 84 | 0.034 |
| 85 | 0.049 |
| 86 | 0.040 |
| 87 | 0.111 |
| 88 | 0.056 |
| 89 | 0.108 |
| 90 | 0.045 |
| 91 | 0.042 |
| 92 | 0.058 |
| 93 | 0.053 |
| 94 | 0.045 |
| 95 | 0.107 |
| 96 | 0.144 |
| 97 | 0.023 |
| 98 | 0.035 |
| 99 | 0.038 |
| 100 | 0.058 |
| 101 | 0.047 |
| 102 | 0.131 |
| 103 | 0.025 |
| 104 | 0.093 |
| 105 | 0.099 |
| 106 | 0.061 |
| 107 | 0.086 |
| 108 | 0.073 |
| 109 | 0.175 |
| 110 | 0.137 |
| 111 | 0.076 |
| 112 | 0.145 |
| 113 | 0.052 |
| 114 | 0.081 |
| 115 | 0.064 |
| 116 | 0.095 |
| 117 | 0.117 |
| 118 | 0.278 |
| 119 | 0.020 |
| 120 | 0.065 |
| 121 | 0.753 |
| 122 | 0.057 |
| 123 | 0.084 |
| 124 | 0.025 |
| 125 | 0.125 |
| 126 | 0.110 |
| 127 | 0.082 |
| 128 | 0.130 |
| 129 | 0.194 |
| 130 | 0.178 |
| 131 | 0.156 |
| 132 | 0.179 |
| 133 | 0.121 |
| 134 | 0.155 |
| 135 | 0.096 |
| 136 | 0.077 |
| 137 | 0.037 |
| 138 | 0.147 |
| 139 | 0.180 |
| 140 | 0.106 |
| 141 | 0.101 |
| 142 | 0.180 |
| 143 | 0.181 |
| 149 | 0.168 |
| 150 | 0.046 |
| 151 | 0.104 |
| 152 | 0.127 |
| 154 | 0.043 |
| 155 | 0.171 |
| 156 | 0.079 |
| 157 | 0.080 |
| 181 | 0.127 |
| 186 | 0.299 |
| 187 | 0.018 |
| 188 | 0.027 |
| 189 | 0.018 |
| 190 | 0.085 |
| 191 | 0.081 |
| 192 | 0.028 |
| 193 | 0.033 |
| 194 | 0.146 |
| 195 | 0.016 |
| 196 | 0.053 |
| 197 | 0.120 |
| 198 | 0.026 |
| 199 | 0.159 |
| 200 | 0.021 |
| 201 | 0.057 |
| 202 | 0.024 |
| 203 | 0.046 |
| 204 | 0.139 |
| 205 | 0.041 |
| 206 | 0.145 |
| 207 | 0.070 |
| 208 | 0.071 |
| 209 | 0.069 |
| 210 | 0.018 |
| 211 | 0.026 |
| 212 | 0.035 |
| 213 | 0.021 |
| 214 | 0.155 |
| 215 | 0.183 |
| 216 | 0.101 |
| 217 | 0.027 |
| 218 | 0.057 |
| 220 | 0.043 |
| 221 | 0.077 |
| 222 | 0.094 |
| 223 | 0.088 |
| 224 | 0.078 |
| 225 | 0.112 |
| 226 | 0.053 |
| 227 | 0.077 |
| 228 | 0.041 |
| 229 | 0.013 |
| 230 | 0.021 |
| 231 | 0.066 |
| 232 | 0.019 |
| 233 | 0.063 |
| 234 | 0.049 |
| 235 | 0.058 |
| 236 | 0.057 |
| 237 | 0.018 |
| 238 | 0.104 |
| 239 | 0.119 |
| 240 | 0.015 |
| 241 | 0.028 |
| 242 | 0.030 |
| 243 | 0.008 |
| 244 | 0.019 |
| 245 | 0.013 |
| 246 | 0.006 |
| 247 | 0.009 |
| 248 | 0.020 |
| 249 | 0.008 |
| 250 | 0.008 |
| 251 | 0.020 |
| 252 | 0.058 |

## Claims

1. A compound of Formula (III): 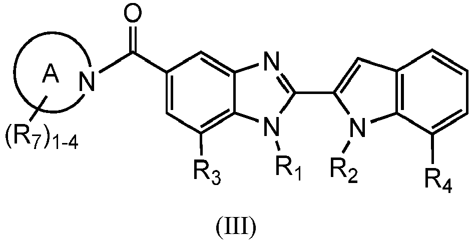
or a pharmaceutically acceptable salt thereof, wherein: 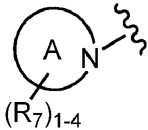 is selected from 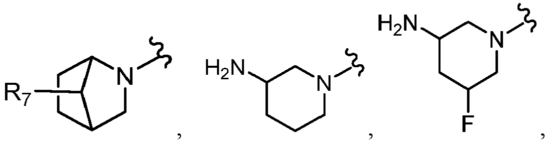 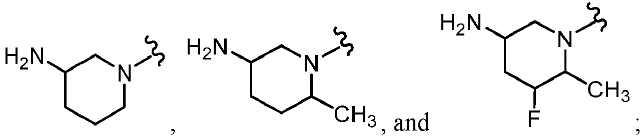
R₁ is selected from -CH₃, -CD₃, and -CH₂-5-6 membered heterocyclyl comprising carbon atoms and 1-3 heteroatoms selected from N, NH, and NC₁₋₃alkyl;
R₂ is selected from CH₃, CH₃CH₂, and -CH₂-cyclopropyl substituted with 0-3 Rₑ;
R₃ is selected from H, F, Cl, Br, and -OC₁₋₄ alkyl;
R₄ is selected from 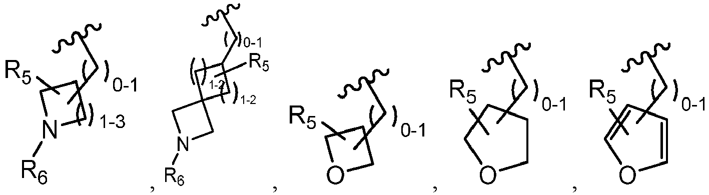 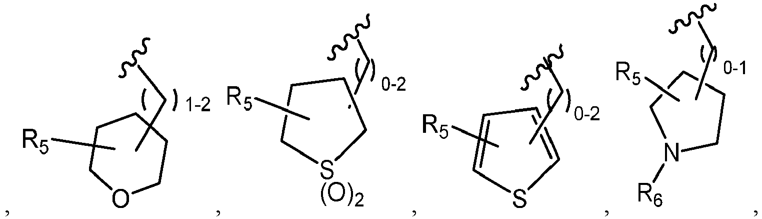 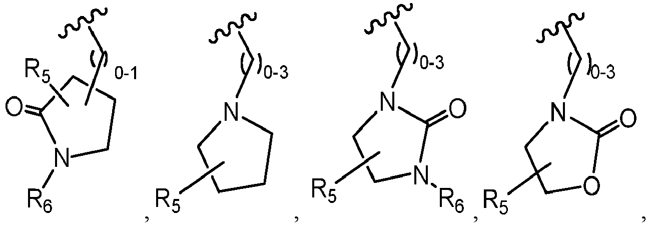 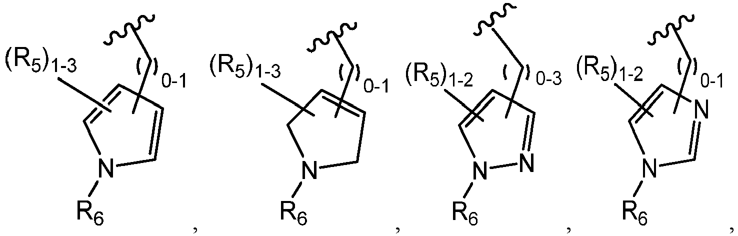 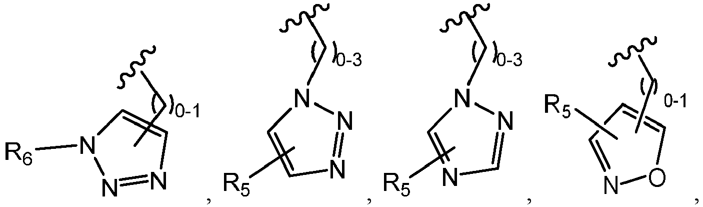 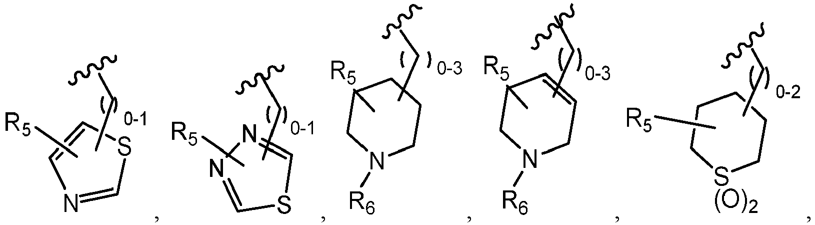 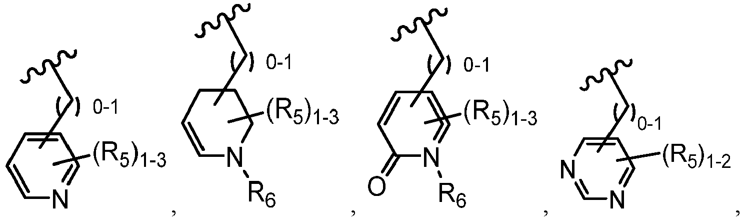 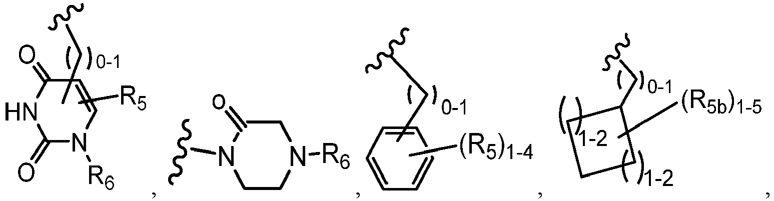 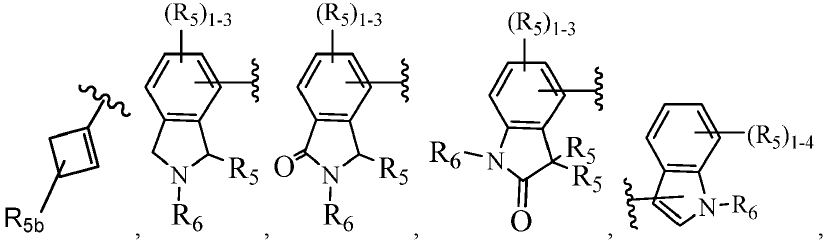 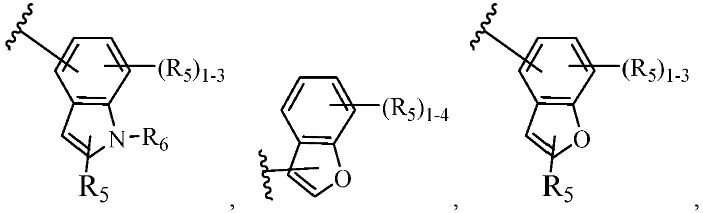 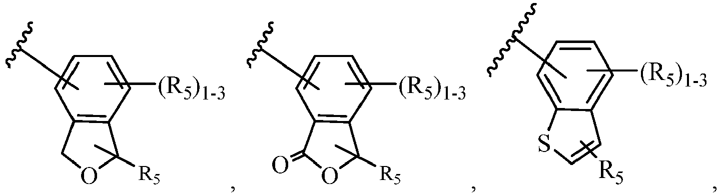 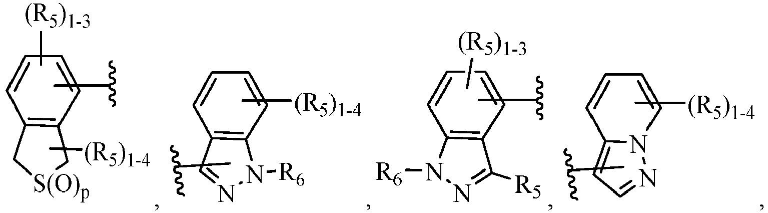 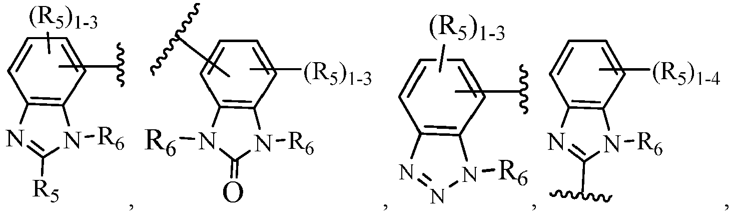 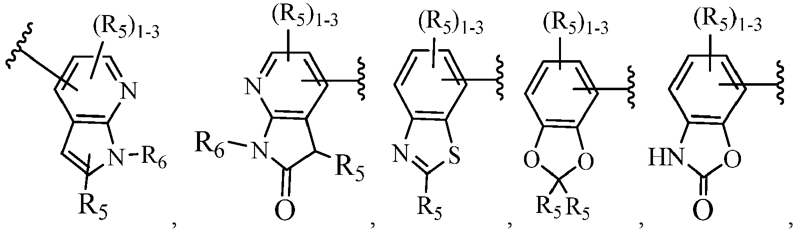 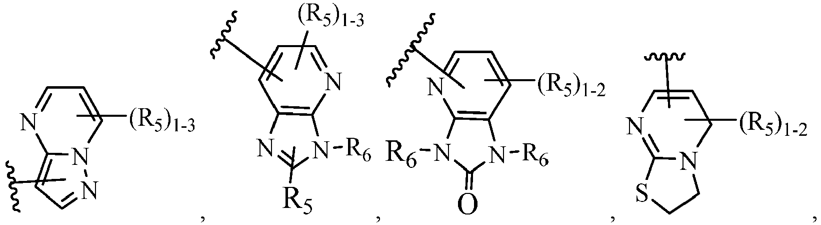 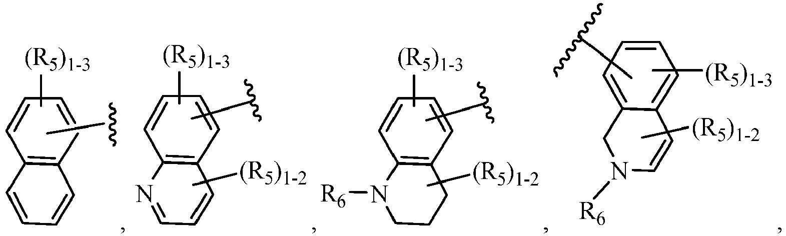 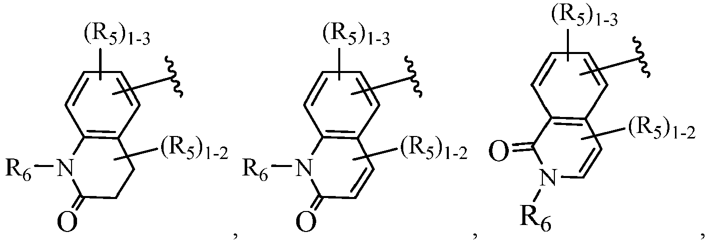 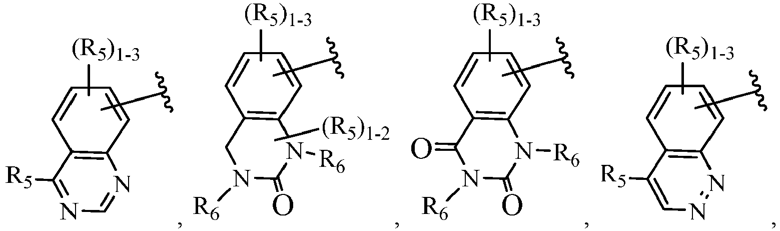 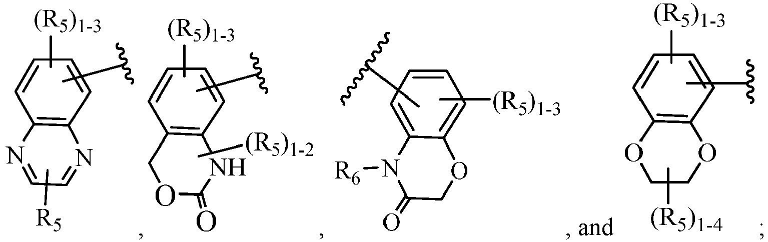
R₅, at each occurrence, is independently selected from H, F, Cl, Br, C₁-₄alkyl substituted
with 0-5 Rₑ, C₂-₄alkenyl, C₂-₄alkynyl,
nitro, -(CH₂)ᵣOR_{b}, -CN, -NRₐRₐ, -(CH₂)ᵣNRₐC(=O)R_{b}, -NRₐC(=O)NRₐRₐ, - C(=O)OR_{b}, -C(=O)R_{b}, -OC(=O)R_{b}, -C(=O)NRₐRₐ, -P(=O)(C₁₋₄alkyl)₂, -S(O)*ₚ*R_{c}, -S(O)*ₚ*NRₐRₐ, -NRₐS(O)*ₚ*R_{c}, -C₃₋₆ cycloalkyl substituted with 0-4 Rₑ, aryl substituted with 0-4 Rₑ, and heterocyclyl substituted with 0-4 Rₑ;
R_{5b}, at each occurrence, is independently selected from OR_{b}, -C(=O)OR_{b}, -C(=O)NRₐRₐ, - NRₐRₐ, -NRₐC(=O)R_{b}, -NRₐC(=O)OR_{b}, -S(=O)ₚR_{c}, and -NRₐS(=O)ₚR_{c};
R₆, at each occurrence, is independently selected from H, C₁-₃alkyl substituted with 0-4 Rₑ, -C(=O)R_{b}, -C(=O)(CH₂)ᵣOR_{b}, -C(=O)(CH₂)ᵣNRₐRₐ, -S(O)ₚR_{c}, -S(O)ₚNRₐRₐ, - (CH₂)ᵣ-aryl substituted with 0-4 Rₑ, and -(CH₂)ᵣ-heterocyclyl substituted with 0-4 Rₑ;
R₇ is selected from H, F, Cl, CN, C₁₋₃ alkyl, =N-OR_{b}, -(CH₂)ᵣOR_{b}, -(CH₂)ᵣNRₐRₐ, - NRₐC(=NH)C₁₋₃alkyl, -NRₐC(=O)OR_{b}, a carbocyclyl, and heterocyclyl;
Rₐ, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, - (CH₂)ᵣ-C₃-₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, - (CH₂)ᵣ-C₃-₁₀carbocyclyl substituted with 0-5 Rₑ, and -(CH₂)ᵣ-heterocyclyl substituted with 0-5 Rₑ;
R_{c}, at each occurrence, is independently selected from C₁-₆ alkyl substituted with 0-5 Rₑ, C₂-₆alkenyl substituted with 0-5 Rₑ, C₂-₆ alkynyl substituted with 0-5 Rₑ, -(CH₂)ᵣ-C₃-₆ carbocyclyl substituted with 0-5 Rₑ, and -(CH₂)ᵣ-heterocyclyl substituted with 0-5 Rₑ;
Rₑ, at each occurrence, is independently selected from C₁-₆ alkyl substituted with 0-5 Rε, C₂-₆ alkenyl, C₂-₆ alkynyl, -(CH₂)ᵣ-C₃-₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, -(CH₂)ᵣOC₁₋₄alkyl, -(CH₂)ᵣOC₂₋₄alkenyl, -(CH₂)ᵣOC₂₋₄alkynyl, and NH₂;
Rε, at each occurrence, is independently selected from H, F, Cl, Br, CN, OH, C₁-₅ alkyl optionally substituted with OH, C₃-₆ cycloalkyl, and phenyl;
p, at each occurrence, is independently selected from zero, 1, and 2; and
r, at each occurrence, is independently selected from zero, 1, 2, 3, and 4.

2. The compound according to claim 1, or a pharmaceutically acceptable salt thereof, wherein: 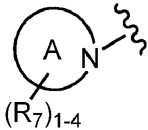 is selected from 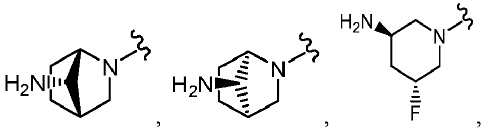 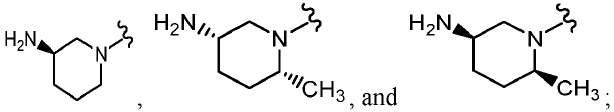
R₁ is selected from -CH₃ and -CD₃;
R₂ is selected from -CH₃ and -CH₂-cyclopropyl substituted with 0-2 F or Cl;
R₃ is selected from H, F and -OC₁₋₄ alkyl;
R₄ is selected from 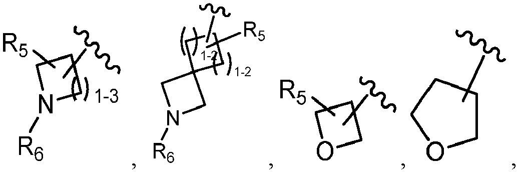 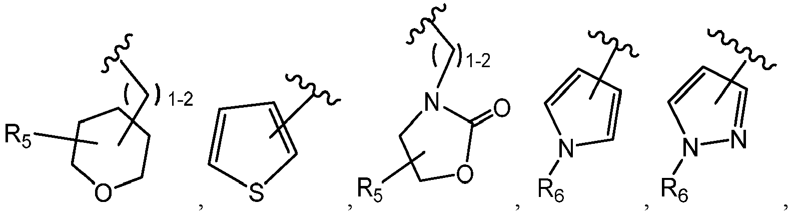 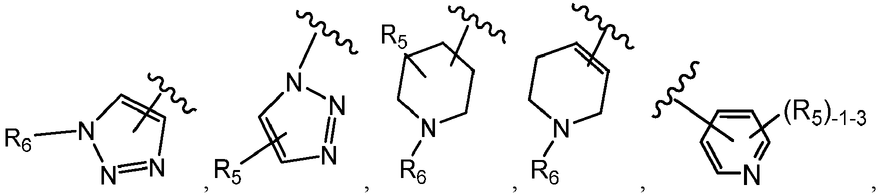 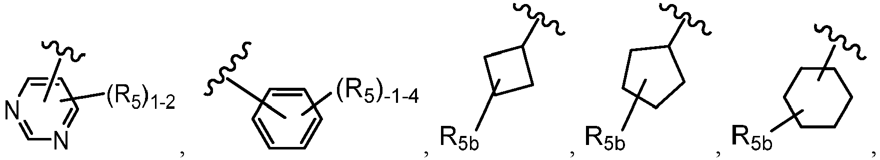 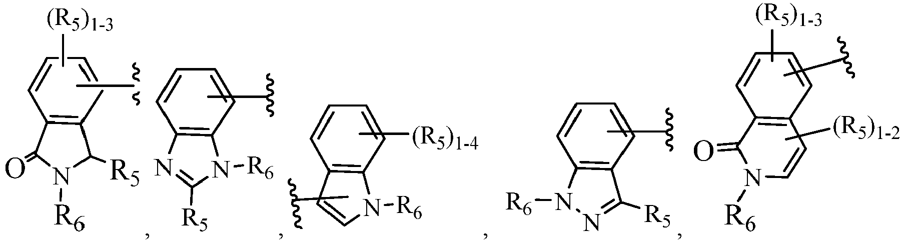 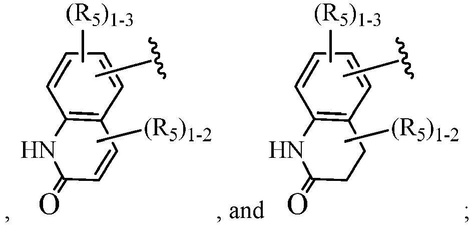
R₅, at each occurrence, is independently selected from H, F, Cl, Br, C₁-₄alkyl, -(CH₂)ᵣOR_{b}, -CN, -NRₐRₐ, -(CH₂)ᵣNRₐC(=O)R_{b}, -NRₐC(=O)NRₐRₐ, - C(=O)OR_{b}, -C(=O)R_{b}, -OC(=O)R_{b}, - C(=O)NRₐRₐ, -S(O)*ₚ*R_{c}, -S(O)*ₚ*NRₐRₐ, -NRₐS(O)*ₚ*R_{c}, C₃₋₆cycloalkyl substituted with 0-4 Rₑ, aryl substituted with 0-4 Rₑ, and heterocyclyl substituted with 0-4 Rₑ;
R_{5b}, at each occurrence, is independently selected from OH, -C(=O)OR_{b}, -C(=O)NRₐRₐ, - NRₐRₐ, -NRₐC(=O)R_{b}, and -NRₐC(=O)OR_{b};
R₆, at each occurrence, is independently selected from H, C₁-₃alkyl, -S(O)ₚR_{c}, -C(=O)R_{b}, -C(=O)OR_{b}, -C(=O)(CH₂)ᵣNRₐRₐ, -S(O)ₚNRₐRₐ, aryl substituted with 0-4 Rₑ, and heterocyclyl substituted with 0-4 Rₑ;
Rₐ, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, -(CH₂)ᵣ-C₃-₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, -(CH₂)ᵣ-C₃-₁₀carbocyclyl substituted with 0-5 Rₑ, and -(CH₂)ᵣ-heterocyclyl substituted with 0-5 Rₑ;
R_{c}, at each occurrence, is independently selected from C₁-₆ alkyl substituted with 0-5 Rₑ, C₂-₆alkenyl substituted with 0-5 Rₑ, C₂-₆ alkynyl substituted with 0-5 Rₑ, -(CH₂)ᵣ-C₃-₆ carbocyclyl substituted with 0-5 Rₑ, and -(CH₂)ᵣ-heterocyclyl substituted with 0-5 Rₑ;
Rₑ, at each occurrence, is independently selected from C₁-₆ alkyl substituted with 0-5 Rε, C₂-₆ alkenyl, C₂-₆ alkynyl, -(CH₂)ᵣ-C₃-₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, -(CH₂)ᵣOC₁₋₄alkyl, -(CH₂)ᵣOC₂₋₄alkenyl, -(CH₂)ᵣOC₂₋₄alkynyl, and NH₂;
Rε, at each occurrence, is independently selected from H, F, Cl, Br, CN, OH, C₁-₅ alkyl optionally substituted with OH, C₃-₆ cycloalkyl, and phenyl;
p, at each occurrence, is independently selected from zero, 1, and 2; and
r, at each occurrence, is independently selected from zero, 1, 2, 3, and 4.

3. The compound according to claim 2 or a pharmaceutically acceptable salt thereof, wherein:
R₁ is -CH₃;
R₂ is selected from -CH₃ and -CH₂-cyclopropyl;
R₃ is -OC₁₋₄ alkyl;
R₄ is selected from 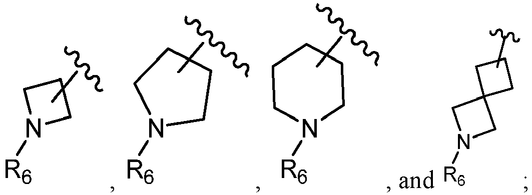
R₆ is selected from H, C₁-₃ alkyl, -C(=O)R_{b}, -C(=O)OR_{b}, -C(=O)NRₐRₐ, and - C(=O)CH₂NRₐRₐ, and -S(O)ₚR_{c};
Rₐ, at each occurrence, is independently selected from H, C₁-₆ alkyl substituted with 0-5 Rₑ, -(CH₂)ᵣ-C₃-₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, - (CH₂)ᵣ-C₃-₁₀carbocyclyl substituted with 0-5 Rₑ, and -(CH₂)ᵣ-heterocyclyl substituted with 0-5 Rₑ;
R_{c}, at each occurrence, is independently C₁-₆ alkyl substituted with 0-5 Rₑ, -(CH₂)ᵣ-C₃-₆ carbocyclyl substituted with 0-5 Rₑ, or -(CH₂)ᵣ-heterocyclyl substituted with 0-5 Rₑ;
Rₑ, at each occurrence, is independently selected from C₁-₆ alkyl substituted with 0-5 Rε, C₂-₆ alkenyl, C₂-₆ alkynyl, -(CH₂)ᵣ-C₃-₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl;
Rε, at each occurrence, is independently selected from H, F, Cl, Br, CN, OH, C₁-₅ alkyl optionally substituted with OH, C₃-₆ cycloalkyl, and phenyl;
p, at each occurrence, is independently selected from zero, 1, and 2; and
r, at each occurrence, is independently selected from zero, 1, 2, 3, and 4.

4. The compound according to claim 2, or a pharmaceutically acceptable salt thereof, wherein:
R₄ is selected from 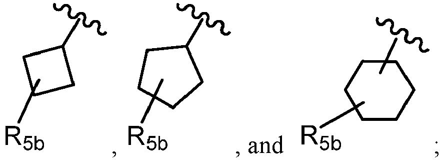
R_{5b}, at each occurrence, is independently selected from OH, -C(=O)OR_{b}, -C(=O)NRₐRₐ, - NRₐRₐ, and -NRₐC(=O)R_{b};
Rₐ, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, - (CH₂)ᵣ-C₃-₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, - (CH₂)ᵣ-C₃-₁₀carbocyclyl substituted with 0-5 Rₑ, and -(CH₂)ᵣ-heterocyclyl substituted with 0-5 Rₑ;
Rₑ, at each occurrence, is independently selected from C₁-₆ alkyl substituted with 0-5 Rε, C₂-₆ alkenyl, C₂-₆ alkynyl, -(CH₂)ᵣ-C₃-₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl;
Rε, at each occurrence, is independently selected from H, F, Cl, Br, CN, OH, C₁-₅ alkyl optionally substituted with OH, C₃-₆ cycloalkyl, and phenyl;
p, at each occurrence, is independently selected from zero, 1, and 2; and
r, at each occurrence, is independently selected from zero, 1, 2, 3, and 4.

5. The compound according to claim 4 or a pharmaceutically acceptable salt thereof, wherein:
R₄ is 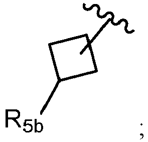
R_{5b} is selected from -C(=O)OR_{b}, -C(=O)NRₐRₐ, and -NHC(=O)R_{b};
Rₐ, at each occurrence, is independently selected from H and C₁-₆ alkyl substituted with 0-5 Rₑ, or Rₐ and Rₐ together with the nitrogen atom to which they are both attached form a heterocyclic ring selected from 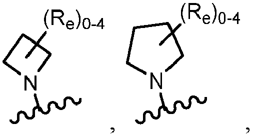 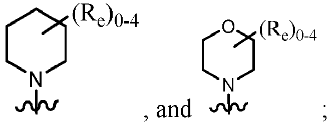
R_{b} is selected from H and C₁-₆ alkyl substituted with 0-5 Rₑ;
Rₑ, at each occurrence, is independently selected from C₁-₆ alkyl substituted with 0-5 Rε, C₂-₆ alkenyl, C₂-₆ alkynyl, -(CH₂)ᵣ-C₃-₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl; and
Rε, at each occurrence, is independently selected from H, F, Cl, Br, CN, OH, C₁-₅ alkyl optionally substituted with OH, C₃-₆ cycloalkyl, and phenyl.

6. The compound according to claim 2 or a pharmaceutically acceptable salt thereof, wherein:
R₄ is selected from 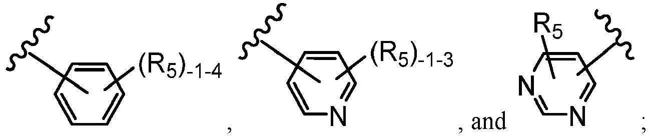
Rs, at each occurrence, is independently selected from H, F, Cl, Br, C₁-₄alkyl substituted with 0-5 Rₑ, C₂-₄alkenyl, C₂-₄alkynyl, -(CH₂)₀₋₁OR_{b}, -CN, -NRₐRₐ, -(CH₂)₀₋₁-NHC(=O)R_{b}, -NRₐC(=O)NRₐRₐ, -C(=O)OR_{b}, -C(=O)R_{b}, -OC(=O)R_{b}, - C(=O)NRₐRₐ, -S(O)*ₚ*R_{c}, -S(O)*ₚ*NRₐRₐ, -NRₐS(O)*ₚ*R_{c}, C₃₋₆ cycloalkyl, heterocyclyl, and aryl, wherein said alkyl, cycloalkyl, heterocyclyl, or aryl is substituted with 0-4 Rₑ;
Rₐ, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, -(CH₂)ᵣ-C₃-₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, - (CH₂)ᵣ-C₃-₁₀carbocyclyl substituted with 0-5 Rₑ, and -(CH₂)ᵣ-heterocyclyl substituted with 0-5 Rₑ;
R_{c}, at each occurrence, is independently selected from C₁-₆ alkyl substituted with 0-5 Rₑ, C₂-₆alkenyl substituted with 0-5 Rₑ, C₂-₆ alkynyl substituted with 0-5 Rₑ, -(CH₂)ᵣ-C₃-₆ carbocyclyl substituted with 0-5 Rₑ, and -(CH₂)ᵣ-heterocyclyl substituted with 0-5 Rₑ;
Rₑ, at each occurrence, is independently selected from C₁-₆ alkyl substituted with 0-5 Rε, C₂-₆ alkenyl, C₂-₆ alkynyl, -(CH₂)ᵣ-C₃-₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl;
Rε, at each occurrence, is independently selected from H, F, Cl, Br, CN, OH, C₁-₅ alkyl optionally substituted with OH, C₃-₆ cycloalkyl, and phenyl;
p, at each occurrence, is independently selected from zero, 1, and 2; and
r, at each occurrence, is independently selected from zero, 1, 2, 3, and 4.

7. The compound according to claim 2 or a pharmaceutically acceptable salt thereof, wherein:
R₂ is selected from -CH₃ and -CH₂-cyclopropyl substituted with 0-2 F or Cl;
R₄, at each occurrence, is selected from 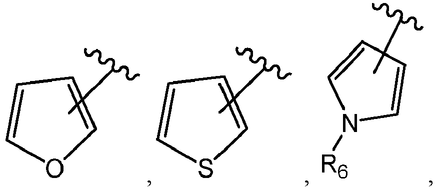 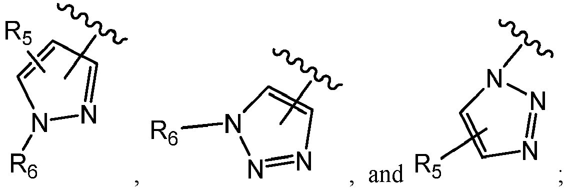
R₅, at each occurrence, is independently selected from H, F, Cl, Br, C₁-₄alkyl substituted with 0-5 Rₑ, C₂-₄alkenyl, C₂-₄alkynyl, nitro, -OR_{b}, -CN, -NRₐRₐ, -S(O)*ₚ*R_{c}, - S(O)*ₚ*NRₐRₐ, -NRₐS(O)*ₚ*R_{c}, -(CH₂)ᵣ-NRₐC(=O)R_{b}, -NRₐC(=O)NRₐRₐ, C(=O)OR_{b}, -C(=O)R_{b}, -OC(=O)R_{b}, -C(=O)NRₐRₐ, P(=O)(OC₁₋₄alkyl)₂, C₃₋₆ cycloalkyl substituted with 0-4 Rₑ, aryl substituted with 0-4 Rₑ, heterocyclyl, substituted with 0-4 Rₑ;
R₆, at each occurrence, is independently selected from H, C₁-₃alkyl substituted with 0-4 Rₑ, and heterocyclyl substituted with 0-4 Rₑ;
Rₐ, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, -(CH₂)ᵣ-C₃-₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, - (CH₂)ᵣ-C₃-₁₀carbocyclyl substituted with 0-5 Rₑ, and -(CH₂)ᵣ-heterocyclyl substituted with 0-5 Rₑ;
R_{c}, at each occurrence, is independently selected from C₁-₆ alkyl substituted with 0-5 Rₑ, C₂-₆alkenyl substituted with 0-5 Rₑ, C₂-₆ alkynyl substituted with 0-5 Rₑ, -(CH₂)ᵣ-C₃-₆ carbocyclyl substituted with 0-5 Rₑ, and -(CH₂)ᵣ-heterocyclyl substituted with 0-5 Rₑ;
Rₑ, at each occurrence, is independently selected from C₁-₆ alkyl substituted with 0-5 Rε, C₂-₆ alkenyl, C₂-₆ alkynyl, -(CH₂)ᵣ-C₃-₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl, -(CH₂)ᵣOC₂₋₄alkenyl, -(CH₂)ᵣOC₂₋₄alkynyl, and NH₂;
Rε, at each occurrence, is independently selected from H, F, Cl, Br, CN, OH, C₁-₅ alkyl optionally substituted with OH, C₃-₆ cycloalkyl, and phenyl;
p, at each occurrence, is independently selected from zero, 1, and 2; and
r, at each occurrence, is independently selected from zero, 1, 2, 3, and 4.

8. The compound according to claim 7 or a pharmaceutically acceptable salt thereof, wherein:
R₂ is selected from -CH₃ and -CH₂-cyclopropyl substituted with 0-2 F and Cl;
R₄ is 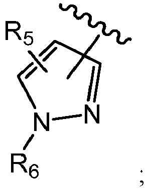
R₅, at each occurrence, is independently selected from H, F, Cl, Br, C₁-₄alkyl, aryl substituted with 0-4 Rₑ, and heterocyclyl substituted with 0-4 Rₑ;
R₆, at each occurrence, is independently selected from H, C₁-₃alkyl substituted with 0-4 Rₑ, and heterocyclyl substituted with 0-4 Rₑ;
Rₑ, at each occurrence, is independently selected from C₁-₆ alkyl, F, Cl, Br, CN, -OH, and -(CH₂)ᵣOC₂₋₄alkynyl; and
r, at each occurrence, is independently selected from zero, 1 and 2.

9. The compound according to claim 2 or a pharmaceutically acceptable salt thereof, wherein:
R₄ is selected from 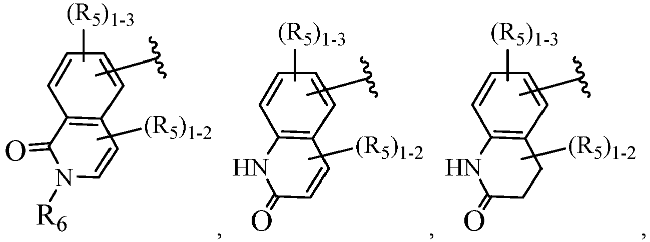 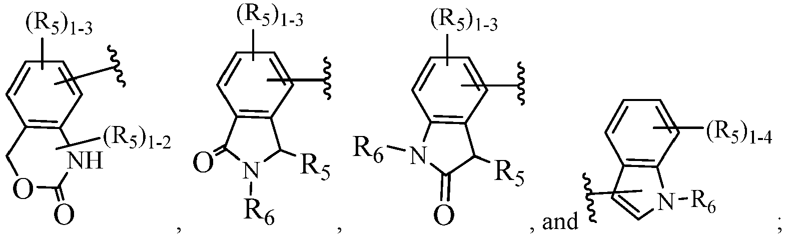
R₅, at each occurrence, is independently selected from H, F, Cl, Br, and C₁-₄alkyl substituted with 0-5 Rₑ, OR_{b}, -CN, -C(=O)R_{b}, -C(=O)OR_{b}, -OC(=O)R_{b}, - C(=O)NRₐRₐ;
R₆ is selected from H and C₁-₃alkyl;
Rₐ, at each occurrence, is independently selected from H and C₁-₄alkyl substituted with 0-5 Rₑ;
R_{b}, at each occurrence, is independently selected from H and C₁-₄alkyl substituted with 0-5 Rₑ;
Rₑ, at each occurrence, is independently selected from C₁-₆ alkyl F, Cl, Br, CN, NO₂, =O, -C(=O)OH, -C(=O)OC₁₋₄alkyl, -(CH₂)ᵣOH, and -(CH₂)ᵣOC₁₋₄alkyl; and
r, at each occurrence, is independently selected from zero, 1 and 2.

10. The compound of claim 1, or a pharmaceutically acceptable salt thereof, wherein: 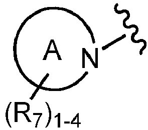 is selected from 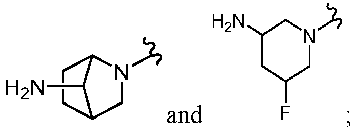
R₃ is selected from H, F, Cl, and -OC₁₋₄ alkyl;
R₄ is selected from 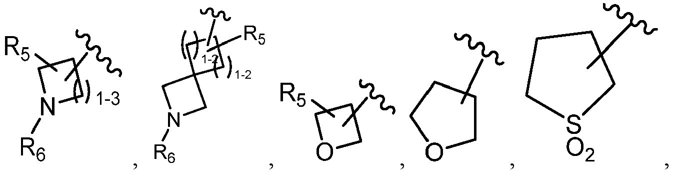 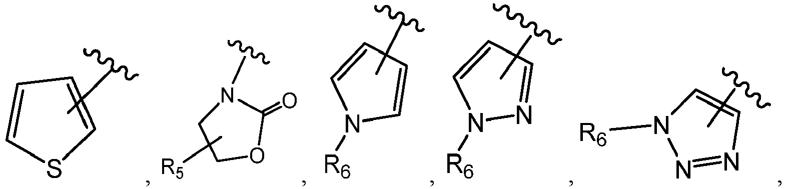 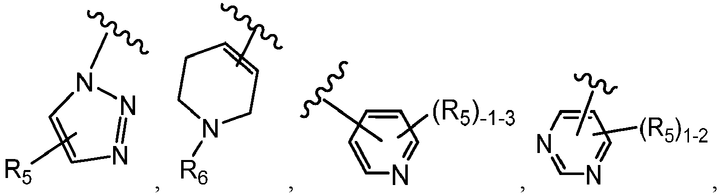 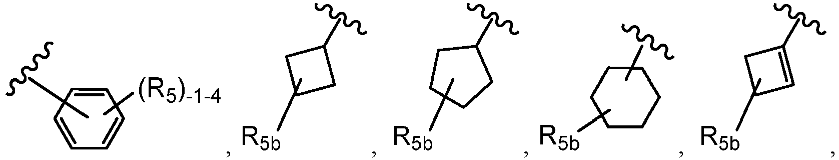 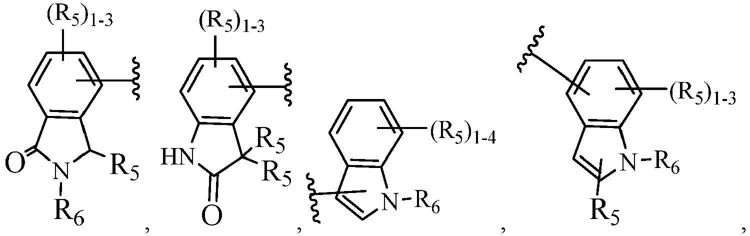 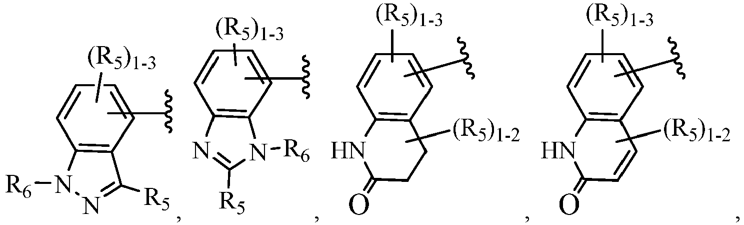 and 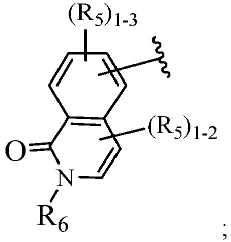
R₅, at each occurrence, is independently selected from H, F, Cl, Br, C₁-₄alkyl substituted with 0-3 Rₑ, -OR_{b}, -CN, -NRₐRₐ, -NRₐC(=O)R_{b}, -NRₐC(=O)NRₐRₐ, -C(=O)OR_{b}, - C(=O)R_{b}, -OC(=O)R_{b}, -C(=O)NRₐRₐ, -S(O)*ₚ*R_{c}, -S(O)*ₚ*NRₐRₐ, -NRₐS(O)*ₚ*R_{c}, C₃₋₆cycloalkyl substituted with 0-4 Rₑ, aryl substituted with 0-4 Rₑ, and heterocyclyl substituted with 0-4 Rₑ;
R_{5b}, at each occurrence, is independently selected from OH, -C(=O)OR_{b}, -C(=O)NRₐRₐ, and -NRₐC(=O)R_{b};
R₆, at each occurrence, is independently selected from H, C₁-₃alkyl, -C(=O)R_{b}, - C(=O)(CH₂)ᵣOR_{b}, -C(=O)(CH₂)ᵣNRₐRₐ, -S(O)ₚR_{c}, -S(O)ₚNRₐRₐ, aryl substituted with 0-4 Rₑ, and heterocyclyl substituted with 0-4 Rₑ;
Rₐ, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, - (CH₂)ᵣ-C₃-₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, -(CH₂)ᵣ-C₃-₁₀carbocyclyl substituted with 0-5 Rₑ, and -(CH₂)ᵣ-heterocyclyl substituted with 0-5 Rₑ;
R_{c}, at each occurrence, is independently selected from C₁-₆ alkyl substituted with 0-5 Rₑ, C₂-₆alkenyl substituted with 0-5 Rₑ, C₂-₆ alkynyl substituted with 0-5 Rₑ, -(CH₂)ᵣ-C₃-₆ carbocyclyl substituted with 0-5 Rₑ, and -(CH₂)ᵣ-heterocyclyl substituted with 0-5 Rₑ;
Rₑ, at each occurrence, is independently selected from C₁-₆ alkyl substituted with 0-5 Rε, C₂-₆ alkenyl, C₂-₆ alkynyl, -(CH₂)ᵣ-C₃-₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl;
Rε, at each occurrence, is independently selected from H, F, Cl, Br, CN, OH, C₁-₅ alkyl optionally substituted with OH, C₃-₆ cycloalkyl, and phenyl;
p, at each occurrence, is independently selected from zero, 1, and 2; and
r, at each occurrence, is independently selected from zero, 1, 2, 3, and 4.

11. The compound according to claim 10, or a pharmaceutically acceptable salt thereof, wherein: 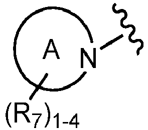 is selected from 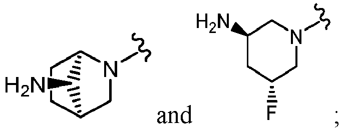
R₁ is selected from -CH₃, -CD₃, and -CH₂-5-6 membered heterocyclyl comprising carbon atoms and 1-3 heteroatoms selected from N, NH, and NC₁₋₃alkyl;
R₂ is selected from -CH₃ and -CH₂-cyclopropyl;
R₃ is selected from H, F, and -OC₁₋₄ alkyl;
R₄ is selected from 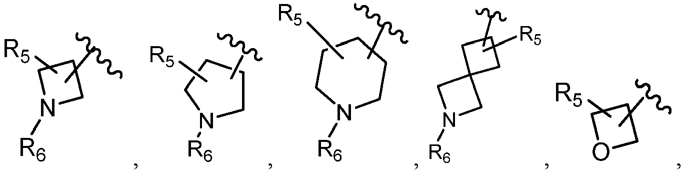 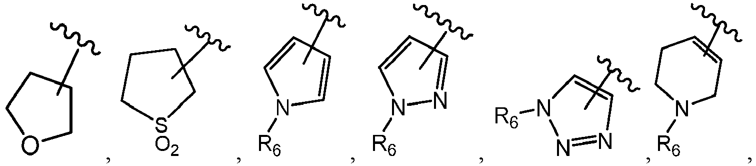 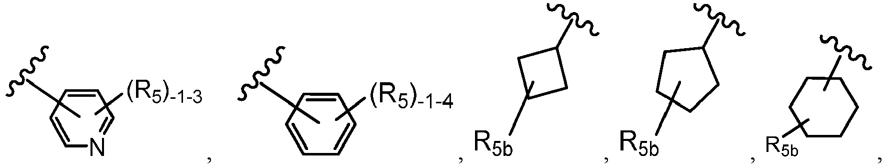 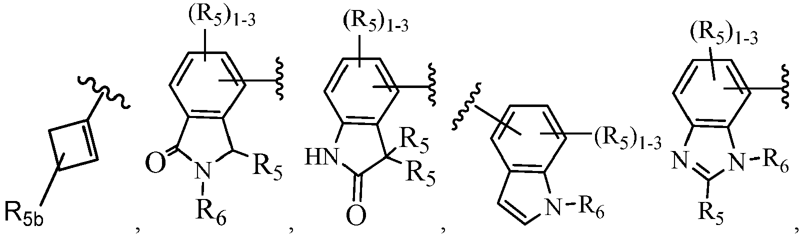 and 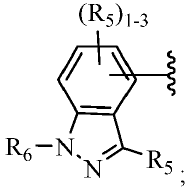
R₅, at each occurrence, is independently selected from H, F, Cl, Br, C₁-₄alkyl substituted with 0-3 Rₑ, -OR_{b}, -CN, -NRₐRₐ;
R_{5b}, at each occurrence, is independently selected from OH, -C(=O)OR_{b}, -C(=O)NRₐRₐ, and -NRₐC(=O)R_{b};
R₆, at each occurrence, is independently selected from H, C₁-₃alkyl, -C(=O)R_{b}, - C(=O)(CH₂)ᵣOR_{b}, -C(=O)(CH₂)ᵣNRₐRₐ, -S(O)ₚR_{c}, -S(O)ₚNRₐRₐ, aryl substituted with 0-4 Rₑ, and heterocyclyl substituted with 0-4 Rₑ;
Rₐ, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, -(CH₂)ᵣ-C₃-₁₀carbocyclyl substituted with 0-5 Rₑ, and -(CH₂)ᵣ-heterocyclyl substituted with 0-5 Rₑ; or Rₐ and Rₐ together with the nitrogen atom to which they are both attached form a heterocyclic ring substituted with 0-5 Rₑ;
R_{b}, at each occurrence, is independently selected from H, C₁-₆ alkyl substituted with 0-5 Rₑ, C₂-₆ alkenyl substituted with 0-5 Rₑ, C₂-₆ alkynyl substituted with 0-5 Rₑ, -(CH₂)ᵣ-C₃-₁₀carbocyclyl substituted with 0-5 Rₑ, and -(CH₂)ᵣ-heterocyclyl substituted with 0-5 Rₑ;
R_{c}, at each occurrence, is independently selected from C₁-₆ alkyl substituted with 0-5 Rₑ, C₂-₆alkenyl substituted with 0-5 Rₑ, C₂-₆ alkynyl substituted with 0-5 Rₑ, -(CH₂)ᵣ-C₃-₆ carbocyclyl substituted with 0-5 Rₑ, and -(CH₂)ᵣ-heterocyclyl substituted with 0-5 Rₑ;
Rₑ, at each occurrence, is independently selected from C₁-₆ alkyl substituted with 0-5 R_{f}, C₂-₆ alkenyl, C₂-₆ alkynyl, -(CH₂)ᵣ-C₃-₆ cycloalkyl, -(CH₂)ᵣ-aryl, F, Cl, Br, CN, NO₂, =O, -C(=O)OH, -C(=O)OC₁₋₄alkyl, -(CH₂)ᵣOH, and -(CH₂)ᵣOC₁₋₄alkyl;
R_{f}, at each occurrence, is independently selected from H, F, Cl, Br, CN, OH, C₁-₅ alkyl optionally substituted with OH, C₃-₆ cycloalkyl, and phenyl;
p, at each occurrence, is independently selected from zero, 1, and 2; and
r, at each occurrence, is independently selected from zero, 1, 2, 3, and 4.

12. The compound according to claim 1, having Formula (V): 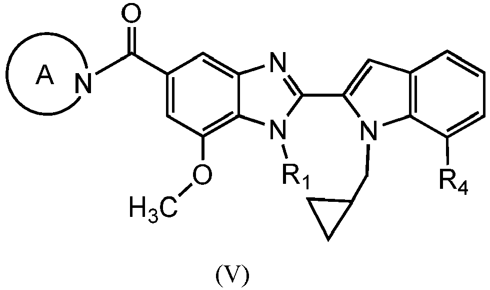
or a pharmaceutically acceptable salt thereof, wherein: 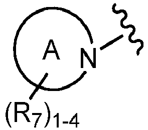 is selected from 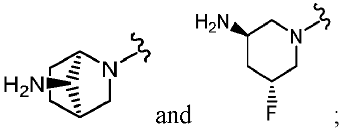
R₁ is selected from -CH₃ and 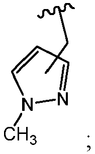
R₄ is selected from 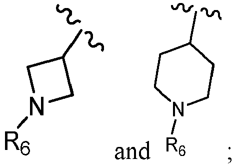
R₆, at each occurrence, is independently selected from -C(=O)R_{b}, -C(=O)(CH₂)₁₋₃OR_{b}, and -C(=O)NRₐRₐ;
Rₐ, at each occurrence, is independently selected from H, C₁-₃ alkyl substituted with 0-3 Rₑ, C₃₋₆ cycloalkyl, phenyl substituted with 0-3 Rₑ, and pyridyl;
R_{b} is selected from Hand C₁-₃ alkyl substituted with 0-3 Rₑ; and
Rₑ, at each occurrence, is independently selected from F, Cl, -OH, -OC₁₋₄alkyl, C(=O)OH, and phenyl.

13. The compound according to claim 12, having Formula (VI): 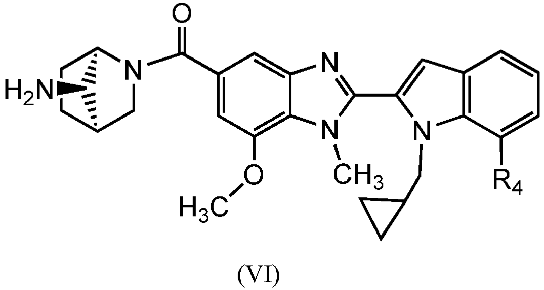
or a pharmaceutically acceptable salt thereof, wherein:
R₄ is selected from 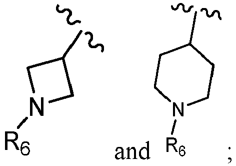 and
R₆, at each occurrence, is independently selected from -C(=O)CH₃, C(=O)(CH₂)₁₋₂OH, - C(=O)CH₂OCH₂CF₃, -C(=O)(CH₂)₁₋₃OCH₃, -C(=O)(CH₂)₁₋₃-phenyl, - C(=O)(CH₂)₁₋₃-pyridyl, -C(=O)(CH₂)₁₋₃-tetrazolyl, -C(=O)NH₂, -C(=O)NHC₁₋₃alkyl, -C(=O)NH-pyridyl, -C(=O)NH-cyclopropyl, and -C(=O)NH-phenyl substituted with 0-1 F, Cl, C₁₋₂ alkyl, and OC₁₋₂ alkyl.

14. The compound according to claim 4, having Formula (IX): 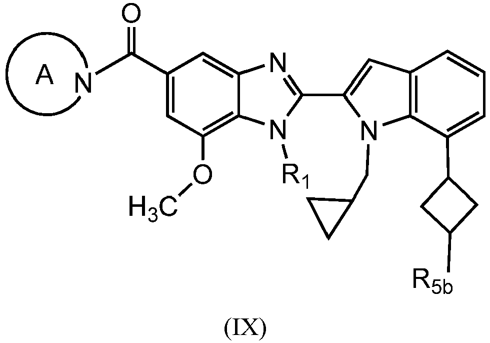
or a pharmaceutically acceptable salt thereof, wherein: 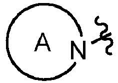 is selected from 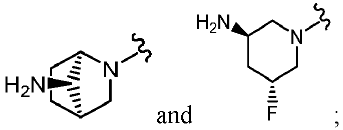
R₁ is selected from -CH₃ and 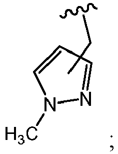
R_{5b} is selected from OH, -C(=O)NRₐRₐ, -C(=O)OR_{b}, NHC(=O)R_{b}, and NH₂,
Rₐ, at each occurrence, is independently selected from H, CH₃, and CD₃; or Rₐ and Rₐ together with the nitrogen atom to which they are both attached form a heterocyclic ring substituted with 0-2 OH; and
R_{b} is selected from H, CH₃, and CD₃.

15. The compound according to claim 4, having Formula (XI): 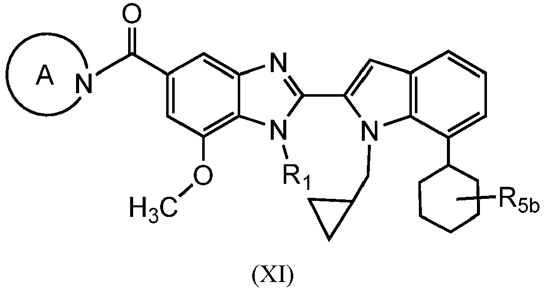
or a pharmaceutically acceptable salt thereof, wherein: 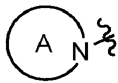 is selected from 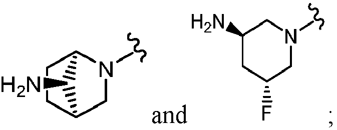
R₁ is selected from -CH₃ and 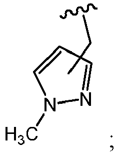 and
R_{5b}, is selected from -OH, -NH₂, and CONH₂.

16. A compound according to claim 1 which is:
(1R,4R,7R)-2-{2-[1-(cyclopropylmethyl)-7-(piperidin-4-yl)-1H-indol-2-yl]-7-methoxy-1-methyl-1H-1,3-benzodiazole-5-carbonyl}-2-azabicyclo[2.2.1]heptan-7-amine;
(1R,4R,7R)-2-{2-[7-(azetidin-3-yl)-1-(cyclopropylmethyl)-1H-indol-2-yl]-7-methoxy-1-methyl-1H-1,3-benzodiazole-5-carbonyl}-2-azabicyclo[2.2.1]heptan-7-amine;
(1R,4R,7R)-2-{2-[7-(3-aminocyclobutyl)-1-(cyclopropylmethyl)-1H-indol-2-yl]-7-methoxy-1-methyl-1H-1,3-benzodiazole-5-carbonyl}-2-azabicyclo[2.2.1]heptan-7-amine;
(1R,4R,7R)-2-{2-[1-(cyclopropylmethyl)-7-[(piperidin-4-yl)methyl]-1H-indol-2-yl]-7-methoxy-1-methyl-1H-1,3-benzodiazole-5-carbonyl}-2-azabicyclo[2.2.1]heptan-7-amine;
(1R,4R,7R)-2-{2-[1-(cyclopropylmethyl)-7-(piperidin-3-yl)-1H-indol-2-yl]-7-methoxy-1-methyl-1H-1,3-benzodiazole-5-carbonyl}-2-azabicyclo[2.2.1]heptan-7-amine;
(1R,4R,7R)-2-{2-[7-(4-aminocyclohexyl)-1-(cyclopropylmethyl)-1H-indol-2-yl]-7-methoxy-1-methyl-1H-1,3-benzodiazole-5-carbonyl}-2-azabicyclo[2.2.1]heptan-7-amine;
((1R,4R,7R)-7-amino-2-azabicyclo[2.2.1]heptan-2-yl)(2-(7-(((*trans*)-4-aminocyclohexyl)methyl)-1-(cyclopropylmethyl)-1H-indol-2-yl)-7-methoxy-1-methyl-1H-benzo[d]imidazol-5-yl)methanone;
1-[4-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)piperidin-1-yl]ethan-1-one;
*cis-* and *trans*-3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobutan-1-ol;
(1R,4R,7R)-2-{2-[1-(cyclopropylmethyl)-7-(1-methylazetidin-3-yl)-1H-indol-2-yl]-7-methoxy-1-methyl-1H-1,3-benzodiazole-5-carbonyl}-2-azabicyclo[2.2.1]heptan-7-amine;
3-[2-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)ethyl]-1,3-oxazolidin-2-one;
4-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclohexan-1-ol;
(1R,4R,7R)-2-{2-[1-(cyclopropylmethyl)-7-(oxetan-3-yl)-1H-indol-2-yl]-7-methoxy-1-methyl-1H-1,3-benzodiazole-5-carbonyl}-2-azabicyclo[2.2.1]heptan-7-amine
(1R,4R,7R)-2-(2-{7-[(azetidin-3-yl)methyl]-1-(cyclopropylmethyl)-1H-indol-2-yl}-7-methoxy-1-methyl-1H-1,3-benzodiazole-5-carbonyl)-2-azabicyclo[2.2.1]heptan-7-amine;
benzyl 3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)pyrrolidine-1-carboxylate;
(1R,4R,7R)-2-{2-[1-(cyclopropylmethyl)-7-[2-(1H-1,2,4-triazol-1-yl)ethyl]-1H-indol-2-yl]-7-methoxy-1-methyl-1H-1,3-benzodiazole-5-carbonyl}-2-azabicyclo[2.2.1]heptan-7-amine;
((1R,4R,7R)-7-amino-2-azabicyclo[2.2.1]heptan-2-yl)(2-(1-(cyclopropylmethyl)-7-(1,1-dioxidotetrahydrothiophen-3-yl)-1H-indol-2-yl)-7-methoxy-1-methyl-1H-benzo[d]imidazol-5-yl)methanone;
(1R,4R,7R)-2-{2-[1-(cyclopropylmethyl)-7-[(oxan-4-yl)methyl]-1H-indol-2-yl]-7-methoxy-1-methyl-1H-1,3-benzodiazole-5-carbonyl}-2-azabicyclo[2.2.1]heptan-7-amine;
(1R,4R,7R)-2-{2-[1-(cyclopropylmethyl)-7-[2-(piperidin-3-yl)ethyl]-1H-indol-2-yl]-7-methoxy-1-methyl-1H-1,3-benzodiazole-5-carbonyl}-2-azabicyclo[2.2.1]heptan-7-amine;
(1R,4R,7R)-2-{2-[1-(cyclopropylmethyl)-7-[2-(3,5-dimethyl-1H-pyrazol-4-yl)ethyl]-1H-indol-2-yl]-7-methoxy-1-methyl-1H-1,3-benzodiazole-5-carbonyl}-2-azabicyclo[2.2.1]heptan-7-amine;
(1R,4R,7R)-2-{2-[1-(cyclopropylmethyl)-7-[(oxan-2-yl)methyl]-1H-indol-2-yl]-7-methoxy-1-methyl-1H-1,3-benzodiazole-5-carbonyl}-2-azabicyclo[2.2.1]heptan-7-amine;
1-(3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)azetidin-1-yl)ethan-1-one;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)piperidin-1-yl]ethan-1-one;
3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)piperidine-1-carboxamide;
Methyl 3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)azetidine-1-carboxylate;
1-[3-(2-{5-[(3R,5R)-3-amino-5-fluoropiperidine-1-carbonyl]-7-methoxy-1-methyl-1H-1,3-benzodiazol-2-yl}-1-(cyclopropylmethyl)-1H-indol-7-yl)azetidin-1-yl]ethan-1-one;
1-(3-(2-(5-((2S,5R)-5-amino-2-methylpiperidine-1-carbonyl)-7-methoxy-1-methyl-1H-benzo[d]imidazol-2-yl)-1-(cyclopropylmethyl)-1H-indol-7-yl)azetidin-1-yl)ethan-1-one;
3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-methylcyclobutane-1-carboxamide;
3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobutane-1-carboxamide;
3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-N,N-dimethylcyclobutane-1-carboxamide;
(3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)(pyrrolidin-1-yl)methanone;
(3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)((R)-3-hydroxypyrrolidin-1-yl)methanone;
(3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)((S)-3-hydroxypyrrolidin-1-yl)methanone;
3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobutane-1-carboxylic acid;
N-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclobutyl]acetamide;
(1R,4R,7R)-2-[2-(7-{2-azaspiro[3.3]heptan-6-yl}-1-(cyclopropylmethyl)-1H-indol-2-yl)-7-methoxy-1-methyl-1H-1,3-benzodiazole-5-carbonyl]-2-azabicyclo[2.2.1]heptan-7-amine;
1-[6-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-2-azaspiro[3.3]heptan-2-yl]ethan-1-one;
1-{3-[(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)methyl]azetidin-1-yl}ethan-1-one;
(1R,4R,7R)-2-{2-[1-(cyclopropylmethyl)-7-(1-methanesulfonylazetidin-3-yl)-1H-indol-2-yl]-7-methoxy-1-methyl-1H-1,3-benzodiazole-5-carbonyl}-2-azabicyclo[2.2.1]heptan-7-amine;
2-amino-1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]ethan-1-one;
3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidine-1-carboxamide;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2,2-dimethylpropan-1-one;
4-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-4-oxobutanoic acid;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-hydroxy-2-methylpropan-1-one;
3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-N-phenylazetidine-1-carboxamide;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-methoxyethan-1-one;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-phenylethan-1-one;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(pyridin-4-yl)ethan-1-one;
(1R,4R,7R)-2-{2-[7-(1-benzoylazetidin-3-yl)-1-(cyclopropylmethyl)-1H-indol-2-yl]-7-methoxy-1-methyl-1H-1,3-benzodiazole-5-carbonyl}-2-azabicyclo[2.2.1]heptan-7-amine;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(pyridin-2-yl)ethan-1-one;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(pyrazin-2-yl)ethan-1-one;
3-[3-(2-{5-[(1R,4R,7R)-7-amino-2-azabicyclo[2.2.1]heptane-2-carbonyl]-7-methoxy-1 -methyl- 1H-1,3 -benzodiazol-2-yl} -1 -(cyclopropylmethyl)- 1H-indol-7-yl)azetidin-1-yl]-3-oxopropanenitrile;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(pyridin-3-yl)ethan-1-one;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-3-phenylpropan-1-one;
1-[3-(2-{5-[(1R,4R,7R)-7-amino-2-azabicyclo[2.2.1]heptane-2-carbonyl]-7-methoxy-1 -methyl- 1H-1,3 -benzodiazol-2-yl} -1 -(cyclopropylmethyl)- 1H-indol-7-yl)azetidin-1-yl]-3-hydroxypropan-1-one;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-(1H-1,2,3,4-tetrazol-5-yl)ethan-1-one;
1-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)azetidin-1-yl]-2-hydroxyethan-1-one
(1R,4R,7R)-2-(2-{7-[1-(benzenesulfonyl)azetidin-3-yl]-1-(cyclopropylmethyl)-1H-indol-2-yl}-7-methoxy-1-methyl-1H-1,3-benzodiazole-5-carbonyl)-2-azabicyclo[2.2.1]heptan-7-amine
(1R,4R,7R)-2-{2-[1-(cyclopropylmethyl)-7-(1,2,3,6-tetrahydropyridin-4-yl)-1H-indol-2-yl]-7-methoxy-1-methyl-1H-1,3-benzodiazole-5-carbonyl}-2-azabicyclo[2.2.1]heptan-7-amine
3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)cyclohexane-1-carboxamide;
2-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)phenol
N-[3-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)phenyl]prop-2-enamide
N-{[4-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)phenyl]methyl}acetamide
4-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-2-fluorophenol
4-(2-{5-[(1R,4R,7R)-7-amino-2-azabicyclo[2.2.1]heptane-2-carbonyl]-7-methoxy-1 -methyl- 1H-1,3 -benzodiazol-2-yl} -1 -(cyclopropylmethyl)- 1H-indol-7-yl)-2-methoxyphenol;
4-(2-{5-[(1R,4R,7R)-7-amino-2-azabicyclo[2.2.1]heptane-2-carbonyl]-7-methoxy-1 -methyl- 1H-1,3 -benzodiazol-2-yl} -1 -(cyclopropylmethyl)- 1H-indol-7-yl)-2-chlorophenol;
4-(2-{5-[(1R,4R,7R)-7-amino-2-azabicyclo[2.2.1]heptane-2-carbonyl]-7-methoxy-1 -methyl- 1H-1,3 -benzodiazol-2-yl} -1 -(cyclopropylmethyl)- 1H-indol-7-yl)-3-chlorophenol;
4-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-2,5-difluorophenol;
6-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-4-methyl-1,2-dihydroquinolin-2-one;
4-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-2,3-dihydro-1H-isoindol-1-one;
2'-{5-[(1R,4R,7R)-7-amino-2-azabicyclo[2.2.1]heptane-2-carbonyl]-7-methoxy-1-methyl-1H-1,3-benzodiazol-2-yl}-1'-(cyclopropylmethyl)-2,3-dihydro-1H,1'H-[5,7'-biindole]-2-one;
6-(2-{5-[(1R,4R,7R)-7-amino-2-azabicyclo[2.2.1]heptane-2-carbonyl]-7-methoxy-1 -methyl- 1H-1,3 -benzodiazol-2-yl} -1 -(cyclopropylmethyl)- 1H-indol-7-yl)-2,3-dihydro-1H-isoindol-1-one;
6-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-1,2,3,4-tetrahydroquinolin-2-one;
6-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-1,2-dihydroquinolin-2-one;
methyl 6-(2-{ 5-[(1R,4R,7R)-7-amino-2-azabicyclo[2.2. 1]heptane-2-carbonyl]-7-methoxy-1-methyl-1H-1,3-benzodiazol-2-yl}-1-(cyclopropylmethyl)-1H-indol-7-yl)-2-oxo-1,2-dihydroquinoline-4-carboxylate;
6-(2-{5-[(1R,4R,7R)-7-amino-2-azabicyclo[2.2.1]heptane-2-carbonyl]-7-methoxy-1 -methyl- 1H-1,3 -benzodiazol-2-yl} -1 -(cyclopropylmethyl)- 1H-indol-7-yl)-1,2-dihydroisoquinolin-1-one;
5-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)-2,3-dihydro-1H-isoindol-1-one;
4-(2-{5-[(1R,4R,7R)-7-amino-2-azabicyclo[2.2.1]heptane-2-carbonyl]-7-methoxy-1 -methyl- 1H-1,3 -benzodiazol-2-yl} -1 -(cyclopropylmethyl)- 1H-indol-7-yl)-2-chlorobenzamide;
methyl N-[5-(2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)pyridin-2-yl]carbamate;
N-[5-(2-{5-[(1R,4R,7R)-7-amino-2-azabicyclo[2.2.1]heptane-2-carbonyl]-7-methoxy-1 -methyl- 1H-1,3 -benzodiazol-2-yl} -1 -(cyclopropylmethyl)- 1H-indol-7-yl)pyridin-2-yl]acetamide;
diethyl (2-{5-[(1R,4R,7R)-7-amino-2-azabicyclo[2.2.1]heptane-2-carbonyl]-7-methoxy-1-methyl-1H-1,3-benzodiazol-2-yl}-1-(cyclopropylmethyl)-1H-indol-7-yl)phosphonate;
2-{4-[2-(5-{7-amino-2-azabicyclo[2.2.1]heptane-2-carbonyl}-7-methoxy-1-methyl-1H-1,3-benzodiazol-2-yl)-1-(cyclopropylmethyl)-1H-indol-7-yl]-1H-1,2,3 -tri azol -1 -yl} ethan-1 -ol;
(1R,4R,7R)-2-{2-[1-(cyclopropylmethyl)-7-{4-[(1r,4r)-4-aminocyclohexyl]-1H-1,2,3-triazol-1-yl}-1H-indol-2-yl]-7-methoxy-1-methyl-1H-1,3-benzodiazole-5-carbonyl}-2-azabicyclo[2.2.1]heptan-7-amine;
(1R,4R,7R)-2-(2-{7-[4-(azetidin-3-yl)-1H-1,2,3-triazol-1-yl]-1-(cyclopropylmethyl)-1H-indol-2-yl}-7-methoxy-1-methyl-1H-1,3-benzodiazole-5-carbonyl)-2-azabicyclo[2.2.1]heptan-7-amine;
3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N-methylcyclobutane-1-carboxamide;
3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)cyclobutane-1-carboxamide;
((3R,5R)-3-amino-5-fluoropiperidin-1-yl)(2-(1-(cyclopropylmethyl)-7-(3-(pyrrolidine-1-carbonyl)cyclobutyl)-1H-indol-2-yl)-7-methoxy-1-methyl-1H-benzo[d]imidazol-5-yl)methanone;
3-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-N,N-dimethylcyclobutane-1-carboxamide;
((1R,4R,7R)-7-amino-2-azabicyclo[2.2.1]heptan-2-yl)(2-(1-(cyclopropylmethyl)-7-(4-hydroxy-3-(hydroxymethyl)phenyl)-1H-indol-2-yl)-7-methoxy-1-methyl-1H-benzo[d]imidazol-5-yl)methanone;
5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzamide;
5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-hydroxybenzoic acid;
((1R,4R,7R)-7-amino-2-azabicyclo[2.2.1]heptan-2-yl)(2-(1-(cyclopropylmethyl)-7-(1-methyl-1H-pyrazol-4-yl)-1H-indol-2-yl)-7-methoxy-1-methyl-1H-benzo[d]imidazol-5-yl)methanone;
ethyl 2-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)benzoate;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)benzamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N,N-dimethylbenzamide;
((7R)-7-amino-2-azabicyclo[2.2.1]heptan-2-yl)(2-(1'-(cyclopropylmethyl)-1-methyl-1H,1'H-[6,7'-biindol]-2'-yl)-7-methoxy-1-methyl-1H-benzo[d]imidazol-5-yl)methanone;
((1R,4R,7R)-7-amino-2-azabicyclo[2.2.1]heptan-2-yl)(2-(1-(cyclopropylmethyl)-7-(1H-pyrazol-3-yl)-1H-indol-2-yl)-7-methoxy-1-methyl-1H-benzo[d]imidazol-5-yl)methanone;
((7R)-7-amino-2-azabicyclo[2.2.1]heptan-2-yl)(2-(1-(cyclopropylmethyl)-7-(1-(2-hydroxy-3-(prop-2-yn-1-yloxy)propyl)-1H-pyrazol-4-yl)-1H-indol-2-yl)-7-methoxy-1-methyl-1H-benzo[d]imidazol-5-yl)methanone;
((7R)-7-amino-2-azabicyclo[2.2.1]heptan-2-yl)(2-(1-(cyclopropylmethyl)-7-(furan-3-yl)-1H-indol-2-yl)-7-methoxy-1-methyl-1H-benzo[d]imidazol-5-yl)methanone;
((7R)-7-amino-2-azabicyclo[2.2.1]heptan-2-yl)(2-(1-(cyclopropylmethyl)-7-(furan-2-yl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-(cyclopropylmethyl)-7-(thiophen-2-yl)-1H-indol-2-yl)-7-methoxy-1-methyl-1H-benzo[d]imidazol-5-yl)methanone;
((7R)-7-amino-2-azabicyclo[2.2.1]heptan-2-yl)(2-(1-(cyclopropylmethyl)-7-(1-(tetrahydro-2H-pyran-4-yl)-1H-pyrazol-4-yl)-1H-indol-2-yl)-7-methoxy-1-methyl-1H-benzo[d]imidazol-5-yl)methanone;
N-(4-(2-(5-((7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)phenyl)acetamide;
((7R)-7-amino-2-azabicyclo[2.2.1]heptan-2-yl)(2-(1-(cyclopropylmethyl)-7-(pyridin-3-yl)-1H-indol-2-yl)-7-methoxy-1-methyl-1H-benzo[d]imidazol-5-yl)methanone;
((7R)-7-amino-2-azabicyclo[2.2.1]heptan-2-yl)(2-(1-(cyclopropylmethyl)-7-(3-(2-fluorophenyl)-1-methyl-1H-pyrazol-4-yl)-1H-indol-2-yl)-7-methoxy-1-methyl-1H-benzo[d]imidazol-5-yl)methanone;
((7R)-7-amino-2-azabicyclo[2.2.1]heptan-2-yl)(2-(1-(cyclopropylmethyl)-7-(3-(hydroxymethyl)phenyl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-(cyclopropylmethyl)-7-(1H-pyrazol-4-yl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-(cyclopropylmethyl)-7-(1-((R)-2-hydroxypropyl)-1H-pyrazol-4-yl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-(cyclopropylmethyl)-7-(1-(2-hydroxy-2-methylpropyl)-3-methyl-1H-pyrazol-4-yl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-(cyclopropylmethyl)-7-(1-(2-hydroxy-2-methylpropyl)-1H-pyrazol-4-yl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-(cyclopropylmethyl)-7-(1-(2-hydroxyethyl)-1H-pyrazol-4-yl)-1H-indol-2-yl)-7-methoxy-1-methyl-1H-benzo[d]imidazol-5-yl)methanone;
6-(2-(5-((1R,4S)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-4,4-dimethyl-1,4-dihydro-2H-benzo[d][1,3]oxazin-2-one;
2-(2-(5-((1R,4S)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)benzonitrile;
5-(2-(5-((1R,4R,7R)-7-amino-2-azabicyclo[2.2.1]heptane-2-carbonyl)-7-methoxy-1-methyl-1H-benzo[d]imidazol-2-yl)-1-((2,2-difluorocyclopropyl)methyl)-1H-indol-7-yl)pyrimidine-2-carbonitrile;
((1R,4R,7R)-7-amino-2-azabicyclo[2.2.1]heptan-2-yl)(2-(1-((2,2-difluorocyclopropyl)methyl)-7-(1H-pyrazol-4-yl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-((2,2-dichlorocyclopropyl)methyl)-7-(1H-pyrazol-4-yl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-((2,2-dichlorocyclopropyl)methyl)-7-(1H-pyrazol-3-yl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-(cyclopropylmethyl)-5-methyl-7-(1H-pyrazol-4-yl)-1H-indol-2-yl)-7-methoxy-1-methyl-1H-benzo[d]imidazol-5-yl)methanone;
2-(2-(5-((1R,4R,7R)-7-amino-2-azabicyclo[2.2.1]heptane-2-carbonyl)-7-methoxy-1-methyl-1H-benzo[d]imidazol-2-yl)-1-((2-methylcyclopropyl)methyl)-1H-indol-7-yl)benzonitrile;
((1R,4R,7R)-7-amino-2-azabicyclo[2.2.1]heptan-2-yl)(7-methoxy-1-methyl-2-(1-((2-methylcyclopropyl)methyl)-7-(1H-pyrazol-4-yl)-1H-indol-2-yl)-1H-benzo[d]imidazol-5-yl)methanone;
((1R,4R,7R)-7-amino-2-azabicyclo[2.2.1]heptan-2-yl)(7-methoxy-1-methyl-2-(1-((2-methylcyclopropyl)methyl)-7-(1H-pyrazol-3-yl)-1H-indol-2-yl)-1H-benzo[d]imidazol-5-yl)methanone;
((1R,4R,7R)-7-amino-2-azabicyclo[2.2.1]heptan-2-yl)(2-(1-(cyclopropylmethyl)-7-(2-(hydroxymethyl)phenyl)-1H-indol-2-yl)-7-methoxy-1-methyl-1H-benzo[d]imidazol-5-yl)methanone;
((1R,4R,7R)-7-amino-2-azabicyclo[2.2.1]heptan-2-yl)(2-(1-(cyclopropylmethyl)-7-phenyl-1H-indol-2-yl)-7-methoxy-1-methyl-1H-benzo[d]imidazol-5-yl)methanonemethyl-1H-benzo[d]imidazol-5-yl)methanone;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-phenylpiperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N,N-dimethylpiperidine-1-carboxamide;
1-(4-(2-(5-((3R,SR)-3-amino-5-fluoropiperidine-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)piperidin-1-yl)ethan-1-one;
5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-3,3-difluoroindolin-2-one;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-3-fluorobenzamide;
5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-3-hydroxy-3-methylindolin-2-one;
2-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-5-hydroxybenzonitrile;
5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)picolinamide;
3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N,N-bis(methyl-d3)cyclobutane-1-carboxamide;
((1R,4R,7R)-7-amino-2-azabicyclo[2.2.1]heptan-2-yl)(2-(1-(cyclopropylmethyl)-7-(6-hydroxypyridin-3-yl)-1H-indol-2-yl)-7-methoxy-1-methyl-1H-benzo[d]imidazol-5-yl)methanone;
5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-3-hydroxy-3-(trifluoromethyl)indolin-2-one;
5-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-3-fluoropicolinamide;
(3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)(3-fluoroazetidin-1-yl)methanone
(3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)cyclobutyl)(azetidin-1-yl)methanone;
6-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-7-methoxy-3,4-dihydroquinolin-2(1H)-one;
1-(4-(2-(5-((3R,SR)-3-amino-5-fluoropiperidine-1-carbonyl)-7-methoxy-1-methyl-1H-benzo[d]imidazol-2-yl)-1-(cyclopropylmethyl)-1H-indol-7-yl)piperidin-1-yl)ethan-1-one;
((3R,5R)-3-amino-5-fluoropiperidin-1-yl)(2-(1-(cyclopropylmethyl)-7-(3-fluoro-4-hydroxyphenyl)-1H-indol-2-yl)-7-methoxy-1-methyl-1H-benzo[d]imidazol-5-yl)methanone;
5-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)indolin-2-one;
5-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)-3-hydroxy-3-methylindolin-2-one;
5-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)isoindolin-1-one;
1-(3-(2-(5-((1R,4R,7R)-7-amino-2-azabicyclo[2.2.1]heptane-2-carbonyl)-7-methoxy-1-((1-methyl-1H-pyrazol-4-yl)methyl)-1H-benzo[d]imidazol-2-yl)-1-(cyclopropylmethyl)-1H-indol-7-yl)azetidin-1-yl)ethan-1-one;
methyl 3-(2-(5-((3R,5R)-3-amino-5-fluoropiperidine-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)azetidine-1-carboxylate;
1-(3-(2-(5-((3R,5R)-3-amino-5-fluoropiperidine-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)azetidin-1-yl)ethan-1-one;
methyl 3-(2-(5-((1R,4R,7R)-7-amino-2-azabicyclo[2.2.1]heptane-2-carbonyl)-7-methoxy-1-((1-methyl-1H-pyrazol-4-yl)methyl)-1H-benzo[d]imidazol-2-yl)-1-(cyclopropylmethyl)-1H-indol-7-yl)azetidine-1-carboxylate;
1-(3-(2-(5-((1R,4R,7R)-7-amino-2-azabicyclo[2.2.1]heptane-2-carbonyl)-7-methoxy-1-((1-methyl-1H-pyrazol-4-yl)methyl)-1H-benzo[d]imidazol-2-yl)-1-methyl-1H-indol-7-yl)azetidin-1-yl)ethan-1-one;
1-(3-(2-(5-((3R,5R)-3-amino-5-fluoropiperidine-1-carbonyl)-7-methoxy-1-((1-methyl-1H-pyrazol-4-yl)methyl)-1H-benzo[d]imidazol-2-yl)-1-methyl-1H-indol-7-yl)azetidin-1-yl)ethan-1-one;
methyl 3-(2-(5-((1R,4R,7R)-7-amino-2-azabicyclo[2.2.1]heptane-2-carbonyl)-7-methoxy-1-((1-methyl-1H-pyrazol-4-yl)methyl)-1H-benzo[d]imidazol-2-yl)-1-methyl-1H-indol-7-yl)azetidine-1-carboxylate;
methyl 4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidine-1-carboxylate;
((1R,4R,7R)-7-amino-2-azabicyclo[2.2.1]heptan-2-yl)(2-(1-(cyclopropylmethyl)-7-(1-hydroxycyclobutyl)-1H-indol-2-yl)-7-methoxy-1-methyl-1H-benzo[d]imidazol-5-yl)methanone;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-3,6-dihydropyridin-1(2H)-yl)ethan-1-one;
methyl 4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-3,6-dihydropyridine-1(2H)-carboxylate;
((1R,4R,7R)-7-amino-2-azabicyclo[2.2.1]heptan-2-yl)(2-(1-(cyclopropylmethyl)-7-(3-(1,2-dihydroxyethyl)phenyl)-1H-indol-2-yl)-7-methoxy-1-methyl-1H-benzo[d]imidazol-5-yl)methanone;
N-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-2-(hydroxymethyl)benzyl)acetamide;
(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)(spiro[2.2]pentan-1-yl)methanone;
(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)(cyclopropyl)methanone;
1-(4-(2-(5-((3R,SR)-3-amino-5-fluoropiperidine-1-carbonyl)-7-methoxy-1-methyl-1H-benzo[d]imidazol-2-yl)-1-(cyclopropylmethyl)-1H-indol-7-yl)piperidin-1-yl)-2-methoxyethan-1-one;
1-(4-(2-(5-((3R,5R)-3-amino-5-fluoropiperidine-1-carbonyl)-7-methoxy-1-methyl-1H-benzo[d]imidazol-2-yl)-1-(cyclopropylmethyl)-1H-indol-7-yl)piperidin-1-yl)ethan-1-one;
3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-phenylazetidine-1-carboxamide;
3-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-(4-fluorophenyl)azetidine-1-carboxamide;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-2-methoxyethan-1-one;
3-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-3-oxopropanenitrile;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-2-(2,2,2-trifluoroethoxy)ethan-1-one;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-2-cyclopropyl-2-methoxyethan-1-one;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-2-ethoxyethan-1-one;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-2-phenoxyethan-1-one;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-3-methoxypropan-1-one;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-4-methoxybutan-1-one;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-2-methoxypropan-1-one;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-2-isopropoxyethan-1-one;
1-(4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidin-1-yl)-2-hydroxyethan-1-one;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-(4-fluorophenyl)piperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-(3-fluorophenyl)piperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-(2-fluorophenyl)piperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-(pyridin-3-yl)piperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-(pyridin-4-yl)piperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-(4-methoxyphenyl)piperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-(2-methoxyphenyl)piperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-(3-methoxyphenyl)piperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-cyclopropylpiperidine-1-carboxamide;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-ethylpiperidine-1-carboxamid;;
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)piperidine-1-carboxamide; or
4-(2-(5-((1R,4R,7R)-7-amino-2-azabicyclo[2.2.1]heptane-2-carbonyl)-7-methoxy-1-methyl-1H-benzo[d]imidazol-2-yl)-1-(cyclopropylmethyl)-1H-indol-7-yl)-N-isopropylpiperidine-1-carboxamide.

17. A pharmaceutically acceptable composition comprising the compound according to any of claims 1 to 16, and a pharmaceutically acceptable carrier, adjuvant, or vehicle, wherein the composition is optionally in combination with an additional therapeutic agent.

18. A method of inhibiting PAD4 in a biological sample in vitro comprising the step of contacting the PAD4 with a compound according to any of claims 1 to 16.

19. A compound according to any one of claims 1 to 16 or composition according to claim 17 for use in therapy, preferably for use in a method of treating a PAD4-mediated disease, disorder, or condition selected from the group consisting of acute lymphocytic leukemia, ankylosing spondylitis, cancer, chronic lymphocytic leukemia, colitis, lupus, rheumatoid arthritis, multiple sclerosis, and ulcerative colitis, in a subject having the PAD4-mediated disease, disorder, or condition, the method comprising the step of administering to said subject the compound or composition, more preferably wherein the PAD4-mediated disease, disorder, or condition is selected from rheumatoid arthritis, systemic lupus erythematosus, cutaneous lupus erythematosus, ulcerative colitis, and cancer.

## Patentansprüche

1. Verbindung der Formel (III): 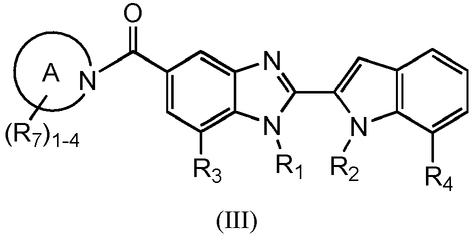
oder ein pharmazeutisch verträgliches Salz davon, wobei: 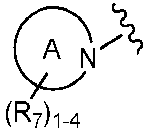 ausgewählt ist aus 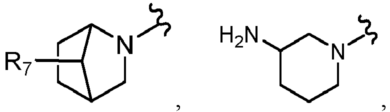 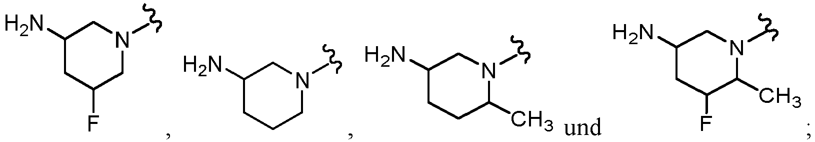
R₁ ausgewählt ist aus -CH₃, -CD₃ und -CH₂-5- bis -6-gliedriges-Heterocyclyl, umfassend Kohlenstoffatome und 1-3 Heteroatome, ausgewählt aus N, NH und NC₁₋₃-Alkyl;
R₂ ausgewählt ist aus CH₃, CH₃CH₂ und -CH₂-Cyclopropyl, substituiert mit 0-3 Rₑ;
R₃ ausgewählt ist aus H, F, Cl, Br und -OC₁₋₄-Alkyl;
R₄ ausgewählt ist aus 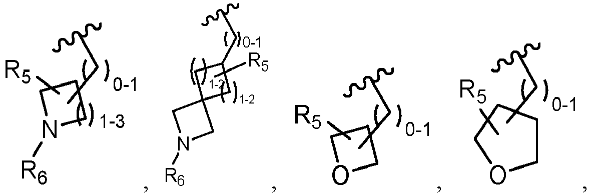 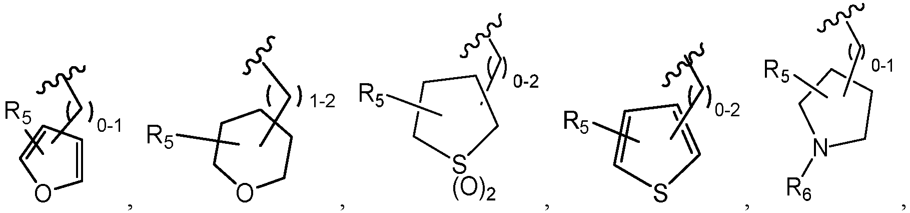 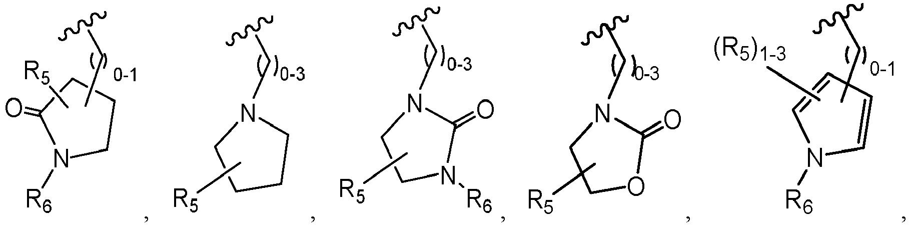 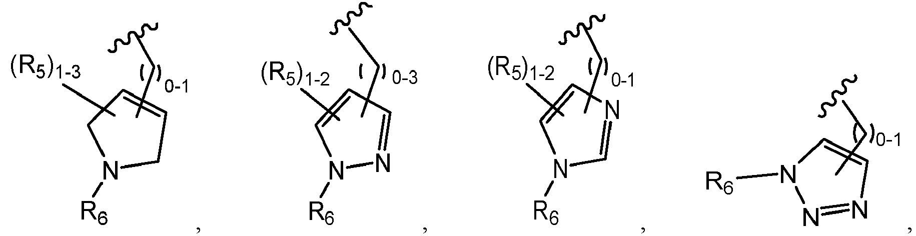 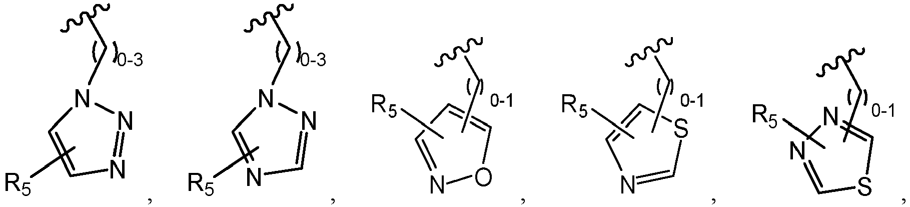 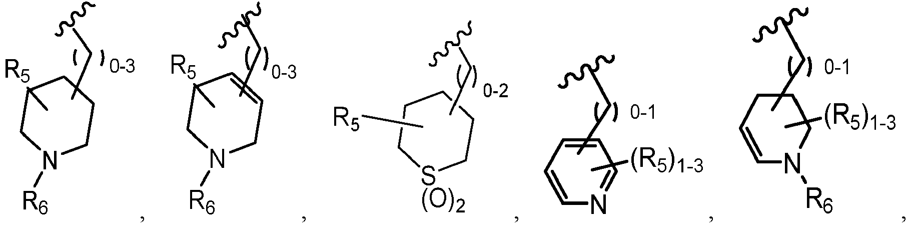 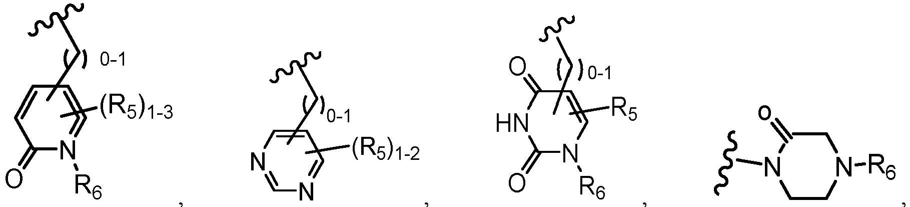 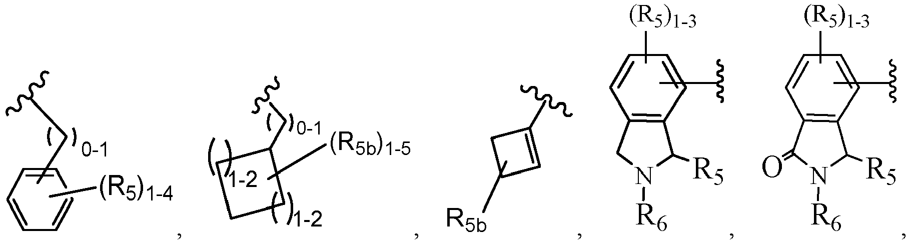 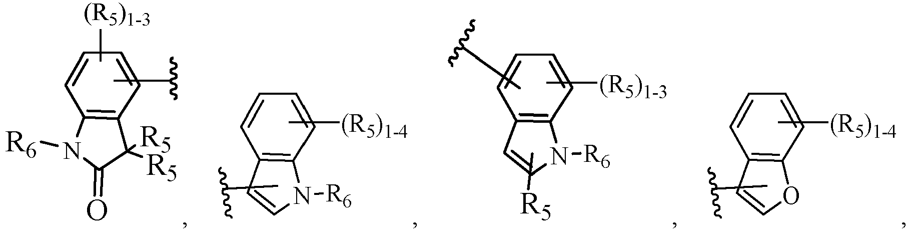 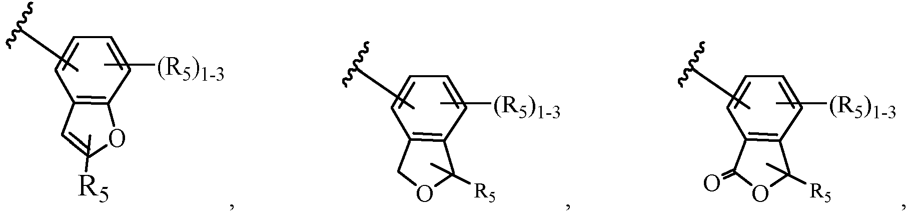 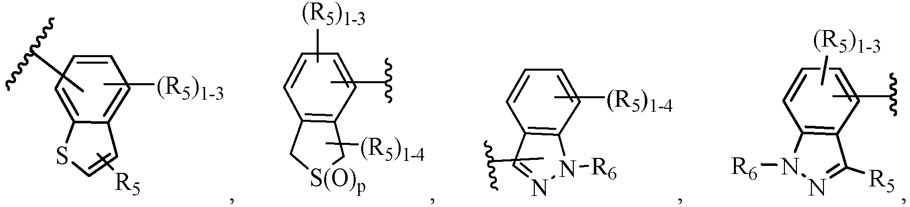 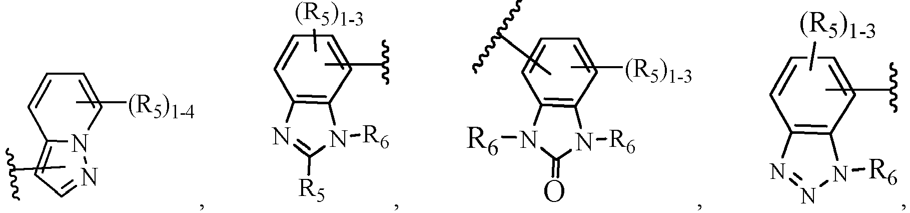 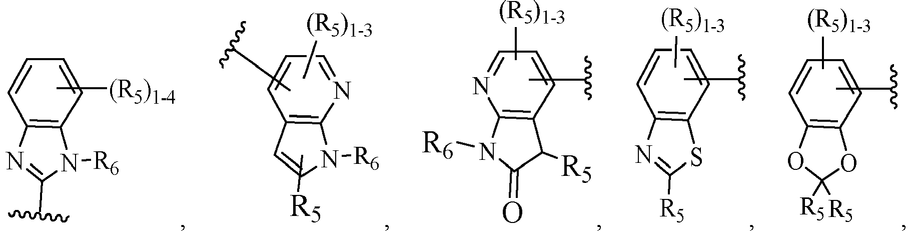 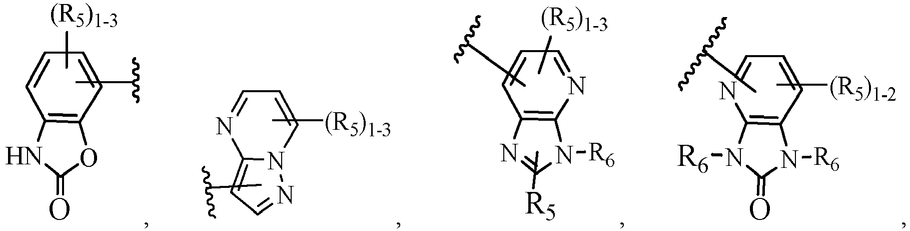 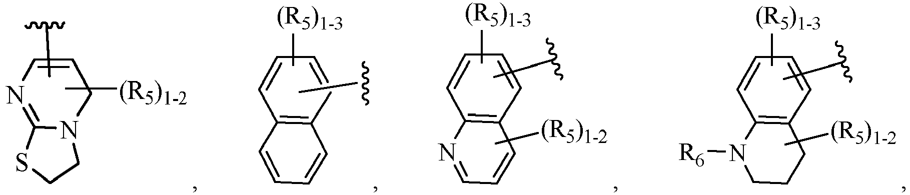 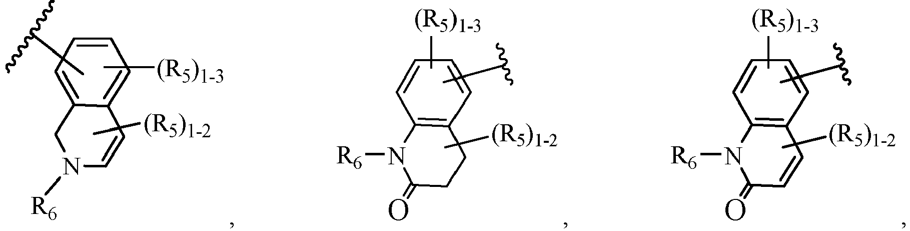 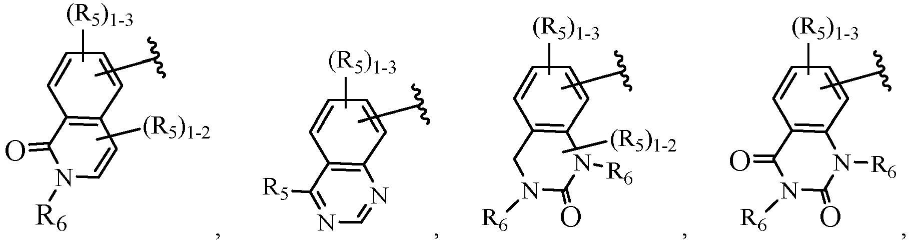 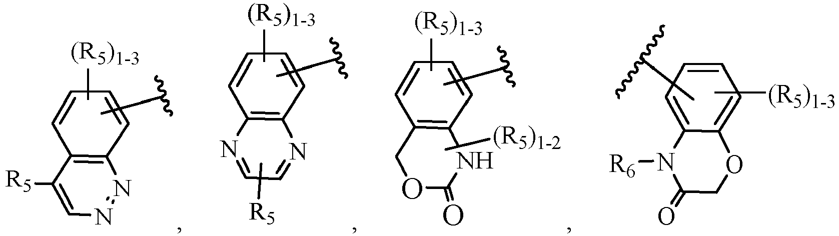 und 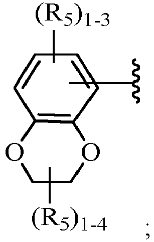
R₅, bei jedem Auftreten, unabhängig ausgewählt ist aus H, F, Cl, Br, C₁₋₄-Alkyl, substituiert mit 0-5 Rₑ, C₂₋₄-Alkenyl, C₂₋₄-Alkinyl, Nitro, -(CH₂)ᵣOR_{b}, -CN, -NRₐRₐ, -(CH₂)ᵣNRₐC(=O)R_{b}, -NRₐC(=O)NRₐRₐ, -C(=O)OR_{b}, -C(=O)R_{b}, -OC(=O)R_{b}, -C(=O)NRₐRₐ, -P(=O)(C₁₋₄-Alkyl)₂, -S(O)*ₚ*R_{c}, -S(O)*ₚ*NRₐRₐ, -NRₐS(O)*ₚ*R_{c}, -C₃₋₆-Cycloalkyl, substituiert mit 0-4 Rₑ, Aryl, substituiert mit 0-4 Rₑ, und Heterocyclyl, substituiert mit 0-4 Rₑ;
R_{5b}, bei jedem Auftreten, unabhängig ausgewählt ist aus OR_{b}, -C(=O)OR_{b}, -C(=O)NRₐRₐ, -NRₐRₐ, -NRₐC(=O)R_{b}, -NRₐC(=O)OR_{b}, -S(=O)ₚR_{c} und -NRₐS(=O)ₚR_{c};
R₆, bei jedem Auftreten, unabhängig ausgewählt ist aus H, C₁₋₃-Alkyl, substituiert mit 0-4 Rₑ, -C(=O)R_{b}, -C(=O)(CH₂)ᵣOR_{b}, -C(=O)(CH₂)ᵣNRₐRₐ, -S(O)ₚR_{c}, -S(O)ₚNRₐRₐ, -(CH₂)ᵣ-Aryl, substituiert mit 0-4 Rₑ, und -(CH₂)ᵣ-Heterocyclyl, substituiert mit 0-4 Rₑ;
R₇ ausgewählt ist aus H, F, Cl, CN, C₁₋₃-Alkyl, =N-ORb, -(CH₂)ᵣOR_{b}, -(CH₂)ᵣNRₐRₐ, -NRₐC(=NH)C₁₋₃-Alkyl, -NRₐC(=O)OR_{b}, einem Carbocyclyl und Heterocyclyl;
Rₐ, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ; oder Rₐ und Rₐ zusammen mit dem Stickstoffatom, an das sie beide gebunden sind, einen heterocyclischen Ring bilden, der substituiert ist mit 0-5 Rₑ;
R_{b}, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ;
R_{c}, bei jedem Auftreten, unabhängig ausgewählt ist aus C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₆-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig ausgewählt ist aus C₁₋₆-Alkyl, substituiert mit 0-5 Rf, C₂₋₆-Alkenyl, C₂₋₆-Alkinyl, -(CH₂)ᵣ-C₃₋₆-Cycloalkyl, -(CH₂)ᵣ-Aryl, F, Cl, Br, CN, NO₂, =O, -C(=O)OH, -C(=O)OC₁₋₄-Alkyl, -(CH₂)ᵣOH, -(CH₂)ᵣOC₁₋₄-Alkyl, -(CH₂)ᵣOC₂₋₄-Alkenyl, -(CH₂)ᵣOC₂₋₄-Alkinyl und NH₂;
Rf, bei jedem Auftreten, unabhängig ausgewählt ist aus H, F, Cl, Br, CN, OH, C₁₋₅-Alkyl, wahlweise substituiert mit OH, C₃₋₆-Cycloalkyl und Phenyl;
p, bei jedem Auftreten, unabhängig ausgewählt ist aus null, 1 und 2; und
r, bei jedem Auftreten, unabhängig ausgewählt ist aus null, 1, 2, 3 und 4.

2. Verbindung nach Anspruch 1, oder ein pharmazeutisch verträgliches Salz davon, wobei: 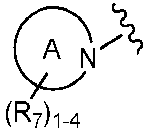 ausgewählt ist aus 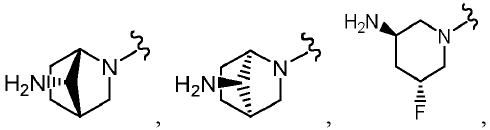 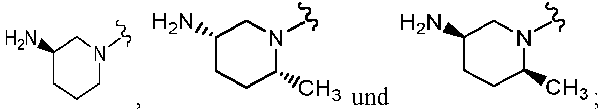
R₁ ausgewählt ist aus -CH₃ und -CD₃;
R₂ ausgewählt ist aus -CH₃ und -CH₂-Cyclopropyl, substituiert mit 0-2 F oder Cl;
R₃ ausgewählt ist aus H, F und -OC₁₋₄-Alkyl;
R₄ ausgewählt ist aus 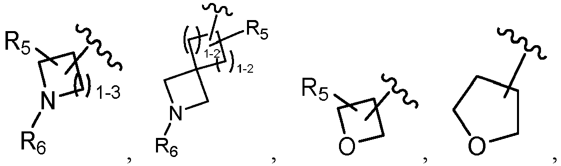 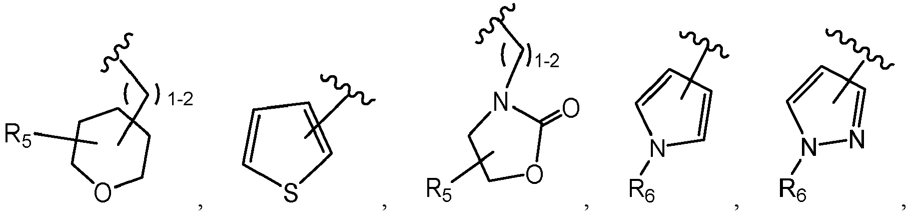 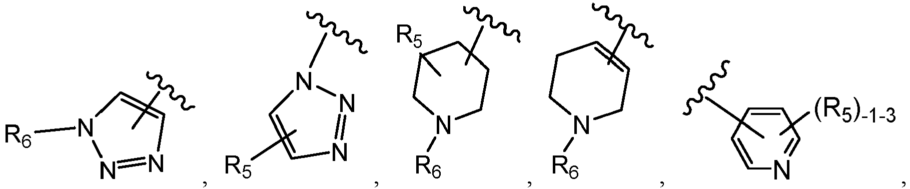 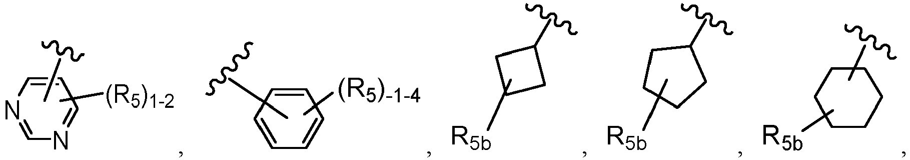 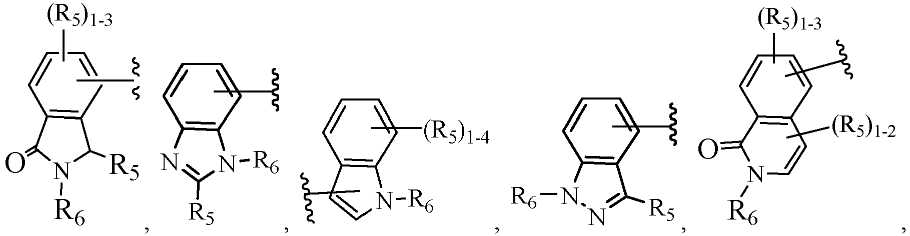 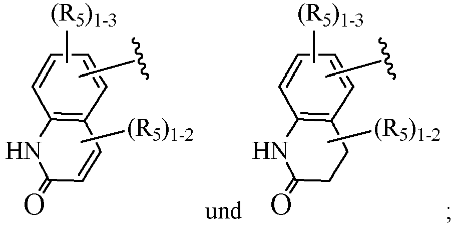
R₅, bei jedem Auftreten, unabhängig ausgewählt ist aus H, F, Cl, Br, C₁₋₄-Alkyl, -(CH₂)ᵣOR_{b}, -CN, -NRₐRₐ, -(CH₂)ᵣNRₐC(=O)R_{b}, -NRₐC(=O)NRₐRₐ, -C(=O)OR_{b}, -C(=O)R_{b}, -OC(=O)R_{b}, -C(=O)NRₐRₐ, -S(O)*ₚ*R_{c}, -S(O)*ₚ*NRₐRₐ, -NRₐS(O)*ₚ*R_{c}, C₃₋₆-Cycloalkyl, substituiert mit 0-4 Rₑ, Aryl, substituiert mit 0-4 Rₑ, und Heterocyclyl, substituiert mit 0-4 Rₑ;
R_{5b}, bei jedem Auftreten, unabhängig ausgewählt ist aus OH, -C(=O)OR_{b}, -C(=O)NRₐRₐ, -NRₐRₐ, -NRₐC(=O)R_{b} und -NRₐC(=O)OR_{b};
R₆, bei jedem Auftreten, unabhängig ausgewählt ist aus H, C₁₋₃-Alkyl, -S(O)ₚR_{c}, -C(=O)R_{b}, -C(=O)OR_{b}, -C(=O)(CH₂)ᵣNRₐRₐ, -S(O)*ₚ*NRₐRₐ, Aryl, substituiert mit 0-4 Rₑ, und Heterocyclyl, substituiert mit 0-4 Rₑ;
Rₐ, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ; oder Rₐ und Rₐ zusammen mit dem Stickstoffatom, an das sie beide gebunden sind, einen heterocyclischen Ring bilden, der substituiert ist mit 0-5 Rₑ;
R_{b}, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ;
R_{c}, bei jedem Auftreten, unabhängig ausgewählt ist aus C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₆-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig ausgewählt ist aus C₁₋₆-Alkyl, substituiert mit 0-5 Rf, C₂₋₆-Alkenyl, C₂₋₆-Alkinyl, -(CH₂)ᵣ-C₃₋₆-Cycloalkyl, -(CH₂)ᵣ-Aryl, F, Cl, Br, CN, NO₂, =O, -C(=O)OH, -C(=O)OC₁₋₄-Alkyl, -(CH₂)ᵣOH, -(CH₂)ᵣOC₁₋₄-Alkyl, -(CH₂)ᵣOC₂₋₄-Alkenyl, -(CH₂)ᵣOC₂₋₄-Alkinyl und NH₂;
Rf, bei jedem Auftreten, unabhängig ausgewählt ist aus H, F, Cl, Br, CN, OH, C₁₋₅-Alkyl, wahlweise substituiert mit OH, C₃₋₆-Cycloalkyl und Phenyl;
p, bei jedem Auftreten, unabhängig ausgewählt ist aus null, 1 und 2; und
r, bei jedem Auftreten, unabhängig ausgewählt ist aus null, 1, 2, 3 und 4.

3. Verbindung nach Anspruch 2, oder ein pharmazeutisch verträgliches Salz davon, wobei:
R₁ -CH₃ ist;
R₂ ausgewählt ist aus -CH₃ und -CH₂-Cyclopropyl;
R₃ -OC₁₋₄-Alkyl ist;
R₄ ausgewählt ist aus 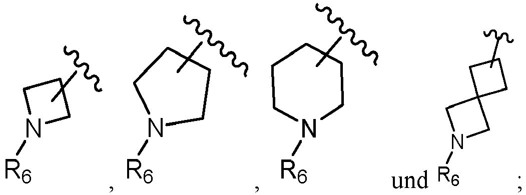 R₆ ausgewählt ist aus H, C₁₋₃-Alkyl, -C(=O)R_{b}, -C(=O)OR_{b}, -C(=O)NRₐRₐ und -C(=O)CH₂NRₐRₐ und -S(O)ₚR_{c};
Rₐ, bei jedem Auftreten, unabhängig ausgewählt ist aus H, C₁₋₆-Alkyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ; oder Rₐ und Rₐ zusammen mit dem Stickstoffatom, an das sie beide gebunden sind, einen heterocyclischen Ring bilden, der substituiert ist mit 0-5 Rₑ;
R_{b}, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig C₁₋₆-Alkyl, substituiert mit 0-5 Rₑ, -(CH₂)ᵣ-C₃₋₆-Carbocyclyl, substituiert mit 0-5 Rₑ, oder -(CH₂)ᵣ-Heterocyclyl, substituiert mit 0-5 Rₑ, ist;
Rₑ, bei jedem Auftreten, unabhängig ausgewählt ist aus C₁₋₆-Alkyl, substituiert mit 0-5 Rf, C₂₋₆-Alkenyl, C₂₋₆-Alkinyl, -(CH₂)ᵣ-C₃₋₆-Cycloalkyl, -(CH₂)ᵣ-Aryl, F, Cl, Br, CN, NO₂, =O, -C(=O)OH, -C(=O)OC₁₋₄-Alkyl, -(CH₂)ᵣOH und -(CH₂)ᵣOC₁₋₄-Alkyl;
Rf, bei jedem Auftreten, unabhängig ausgewählt ist aus H, F, Cl, Br, CN, OH, C₁₋₅-Alkyl, wahlweise substituiert mit OH, C₃₋₆-Cycloalkyl und Phenyl;
p, bei jedem Auftreten, unabhängig ausgewählt ist aus null, 1 und 2; und
r, bei jedem Auftreten, unabhängig ausgewählt ist aus null, 1, 2, 3 und 4.

4. Verbindung nach Anspruch 2, oder ein pharmazeutisch verträgliches Salz davon, wobei:
R₄ ausgewählt ist aus 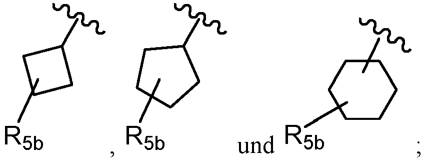
R_{5b}, bei jedem Auftreten, unabhängig ausgewählt ist aus OH, -C(=O)OR_{b}, -C(=O)NRₐRₐ, -NRₐRₐ und -NRₐC(=O)R_{b};
Rₐ, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ; oder Rₐ und Rₐ zusammen mit dem Stickstoffatom, an das sie beide gebunden sind, einen heterocyclischen Ring bilden, der substituiert ist mit 0-5 Rₑ;
R_{b}, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig ausgewählt ist aus C₁₋₆-Alkyl, substituiert mit 0-5 Rf, C₂₋₆-Alkenyl, C₂₋₆-Alkinyl, -(CH₂)ᵣ-C₃₋₆-Cycloalkyl, -(CH₂)ᵣAryl, F, Cl, Br, CN, NO₂, =O, -C(=O)OH, -C(=O)OC₁₋₄-Alkyl, -(CH₂)ᵣOH und -(CH₂)ᵣOC₁₋₄-Alkyl;
Rf, bei jedem Auftreten, unabhängig ausgewählt ist aus H, F, Cl, Br, CN, OH, C₁₋₅-Alkyl, wahlweise substituiert mit OH, C₃₋₆-Cycloalkyl und Phenyl;
p, bei jedem Auftreten, unabhängig ausgewählt ist aus null, 1 und 2; und
r, bei jedem Auftreten, unabhängig ausgewählt ist aus null, 1, 2, 3 und 4.

5. Verbindung nach Anspruch 4, oder ein pharmazeutisch verträgliches Salz davon, wobei: 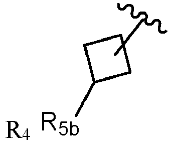 ist;
R_{5b} ausgewählt ist aus -C(=O)OR_{b}, -C(=O)NRₐRₐ und -NHC(=O)R_{b};
Rₐ, bei jedem Auftreten, unabhängig ausgewählt ist aus H und C₁₋₆-Alkyl, substituiert mit 0-5 Rₑ, oder Rₐ und Rₐ zusammen mit dem Stickstoffatom, an das sie beide gebunden sind, einen heterocyclischen Ring bilden, der ausgewählt ist aus 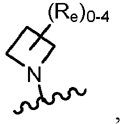 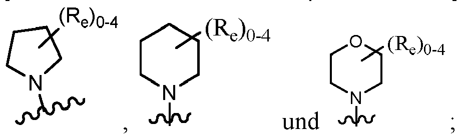
R_{b} ausgewählt ist aus H und C₁₋₆-Alkyl, substituiert mit 0-5 Rₑ;
Rₑ, bei jedem Auftreten, unabhängig ausgewählt ist aus C₁₋₆-Alkyl, substituiert mit 0-5 Rf, C₂₋₆-Alkenyl, C₂₋₆-Alkinyl, -(CH₂)ᵣ-C₃₋₆-Cycloalkyl, -(CH₂)ᵣAryl, F, Cl, Br, CN, NO₂, =O, -C(=O)OH, -C(=O)OC₁₋₄-Alkyl, -(CH₂)ᵣOH und -(CH₂)ᵣOC₁₋₄-Alkyl; und
Rf, bei jedem Auftreten, unabhängig ausgewählt ist aus H, F, Cl, Br, CN, OH, C₁₋₅-Alkyl, wahlweise substituiert mit OH, C₃₋₆-Cycloalkyl und Phenyl.

6. Verbindung nach Anspruch 2, oder ein pharmazeutisch verträgliches Salz davon, wobei:
R₄ ausgewählt ist aus 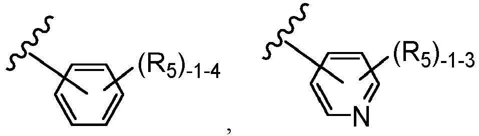 und 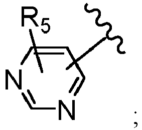
R₅, bei jedem Auftreten, unabhängig ausgewählt ist aus H, F, Cl, Br, C₁₋₄-Alkyl, substituiert mit 0-5 Rₑ, C₂₋₄-Alkenyl, C₂₋₄-Alkinyl, -(CH₂)₀₋₁OR_{b}, -CN, -NRₐRₐ, -(CH₂)₀₋₁-NHC(=O)R_{b}, -NRₐC(=O)NRₐRₐ, -C(=O)OR_{b}, -C(=O)R_{b}, -OC(=O)R_{b}, -C(=O)NRₐRₐ, -S(O)*ₚ*R_{c}, -S(O)*ₚ*NRₐRₐ, -NRₐS(O)*ₚ*R_{c}, C₃₋₆-Cycloalkyl, Heterocyclyl und Aryl, wobei das Alkyl, Cycloalkyl, Heterocyclyl oder Aryl substituiert ist mit 0-4 Rₑ;
Rₐ, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ; oder Rₐ und Rₐ zusammen mit dem Stickstoffatom, an das sie beide gebunden sind, einen heterocyclischen Ring bilden, der substituiert ist mit 0-5 Rₑ;
R_{b}, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig ausgewählt ist aus C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₆-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig ausgewählt ist aus C₁₋₆-Alkyl, substituiert mit 0-5 Rf, C₂₋₆-Alkenyl, C₂₋₆-Alkinyl, -(CH₂)ᵣ-C₃₋₆-Cycloalkyl, -(CH₂)ᵣ-Aryl, F, Cl, Br, CN, NO₂, =O, -C(=O)OH, -C(=O)OC₁₋₄-Alkyl, -(CH₂)ᵣOH und -(CH₂)ᵣOC₁₋₄-Alkyl;
Rf, bei jedem Auftreten, unabhängig ausgewählt ist aus H, F, Cl, Br, CN, OH, C₁₋₅-Alkyl, wahlweise substituiert mit OH, C₃₋₆-Cycloalkyl und Phenyl;
p, bei jedem Auftreten, unabhängig ausgewählt ist aus null, 1 und 2; und
r, bei jedem Auftreten, unabhängig ausgewählt ist aus null, 1, 2, 3 und 4.

7. Verbindung nach Anspruch 2, oder ein pharmazeutisch verträgliches Salz davon, wobei:
R₂ ausgewählt ist aus -CH₃ und -CH₂-Cyclopropyl, substituiert mit 0-2 F oder Cl;
R₄, bei jedem Auftreten, ausgewählt ist aus 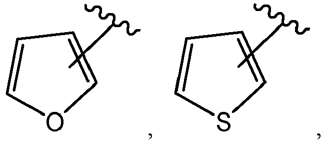 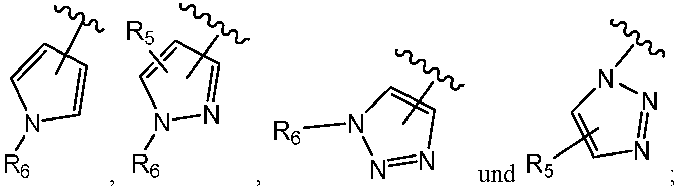
R₅, bei jedem Auftreten, unabhängig ausgewählt ist aus H, F, Cl, Br, C₁₋₄-Alkyl, substituiert mit 0-5 Rₑ, C₂₋₄-Alkenyl, C₂₋₄-Alkinyl, Nitro, -OR_{b}, -CN, -NRₐRₐ, -S(O)*ₚ*R_{c}, -S(O)*ₚ*NRₐRₐ, -NRₐS(O)*ₚ*R_{c}, -(CH₂)ᵣ-NRₐC(=O)R_{b}, -NRₐC(=O)NRₐRₐ, C(=O)OR_{b}, -C(=O)R_{b}, -OC(=O)R_{b}, -C(=O)NRₐRₐ, P(=O)(OC₁₋₄-Alkyl)₂, C₃₋₆-Cycloalkyl, substituiert mit 0-4 Rₑ, Aryl, substituiert mit 0-4 Rₑ, Heterocyclyl, substituiert mit 0-4 Rₑ;
R₆, bei jedem Auftreten, unabhängig ausgewählt ist aus H, C₁₋₃-Alkyl, substituiert mit 0-4 Rₑ, und Heterocyclyl, substituiert mit 0-4 Rₑ;
Rₐ, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ; oder Rₐ und Rₐ zusammen mit dem Stickstoffatom, an das sie beide gebunden sind, einen heterocyclischen Ring bilden, der substituiert ist mit 0-5 Rₑ;
R_{b}, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ;
R_{c}, bei jedem Auftreten, unabhängig ausgewählt ist aus C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₆-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig ausgewählt ist aus C₁₋₆-Alkyl, substituiert mit 0-5 Rf, C₂₋₆-Alkenyl, C₂₋₆-Alkinyl, -(CH₂)ᵣ-C₃₋₆-Cycloalkyl, -(CH₂)ᵣ-Aryl, F, Cl, Br, CN, NO₂, =O, -C(=O)OH, -C(=O)OC₁₋₄-Alkyl, -(CH₂)ᵣOH und -(CH₂)ᵣOC₁₋₄-Alkyl, -(CH₂)ᵣOC₂₋₄-Alkenyl, -(CH₂)ᵣOC₂₋₄-Alkinyl und NH₂;
Rf, bei jedem Auftreten, unabhängig ausgewählt ist aus H, F, Cl, Br, CN, OH, C₁₋₅-Alkyl, wahlweise substituiert mit OH, C₃₋₆-Cycloalkyl und Phenyl;
p, bei jedem Auftreten, unabhängig ausgewählt ist aus null, 1 und 2; und
r, bei jedem Auftreten, unabhängig ausgewählt ist aus null, 1, 2, 3 und 4.

8. Verbindung nach Anspruch 7, oder ein pharmazeutisch verträgliches Salz davon, wobei:
R₂ ausgewählt ist aus -CH₃ und -CH₂-Cyclopropyl, substituiert mit 0-2 F und Cl;
R₄ 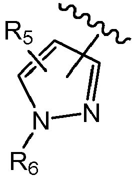 ist;
R₅, bei jedem Auftreten, unabhängig ausgewählt ist aus H, F, Cl, Br, C₁₋₄-Alkyl, Aryl, substituiert mit 0-4 Rₑ, und Heterocyclyl, substituiert mit 0-4 Rₑ;
R₆, bei jedem Auftreten, unabhängig ausgewählt ist aus H, C₁₋₃-Alkyl, substituiert mit 0-4 Rₑ, und Heterocyclyl, substituiert mit 0-4 Rₑ;
Rₑ, bei jedem Auftreten, unabhängig ausgewählt ist aus C₁₋₆-Alkyl, F, Cl, Br, CN, -OH und -(CH₂)ᵣOC₂₋₄-Alkinyl; und
r, bei jedem Auftreten, unabhängig ausgewählt ist aus null, 1 und 2.

9. Verbindung nach Anspruch 2, oder ein pharmazeutisch verträgliches Salz davon, wobei:
R₄ ausgewählt ist aus 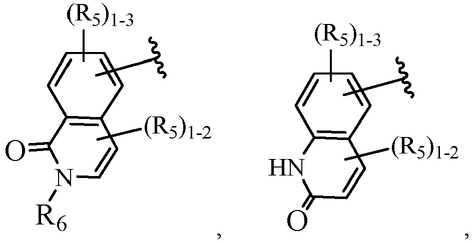 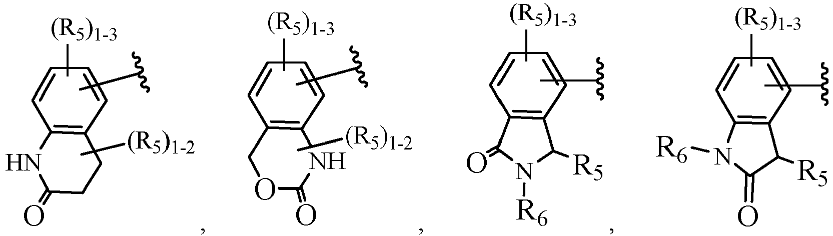 und 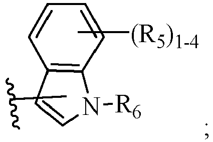
R₅, bei jedem Auftreten, unabhängig ausgewählt ist aus H, F, Cl, Br und C₁₋₄-Alkyl, substituiert mit 0-5 Rₑ, OR_{b}, -CN, -C(=O)R_{b}, -C(=O)OR_{b}, -OC(=O)R_{b}, -C(=O)NRₐRₐ;
R₆ ausgewählt ist aus H und C₁₋₃-Alkyl;
Rₐ, bei jedem Auftreten, unabhängig ausgewählt ist aus H und C₁₋₄-Alkyl, substituiert mit 0-5 Rₑ;
R_{b}, bei jedem Auftreten, unabhängig ausgewählt ist aus H und C₁₋₄-Alkyl, substituiert mit 0-5 Rₑ;
Rₑ, bei jedem Auftreten, unabhängig ausgewählt ist aus C₁₋₆-Alkyl, F, Cl, Br, CN, NO₂, =O, -C(=O)OH, -C(=O)OC₁₋₄-Alkyl, -(CH₂)ᵣOH und -(CH₂)ᵣOC₁₋₄-Alkyl; und
r, bei jedem Auftreten, unabhängig ausgewählt ist aus null, 1 und 2.

10. Verbindung nach Anspruch 1, oder ein pharmazeutisch verträgliches Salz davon, wobei: 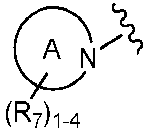 ausgewählt ist aus 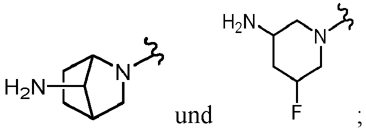
R₃ ausgewählt ist aus H, F, Cl und -OC₁₋₄-Alkyl;
R₄ ausgewählt ist aus 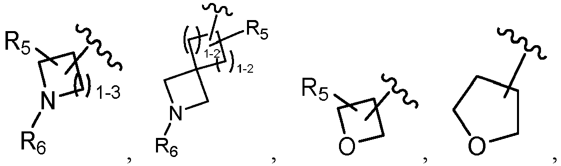 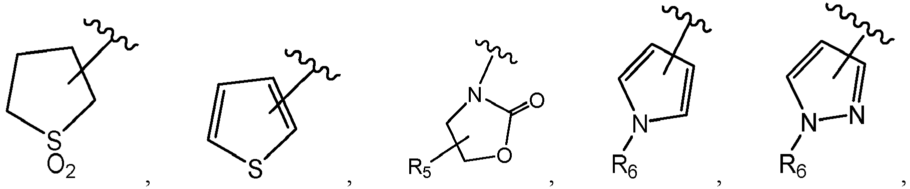 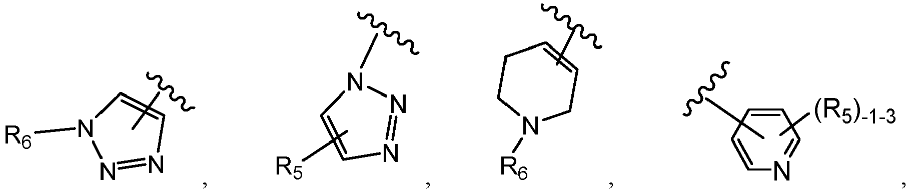 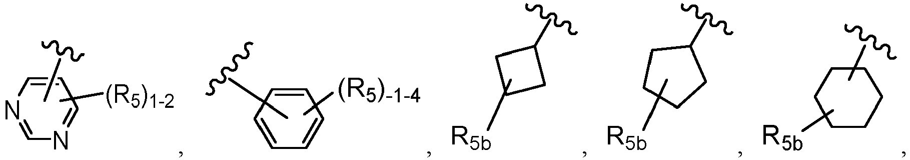 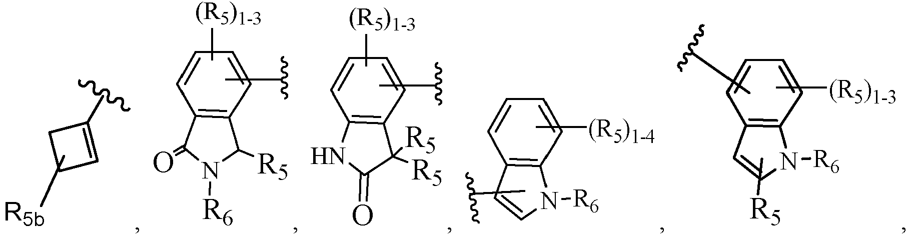 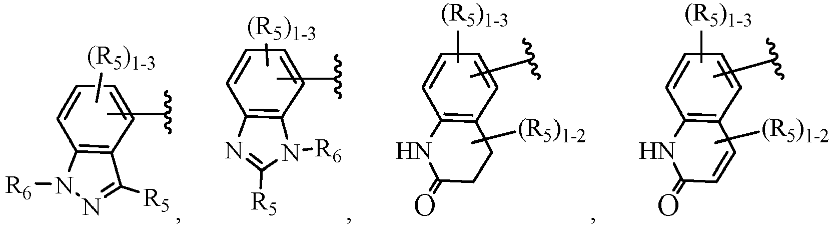 und 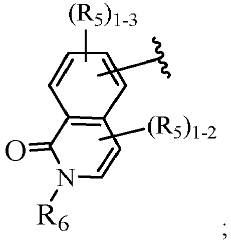
R₅, bei jedem Auftreten, unabhängig ausgewählt ist aus H, F, Cl, Br, C₁₋₄-Alkyl, substituiert mit 0-3 Rₑ, -OR_{b}, -CN, -NRₐRₐ, -NRₐC(=O)R_{b}, -NRₐC(=O)NRₐRₐ, -C(=O)OR_{b}, -C(=O)R_{b}, -OC(=O)R_{b}, -C(=O)NRₐRₐ, -S(O)*ₚ*R_{c}, -S(O)*ₚ*NRₐRₐ, -NRₐS(O)*ₚ*R_{c}, C₃₋₆-Cycloalkyl, substituiert mit 0-4 Rₑ, Aryl, substituiert mit 0-4 Rₑ, und Heterocyclyl, substituiert mit 0-4 Rₑ;
R_{5b}, bei jedem Auftreten, unabhängig ausgewählt ist aus OH, -C(=O)OR_{b}, -C(=O)NRₐRₐ und -NRₐC(=O)R_{b};
R₆, bei jedem Auftreten, unabhängig ausgewählt ist aus H, C₁₋₃-Alkyl, -C(=O)R_{b}, -C(=O)(CH₂)ᵣOR_{b}, -C(=O)(CH₂)ᵣNRₐRₐ, -S(O)ₚR_{c}, -S(O)ₚNRₐRₐ, Aryl, substituiert mit 0-4 Rₑ, und Heterocyclyl, substituiert mit 0-4 Rₑ;
Rₐ, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ; oder Rₐ und Rₐ zusammen mit dem Stickstoffatom, an das sie beide gebunden sind, einen heterocyclischen Ring bilden, der substituiert ist mit 0-5 Rₑ;
R_{b}, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ;
R_{c}, bei jedem Auftreten, unabhängig ausgewählt ist aus C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₆-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig ausgewählt ist aus C₁₋₆-Alkyl, substituiert mit 0-5 Rf, C₂₋₆-Alkenyl, C₂₋₆-Alkinyl, -(CH₂)ᵣ-C₃₋₆-Cycloalkyl, -(CH₂)ᵣ-Aryl, F, Cl, Br, CN, NO2, =O, -C(=O)OH, -C(=O)OC1-4-Alkyl, -(CH₂)ᵣOH und -(CH2)rOCi-4-Alkyl;
Rf, bei jedem Auftreten, unabhängig ausgewählt ist aus H, F, Cl, Br, CN, OH, C₁₋₅-Alkyl, wahlweise substituiert mit OH, C₃₋₆-Cycloalkyl und Phenyl;
p, bei jedem Auftreten, unabhängig ausgewählt ist aus null, 1 und 2; und
r, bei jedem Auftreten, unabhängig ausgewählt ist aus null, 1, 2, 3 und 4.

11. Verbindung nach Anspruch 10, oder ein pharmazeutisch verträgliches Salz davon, wobei: 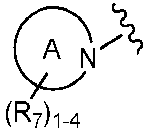 ausgewählt ist aus 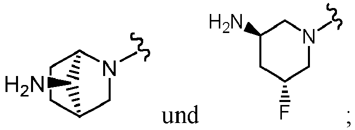
R₁ ausgewählt ist aus -CH₃, -CD₃ und -CH₂-5- bis -6-gliedriges-Heterocyclyl, umfassend Kohlenstoffatome und 1-3 Heteroatome, ausgewählt aus N, NH und NC₁₋₃-Alkyl;
R₂ ausgewählt ist aus -CH₃ und -CH₂-Cyclopropyl;
R₃ ausgewählt ist aus H, F und -OC₁₋₄-Alkyl;
R₄ ausgewählt ist aus 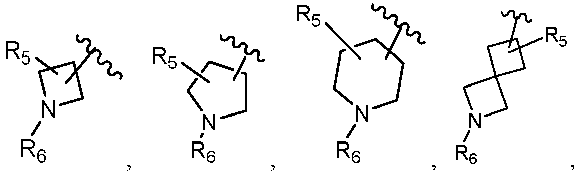 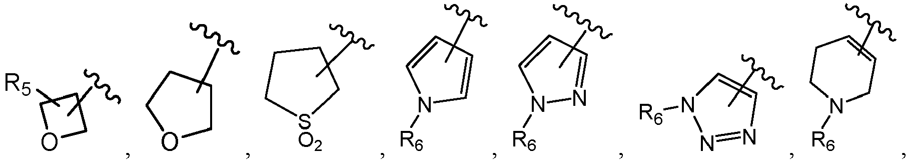 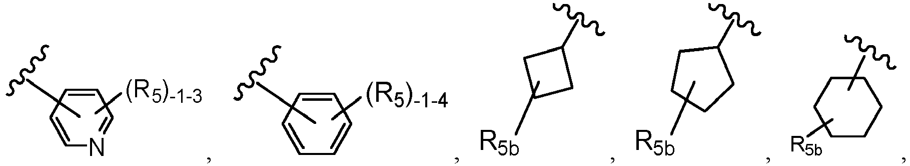 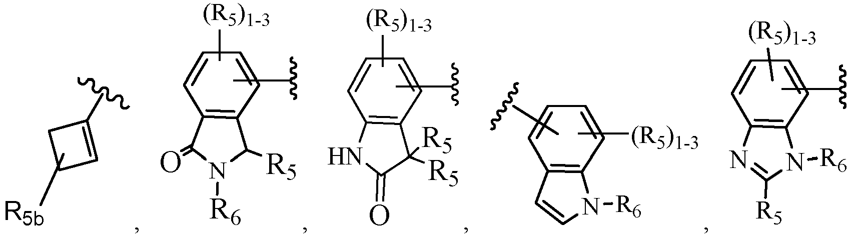 und 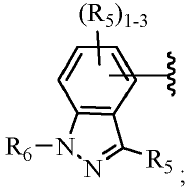
Rs, bei jedem Auftreten, unabhängig ausgewählt ist aus H, F, Cl, Br, C₁₋₄-Alkyl, substituiert mit 0-3 Rₑ, -OR_{b}, -CN, -NRₐRₐ;
R_{5b}, bei jedem Auftreten, unabhängig ausgewählt ist aus OH, -C(=O)OR_{b}, -C(=O)NRₐRₐ und -NRₐC(=O)R_{b};
R₆, bei jedem Auftreten, unabhängig ausgewählt ist aus H, C₁₋₃-Alkyl, -C(=O)R_{b}, -C(=O)(CH₂)ᵣOR_{b}, -C(=O)(CH₂)ᵣNRₐRₐ, -S(O)ₚR_{c}, -S(O)ₚNRₐRₐ, Aryl, substituiert mit 0-4 Rₑ, und Heterocyclyl, substituiert mit 0-4 Rₑ;
Rₐ, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ; oder Rₐ und Rₐ zusammen mit dem Stickstoffatom, an das sie beide gebunden sind, einen heterocyclischen Ring bilden, der substituiert ist mit 0-5 Rₑ;
R_{b}, bei jedem Auftreten, unabhängig ausgewählt ist aus H, C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₁₀-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig ausgewählt ist aus C₁₋₆-Alkyl, substituiert mit 0-5 Rₑ, C₂₋₆-Alkenyl, substituiert mit 0-5 Rₑ, C₂₋₆-Alkinyl, substituiert mit 0-5 Rₑ, -(CH₂)ᵣ-C₃₋₆-Carbocyclyl, substituiert mit 0-5 Rₑ, und -(CH₂)ᵣ-Heterocyclyl, substituiert mit 0-5 Rₑ;
Rₑ, bei jedem Auftreten, unabhängig ausgewählt ist aus C₁₋₆-Alkyl, substituiert mit 0-5 Rf, C₂₋₆-Alkenyl, C₂₋₆-Alkinyl, -(CH₂)ᵣ-C₃₋₆-Cycloalkyl, -(CH₂)ᵣ-Aryl, F, Cl, Br, CN, NO₂, =O, -C(=O)OH, -C(=O)OC₁₋₄-Alkyl, -(CH₂)ᵣOH und -(CH₂)ᵣOC₁₋₄-Alkyl;
Rf, bei jedem Auftreten, unabhängig ausgewählt ist aus H, F, Cl, Br, CN, OH, C₁₋₅-Alkyl, wahlweise substituiert mit OH, C₃₋₆-Cycloalkyl und Phenyl;
p, bei jedem Auftreten, unabhängig ausgewählt ist aus null, 1 und 2; und
r, bei jedem Auftreten, unabhängig ausgewählt ist aus null, 1, 2, 3 und 4.

12. Verbindung nach Anspruch 1, mit Formel (V): 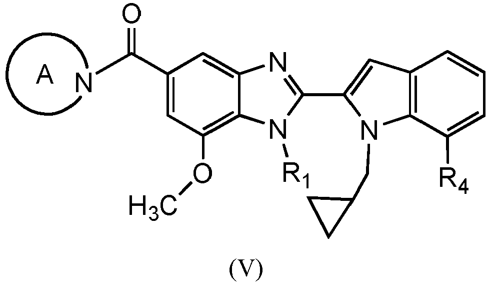
oder ein pharmazeutisch verträgliches Salz davon, wobei: 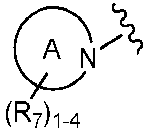 ausgewählt ist aus 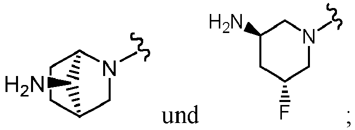
R₁ ausgewählt ist aus -CH₃ und 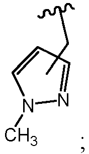
R₄ ausgewählt ist aus 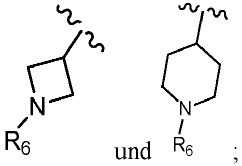
R₆, bei jedem Auftreten, unabhängig ausgewählt ist aus -C(=O)R_{b}, -C(=O)(CH₂)₁₋₃OR_{b} und -C(=O)NRₐRₐ;
Rₐ, bei jedem Auftreten, unabhängig ausgewählt ist aus H, C₁₋₃-Alkyl, substituiert mit 0-3 Rₑ, C₃₋₆-Cycloalkyl, Phenyl, substituiert mit 0-3 Rₑ, und Pyridyl;
R_{b} ausgewählt ist aus H und C₁₋₃-Alkyl, substituiert mit 0-3 Rₑ; und
Rₑ, bei jedem Auftreten, unabhängig ausgewählt ist aus F, Cl, -OH, -OC₁₋₄-Alkyl, C(=O)OH und Phenyl.

13. Verbindung nach Anspruch 12, mit Formel (VI): 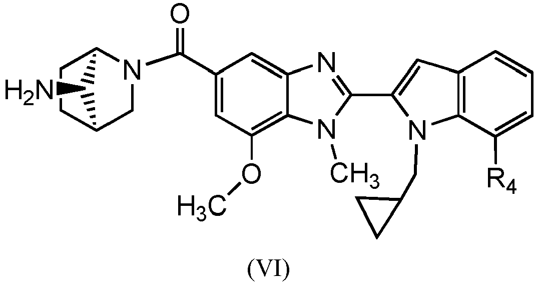
oder ein pharmazeutisch verträgliches Salz davon, wobei:
R₄ ausgewählt ist aus 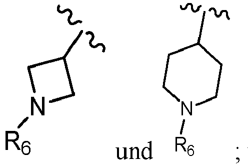 und
R₆, bei jedem Auftreten, unabhängig ausgewählt ist aus -C(=O)CH₃, C(=O)(CH₂)₁₋₂OH, -C(=O)CH₂OCH₂CF₃, -C(=O)(CH₂)₁₋₃OCH₃, -C(=O)(CH₂)₁₋₃-Phenyl, -C(=O)(CH₂)₁₋₃-Pyridyl, -C(=O)(CH₂)₁₋₃-Tetrazolyl, -C(=O)NH₂, -C(=O)NHC₁₋₃-Alkyl, -C(=O)NH-Pyridyl, -C(=O)NH-Cyclopropyl und -C(=O)NH-Phenyl, substituiert mit 0-1 F, Cl, C₁₋₂-Alkyl, und OC₁₋₂-Alkyl.

14. Verbindung nach Anspruch 4, mit Formel (IX): 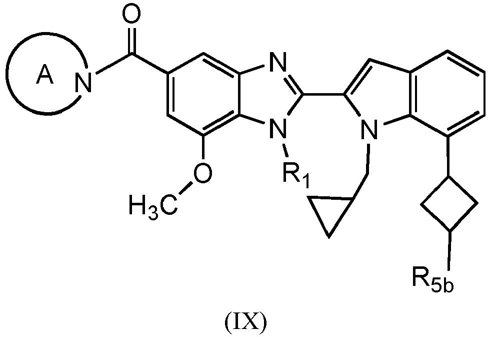
oder ein pharmazeutisch verträgliches Salz davon, wobei: 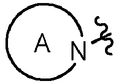 ausgewählt ist aus 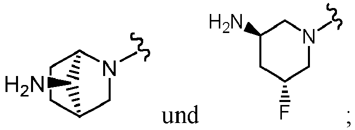
R₁ ausgewählt ist aus -CH₃ und 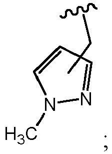
R_{5b} ausgewählt ist aus OH, -C(=O)NRₐRₐ, -C(=O)OR_{b}, NHC(=O)R_{b} und NH₂,
Rₐ, bei jedem Auftreten, unabhängig ausgewählt ist aus H, CH₃ und CD₃; oder Rₐ und Rₐ zusammen mit dem Stickstoffatom, an das sie beide gebunden sind, einen heterocyclischen Ring bilden, der substituiert ist mit 0-2 OH; und
R_{b} ausgewählt ist aus H, CH₃ und CD₃.

15. Verbindung nach Anspruch 4, mit Formel (XI): 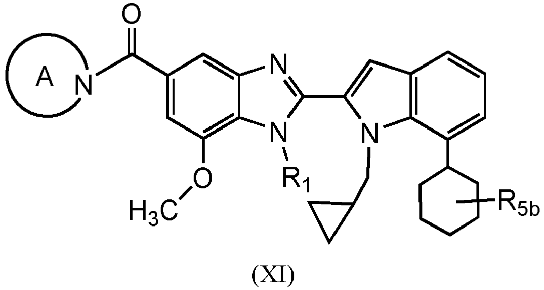
oder ein pharmazeutisch verträgliches Salz davon, wobei: 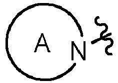 ausgewählt ist aus 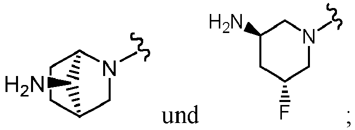
R₁ ausgewählt ist aus -CH₃ und 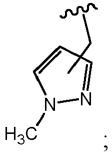 und
R_{5b} ausgewählt ist aus -OH, -NH₂ und CONH₂.

16. Verbindung nach Anspruch 1, welche ist:
(1R,4R,7R)-2-{2-[1-(Cyclopropylmethyl)-7-(piperidin-4-yl)-1H-indol-2-yl]-7-methoxy-1-methyl-1H-1,3-benzodiazol-5-carbonyl}-2-azabicyclo[2.2.1]heptan-7-amin;
(1R,4R,7R)-2-{2-[7-(Azetidin-3-yl)-1-(cyclopropylmethyl)-1H-indol-2-yl]-7-methoxy-1-methyl-1H-1,3-benzodiazol-5-carbonyl}-2-azabicyclo[2.2.1]heptan-7-amin;
(1R,4R,7R)-2-{2-[7-(3-Aminocyclobutyl)-1-(cyclopropylmethyl)-1H-indol-2-yl]-7-methoxy-1-methyl-1H-1,3-benzodiazol-5-carbonyl}-2-azabicyclo[2.2.1]heptan-7-amin;
(1R,4R,7R)-2-{2-[1-(Cyclopropylmethyl)-7-[(piperidin-4-yl)methyl]-1H-indol-2-yl]-7-methoxy-1-methyl-1H-1,3-benzodiazol-5-carbonyl}-2-azabicyclo[2.2.1]heptan-7-amin;
(1R,4R,7R)-2-{2-[1-(Cyclopropylmethyl)-7-(piperidin-3-yl)-1H-indol-2-yl]-7-methoxy-1-methyl-1H-1,3-benzodiazol-5-carbonyl}-2-azabicyclo[2.2.1]heptan-7-amin;
(1R,4R,7R)-2-{2-[7-(4-Aminocyclohexyl)-1-(cyclopropylmethyl)-1H-indol-2-yl]-7-methoxy-1-methyl-1H-1,3-benzodiazol-5-carbonyl}-2-azabicyclo[2.2.1]heptan-7-amin;
((lR,4R,7R)-7-Amino-2-azabicyclo[2.2.1]heptan-2-yl)(2-(7-(((trans)-4-aminocyclohexyl)methyl)-1-(cyclopropylmethyl)-1H-indol-2-yl)-7-methoxy-1-methyl-1H-benzo[d]imidazol-5-yl)methanon;
1-[4-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)piperidin-1-yl]ethan-1-on;
cis- und trans-3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)cyclobutan-1-ol;
(1R,4R,7R)-2-{2-[1-(Cyclopropylmethyl)-7-(1 -methyl azetidin-3 -yl)-1H-indol-2-yl]-7-methoxy-1-methyl-1H-1,3-benzodiazol-5-carbonyl}-2-azabicyclo[2.2.1]heptan-7-amin;
3-[2-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)ethyl]-1,3-oxazolidin-2-on;
4-(2-{5-[(1R,4R,7R)-7-Amino-2-azabicyclo[2.2.1]heptan-2-carbonyl]-7-methoxy-1 -methyl- 1H-1,3 -benzodiazol-2-yl} -1 -(cyclopropylmethyl)- 1H-indol-7-yl)cyclohexan-1-ol;
(1R,4R,7R)-2-{2-[1-(Cyclopropylmethyl)-7-(oxetan-3-yl)-1H-indol-2-yl]-7-methoxy-1-methyl-1H-1,3-benzodiazol-5-carbonyl}-2-azabicyclo[2.2.1]heptan-7-amin
(1R,4R,7R)-2-(2-{7-[(Azetidin-3-yl)methyl]-1-(cyclopropylmethyl)-1H-indol-2-yl}-7-methoxy-1-methyl-1H-1,3-benzodiazol-5-carbonyl)-2-azabicyclo[2.2.1]heptan-7-amin;
Benzyl-3-(2-{5-[(1R,4R,7R)-7-amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)pyrrolidin-1-carboxylat;
(1R,4R,7R)-2-{2-[1-(Cyclopropylmethyl)-7-[2-(1H-1,2,4-triazol-1-yl)ethyl]-1H-indol-2-yl]-7-methoxy-1-methyl-1H-1,3-benzodiazol-5-carbonyl}-2-azabicyclo[2.2. 1]heptan-7-amin;
((1R,4R,7R)-7-Amino-2-azabicyclo[2.2.1]heptan-2-yl)(2-(1-(cyclopropylmethyl)-7-(1,1-dioxidotetrahydrothiophen-3-yl)-1H-indol-2-yl)-7-methoxy-1-methyl-1H-benzo[d]imidazol-5-yl)methanon;
(1R,4R,7R)-2-{2-[1-(Cyclopropylmethyl)-7-[(oxan-4-yl)methyl]-1H-indol-2-yl]-7-methoxy-1-methyl-1H-1,3-benzodiazol-5-carbonyl}-2-azabicyclo[2.2.1]heptan-7-amin;
(1R,4R,7R)-2-{2-[1-(Cyclopropylmethyl)-7-[2-(piperidin-3-yl)ethyl]-1H-indol-2-yl]-7-methoxy-1-methyl-1H-1,3-benzodiazol-5-carbonyl}-2-azabicyclo[2.2.1]heptan-7-amin;
(1R,4R,7R)-2-{2-[1-(Cyclopropylmethyl)-7-[2-(3,5-dimethyl-1H-pyrazol-4-yl)ethyl]-1H-indol-2-yl]-7-methoxy-1-methyl-1H-1,3-benzodiazol-5-carbonyl }-2-azabicyclo[2.2. 1]heptan-7-amin;
(1R,4R,7R)-2-{2-[1-(Cyclopropylmethyl)-7-[(oxan-2-yl)methyl]-1H-indol-2-yl]-7-methoxy-1-methyl-1H-1,3-benzodiazol-5-carbonyl}-2-azabicyclo[2.2.1]heptan-7-amin;
1-(3-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)azetidin-1-yl)ethan-1-on;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)piperidin-1-yl]ethan-1-on;
3-(2-{5-[(1R,4R,7R)-7-Amino-2-azabicyclo[2.2.1]heptan-2-carbonyl]-7-methoxy-1 -methyl- 1H-1,3 -benzodiazol-2-yl} -1 -(cyclopropylmethyl)- 1H-indol-7-yl)piperidin-1-carboxamid;
Methyl-3-(2-(5-((1R,4R,7R)-7-amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)azetidin-1-carboxylat;
1-[3-(2-{5-[(3R,5R)-3-Amino-5-fluorpiperidin-1-carbonyl]-7-methoxy-1-methyl-1H-1 ,3 -benzodiazol-2-yl} -1 -(cyclopropylmethyl)-1H-indol-7-yl)azetidin-1 -yl] ethan-1 -on;
1-(3 -(2-(5-((2S, 5R)-5-Amino-2-methylpiperidin-1 -carbonyl)-7-methoxy-1 - methyl-1 H-benzo[d]imidazol-2-yl)-1 -(cyclopropylmethyl)- 1H-indol-7-yl)azetidin-1 - yl)ethan-1-on;
3-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)-N-methylcyclobutan-1-carboxamid;
3-(2-{5-[(1R,4R,7R)-7-Amino-2-azabicyclo[2.2.1]heptan-2-carbonyl]-7-methoxy-1 -methyl- 1H-1,3 -benzodiazol-2-yl} -1 -(cyclopropylmethyl)- 1H-indol-7-yl)cyclobutan-1-carboxamid;
3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-N,N-dimethylcyclobutan-1-carboxamid;
(3-(2-(5-((1R,4R,7R)-7-Amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)cyclobutyl)(pyrrolidin-1-yl)methanon;
(3-(2-(5-((1R,4R,7R)-7-Amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)cyclobutyl)((R)-3-hydroxypyrrolidin-1-yl)methanon;
(3-(2-(5-((1R,4R,7R)-7-Amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)cyclobutyl)((S)-3-hydroxypyrrolidin-1-yl)methanon;
3-(2-{5-[(1R,4R,7R)-7-Amino-2-azabicyclo[2.2.1]heptan-2-carbonyl]-7-methoxy-1 -methyl- 1H-1,3 -benzodiazol-2-yl} -1 -(cyclopropylmethyl)- 1H-indol-7-yl)cyclobutan-1-carbonsäure;
N-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)cyclobutyl]acetamid;
(1R,4R,7R)-2-[2-(7- f 2-Azaspiro[3.3]heptan-6-yl}-1-(cyclopropylmethyl)-1H-indol-2-yl)-7-methoxy-1-methyl-1H-1,3-benzodiazol-5-carbonyl]-2-azabicyclo[2.2. 1]heptan-7-amin;
1-[6-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-2-azaspiro[3.3]heptan-2-yl]ethan-1-on;
1-{3-[(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)methyl]azetidin-1-yl}ethan-1-on;
(1R,4R,7R)-2-{2-[1-(Cyclopropylmethyl)-7-(1-methansulfonylazetidin-3-yl)-1H-indol-2-yl]-7-methoxy-1-methyl-1H-1,3-benzodiazol-5-carbonyl}-2-azabicyclo[2.2. 1]heptan-7-amin;
2-Amino-1-[3-(2-{5-[(1R,4R,7R)-7-amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]ethan-1-on;
3-(2-{5-[(1R,4R,7R)-7-Amino-2-azabicyclo[2.2.1]heptan-2-carbonyl]-7-methoxy-1 -methyl- 1H-1,3 -benzodiazol-2-yl} -1 -(cyclopropylmethyl)- 1H-indol-7-yl)azetidin-1-carboxamid;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2,2-dimethylpropan-1-on;
4-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-4-oxobutansäure;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2-hydroxy-2-methylpropan-1-on;
3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-N-phenylazetidin-1-carboxamid;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2-methoxyethan-1-on;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2-phenylethan-1-on;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2-(pyridin-4-yl)ethan-1-on;
(1R,4R,7R)-2- { 2- [7-(1 -Benzoylazetidin-3-yl)-1-(cyclopropylmethyl)-1H-indol-2-yl]-7-methoxy-1-methyl-1H-1,3-benzodiazol-5-carbonyl}-2-azabicyclo[2.2.1]heptan-7-amin;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2-(pyridin-2-yl)ethan-1-on;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2-(pyrazin-2-yl)ethan-1-on;
3-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-3-oxopropannitril,
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2-(pyridin-3-yl)ethan-1-on;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-3-phenylpropan-1-on;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-3-hydroxypropan-1-on;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2-(1H-1,2,3,4-tetrazol-5-yl)ethan-1-on;
1-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)azetidin-1-yl]-2-hydroxyethan-1-on
(1R,4R,7R)-2-(2-{7-[1-(Benzolsulfonyl)azetidin-3-yl]-1-(cyclopropylmethyl)-1H-indol-2-yl} -7-methoxy-1 -methyl- 1H- 1,3 -benzodiazol-5-carbonyl)-2-azabicyclo[2.2. 1]heptan-7-amin
(1R,4R,7R)-2-{2-[1-(Cyclopropylmethyl)-7-(1,2,3,6-tetrahydropyridin-4-yl)-1H-indol-2-yl]-7-methoxy-1-methyl-1H-1,3-benzodiazol-5-carbonyl}-2-azabicyclo[2.2. 1]heptan-7-amin
3-(2-{5-[(1R,4R,7R)-7-Amino-2-azabicyclo[2.2.1]heptan-2-carbonyl]-7-methoxy-1 -methyl- 1H-1,3 -benzodiazol-2-yl} -1 -(cyclopropylmethyl)- 1H-indol-7-yl)cyclohexan-1-carboxamid;
2-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)phenol
N-[3-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)phenyl]prop-2-enamid
N-{[4-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)phenyl]methyl}acetamid
4-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-2-fluorphenol 4-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-2-methoxyphenol;
4-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-2-chlorphenol;
4-(2-{5-[(1R,4R,7R)-7-Amino-2-azabicyclo[2.2.1]heptan-2-carbonyl]-7-methoxy-1-methyl-1H-1,3 -benzodiazol-2-yl} -1 -(cyclopropylmethyl)-1H-indol-7-yl)-3 - chlorphenol;
4-(2-{5-[(1R,4R,7R)-7-Amino-2-azabicyclo[2.2.1]heptan-2-carbonyl]-7-methoxy-1-methyl-1H-1,3 -benzodiazol-2-yl} -1 -(cyclopropylmethyl)-1H-indol-7-yl)-2,5 - difluorphenol;
6-(2-{5-[(1R,4R,7R)-7-Amino-2-azabicyclo[2.2.1]heptan-2-carbonyl]-7-methoxy-1 -methyl- 1H-1,3 -benzodiazol-2-yl} -1 -(cyclopropylmethyl)- 1H-indol-7-yl)-4-methyl-1,2-dihydrochinolin-2-on;
4-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-2,3-dihydro-1H-isoindol-1-on;
2'-{5-[(1R,4R,7R)-7-Amino-2-azabicyclo[2.2.1]heptan-2-carbonyl]-7-methoxy-1-methyl-1H-1,3-benzodiazol-2-yl}-1'-(cyclopropylmethyl)-2,3-dihydro-1H,1'H-[5,7'-biindol]-2-on;
6-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-2,3-dihydro-1H-isoindol-1-on;
6-(2-{5-[(1R,4R,7R)-7-Amino-2-azabicyclo[2.2.1]heptan-2-carbonyl]-7-methoxy-1-methyl- 1H-1,3-benzodiazol-2-yl} -1-(cyclopropylmethyl)- 1H-indol-7-yl)-1,2,3,4-tetrahydrochinolin-2-on;
6-(2-{5-[(1R,4R,7R)-7-Amino-2-azabicyclo[2.2.1]heptan-2-carbonyl]-7-methoxy-1 -methyl- 1H-1,3 -benzodiazol-2-yl} -1 -(cyclopropylmethyl)- 1H-indol-7-yl)-1,2-dihydrochinolin-2-on;
Methyl-6-(2-{5-[(1R,4R,7R)-7-amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-2-oxo-1,2-dihydrochinolin-4-carboxylat;
6-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-1,2-dihydroisochinolin-1-on;
5-(2-{5-[(1R,4R,7R)-7-Amino-2-azabicyclo[2.2.1]heptan-2-carbonyl]-7-methoxy-1-methyl-1H-1,3-benzodiazol-2-yl}-1 -(cyclopropylmethyl)-1H-indol-7-yl)-2,3 -dihydro-1H-isoindol-1-on;
4-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)-2-chlorbenzamid;
Methyl-N-[5-(2-{5-[(1R,4R,7R)-7-amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)pyridin-2-yl]carbamat;
N-[5-(2-{5-[(1R,4R,7R)-7-Amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)pyridin-2-yl]acetamid;
Diethyl-(2-{5-[(1R,4R,7R)-7-amino-2-azabicyclo[2.2.1]heptan-2-carbonyl]-7-methoxy-1-methyl-1H-1,3-benzodiazol-2-yl}-1-(cyclopropylmethyl)-1H-indol-7-yl)phosphonat;
2-{4-[2-(5-{7-Amino-2-azabicyclo[2.2.1]heptan-2-carbonyl}-7-methoxy-1-methyl-1H-1,3-benzodiazol-2-yl)-1-(cyclopropylmethyl)-1H-indol-7-yl]-1H-1,2,3-triazol-1-yl}ethan-1-ol;
(1R,4R,7R)-2-{2-[1-(Cyclopropylmethyl)-7-{4-[(1r,4r)-4-aminocyclohexyl]-1H-1,2,3-triazol-1-yl}-1H-indol-2-yl]-7-methoxy-1-methyl-1H-1,3-benzodiazol-5-carbonyl}-2-azabicyclo[2.2.1]heptan-7-amin;
(1R,4R,7R)-2-(2-{7-[4-(Azetidin-3-yl)-1H-1,2,3-triazol-1-yl]-1-(cyclopropylmethyl)-1H-indol-2-yl}-7-methoxy-1-methyl-1H-1,3-benzodiazol-5-carbonyl)-2-azabicyclo[2.2.1]heptan-7-amin;
3-(2-(5-((3R, SR)-3-Amino-5-fluorpiperidin-1-carbonyl)-7-methoxy-1-methyl-1H-benzo[d]imidazol-2-yl)-1-(cyclopropylmethyl)-1H-indol-7-yl)-N-methylcyclobutan-1-carboxamid;
3-(2-(5-((3R, SR)-3-Amino-5-fluorpiperidin-1-carbonyl)-7-methoxy-1-methyl-1H-benzo[d]imidazol-2-yl)-1-(cyclopropylmethyl)-1H-indol-7-yl)cyclobutan-1-carboxamid;
((3R,SR)-3-Amino-5-fluorpiperidin-1-yl)(2-(1-(cyclopropylmethyl)-7-(3-(pyrrolidin-1-carbonyl)cyclobutyl)-1H-indol-2-yl)-7-methoxy-1-methyl-1H-benzo[d]imidazol-5-yl)methanon;
3-(2-(5-((3R, SR)-3-Amino-5-fluorpiperidin-1-carbonyl)-7-methoxy-1-methyl-1H-benzo[d]imidazol-2-yl)-1-(cyclopropylmethyl)-1H-indol-7-yl)-N,N-dimethylcyclobutan-1-carboxamid;
((1R,4R,7R)-7-Amino-2-azabicyclo[2.2.1]heptan-2-yl)(2-(1-(cyclopropylmethyl)-7-(4-hydroxy-3-(hydroxymethyl)phenyl)-1H-indol-2-yl)-7-methoxy-1-methyl-1H-benzo[d]imidazol-5-yl)methanon;
5-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)-2-hydroxybenzamid;
5-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)-2-hydroxybenzoesäure;
((1R,4R,7R)-7-Amino-2-azabicyclo[2.2.1]heptan-2-yl)(2-(1-(cyclopropylmethyl)-7-(1-methyl-1H-pyrazol-4-yl)-1H-indol-2-yl)-7-methoxy-1-methyl-1H-benzo[d]imidazol-5-yl)methanon;
Ethyl-2-(2-(5-((1R,4R,7R)-7-amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)benzoat;
4-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)benzamid;
4-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)-N,N-dimethylbenzamid;
((7R)-7-Amino-2-azabicyclo[2.2.1]heptan-2-yl)(2-(1'-(cyclopropylmethyl)-1-methyl-1H,1'H-[6,7'-biindol]-2'-yl)-7-methoxy-1-methyl-1H-benzo[d]imidazol-5-yl)methanon;
((1R,4R,7R)-7-Amino-2-azabicyclo[2.2.1]heptan-2-yl)(2-(1-(cyclopropylmethyl)-7-(1H-pyrazol-3-yl)-1H-indol-2-yl)-7-methoxy-1-methyl-1H-benzo[d]imidazol-5-yl)methanon;
((7R)-7-Amino-2-azabicyclo[2.2.1]heptan-2-yl)(2-(1-(cyclopropylmethyl)-7-(1-(2-hydroxy-3-(prop-2-in-1-yloxy)propyl)-1H-pyrazol-4-yl)-1H-indol-2-yl)-7-methoxy-1- methyl-1H-benzo[d]imidazol-5-yl)methanon;
((7R)-7-Amino-2-azabicyclo[2.2.1]heptan-2-yl)(2-(1-(cyclopropylmethyl)-7-(furan-3-yl)-1H-indol-2-yl)-7-methoxy-1-methyl-1H-benzo[d]imidazol-5-yl)methanon;
((7R)-7-Amino-2-azabicyclo[2.2.1]heptan-2-yl)(2-(1-(cyclopropylmethyl)-7-(furan-2-yl)-1H-indol-2-yl)-7-methoxy-1-methyl-1H-benzo[d]imidazol-5-yl)methanon;
((1R,4R,7R)-7-Amino-2-azabicyclo[2.2.1]heptan-2-yl)(2-(1-(cyclopropylmethyl)-7-(thiophen-2-yl)-1H-indol-2-yl)-7-methoxy-1-methyl-1H-benzo[d]imidazol-5-yl)methanon;
((7R)-7-Amino-2-azabicyclo[2.2.1]heptan-2-yl)(2-(1-(cyclopropylmethyl)-7-(1-(tetrahydro-2H-pyran-4-yl)-1H-pyrazol-4-yl)-1H-indol-2-yl)-7-methoxy-1-methyl-1H-benzo[d]imidazol-5-yl)methanon;
N-(4-(2-(5-((7R)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)phenyl)acetamid;
((7R)-7-Amino-2-azabicyclo[2.2.1]heptan-2-yl)(2-(1-(cyclopropylmethyl)-7-(pyridin-3-yl)-1H-indol-2-yl)-7-methoxy-1-methyl-1H-benzo[d]imidazol-5-yl)methanon;
((7R)-7-Amino-2-azabicyclo[2.2.1]heptan-2-yl)(2-(1-(cyclopropylmethyl)-7-(3-(2-fluorphenyl)-1-methyl-1H-pyrazol-4-yl)-1H-indol-2-yl)-7-methoxy-1-methyl-1H-benzo[d]imidazol-5-yl)methanon;
((7R)-7-Amino-2-azabicyclo[2.2.1]heptan-2-yl)(2-(1-(cyclopropylmethyl)-7-(3-(hydroxymethyl)phenyl)-1H-indol-2-yl)-7-methoxy-1-methyl-1H-benzo[d]imidazol-5-yl)methanon;
((1R,4R,7R)-7-Amino-2-azabicyclo[2.2.1]heptan-2-yl)(2-(1-(cyclopropylmethyl)-7-(1H-pyrazol-4-yl)-1H-indol-2-yl)-7-methoxy-1-methyl-1H-benzo[d]imidazol-5-yl)methanon;
((1R,4R,7R)-7-Amino-2-azabicyclo[2.2.1]heptan-2-yl)(2-(1-(cyclopropylmethyl)-7-(1-((R)-2-hydroxypropyl)-1H-pyrazol-4-yl)-1H-indol-2-yl)-7-methoxy-1 -methyl-1H-benzo[d]imidazol-5-yl)methanon;
((1R,4R,7R)-7-Amino-2-azabicyclo[2.2.1]heptan-2-yl)(2-(1-(cyclopropylmethyl)-7-(1-(2-hydroxy-2-methylpropyl)-3-methyl-1H-pyrazol-4-yl)-1H-indol-2-yl)-7-methoxy-1-methyl-1H-benzo[d]imidazol-5-yl)methanon;
((1R,4R,7R)-7-Amino-2-azabicyclo[2.2.1]heptan-2-yl)(2-(1-(cyclopropylmethyl)-7-(1-(2-hydroxy-2-methylpropyl)-1H-pyrazol-4-yl)-1H-indol-2-yl)-7-methoxy-1-methyl-1H-benzo[d]imidazol-5-yl)methanon;
((1R,4R,7R)-7-Amino-2-azabicyclo[2.2.1]heptan-2-yl)(2-(1-(cyclopropylmethyl)-7-(1-(2-hydroxyethyl)-1H-pyrazol-4-yl)-1H-indol-2-yl)-7-methoxy-1-methyl-1H-benzo[d]imidazol-5-yl)methanon;
6-(2-(5-((1R,4S)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)-4,4-dimethyl-1,4-dihydro-2H-benzo[d][1,3]oxazin-2-on;
2-(2-(5-((1R,4S)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)benzonitril;
5-(2-(5-((1R,4R,7R)-7-Amino-2-azabicyclo[2.2.1]heptan-2-carbonyl)-7-methoxy-1-methyl-1H-benzo[d]imidazol-2-yl)-1-((2,2-difluorcyclopropyl)methyl)-1H-indol-7-yl)pyrimidin-2-carbonitril;
((1R,4R,7R)-7-Amino-2-azabicyclo[2.2.1]heptan-2-yl)(2-(1-((2,2-difluorcyclopropyl)methyl)-7-(1H-pyrazol-4-yl)-1H-indol-2-yl)-7-methoxy-1-methyl-1H-benzo[d]imidazol-5-yl)methanon;
((1R,4R,7R)-7-Amino-2-azabicyclo[2.2.1]heptan-2-yl)(2-(1-((2,2-dichlorcyclopropyl)methyl)-7-(1H-pyrazol-4-yl)-1H-indol-2-yl)-7-methoxy-1-methyl-1H-benzo[d]imidazol-5-yl)methanon;
((1R,4R,7R)-7-Amino-2-azabicyclo[2.2.1]heptan-2-yl)(2-(1-((2,2-dichlorcyclopropyl)methyl)-7-(1H-pyrazol-3-yl)-1H-indol-2-yl)-7-methoxy-1-methyl-1H-benzo[d]imidazol-5-yl)methanon;
((1R,4R,7R)-7-Amino-2-azabicyclo[2.2.1]heptan-2-yl)(2-(1-(cyclopropylmethyl)-5-methyl-7-(1H-pyrazol-4-yl)-1H-indol-2-yl)-7-methoxy-1-methyl-1H-benzo[d]imidazol-5-yl)methanon;
2-(2-(5-((1R,4R,7R)-7-Amino-2-azabicyclo[2.2.1]heptan-2-carbonyl)-7-methoxy-1 -methyl- 1H-benzo[d]imidazol-2-yl)-1-((2-methylcyclopropyl)methyl)-1H-indol-7-yl)benzonitril;
((1R,4R,7R)-7-Amino-2-azabicyclo[2.2.1]heptan-2-yl)(7-methoxy-1-methyl-2-(1-((2-methylcyclopropyl)methyl)-7-(1H-pyrazol-4-yl)-1H-indol-2-yl)-1H-benzo[d]imidazol-5-yl)methanon;
((1R,4R,7R)-7-Amino-2-azabicyclo[2.2.1]heptan-2-yl)(7-methoxy-1-methyl-2-(1-((2-methylcyclopropyl)methyl)-7-(1H-pyrazol-3-yl)-1H-indol-2-yl)-1H-benzo[d]imidazol-5-yl)methanon;
((1R,4R,7R)-7-Amino-2-azabicyclo[2.2.1]heptan-2-yl)(2-(1-(cyclopropylmethyl)-7-(2-(hydroxymethyl)phenyl)-1H-indol-2-yl)-7-methoxy-1-methyl-1H-benzo[d]imidazol-5-yl)methanon;
((1R,4R,7R)-7-Amino-2-azabicyclo[2.2.1]heptan-2-yl)(2-(1-(cyclopropylmethyl)-7-phenyl-1H-indol-2-yl)-7-methoxy-1-methyl-1H-benzo[d]imidazol-5-yl)methanonmethyl-1H-benzo[d]imidazol-5-yl)methanon;
4-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)-N-phenylpiperidin-1-carboxamid;
4-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)-N,N-dimethylpiperidin-1-carboxamid;
1-(4-(2-(5-((3R,5R)-3-Amino-5-fluorpiperidin-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)piperidin-1-yl)ethan-1-on;
5-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)-3,3-difluorindolin-2-on;
4-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)-3 - fluorbenzamid;
5-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-3 -hydroxy-3 - methylindolin-2-on;
2-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)-5-hydroxybenzonitril;
5-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)picolinamid;
3-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)-N,N-bis(methyl-d3)cyclobutan-1-carboxamid;
((1R,4R,7R)-7-Amino-2-azabicyclo[2.2.1]heptan-2-yl)(2-(1-(cyclopropylmethyl)-7-(6-hydroxypyridin-3-yl)-1H-indol-2-yl)-7-methoxy-1-methyl-1H-benzo[d]imidazol-5-yl)methanon;
5-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)-3 -hydroxy-3 - (trifluormethyl)indolin-2-on;
5-(2-(5-((1R,4R,7R)-7-Amino-2-azabicyclo[2.2.1]heptan-2-carbonyl)-7-methoxy-1 -methyl- 1H-benzo[d]imidazol-2-yl)-1-(cyclopropylmethyl)- 1H-indol-7-yl)-3 - fluorpicolinamid;
(3-(2-(5-((1R,4R,7R)-7-Amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)cyclobutyl)(3-fluorazetidin-1-yl)methanon
(3-(2-(5-((1R,4R,7R)-7-Amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)cyclobutyl)(azetidin-1-yl)methanon;
6-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)-7-methoxy-3,4-dihydrochinolin-2(1H)-on;
1-(4-(2-(5-((3R,5R)-3-Amino-5-fluorpiperidin-1-carbonyl)-7-methoxy-1-methyl-1H-benzo[d]imidazol-2-yl)-1-(cyclopropylmethyl)- 1H-indol-7-yl)piperidin-1-yl)ethan-1-on;
((3R,SR)-3-Amino-5-fluorpiperidin-1-yl)(2-(1-(cyclopropylmethyl)-7-(3-fluor-4-hydroxyphenyl)-1H-indol-2-yl)-7-methoxy-1-methyl-1H-benzo[d]imidazol-5-yl)methanon;
5-(2-(5-((3R,5R)-3-Amino-5-fluorpiperidin-1-carbonyl)-7-methoxy-1-methyl-1H-benzo[d]imidazol-2-yl)-1-(cyclopropylmethyl)-1H-indol-7-yl)indolin-2-on;
5-(2-(5-((3R,5R)-3-Amino-5-fluorpiperidin-1-carbonyl)-7-methoxy-1-methyl-1H-benzo[d]imidazol-2-yl)-1-(cyclopropylmethyl)-1H-indol-7-yl)-3-hydroxy-3-methylindolin-2-on;
5-(2-(5-((3R,5R)-3-Amino-5-fluorpiperidin-1-carbonyl)-7-methoxy-1-methyl-1H-benzo[d]imidazol-2-yl)-1-(cyclopropylmethyl)-1H-indol-7-yl)isoindolin-1-on;
1-(3-(2-(5-((1R,4R,7R)-7-Amino-2-azabicyclo[2.2.1]heptan-2-carbonyl)-7-methoxy-1-((1-methyl- 1H-pyrazol-4-yl)methyl)-1H-benzo[d]imidazol-2-yl)-1-(cyclopropylmethyl)-1H-indol-7-yl)azetidin-1 -yl)ethan-1 -on;
Methyl-3-(2-(5-((3R,5R)-3-amino-5-fluorpiperidin-1-carbonyl)-7-methoxy-1-((1- methyl-1H-pyrazol-4-yl)methyl)-1H-benzo[d]imidazol-2-yl)-1-(cyclopropylmethyl)-1H-indol-7-yl)azetidin-1-carboxylat;
1-(3-(2-(5-((3R,5R)-3-Amino-5-fluorpiperidin-1-carbonyl)-7-methoxy-1-((1-methyl-1H-pyrazol-4-yl)methyl)-1H-benzo[d]imidazol-2-yl)-1-(cyclopropylmethyl)-1H-indol-7-yl)azetidin-1-yl)ethan-1-on;
Methyl-3-(2-(5-((lR,4R,7R)-7-amino-2-azabicyclo[2.2.1]heptan-2-carbonyl)-7-methoxy-1 -((1 -methyl- 1H-pyrazol-4-yl)methyl)-1H-benzo[d]imidazol-2-yl)-1-(cyclopropylmethyl)-1H-indol-7-yl)azetidin-1-carboxylat;
1-(3-(2-(5-((1R,4R,7R)-7-Amino-2-azabicyclo[2.2.1]heptan-2-carbonyl)-7-methoxy-1 -((1 -methyl- 1H-pyrazol-4-yl)methyl)-1H-benzo[d]imidazol-2-yl)-1-methyl-1H-indol-7-yl)azetidin-1-yl)ethan-1-on;
1-(3-(2-(5-((3R,SR)-3-Amino-5-fluorpiperidin-1-carbonyl)-7-methoxy-1-((1-methyl-1H-pyrazol-4-yl)methyl)-1H-benzo[d]imidazol-2-yl)-1-methyl-1H-indol-7-yl)azetidin-1-yl)ethan-1-on;
Methyl-3-(2-(5-((lR,4R,7R)-7-amino-2-azabicyclo[2.2.1]heptan-2-carbonyl)-7-methoxy-1-((1-methyl-1H-pyrazol-4-yl)methyl)-1H-benzo[d]imidazol-2-yl)-1-methyl-1H-indol-7-yl)azetidin-1-carboxylat;
Methyl-4-(2-(5-((lR,4R,7R)-7-amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)piperidin-1-carboxylat;
((1R,4R,7R)-7-Amino-2-azabicyclo[2.2.1]heptan-2-yl)(2-(1-(cyclopropylmethyl)-7-(1-hydroxycyclobutyl)-1H-indol-2-yl)-7-methoxy-1-methyl-1H-benzo[d]imidazol-5-yl)methanon;
1-(4-(2-(5-((1R,4R,7R)-7-Amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)-3,6-dihydropyridin-1(2H)-yl)ethan-1-on;
Methyl-4-(2-(5-((lR,4R,7R)-7-amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)-3,6-dihydropyridin-1(2H)-carboxylat;
((1R,4R,7R)-7-Amino-2-azabicyclo[2.2.1]heptan-2-yl)(2-(1-(cyclopropylmethyl)-7-(3-(1,2-dihydroxyethyl)phenyl)-1H-indol-2-yl)-7-methoxy-1-methyl-1H-benzo[d]imidazol-5-yl)methanon;
N-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)-2-(hydroxymethyl)benzyl)acetamid;
(4-(2-(5-((1R,4R,7R)-7-Amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)piperidin-1-yl)(spiro[2.2]pentan-1-yl)methanon;
(4-(2-(5-((1R,4R,7R)-7-Amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)piperidin-1-yl)(cyclopropyl)methanon;
1-(4-(2-(5-((3R,5R)-3-Amino-5-fluorpiperidin-1-carbonyl)-7-methoxy-1-methyl-1H-benzo[d]imidazol-2-yl)-1-(cyclopropylmethyl)-1H-indol-7-yl)piperidin-1-yl)-2-methoxyethan-1-on;
1-(4-(2-(5-((3R,5R)-3-Amino-5-fluorpiperidin-1-carbonyl)-7-methoxy-1-methyl-1H-benzo[d]imidazol-2-yl)-1-(cyclopropylmethyl)- 1H-indol-7-yl)piperidin-1-yl)ethan-1-on;
3-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-N-phenylazetidin-1-carboxamid;
3-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-N-(4-fluorphenyl)azetidin-1 -carboxamid;
1-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1 -yl)-2-methoxyethan-1 -on;
3-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1-yl)-3-oxopropannitril;
1-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1-yl)-2-(2,2,2-trifluorethoxy)ethan-1-on;
1-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1-yl)-2-cyclopropyl-2-methoxyethan-1-on;
1-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1-yl)-2-ethoxyethan-1-on;
1-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1-yl)-2-phenoxyethan-1-on;
1-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1-yl)-3-methoxypropan-1-on;
1-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1 -yl)-4-methoxybutan-1 -on;
1-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1-yl)-2-methoxypropan-1-on;
1-(4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)- 1H-indol-7-yl)piperidin-1-yl)-2-isopropoxyethan-1-on;
1-(4-(2-(5-((1R,4R,7R)-7-Amino-2-azabicyclo[2.2.1]heptan-2-carbonyl)-7-methoxy-1-methyl- 1H-benzo[d]imidazol-2-yl)-1-(cyclopropylmethyl)- 1H-indol-7-yl)piperidin-1-yl)-2-hydroxyethan-1-on;
4-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-N-(4-fluorphenyl)piperidin-1-carboxamid;
4-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-N-(3 - fluorphenyl)piperidin-1-carboxamid;
4-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-N-(2-fluorphenyl)piperidin-1-carboxamid;
4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)-N-(pyridin-3-yl)piperidin-1-carboxamid;
4-(2-(5-((1R,4R,7R)-7-Amino-2-azabicyclo[2.2.1]heptan-2-carbonyl)-7-methoxy-1-methyl-1H-benzo[d]imidazol-2-yl)-1-(cyclopropylmethyl)-1H-indol-7-yl)-N-(pyridin-4-yl)piperidin-1-carboxamid;
4-(2-(5-((1R,4R,7R)-7-Amino-2-azabicyclo[2.2. 1]heptan-2-carbonyl)-7-methoxy-1 -methyl-1H-benzo[d]imidazol-2-yl)-1-(cyclopropylmethyl)- 1H-indol-7-yl)-N-(4-methoxyphenyl)piperidin-1-carboxamid;
4-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-N-(2-methoxyphenyl)piperidin-1-carboxamid;
4-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-N-(3 - methoxyphenyl)piperidin-1-carboxamid;
4-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)-1H-indol-7-yl)-N-cyclopropylpiperidin-1-carboxamid;
4-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-N-ethylpiperidin-1-carboxamid;
4-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)-1H-indol-7-yl)piperidin-1-carboxamid; oder
4-(2-(5-((1R,4R,7R)-7-Amino-2-azabicyclo[2.2.1]heptan-2-carbonyl)-7-methoxy-1 -methyl-1H-benzo[d]imidazol-2-yl)-1-(cyclopropylmethyl)- 1H-indol-7-yl)-N-isopropylpiperidin-1-carboxamid.

17. Pharmazeutisch verträgliche Zusammensetzung, umfassend die Verbindung nach einem der Ansprüche 1 bis 16, und einen pharmazeutisch verträglichen Träger, ein pharmazeutisch verträgliches Adjuvans oder ein pharmazeutisch verträgliches Vehikel, wobei die Zusammensetzung wahlweise in Kombination mit einem zusätzlichen therapeutischen Mittel ist.

18. Verfahren zur Hemmung von PAD4 in einer biologischen Probe *in vitro,* umfassend den Schritt des In-Kontakt-Bringens der PAD4 mit einer Verbindung nach einem der Ansprüche 1 bis 16.

19. Verbindung nach einem der Ansprüche 1 bis 16 oder Zusammensetzung nach Anspruch 17, zur Verwendung in der Therapie, vorzugsweise zur Verwendung in einem Verfahren zur Behandlung einer/-s PAD4-vermittelten Erkrankung, Störung oder Zustands, ausgewählt aus der Gruppe bestehend aus akuter lymphatischer Leukämie, Spondylitis ankylosans, Krebs, chronischer lymphatischer Leukämie, Colitis, Lupus, rheumatoider Arthritis, multipler Sklerose und Colitis ulcerosa, bei einem Individuum mit der/-m PAD4-vermittelten Erkrankung, Störung oder Zustand, wobei das Verfahren den Schritt des Verabreichens der Verbindung oder Zusammensetzung an das Individuum umfasst, wobei, stärker bevorzugt, die/der PAD4-vermittelte Erkrankung, Störung oder Zustand ausgewählt ist aus rheumatoider Arthritis, systemischem Lupus erythematodes, kutanem Lupus erythematodes, Colitis ulcerosa und Krebs.

## Revendications

1. Composé répondant à la formule (III) : 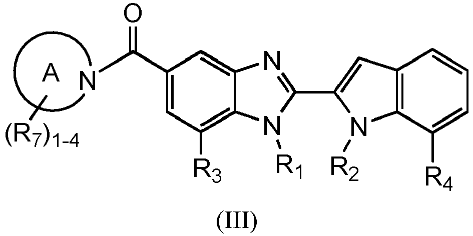
ou un sel pharmaceutiquement acceptable de celui-ci, dans lequel : 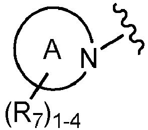 est choisi parmi 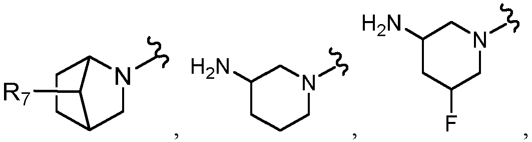 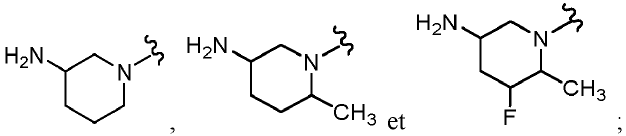
R₁ est choisi parmi -CH₃, -CD₃ et -CH₂-(hétérocyclyle à 5 ou 6 chaînons) comprenant des atomes de carbone et 1 à 3 hétéroatomes choisis parmi N, NH et N(alkyle en C₁₋₃) ;
R₂ est choisi parmi CH₃, CH₃CH₂ et -CH₂-cyclopropyle substitué par 0 à 3 Rₑ ;
R₃ est choisi parmi H, F, Cl, Br et -O(alkyle en C₁₋₄ ;
R₄ est choisi parmi 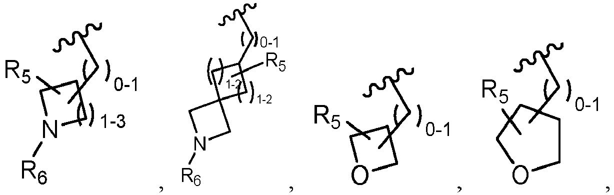 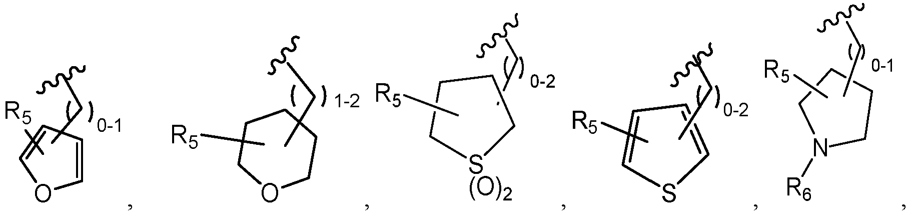 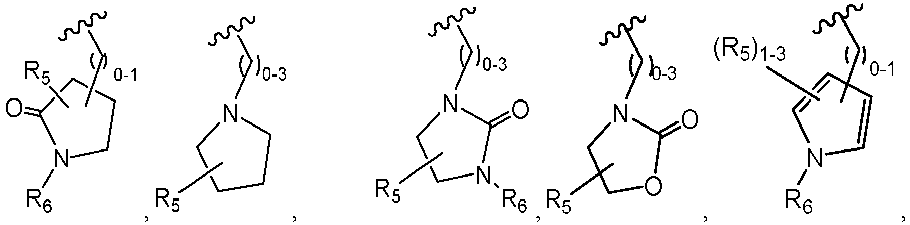 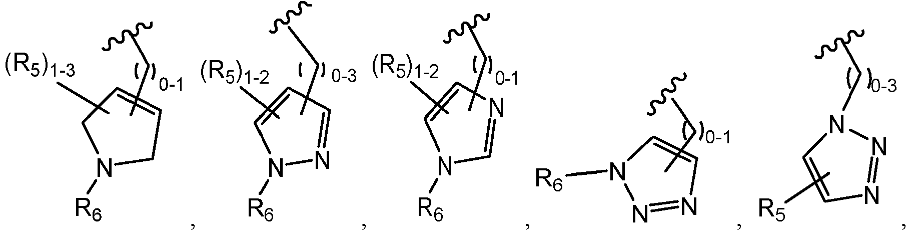 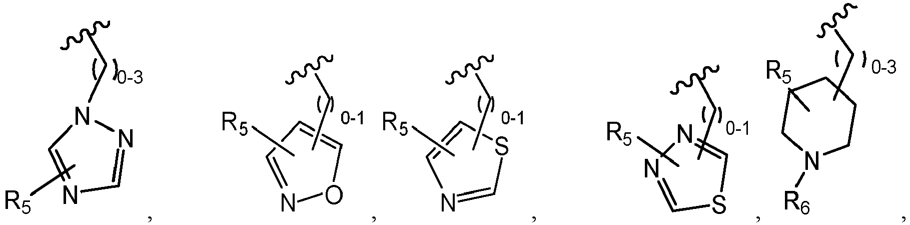 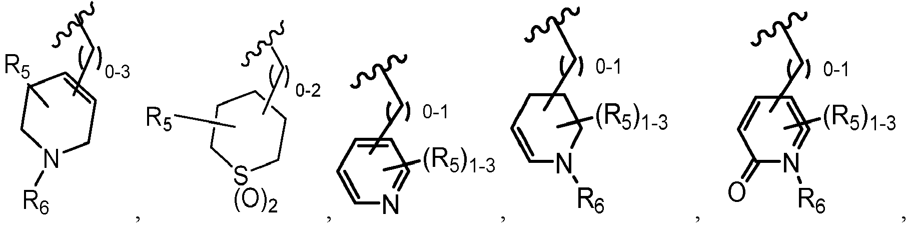 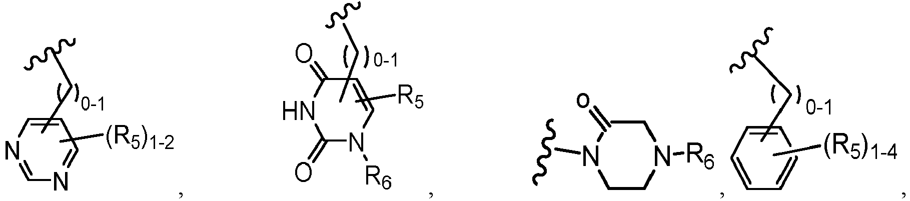 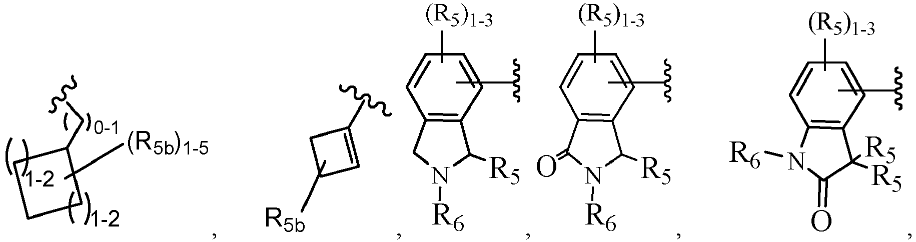 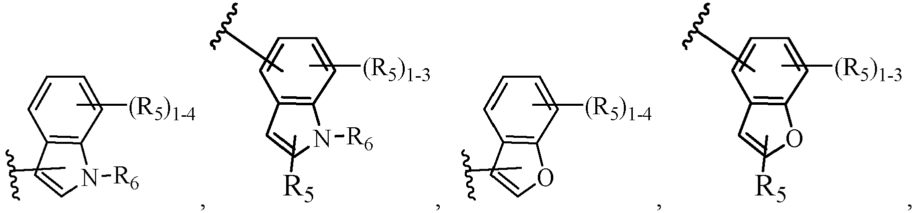 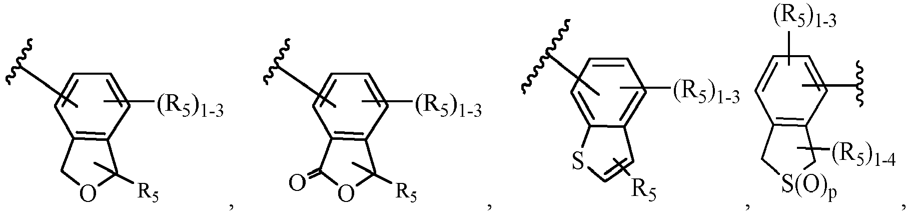 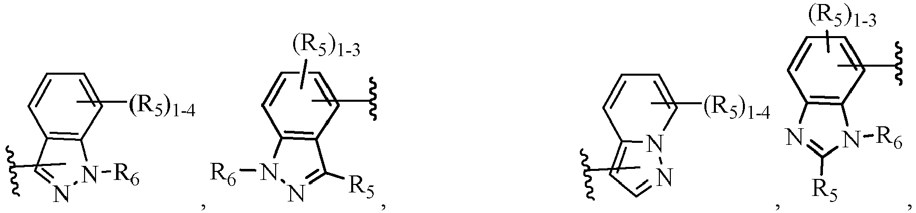 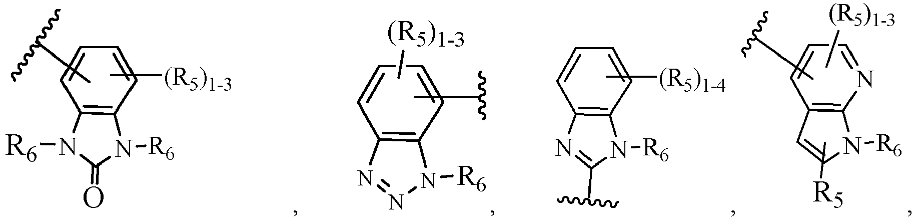 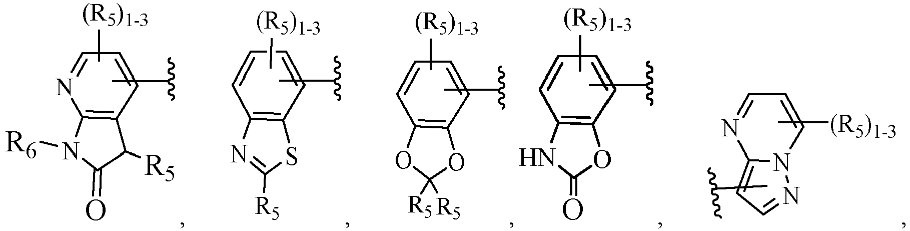 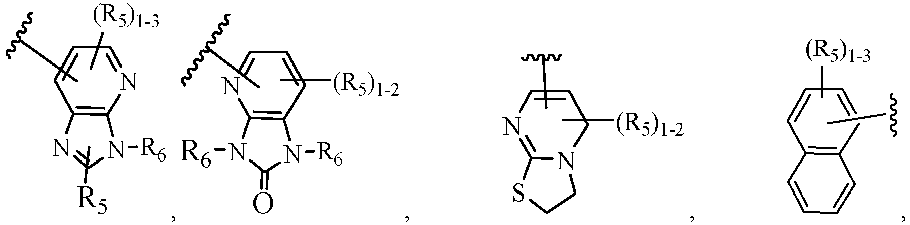 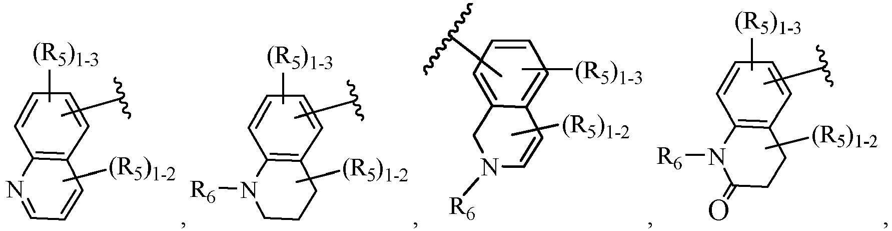 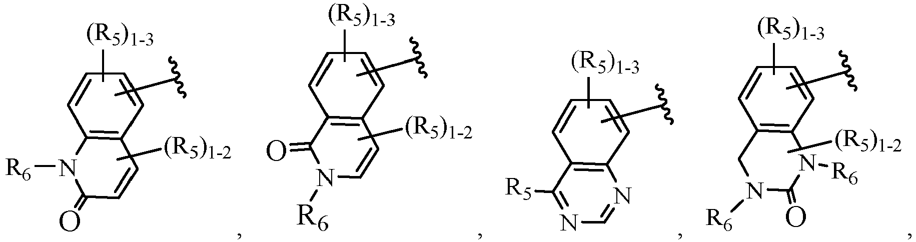 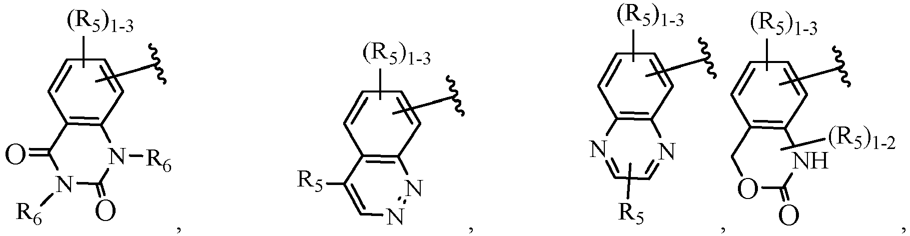 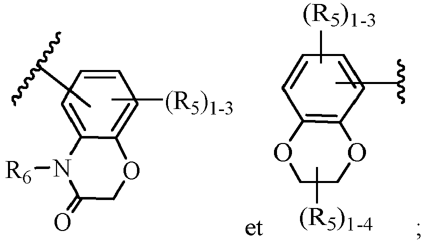
R₅, à chaque occurrence, est indépendamment choisi parmi H, F, Cl, Br, alkyle en C₁₋₄ substitué par 0 à 5 Rₑ, alcényle en C₂₋₄, alcynyle en C₂₋₄, nitro, -(CH₂)ᵣOR_{b}, -CN, -NRₐRₐ, -(CH₂)ᵣNRₐC(=O)R_{b}, -NRₐC(=O)NRₐRₐ, -C(=O)OR_{b}, -C(=O)R_{b}, -OC(=O)R_{b}, -C(=O)NRₐRₐ, -P(=O)(alkyle en C₁₋₄)₂, -S(O)*ₚ*Rₑ, -S(O)ₚNRₐRₐ, -NRₐS(O)*ₚ*Rₑ, cycloalkyle en C₃₋₆ substitué par 0 à 4 Rₑ, aryle substitué par 0 à 4 Rₑ et hétérocyclyle substitué par 0 à 4Rₑ;
R_{5b}, à chaque occurrence, est indépendamment choisi parmi OR_{b}, -C(=O)OR_{b}, -C(=O)NRₐRₐ, -NRₐRₐ, -NRₐC(=O)R_{b}, -NRₐC(=O)OR_{b}, -S(=O)ₚR_{c} et -NRₐS(=O)ₚRₑ ;
R₆, à chaque occurrence, est indépendamment choisi parmi H, alkyle en C₁₋₃ substitué par 0 à 4 Rₑ, -C(=O)R_{b}, -C(=O)(CH₂)ᵣOR_{b}, -C(=O)(CH₂)ᵣNRₐRₐ, -S(O)ₚR_{c}, -S(O)ₚNRₐRₐ, -(CH₂)ᵣ-aryle substitué par 0 à 4 Rₑ et -(CH₂)ᵣ-hétérocyclyle substitué par 0 à 4 Rₑ ;
R₇ est choisi parmi H, F, Cl, CN, alkyle en C₁₋₃, =N-OR_{b}, -(CH₂)ᵣOR_{b}, -(CH₂)ᵣNRₐRₐ, -NRₐC(=NH)(alkyle en C₁₋₃), -NRₐC(=O)OR_{b}, un carbocyclyle et hétérocyclyle ;
Rₐ, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ-hétérocyclyle substitué par 0 à 5 Rₑ ; ou Rₐ et Rₐ, conjointement avec l'atome d'azote auquel ils sont tous deux liés, forment un hétérocycle substitué par 0 à 5 Rₑ ;
R_{b}, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₆) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rf, alcényle en C₂₋₆, alcynyle en C₂₋₆, -(CH₂)ᵣ-(cycloalkyle en C₃₋₆), -(CH₂)ᵣ-aryle, F, Cl, Br, CN, NO₂, =O, -C(=O)OH, -C(=O)O(alkyle en C₁₋₄), -(CH₂)ᵣOH, -(CH₂)ᵣO(alkyle en C₁₋₄), -(CH₂)ᵣO(alcényle en C₂₋₄), -(CH₂)ᵣO(alcynyle en C₂₋₄) et NH₂ ;
Rf, à chaque occurrence, est indépendamment choisi parmi H, F, Cl, Br, CN, OH, alkyle en C₁₋₅ optionnellement substitué par OH, cycloalkyle en C₃₋₆ et phényle ;
p, à chaque occurrence, est indépendamment choisi parmi zéro, 1 et 2 ; et
r, à chaque occurrence, est indépendamment choisi parmi zéro, 1, 2, 3 et 4.

2. Composé selon la revendication 1, ou un sel pharmaceutiquement acceptable de celui-ci, dans lequel : 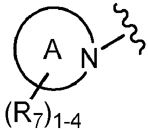 est choisi parmi 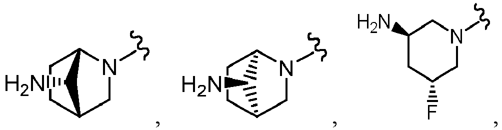 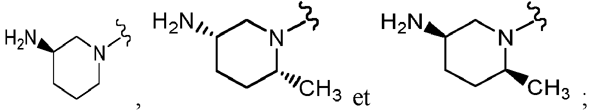
R₁ est choisi parmi -CH₃ et -CD₃ ;
R₂ est choisi parmi -CH₃ et -CH₂-cyclopropyle substitué par 0 à 2 F ou Cl ;
R₃ est choisi parmi H, F et -O(alkyle en C₁₋₄) ;
R₄ est choisi parmi 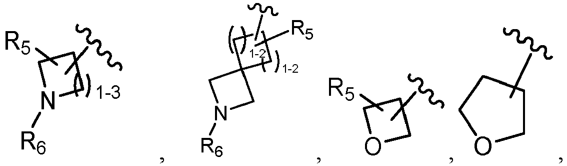 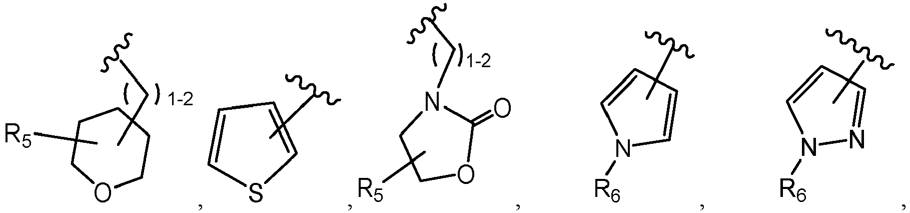 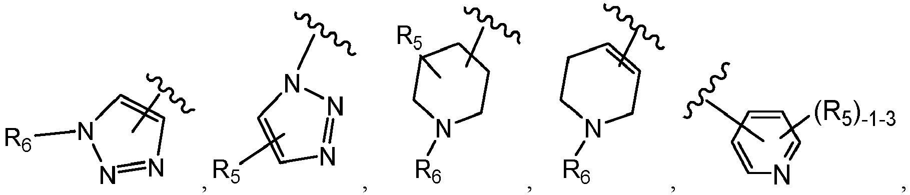 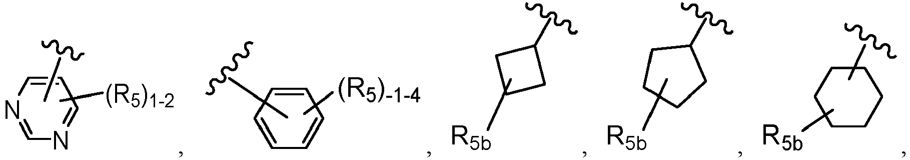 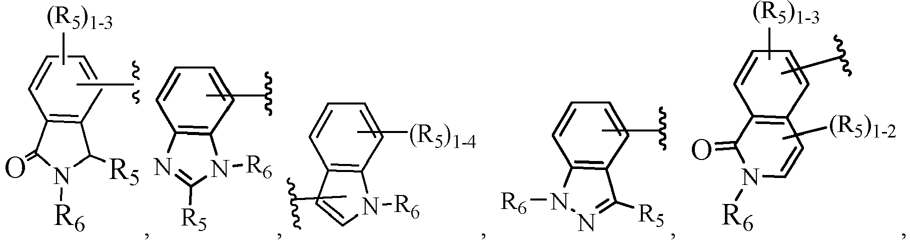 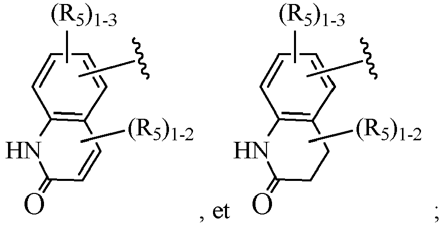
R₅, à chaque occurrence, est indépendamment choisi parmi H, F, Cl, Br, alkyle en C₁₋₄, -(CH₂)ᵣOR_{b}, -CN, -NRₐRₐ, -(CH₂)ᵣNRₐC(=O)R_{b}, -NRₐC(=O)NRₐRₐ, -C(=O)OR_{b}, -C(=O)R_{b}, -OC(=O)R_{b}, -C(=O)NRₐRₐ, -S(O)*ₚ*R_{c}, -S(O)ₚNRₐRₐ, -NRₐS(O)*ₚ*R_{c}, cycloalkyle en C₃₋₆ substitué par 0 à 4 Rₑ, aryle substitué par 0 à 4 Rₑ et hétérocyclyle substitué par 0 à 4 Rₑ ;
R_{5b}, à chaque occurrence, est indépendamment choisi parmi OH, -C(=O)OR_{b}, -C(=O)NRₐRₐ, -NRₐRₐ, -NRₐC(=O)R_{b} et -NRₐC(=O)OR_{b} ;
R₆, à chaque occurrence, est indépendamment choisi parmi H, alkyle en C₁₋₃, -S(O)ₚR_{c}, -C(=O)R_{b}, -C(=O)OR_{b}, -C(=O)(CH₂)ᵣNRₐRₐ, -S(O)ₚNRₐRₐ, aryle substitué par 0 à 4 Rₑ et hétérocyclyle substitué par 0 à 4 Rₑ ;
Rₐ, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ hétérocyclyle substitué par 0 à 5 Rₑ ; ou Rₐ et Rₐ, conjointement avec l'atome d'azote auquel ils sont tous deux liés, forment un hétérocycle substitué par 0 à 5 Rₑ ;
R_{b}, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ hétérocyclyle substitué par 0 à 5 Rₑ ;
Rₑ, à chaque occurrence, est indépendamment choisi parmi alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₆) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rf, alcényle en C₂₋₆, alcynyle en C₂₋₆, -(CH₂)ᵣ-(cycloalkyle en C₃₋₆), -(CH₂)ᵣ-aryle, F, Cl, Br, CN, NO₂, =O, -C(=O)OH, -C(=O)O(alkyle en C₁₋₄), -(CH₂)ᵣOH, -(CH₂)ᵣO(alkyle en C₁₋₄), -(CH₂)ᵣO(alcényle en C₂₋₄), -(CH₂)ᵣO(alcynyle en C₂₋₄) et NH₂ ;
Rf, à chaque occurrence, est indépendamment choisi parmi H, F, Cl, Br, CN, OH, alkyle en C₁₋₅ optionnellement substitué par OH, cycloalkyle en C₃₋₆ et phényle ;
p, à chaque occurrence, est indépendamment choisi parmi zéro, 1 et 2 ; et
r, à chaque occurrence, est indépendamment choisi parmi zéro, 1, 2, 3 et 4.

3. Composé selon la revendication 2 ou un sel pharmaceutiquement acceptable de celui-ci, dans lequel :
R₁ représente -CH₃ ;
R₂ est choisi parmi -CH₃ et -CH₂-cyclopropyle ;
R₃ représente -O(alkyle en C₁₋₄) ;
R₄ est choisi parmi 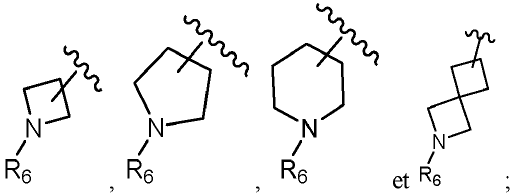
R₆ est choisi parmi H, alkyle en C₁₋₃, -C(=O)R_{b}, -C(=O)OR_{b}, -C(=O)NRₐRₐ et -C(=O)CH₂NRₐRₐ et -S(O)ₚR_{c} ;
Rₐ, à chaque occurrence, est indépendamment choisi parmi H, alkyle en C₁₋₆ substitué par 0 à 5 Rₑ, -(CH₂)ᵣ-(carbocyclyle en C₃₋₁₀) substitué par 0 à 5 Rₑ et -(CH₂)ᵣ hétérocyclyle substitué par 0 à 5 Rₑ ; ou Rₐ et Rₐ, conjointement avec l'atome d'azote auquel ils sont tous deux liés, forment un hétérocycle substitué par 0 à 5 Rₑ ;
R_{b}, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ hétérocyclyle substitué par 0 à 5 Rₑ ;
Rₑ, à chaque occurrence, est indépendamment alkyle en C₁₋₆ substitué par 0 à 5 Rₑ, -(CH₂)ᵣ-(carbocyclyle en C₃₋₆) substitué par 0 à 5 Rₑ ou -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rf, alcényle en C₂₋₆, alcynyle en C₂₋₆, -(CH₂)ᵣ-(cycloalkyle en C₃₋₆), -(CH₂)ᵣ-aryle, F, Cl, Br, CN, NO₂, =O, -C(=O)OH, -C(=O)O(alkyle en C₁₋₄), -(CH₂)ᵣOH et -(CH₂)ᵣO(alkyle en C₁₋₄) ;
Rf, à chaque occurrence, est indépendamment choisi parmi H, F, Cl, Br, CN, OH, alkyle en C₁₋₅ optionnellement substitué par OH, cycloalkyle en C₃₋₆ et phényle ;
p, à chaque occurrence, est indépendamment choisi parmi zéro, 1 et 2 ; et
r, à chaque occurrence, est indépendamment choisi parmi zéro, 1, 2, 3 et 4.

4. Composé selon la revendication 2, ou un sel pharmaceutiquement acceptable de celui-ci, dans lequel :
R₄ est choisi parmi 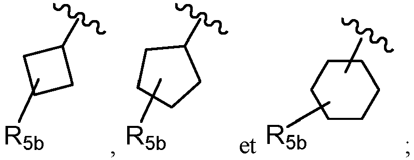 R_{5b}, à chaque occurrence, est indépendamment choisi parmi OH, -C(=O)OR_{b}, -C(=O)NRₐRₐ, -NRₐRₐ et -NRₐC(=O)R_{b} ;
Rₐ, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ-hétérocyclyle substitué par 0 à 5 Rₑ ; ou Rₐ et Rₐ, conjointement avec l'atome d'azote auquel ils sont tous deux liés, forment un hétérocycle substitué par 0 à 5 Rₑ ;
R_{b}, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rf, alcényle en C₂₋₆, alcynyle en C₂₋₆, -(CH₂)ᵣ-(cycloalkyle en C₃₋₆), -(CH₂)ᵣ-aryle, F, Cl, Br, CN, NO₂, =O, -C(=O)OH, -C(=O)O(alkyle en C₁₋₄), -(CH₂)ᵣOH et -(CH₂)ᵣO(alkyle en C₁₋₄) ;
Rf, à chaque occurrence, est indépendamment choisi parmi H, F, Cl, Br, CN, OH, alkyle en C₁₋₅ optionnellement substitué par OH, cycloalkyle en C₃₋₆ et phényle ;
p, à chaque occurrence, est indépendamment choisi parmi zéro, 1 et 2 ; et
r, à chaque occurrence, est indépendamment choisi parmi zéro, 1, 2, 3 et 4.

5. Composé selon la revendication 4 ou un sel pharmaceutiquement acceptable de celui-ci, dans lequel :
R₄ représente 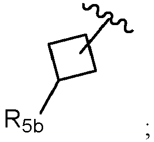
R_{5b} est choisi parmi -C(=O)OR_{b}, -C(=O)NRₐRₐ et -NHC(=O)R_{b} ;
Rₐ, à chaque occurrence, est indépendamment choisi parmi H et alkyle en C₁₋₆ substitué par 0 à 5 Rₑ, ou Rₐ et Rₐ, conjointement avec l'atome d'azote auquel ils sont tous deux liés, forment un hétérocycle choisi parmi 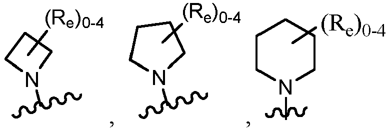 et 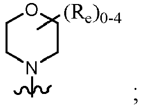
R_{b} est choisi parmi H et alkyle en C₁₋₆ substitué par 0 à 5 Rₑ ;
Rₑ, à chaque occurrence, est indépendamment choisi parmi alkyle en C₁₋₆ substitué par 0 à 5 Rf, alcényle en C₂₋₆, alcynyle en C₂₋₆, -(CH₂)ᵣ-(cycloalkyle en C₃₋₆), -(CH₂)ᵣ-aryle, F, Cl, Br, CN, NO₂, =O, -C(=O)OH, -C(=O)O(alkyle en C₁₋₄), -(CH₂)ᵣOH et -(CH₂)ᵣO(alkyle en C₁₋₄) ; et
Rf, à chaque occurrence, est indépendamment choisi parmi H, F, Cl, Br, CN, OH, alkyle en C₁₋₅ optionnellement substitué par OH, cycloalkyle en C₃₋₆ et phényle.

6. Composé selon la revendication 2 ou un sel pharmaceutiquement acceptable de celui-ci, dans lequel :
R₄ est choisi parmi 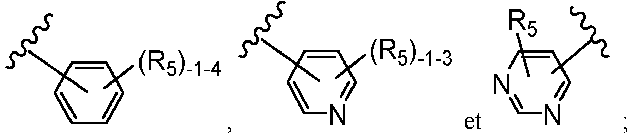
R₅, à chaque occurrence, est indépendamment choisi parmi H, F, Cl, Br, alkyle en C₁₋₄ substitué par 0 à 5 Rₑ, alcényle en C₂₋₄, alcynyle en C₂₋₄, -(CH₂)₀₋₁OR_{b}, -CN, -NRₐRₐ, -(CH₂)₀₋₁-NHC(=O)R_{b}, -NRₐC(=O)NRₐRₐ, -C(=O)OR_{b}, -C(=O)R_{b}, -OC(=O)R_{b}, -C(=O)NRₐRₐ, -S(O)ₚR_{c}, -S(O)ₚNRₐRₐ, -NRₐS(O)*ₚ*Rₑ, cycloalkyle en C₃₋₆, hétérocyclyle et aryle ; ledit alkyle, cycloalkyle, hétérocyclyle ou aryle étant substitué par 0 à 4 Rₑ ;
Rₐ, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ hétérocyclyle substitué par 0 à 5 Rₑ ; ou Rₐ et Rₐ, conjointement avec l'atome d'azote auquel ils sont tous deux liés, forment un hétérocycle substitué par 0 à 5 Rₑ ;
R_{b}, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₆) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rf, alcényle en C₂₋₆, alcynyle en C₂₋₆, -(CH₂)ᵣ-(cycloalkyle en C₃₋₆), -(CH₂)ᵣ-aryle, F, Cl, Br, CN, NO₂, =O, -C(=O)OH, -C(=O)O(alkyle en C₁₋₄), -(CH₂)ᵣOH et -(CH₂)ᵣO(alkyle en C₁₋₄) ;
Rf, à chaque occurrence, est indépendamment choisi parmi H, F, Cl, Br, CN, OH, alkyle en C₁₋₅ optionnellement substitué par OH, cycloalkyle en C₃₋₆ et phényle ;
p, à chaque occurrence, est indépendamment choisi parmi zéro, 1 et 2 ; et
r, à chaque occurrence, est indépendamment choisi parmi zéro, 1, 2, 3 et 4.

7. Composé selon la revendication 2 ou un sel pharmaceutiquement acceptable de celui-ci, dans lequel :
R₂ est choisi parmi -CH₃ et -CH₂-cyclopropyle substitué par 0 à 2 F ou Cl ;
R₄, à chaque occurrence, est choisi parmi 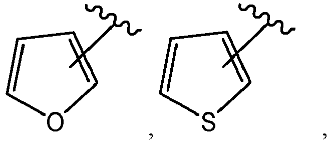 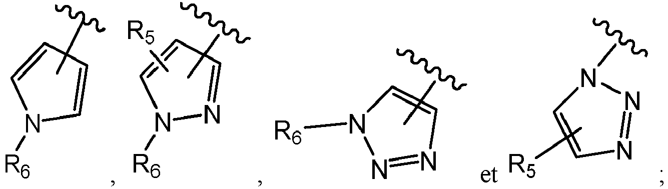
R₅, à chaque occurrence, est indépendamment choisi parmi H, F, Cl, Br, alkyle en C₁₋₄ substitué par 0 à 5 Rₑ, alcényle en C₂₋₄, alcynyle en C₂₋₄, nitro, -OR_{b}, -CN,-NRₐRₐ, -S(O)*ₚ*Rₑ, -S(O)ₚNRₐRₐ, -NRₐS(O)*ₚ*Rₑ, -(CH₂)ᵣ-NRₐC(=O)R_{b}, -NRₐC(=O)NRₐRₐ, C(=O)OR_{b}, -C(=O)R_{b}, -OC(=O)R_{b}, -C(=O)NRₐRₐ, P(=O)(O(alkyle en C₁₋₄))₂, cycloalkyle en C₃₋₆ substitué par 0 à 4 Rₑ, aryle substitué par 0 à 4 Rₑ, hétérocyclyle substitué par 0 à 4 Rₑ;
R₆, à chaque occurrence, est indépendamment choisi parmi H, alkyle en C₁₋₃ substitué par 0 à 4 Rₑ et hétérocyclyle substitué par 0 à 4 Rₑ ;
Rₐ, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ hétérocyclyle substitué par 0 à 5 Rₑ ; ou Rₐ et Rₐ, conjointement avec l'atome d'azote auquel ils sont tous deux liés, forment un hétérocycle substitué par 0 à 5 Rₑ ;
R_{b}, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₆) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rf, alcényle en C₂₋₆, alcynyle en C₂₋₆, -(CH₂)ᵣ-(cycloalkyle en C₃₋₆), -(CH₂)ᵣ-aryle, F, Cl, Br, CN, NO₂, =O, -C(=O)OH, -C(=O)O(alkyle en C₁₋₄), -(CH₂)ᵣOH et -(CH₂)ᵣO(alkyle en C₁₋₄), -(CH₂)ᵣO(alcényle en C₂₋₄), -(CH₂)ᵣO(alcynyle en C₂₋₄) et NH₂ ;
Rf, à chaque occurrence, est indépendamment choisi parmi H, F, Cl, Br, CN, OH, alkyle en C₁₋₅ optionnellement substitué par OH, cycloalkyle en C₃₋₆ et phényle ;
p, à chaque occurrence, est indépendamment choisi parmi zéro, 1 et 2 ; et
r, à chaque occurrence, est indépendamment choisi parmi zéro, 1, 2, 3 et 4.

8. Composé selon la revendication 7 ou un sel pharmaceutiquement acceptable de celui-ci, dans lequel :
R₂ est choisi parmi -CH₃ et -CH₂-cyclopropyle substitué par 0 à 2 F et Cl ;
R₄ représente 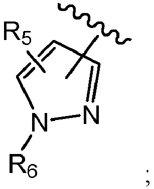
R₅, à chaque occurrence, est indépendamment choisi parmi H, F, Cl, Br, alkyle en C₁₋₄, aryle substitué par 0 à 4 Rₑ et hétérocyclyle substitué par 0 à 4 Rₑ ;
R₆, à chaque occurrence, est indépendamment choisi parmi H, alkyle en C₁₋₃ substitué par 0 à 4 Rₑ et hétérocyclyle substitué par 0 à 4 Rₑ ;
Rₑ, à chaque occurrence, est indépendamment choisi parmi alkyle en C₁₋₆, F, Cl, Br, CN, -OH et -(CH₂)ᵣO(alcynyle en C₂₋₄) ; et
r, à chaque occurrence, est indépendamment choisi parmi zéro, 1 et 2.

9. Composé selon la revendication 2 ou un sel pharmaceutiquement acceptable de celui-ci, dans lequel :
R₄ est choisi parmi 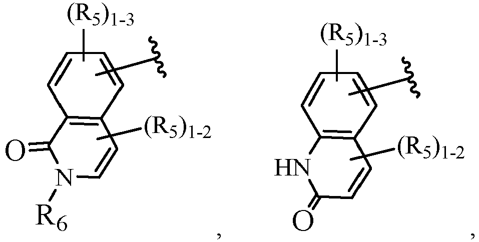 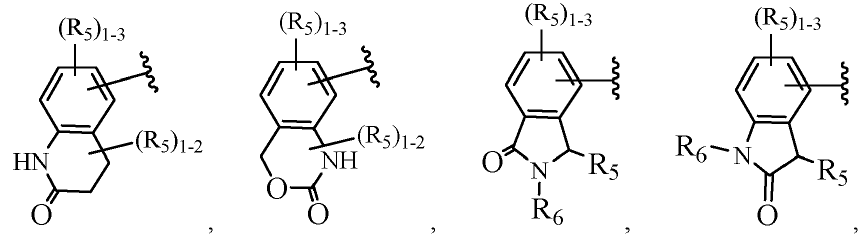 et 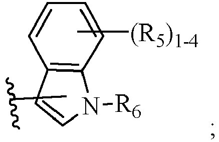
R₅, à chaque occurrence, est indépendamment choisi parmi H, F, Cl, Br et alkyle en C₁₋₄ substitué par 0 à 5 Rₑ, OR_{b}, -CN, -C(=O)R_{b}, -C(=O)OR_{b}, -OC(=O)R_{b}, -C(=O)NRₐRₐ ;
R₆ est choisi parmi H et alkyle en C₁₋₃ ;
Rₐ, à chaque occurrence, est indépendamment choisi parmi H et alkyle en C₁₋₄ substitué par 0 à 5 Rₑ ;
R_{b}, à chaque occurrence, est indépendamment choisi parmi H et alkyle en C₁₋₄ substitué par 0 à 5 Rₑ ;
Rₑ, à chaque occurrence, est indépendamment choisi parmi alkyle en C₁₋₆ F, Cl, Br, CN, NO₂, =O, -C(=O)OH, -C(=O)O(alkyle en C₁₋₄), -(CH₂)ᵣOH et -(CH₂)ᵣO(alkyle en C₁₋₄) ; et
r, à chaque occurrence, est indépendamment choisi parmi zéro, 1 et 2.

10. Composé selon la revendication 1, ou un sel pharmaceutiquement acceptable de celui-ci, dans lequel : 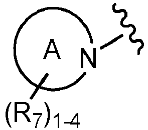 est choisi parmi 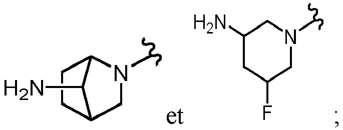
R₃ est choisi parmi H, F, Cl et -O(alkyle en C₁₋₄) ;
R₄ est choisi parmi 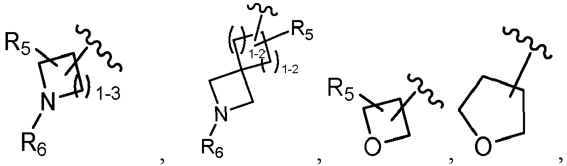 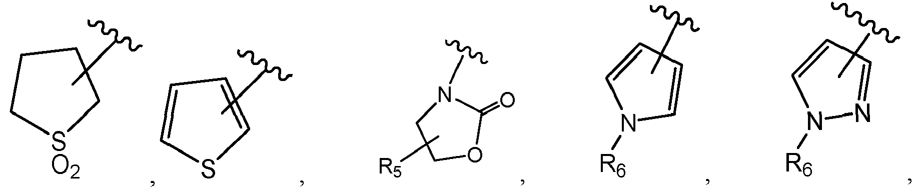 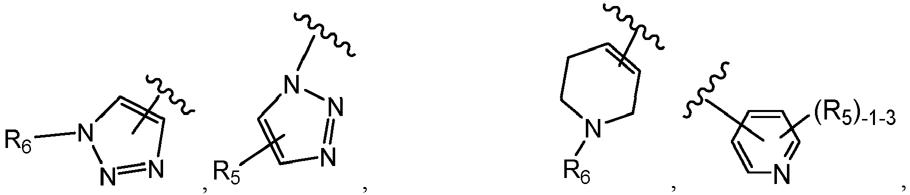 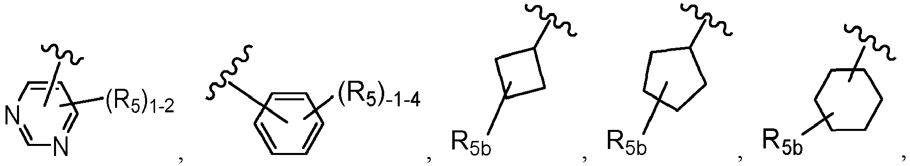 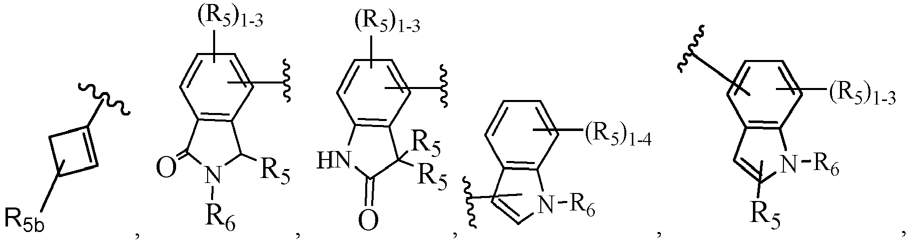 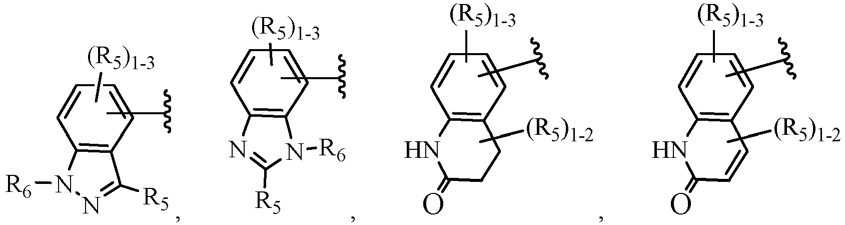 et 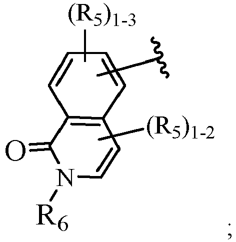
R₅, à chaque occurrence, est indépendamment choisi parmi H, F, Cl, Br, alkyle en C₁₋₄ substitué par 0 à 3 Rₑ, -OR_{b}, -CN, -NRₐRₐ, -NRₐC(=O)R_{b}, -NRₐC(=O)NRₐRₐ, -C(=O)OR_{b}, -C(=O)R_{b}, -OC(=O)R_{b}, -C(=O)NRₐRₐ, -S(O)*ₚ*R_{c}, -S(O)*ₚ*NRₐRₐ, -NRₐS(O)*ₚ*R_{c}, cycloalkyle en C₃₋₆ substitué par 0 à 4 Rₑ, aryle substitué par 0 à 4 Rₑ et hétérocyclyle substitué par 0 à 4 Rₑ ;
R_{5b}, à chaque occurrence, est indépendamment choisi parmi OH, -C(=O)OR_{b}, -C(=O)NRₐRₐ et -NRₐC(=O)R_{b} ;
R₆, à chaque occurrence, est indépendamment choisi parmi H, alkyle en C₁₋₃, -C(=O)R_{b}, -C(=O)(CH₂)ᵣOR_{b}, -C(=O)(CH₂)ᵣNRₐRₐ, -S(O)ₚR_{c}, -S(O)*ₚ*NRₐRₐ, aryle substitué par 0 à 4 Rₑ et hétérocyclyle substitué par 0 à 4 Rₑ ;
Rₐ, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ hétérocyclyle substitué par 0 à 5 Rₑ ; ou Rₐ et Rₐ, conjointement avec l'atome d'azote auquel ils sont tous deux liés, forment un hétérocycle substitué par 0 à 5 Rₑ ;
R_{b}, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ hétérocyclyle substitué par 0 à 5 Rₑ ;
Rₑ, à chaque occurrence, est indépendamment choisi parmi alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₆) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rf, alcényle en C₂₋₆, alcynyle en C₂₋₆, -(CH₂)ᵣ-(cycloalkyle en C₃₋₆), -(CH₂)ᵣ-aryle, F, Cl, Br, CN, NO₂, =O, -C(=O)OH, -C(=O)O(alkyle en C₁₋₄), -(CH₂)ᵣOH et -(CH₂)ᵣO(alkyle en C₁₋₄) ;
Rf, à chaque occurrence, est indépendamment choisi parmi H, F, Cl, Br, CN, OH, alkyle en C₁₋₅ optionnellement substitué par OH, cycloalkyle en C₃₋₆ et phényle ;
p, à chaque occurrence, est indépendamment choisi parmi zéro, 1 et 2 ; et
r, à chaque occurrence, est indépendamment choisi parmi zéro, 1, 2, 3 et 4.

11. Composé selon la revendication 10, ou un sel pharmaceutiquement acceptable de celui-ci, dans lequel : 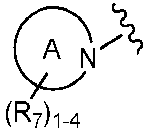 est choisi parmi 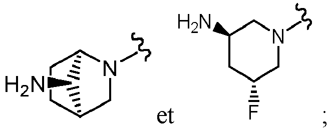
R₁ est choisi parmi -CH₃, -CD₃ et -CH₂-(hétérocyclyle à 5 ou 6 chaînons) comprenant des atomes de carbone et 1 à 3 hétéroatomes choisis parmi N, NH et N(alkyle en C₁₋₃) ;
R₂ est choisi parmi -CH₃ et -CH₂-cyclopropyle ;
R₃ est choisi parmi H, F et -O(alkyle en C₁₋₄) ;
R₄ est choisi parmi 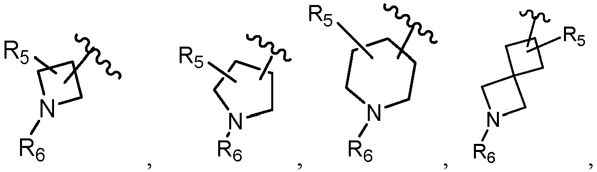 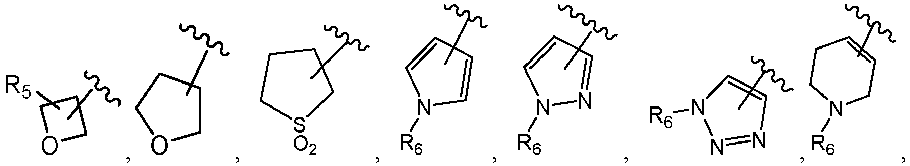 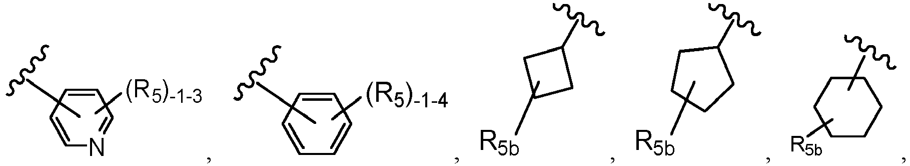 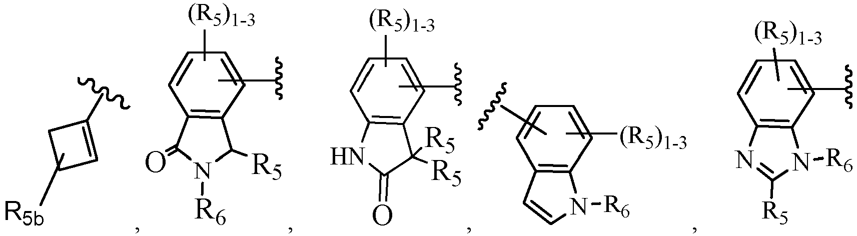 et 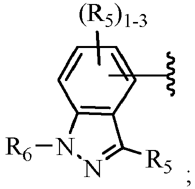
R₅, à chaque occurrence, est indépendamment choisi parmi H, F, Cl, Br, alkyle en C₁₋₄ substitué par 0 à 3 Rₑ, -OR_{b}, -CN, -NRₐRₐ ;
R_{5b}, à chaque occurrence, est indépendamment choisi parmi OH, -C(=O)OR_{b}, -C(=O)NRₐRₐ et -NRₐC(=O)R_{b} ;
R₆, à chaque occurrence, est indépendamment choisi parmi H, alkyle en C₁₋₃, -C(=O)R_{b}, -C(=O)(CH₂)ᵣOR_{b}, -C(=O)(CH₂)ᵣNRₐRₐ, -S(O)ₚR_{c}, -S(O)ₚNRₐRₐ, aryle substitué par 0 à 4 Rₑ et hétérocyclyle substitué par 0 à 4 Rₑ ;
Rₐ, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ-hétérocyclyle substitué par 0 à 5 Rₑ ; ou Rₐ et Rₐ, conjointement avec l'atome d'azote auquel ils sont tous deux liés, forment un hétérocycle substitué par 0 à 5 Rₑ ;
R_{b}, à chaque occurrence, est indépendamment choisi parmi H, alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₁₀) substitué par 0 à 5 Rₑ et -(CH₂)ᵣ hétérocyclyle substitué par 0 à 5 Rₑ ;
Rₑ, à chaque occurrence, est indépendamment choisi parmi alkyle en C₁₋₆ substitué par 0 à 5 Rₑ, alcényle en C₂₋₆ substitué par 0 à 5 Rₑ, alcynyle en C₂₋₆ substitué par 0 à 5 Rₑ, -(CH₂)ᵣ-(carbocyclyle en C₃₋₆) substitué par 0 à 5 Rₑ et -(CH₂)ᵣ-hétérocyclyle substitué par 0 à 5 Rₑ ;
Rₑ, à chaque occurrence, est indépendamment choisi parmi alkyle en C₁₋₆ substitué par 0 à 5 Rf, alcényle en C₂₋₆, alcynyle en C₂₋₆, -(CH₂)ᵣ-(cycloalkyle en C₃₋₆), -(CH₂)ᵣ-aryle, F, Cl, Br, CN, NO₂, =O, -C(=O)OH, -C(=O)O(alkyle en C₁₋₄), -(CH₂)ᵣOH et -(CH₂)ᵣO(alkyle en C₁₋₄) ;
Rf, à chaque occurrence, est indépendamment choisi parmi H, F, Cl, Br, CN, OH, alkyle en C₁₋₅ optionnellement substitué par OH, cycloalkyle en C₃₋₆ et phényle ;
p, à chaque occurrence, est indépendamment choisi parmi zéro, 1 et 2 ; et
r, à chaque occurrence, est indépendamment choisi parmi zéro, 1, 2, 3 et 4.

12. Composé selon la revendication 1, présentant la formule (V) : 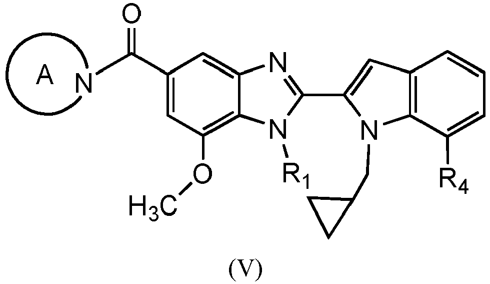
ou un sel pharmaceutiquement acceptable de celui-ci, dans lequel : 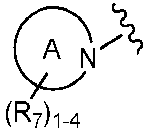 est choisi parmi 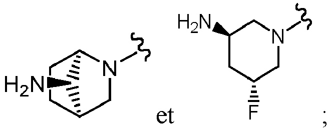
R₁ est choisi parmi -CH₃ et 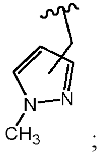
R₄ est choisi parmi 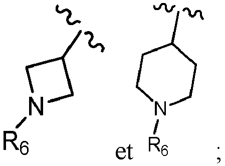
R₆, à chaque occurrence, est indépendamment choisi parmi -C(=O)R_{b}, -C(=O)(CH₂)₁₋₃OR_{b} et -C(=O)NRₐRₐ ;
Rₐ, à chaque occurrence, est indépendamment choisi parmi H, alkyle en C₁₋₃ substitué par 0 à 3 Rₑ, cycloalkyle en C₃₋₆, phényle substitué par 0 à 3 Rₑ et pyridyle ;
R_{b} est choisi parmi H et alkyle en C₁₋₃ substitué par 0 à 3 Rₑ ; et
Rₑ, à chaque occurrence, est indépendamment choisi parmi F, Cl, -OH, -O(alkyle en C₁₋₄), C(=O)OH et phényle.

13. Composé selon la revendication 12, présentant la formule (VI) : 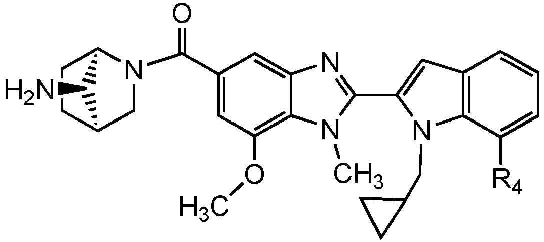 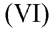
ou un sel pharmaceutiquement acceptable de celui-ci, dans lequel :
R₄ est choisi parmi 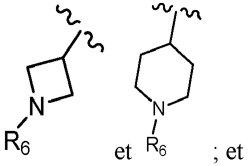
R₆, à chaque occurrence, est indépendamment choisi parmi -C(=O)CH₃, C(=O)(CH₂)₁₋₂OH, -C(=O)CH₂OCH₂CF₃, -C(=O)(CH₂)₁₋₃OCH₃, -C(=O)(CH₂)₁₋₃-phényle, -C(=O)(CH₂)₁₋₃-pyridyle, -C(=O)(CH₂)₁₋₃-tétrazolyle, -C(=O)NH₂, -C(=O)NHalkyle en C₁₋₃, -C(=O)NH-pyridyle, -C(=O)NH-cyclopropyle et -C(=O)NH-phényle substitué par 0 à 1 F, Cl, alkyle en C₁₋₂ et O(alkyle en C₁₋₂).

14. Composé selon la revendication 4, présentant la formule (IX) : 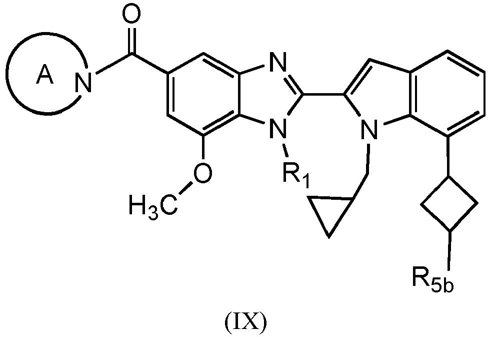
ou un sel pharmaceutiquement acceptable de celui-ci, dans lequel : 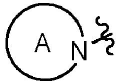 est choisi parmi 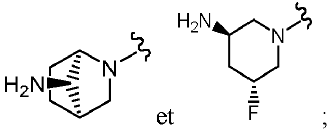
R₁ est choisi parmi -CH₃ et 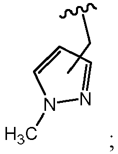
R_{5b} est choisi parmi OH, -C(=O)NRₐRₐ, -C(=O)OR_{b}, NHC(=O)R_{b} et NH₂,
Rₐ, à chaque occurrence, est indépendamment choisi parmi H, CH₃ et CD₃ ; ou Rₐ et Rₐ, conjointement avec l'atome d'azote auquel ils sont tous deux liés, forment un hétérocycle substitué par 0 à 2 OH ; et
R_{b} est choisi parmi H, CH₃ et CD₃.

15. Composé selon la revendication 4, présentant la formule (XI) : 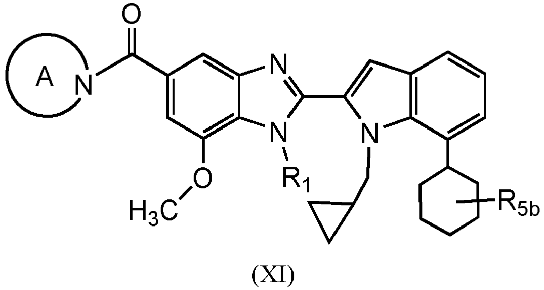
ou un sel pharmaceutiquement acceptable de celui-ci, dans lequel : 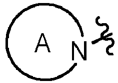 est choisi parmi 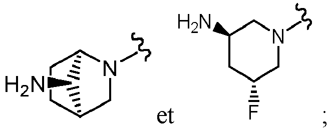
R₁ est choisi parmi -CH₃ et 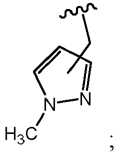 et
R_{5b}, est choisi parmi -OH, -NH₂ et CONH₂.

16. Composé selon la revendication 1, qui est :
(1R,4R,7R)-2- f 2-[1-(cyclopropylméthyl)-7-(pipéridin-4-yl)-1H-indol-2-yl]-7-méthoxy-1-méthyl-1H-1,3-benzodiazole-5-carbonyl}-2-azabicyclo[2.2.1]heptan-7-amine ;
(1R,4R,7R)-2-{2-[7-(azétidin-3-yl)-1-(cyclopropylméthyl)-1H-indol-2-yl]-7-méthoxy-1-méthyl-1H-1,3-benzodiazole-5-carbonyl}-2-azabicyclo[2.2.1]heptan-7-amine ;
(1R,4R,7R)-2- {2-[7-(3-aminocyclobutyl)-1-(cyclopropylméthyl)-1H-indol-2-yl] - 7-méthoxy-1-méthyl-1H-1,3-benzodiazole-5-carbonyl}-2-azabicyclo[2.2.1]heptan-7-amine ;
(1R,4R,7R)-2-{2-[1-(cyclopropylméthyl)-7-[(pipéridin-4-yl)méthyl]-1H-indol-2-yl]-7-méthoxy-1-méthyl-1H-1,3-benzodiazole-5-carbonyl}-2-azabicyclo[2.2.1]heptan-7-amine ;
(1R,4R,7R)-2-{2-[1-(cyclopropylméthyl)-7-(pipéridin-3-yl)-1H-indol-2-yl]-7-méthoxy-1-méthyl-1H-1,3-benzodiazole-5-carbonyl}-2-azabicyclo[2.2.1]heptan-7-amine ;
(1R,4R,7R)-2- f 2-[7-(4-aminocyclohexyl)-1-(cyclopropylméthyl)-1H-indol-2-yl]-7-méthoxy-1-méthyl-1H-1,3-benzodiazole-5-carbonyl}-2-azabicyclo[2.2.1]heptan-7-amine ;
((lR,4R,7R)-7-amino-2-azabicyclo[2.2.1]heptan-2-yl)(2-(7-(((trans)-4-aminocyclohexyl)méthyl)-1-(cyclopropylméthyl)-1H-indol-2-yl)-7-méthoxy-1-méthyl-1H-benzo[d]imidazol-5-yl)méthanone ;
1-[4-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)pipéridin-1-yl]éthan-1-one ;
cis- et trans-3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)cyclobutan-1-ol ;
(1R,4R,7R)-2-{2-[1-(cyclopropylméthyl)-7-(1-méthylazétidin-3-yl)-1H-indol-2-yl]-7-méthoxy-1-méthyl-1H-1,3-benzodiazole-5-carbonyl}-2-azabicyclo[2.2.1]heptan-7-amine ;
3-[2-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)éthyl]-1,3-oxazolidin-2-one ;
4-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)cyclohexan-1-ol ;
(1R,4R,7R)-2-{2-[1-(cyclopropylméthyl)-7-(oxétan-3-yl)-1H-indol-2-yl]-7-méthoxy-1-méthyl-1H-1,3-benzodiazole-5-carbonyl}-2-azabicyclo[2.2.1]heptan-7-amine
(1R,4R,7R)-2-(2-{7-[(azétidin-3-yl)méthyl]-1-(cyclopropylméthyl)-1H-indol-2-yl}-7-méthoxy-1-méthyl-1H-1,3-benzodiazole-5-carbonyl)-2-azabicyclo[2.2.1]heptan-7-amine ;
3-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)pyrrolidine-1-carboxylate de benzyle ;
(1R,4R,7R)-2-{2-[1-(cyclopropylméthyl)-7-[2-(1H-1,2,4-triazol-1-yl)éthyl]-1H-indol-2-yl]-7-méthoxy-1-méthyl-1H-1,3-benzodiazole-5-carbonyl}-2-azabicyclo[2.2. 1]heptan-7-amine ;
((1R,4R,7R)-7-amino-2-azabicyclo[2.2.1]heptan-2-yl)(2-(1-(cyclopropylméthyl)-7-(1,1-dioxidotétrahydrothiophén-3-yl)-1H-indol-2-yl)-7-méthoxy-1-méthyl-1H-benzo[d]imidazol-5-yl)méthanone ;
(1R,4R,7R)-2- f 2-[1-(cyclopropylméthyl)-7-[(oxan-4-yl)méthyl]-1H-indol-2-yl]-7-méthoxy-1-méthyl-1H-1,3-benzodiazole-5-carbonyl}-2-azabicyclo[2.2.1]heptan-7-amine ;
(1R,4R,7R)-2-{2-[1-(cyclopropylméthyl)-7-[2-(pipéridin-3-yl)éthyl]-1H-indol-2-yl]-7-méthoxy-1-méthyl-1H-1,3-benzodiazole-5-carbonyl}-2-azabicyclo[2.2.1]heptan-7-amine ;
(1R,4R,7R)-2-{2-[1-(cyclopropylméthyl)-7-[2-(3,5-diméthyl-1H-pyrazol-4-yl)éthyl]-1H-indol-2-yl]-7-méthoxy-1-méthyl-1H-1,3-benzodiazole-5-carbonyl }-2-azabicyclo[2.2. 1]heptan-7-amine ;
(1R,4R,7R)-2- f 2-[1-(cyclopropylméthyl)-7-[(oxan-2-yl)méthyl]-1H-indol-2-yl]-7-méthoxy-1-méthyl-1H-1,3-benzodiazole-5-carbonyl}-2-azabicyclo[2.2.1]heptan-7-amine ;
1-(3-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)azétidin-1-yl)éthan-1-one ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)pipéridin-1-yl]éthan-1-one ;
3-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)pipéridine-1-carboxamide ;
3-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)azétidine-1-carboxylate de méthyle ;
1-[3-(2-{5-[(3R,5R)-3-amino-5-fluoropipéridine-1-carbonyl]-7-méthoxy-1-méthyl-1H-1,3 -benzodiazol-2-yl}-1-(cyclopropylméthyl)-1H-indol-7-yl)azétidin-1-yl]éthan-1-one ;
1-(3-(2-(5-((2S,5R)-5-amino-2-méthylpipéridine-1-carbonyl)-7-méthoxy-1-méthyl-1H-benzo[d]imidazol-2-yl)-1-(cyclopropylméthyl)-1H-indol-7-yl)azétidin-1 - yl)éthan-1-one ;
3-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-méthylcyclobutane-1-carboxamide ;
3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)cyclobutane-1-carboxamide ;
3-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)-N,N-diméthylcyclobutane-1-carboxamide ;
(3-(2-(5-((lR,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)cyclobutyl)(pyrrolidin-1-yl)méthanone ;
(3-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)cyclobutyl)((R)-3-hydroxypyrrolidin-1-yl)méthanone ;
(3-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)cyclobutyl)((S)-3-hydroxypyrrolidin-1-yl)méthanone ;
acide 3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)cyclobutane-1-carboxylique ;
N-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)cyclobutyl]acétamide ;
(1R,4R,7R)-2-[2-(7-{2-azaspiro[3.3]heptan-6-yl}-1-(cyclopropylméthyl)-1H-indol-2-yl)-7-méthoxy-1-méthyl-1H-1,3-benzodiazole-5-carbonyl]-2-azabicyclo[2.2.1]heptan-7-amine ;
1-[6-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-2-azaspiro[3.3]heptan-2-yl]éthan-1-one ;
1-{3-[(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)méthyl]azétidin-1-yl}éthan-1-one ;
(1R,4R,7R)-2-{2-[1-(cyclopropylméthyl)-7-(1-méthanesulfonylazétidin-3-yl)-1H-indol-2-yl]-7-méthoxy-1-méthyl-1H-1,3-benzodiazole-5-carbonyl}-2-azabicyclo[2.2.1]heptan-7-amine ;
2-amino-1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]éthan-1-one ;
3-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)azétidine-1-carboxamide ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2,2-diméthylpropan-1-one ;
acide 4-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-4-oxobutanoïque ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2-hydroxy-2-méthylpropan-1-one ;
3-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)-N-phénylazétidine-1-carboxamide ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2-méthoxyéthan-1-one ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2-phényléthan-1-one ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2-(pyridin-4-yl)éthan-1-one ;
(1R,4R,7R)-2-{2-[7-(1-benzoylazétidin-3-yl)-1-(cyclopropylméthyl)-1H-indol-2-yl]-7-méthoxy-1-méthyl-1H-1,3-benzodiazole-5-carbonyl}-2-azabicyclo[2.2.1]heptan-7-amine ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2-(pyridin-2-yl)éthan-1-one ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2-(pyrazin-2-yl)éthan-1-one ;
3-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-3-oxopropanenitrile ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2-(pyridin-3-yl)éthan-1-one ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-3-phénylpropan-1-one ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-3-hydroxypropan-1-one ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2-(1H-1,2,3,4-tétrazol-5-yl)éthan-1-one ;
1-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)azétidin-1-yl]-2-hydroxyéthan-1-one
(1R,4R,7R)-2-(2-{7-[1-(benzènesulfonyl)azétidin-3-yl]-1-(cyclopropylméthyl)-1H-indol-2-yl}-7-méthoxy-1-méthyl-1H-1,3-benzodiazole-5-carbonyl)-2-azabicyclo[2.2. 1]heptan-7-amine
(1R,4R,7R)-2-{2-[1-(cyclopropylméthyl)-7-(1,2,3,6-tétrahydropyridin-4-yl)-1H-indol-2-yl]-7-méthoxy-1-méthyl-1H-1,3-benzodiazole-5-carbonyl}-2-azabicyclo[2.2. 1]heptan-7-amine
3-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)cyclohexane-1-carboxamide ;
2-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)phénol
N-[3-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)phényl]prop-2-enamide
N-{[4-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)phényl]méthyl}acétamide
4-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)-2-fluorophénol
4-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-2-méthoxyphénol ;
4-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-2-chlorophénol ;
4-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-3 - chlorophénol ;
4-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-2,5 - difluorophénol ;
6-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-4-méthyl-1,2-dihydroquinolin-2-one ;
4-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-2,3-dihydro-1H-isoindol-1-one ;
2'-{5-[(1R,4R,7R)-7-amino-2-azabicyclo[2.2.1]heptane-2-carbonyl]-7-méthoxy-1-méthyl-1H-1,3-benzodiazol-2-yl}-1'-(cyclopropylméthyl)-2,3-dihydro-1H,1'H-[5,7'-biindole]-2-one ;
6-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-2,3 -dihydro-1H-isoindol-1-one ;
6-(2-{5-[(1,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-1,2,3,4-tétrahydroquinolin-2-one ;
6-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-1,2-dihydroquinolin-2-one ;
6-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-2-oxo-1,2-dihydroquinoline-4-carboxylate de méthyle ;
6-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-1,2-dihydroisoquinolin-1-one ;
5-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)-2,3 -dihydro-1H-isoindol-1-one ;
4-(2-{5-[(1R,4R,7R)-7-amino-2-azabicyclo[2.2.1]heptane-2-carbonyl]-7-méthoxy-1-méthyl-1H-1,3 -benzodiazol-2-yl}-1-(cyclopropylméthyl)-1H-indol-7-yl)-2-chlorobenzamide ;
N-[5-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)pyridin-2-yl]carbamate de méthyle ;
N-[5-(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)pyridin-2-yl]acétamide ;
(2-{5-[(1R,4R,7R)-7-amino-2-azabicyclo[2.2.1]heptane-2-carbonyl]-7-méthoxy-1-méthyl-1H-1,3-benzodiazol-2-yl}-1-(cyclopropylméthyl)-1H-indol-7-yl)phosphonate de diéthyle ;
2-{4-[2-(5-{7-amino-2-azabicyclo[2.2.1]heptane-2-carbonyl}-7-méthoxy-1-méthyl-1H-1,3-benzodiazol-2-yl)-1-(cyclopropylméthyl)-1H-indol-7-yl]-1H-1,2,3-triazol-1-yl}éthan-1-ol;
(1R,4R,7R)-2-{2-[1-(cyclopropylméthyl)-7-{4-[(1r,4r)-4-aminocyclohexyl]-1H-1,2,3-triazol-1-yl}-1H-indol-2-yl]-7-méthoxy-1-méthyl-1H-1,3-benzodiazole-5-carbonyl}-2-azabicyclo[2.2.1]heptan-7-amine ;
(1R,4R,7R)-2-(2-{7-[4-(azétidin-3-yl)-1H-1,2,3-triazol-1-yl]-1-(cyclopropylméthyl)-1H-indol-2-yl}-7-méthoxy-1-méthyl-1H-1,3-benzodiazole-5-carbonyl)-2-azabicyclo[2.2.1]heptan-7-amine ;
3-(2-(5-((3R,5R)-3-amino-5-fluoropipéridine-1-carbonyl)-7-méthoxy-1-méthyl-1H-benzo[d]imidazol-2-yl)-1-(cyclopropylméthyl)-1H-indol-7-yl)-N-méthylcyclobutane-1-carboxamide ;
3-(2-(5-((3R, SR)-3-amino-5-fluoropipéridine-1-carbonyl)-7-méthoxy-1-méthyl-1H-benzo[d]imidazol-2-yl)-1-(cyclopropylméthyl)-1H-indol-7-yl)cyclobutane-1-carboxamide ;
((3R,5R)-3-amino-5-fluoropipéridin-1-yl)(2-(1-(cyclopropylméthyl)-7-(3-(pyrrolidine-1-carbonyl)cyclobutyl)-1H-indol-2-yl)-7-méthoxy-1-méthyl-1H-benzo[d]imidazol-5-yl)méthanone ;
3-(2-(5-((3R, SR)-3-amino-5-fluoropipéridine-1-carbonyl)-7-méthoxy-1-méthyl-1H-benzo[d]imidazol-2-yl)-1-(cyclopropylméthyl)-1H-indol-7-yl)-N,N-diméthylcyclobutane-1-carboxamide ;
((1R,4R,7R)-7-amino-2-azabicyclo[2.2.1]heptan-2-yl)(2-(1-(cyclopropylméthyl)-7-(4-hydroxy-3-(hydroxyméthyl)phényl)-1H-indol-2-yl)-7-méthoxy-1-méthyl-1H-benzo[d]imidazol-5-yl)méthanone ;
5-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-2-hydroxybenzamide ;
acide 5-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-2-hydroxybenzoïque ;
((1R,4R,7R)-7-amino-2-azabicyclo[2.2.1]heptan-2-yl)(2-(1-(cyclopropylméthyl)-7-(1-méthyl-1H-pyrazol-4-yl)-1H-indol-2-yl)-7-méthoxy-1-méthyl-1H-benzo[d]imidazol-5-yl)méthanone ;
2-(2-(5-((1R,4R,7R)-7-amino-2-azabicyclo[2.2.1]heptane-2-carbonyl)-7-méthoxy-1 -méthyl- 1H-benzo[d]imidazol-2-yl)-1-(cyclopropylméthyl)- 1H-indol-7-yl)benzoate d'éthyle ;
4-(2-(5-((1R,4R,7R)-7-amino-2-azabicyclo[2.2.1]heptane-2-carbonyl)-7-méthoxy-1 -méthyl- 1H-benzo[d]imidazol-2-yl)-1-(cyclopropylméthyl)- 1H-indol-7-yl)benzamide ;
4-(2-(5-((1R,4R,7R)-7-amino-2-azabicyclo[2.2.1]heptane-2-carbonyl)-7-méthoxy-1 -méthyl- 1H-benzo[d]imidazol-2-yl)-1-(cyclopropylméthyl)- 1H-indol-7-yl)-N,N-diméthylbenzamide ;
((7R)-7-amino-2-azabicyclo[2.2.1]heptan-2-yl)(2-(1'-(cyclopropylméthyl)-1-méthyl-1H,1'H-[6,7'-biindol]-2'-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-(cyclopropylméthyl)-7-(1H-pyrazol-3-yl)-1H-indol-2-yl)-7-méthoxy-1-méthyl-1H-benzo[d]imidazol-5-yl)méthanone ;
((7R)-7-amino-2-azabicyclo[2.2.1]heptan-2-yl)(2-(1-(cyclopropylméthyl)-7-(1-(2-hydroxy-3 -(prop-2-yn-1-yloxy)propyl)-1H-pyrazol-4-yl)-1H-indol-2-yl)-7-méthoxy-1-méthyl-1H-benzo[d]imidazol-5-yl)méthanone ;
((7R)-7-amino-2-azabicyclo[2.2.1]heptan-2-yl)(2-(1-(cyclopropylméthyl)-7-(furan-3-yl)-1H-indol-2-yl)-7-méthoxy-1-méthyl-1H-benzo[d]imidazol-5-yl)méthanone ;
((7R)-7-amino-2-azabicyclo[2.2.1]heptan-2-yl)(2-(1-(cyclopropylméthyl)-7-(furan-2-yl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-(cyclopropylméthyl)-7-(thiophén-2-yl)-1H-indol-2-yl)-7-méthoxy-1-méthyl-1H-benzo[d]imidazol-5-yl)méthanone ;
((7R)-7-amino-2-azabicyclo[2.2.1]heptan-2-yl)(2-(1-(cyclopropylméthyl)-7-(1-(tétrahydro-2H-pyran-4-yl)-1H-pyrazol-4-yl)-1H-indol-2-yl)-7-méthoxy-1 -méthyl- 1H-benzo[d]imidazol-5-yl)méthanone ;
N-(4-(2-(5-((7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)phényl)acétamide ;
((7R)-7-amino-2-azabicyclo[2.2.1]heptan-2-yl)(2-(1-(cyclopropylméthyl)-7-(pyridin-3-yl)-1H-indol-2-yl)-7-méthoxy-1-méthyl-1H-benzo[d]imidazol-5-yl)méthanone ;
((7R)-7-amino-2-azabicyclo[2.2.1]heptan-2-yl)(2-(1-(cyclopropylméthyl)-7-(3-(2-fluorophényl)-1-méthyl-1H-pyrazol-4-yl)-1H-indol-2-yl)-7-méthoxy-1 -méthyl- 1H-benzo[d]imidazol-5-yl)méthanone ;
((7R)-7-amino-2-azabicyclo[2.2.1]heptan-2-yl)(2-(1-(cyclopropylméthyl)-7-(3-(hydroxyméthyl)phényl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-(cyclopropylméthyl)-7-(1H-pyrazol-4-yl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-(cyclopropylméthyl)-7-(1-((R)-2-hydroxypropyl)-1H-pyrazol-4-yl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-(cyclopropylméthyl)-7-(1-(2-hydroxy-2-méthylpropyl)-3-méthyl-1H-pyrazol-4-yl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-(cyclopropylméthyl)-7-(1-(2-hydroxy-2-méthylpropyl)-1H-pyrazol-4-yl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-(cyclopropylméthyl)-7-(1-(2-hydroxyéthyl)-1H-pyrazol-4-yl)-1H-indol-2-yl)-7-méthoxy-1-méthyl-1H-benzo[d]imidazol-5-yl)méthanone ;
6-(2-(5-((1R,4S)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-4,4-diméthyl-1,4-dihydro-2H-benzo[d][1,3]oxazin-2-one ;
2-(2-(5-((1R,4S)-7-amino-2-azabicyclo[2.2.1]heptane-2-carbonyl)-7-méthoxy-1-méthyl-1H-benzo[d]imidazol-2-yl)-1-(cyclopropylméthyl)- 1H-indol-7-yl)benzonitrile ;
5-(2-(5-((1R,4R,7R)-7-amino-2-azabicyclo[2.2.1]heptane-2-carbonyl)-7-méthoxy-1 -méthyl- 1H-benzo[d]imidazol-2-yl)-1-((2,2-difluorocyclopropyl)méthyl)- 1H-indol-7-yl)pyrimidine-2-carbonitrile ;
((1R,4R,7R)-7-amino-2-azabicyclo[2.2.1]heptan-2-yl)(2-(1-((2,2-difluorocyclopropyl)méthyl)-7-(1H-pyrazol-4-yl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-((2,2-dichlorocyclopropyl)méthyl)-7-(1H-pyrazol-4-yl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-((2,2-dichlorocyclopropyl)méthyl)-7-(1H-pyrazol-3-yl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-(cyclopropylméthyl)-5-méthyl-7-(1H-pyrazol-4-yl)-1H-indol-2-yl)-7-méthoxy-1-méthyl-1H-benzo[d]imidazol-5-yl)méthanone ;
2-(2-(5-((1R,4R,7R)-7-amino-2-azabicyclo[2.2.1]heptane-2-carbonyl)-7-méthoxy-1-méthyl-1H-benzo[d]imidazol-2-yl)-1-((2-méthylcyclopropyl)méthyl)- 1H-indol-7-yl)benzonitrile ;
((1R,4R,7R)-7-amino-2-azabicyclo[2.2.1]heptan-2-yl)(7-méthoxy-1-méthyl-2-(1-((2-méthylcyclopropyl)méthyl)-7-(1H-pyrazol-4-yl)-1H-indol-2-yl)-1H-benzo[d]imidazol-5-yl)méthanone ;
((1R,4R,7R)-7-amino-2-azabicyclo[2.2.1]heptan-2-yl)(7-méthoxy-1-méthyl-2-(1-((2-méthylcyclopropyl)méthyl)-7-(1H-pyrazol-3-yl)-1H-indol-2-yl)-1H-benzo[d]imidazol-5-yl)méthanone ;
((1R,4R,7R)-7-amino-2-azabicyclo[2.2.1]heptan-2-yl)(2-(1-(cyclopropylméthyl)-7-(2-(hydroxyméthyl)phényl)-1H-indol-2-yl)-7-méthoxy-1-méthyl-1H-benzo[d]imidazol-5-yl)méthanone ;
((1R,4R,7R)-7-amino-2-azabicyclo[2.2.1]heptan-2-yl)(2-(1-(cyclopropylméthyl)-7-phényl-1H-indol-2-yl)-7-méthoxy-1-méthyl-1H-benzo[d]imidazol-5-yl)méthanoneméthyl-1H-benzo[d]imidazol-5-yl)méthanone ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-phénylpipéridine-1-carboxamide ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)- 1H-indol-7-yl)-N,N-diméthylpipéridine-1-carboxamide ;
1-(4-(2-(5-((3R,SR)-3-amino-5-fluoropipéridine-1-carbonyl)-7-méthoxy-1-((1-méthyl-1H-pyrazol-4-yl)méthyl)-1H-benzo[d]imidazol-2-yl)-1-(cyclopropylméthyl)-1H-indol-7-yl)pipéridin-1-yl)éthan-1-one ;
5-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-3,3-difluoroindolin-2-one ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-3-fluorobenzamide ;
5-(2-(5-((1R,4R,7R)-7-amino-2-azabicyclo[2.2.1]heptane-2-carbonyl)-7-méthoxy-1 -méthyl-1H-benzo[d]imidazol-2-yl)-1-(cyclopropylméthyl)- 1H-indol-7-yl)-3 -hydroxy-3 - méthylindolin-2-one ;
2-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-5-hydroxybenzonitrile ;
5-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)picolinamide ;
3-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N,N-bis(méthyl-d3)cyclobutane-1-carboxamide ;
((1R,4R,7R)-7-amino-2-azabicyclo[2.2.1]heptan-2-yl)(2-(1-(cyclopropylméthyl)-7-(6-hydroxypyridin-3-yl)-1H-indol-2-yl)-7-méthoxy-1-méthyl-1H-benzo[d]imidazol-5-yl)méthanone ;
5-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-3 -hydroxy-3 - (trifluorométhyl)indolin-2-one ;
5-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-3 - fluoropicolinamide ;
(3-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)cyclobutyl)(3-fluoroazétidin-1-yl)méthanone
(3-(2-(5-((lR,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)cyclobutyl)(azétidin-1-yl)méthanone ;
6-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-7-méthoxy-3,4-dihydroquinolin-2(1H)-one ;
1-(4-(2-(5-((3R,5R)-3-amino-5-fluoropipéridine-1-carbonyl)-7-méthoxy-1-méthyl-1H-benzo[d]imidazol-2-yl)-1-(cyclopropylméthyl)-1H-indol-7-yl)pipéridin-1-yl)éthan-1-one ;
((3R,SR)-3-amino-5-fluoropipéridin-1-yl)(2-(1-(cyclopropylméthyl)-7-(3-fluoro-4-hydroxyphényl)-1H-indol-2-yl)-7-méthoxy-1-méthyl-1H-benzo[d]imidazol-5-yl)méthanone ;
5-(2-(5-((3R,5R)-3-amino-5-fluoropipéridine-1-carbonyl)-7-méthoxy-1-méthyl-1H-benzo[d]imidazol-2-yl)-1-(cyclopropylméthyl)-1H-indol-7-yl)indolin-2-one ;
5-(2-(5-((3R, SR)-3-amino-5-fluoropipéridine-1-carbonyl)-7-méthoxy-1-méthyl-1H-benzo[d]imidazol-2-yl)-1-(cyclopropylméthyl)-1H-indol-7-yl)-3-hydroxy-3-méthylindolin-2-one ;
5-(2-(5-((3R, SR)-3-amino-5-fluoropipéridine-1-carbonyl)-7-méthoxy-1-méthyl-1H-benzo[d]imidazol-2-yl)-1-(cyclopropylméthyl)-1H-indol-7-yl)isoindolin-1-one ;
1-(3-(2-(5-((1R,4R,7R)-7-amino-2-azabicyclo[2.2.1]heptane-2-carbonyl)-7-méthoxy-1-((1-méthyl-1H-pyrazol-4-yl)méthyl)-1H-benzo[d]imidazol-2-yl)-1-(cyclopropylméthyl)-1H-indol-7-yl)azétidin-1-yl)éthan-1-one ;
3-(2-(5-((3R,5R)-3-amino-5-fluoropipéridine-1-carbonyl)-7-méthoxy-1-((1-méthyl-1H-pyrazol-4-yl)méthyl)-1H-benzo[d]imidazol-2-yl)-1-(cyclopropylméthyl)-1H-indol-7-yl)azétidine-1-carboxylate de méthyle ;
1-(3-(2-(5-((3R,SR)-3-amino-5-fluoropipéridine-1-carbonyl)-7-méthoxy-1-((1-méthyl-1H-pyrazol-4-yl)méthyl)-1H-benzo[d]imidazol-2-yl)-1-(cyclopropylméthyl)-1H-indol-7-yl)azétidin-1-yl)éthan-1-one ;
3-(2-(5-((1R,4R,7R)-7-amino-2-azabicyclo[2.2.1]heptane-2-carbonyl)-7-méthoxy-1-((1-méthyl-1H-pyrazol-4-yl)méthyl)-1H-benzo[d]imidazol-2-yl)-1-(cyclopropylméthyl)-1H-indol-7-yl)azétidine-1-carboxylate de méthyle ;
1-(3-(2-(5-((1R,4R,7R)-7-amino-2-azabicyclo[2.2.1]heptane-2-carbonyl)-7-méthoxy-1-((1-méthyl-1H-pyrazol-4-yl)méthyl)-1H-benzo[d]imidazol-2-yl)-1-méthyl-1H-indol-7-yl)azétidin-1-yl)éthan-1-one ;
1-(3-(2-(5-((3R,SR)-3-amino-5-fluoropipéridine-1-carbonyl)-7-méthoxy-1-((1-méthyl-1H-pyrazol-4-yl)méthyl)-1H-benzo[d]imidazol-2-yl)-1-méthyl-1H-indol-7-yl)azétidin-1-yl)éthan-1-one ;
3-(2-(5-((1R,4R,7R)-7-amino-2-azabicyclo[2.2.1]heptane-2-carbonyl)-7-méthoxy-1-((1-méthyl-1H-pyrazol-4-yl)méthyl)-1H-benzo[d]imidazol-2-yl)-1-méthyl-1H-indol-7-yl)azétidine-1-carboxylate de méthyle ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridine-1-carboxylate de méthyle ;
((1R,4R,7R)-7-amino-2-azabicyclo[2.2.1]heptan-2-yl)(2-(1-(cyclopropylméthyl)-7-(1-hydroxycyclobutyl)-1H-indol-2-yl)-7-méthoxy-1-méthyl-1H-benzo[d]imidazol-5-yl)méthanone ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-3,6-dihydropyridin-1(2H)-yl)éthan-1-one ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-3,6-dihydropyridine-1(2H)-carboxylate de méthyle ;
((1R,4R,7R)-7-amino-2-azabicyclo[2.2.1]heptan-2-yl)(2-(1-(cyclopropylméthyl)-7-(3-(1,2-dihydroxyéthyl)phényl)-1H-indol-2-yl)-7-méthoxy-1-méthyl-1H-benzo[d]imidazol-5-yl)méthanone ;
N-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-2-(hydroxyméthyl)benzyl)acétamide ;
(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridin-1-yl)(spiro[2.2]pentan-1-yl)méthanone ;
(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridin-1-yl)(cyclopropyl)méthanone ;
l-(4-(2-(5-((3R,5R)-3-amino-5-fluoropipéridine-1-carbonyl)-7-méthoxy-1-méthyl-1H-benzo[d]imidazol-2-yl)-1-(cyclopropylméthyl)-1H-indol-7-yl)pipéridin-1-yl)-2-méthoxyéthan-1-one ;
1-(4-(2-(5-((3R,5R)-3-amino-5-fluoropipéridine-1-carbonyl)-7-méthoxy-1-méthyl-1H-benzo[d]imidazol-2-yl)-1-(cyclopropylméthyl)-1H-indol-7-yl)pipéridin-1-yl)éthan-1 - one ;
3-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-phénylazétidine-1-carboxamide ;
3-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)- 1H-indol-7-yl)-N-(4-fluorophényl)azétidine-1-carboxamide ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)- 1H-indol-7-yl)pipéridin-1-yl)-2-méthoxyéthan-1-one ;
3-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridin-1-yl)-3-oxopropanenitrile ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridin-1-yl)-2-(2,2,2-trifluoroéthoxy)éthan-1-one ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridin-1-yl)-2-cyclopropyl-2-méthoxyéthan-1-one ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridin-1-yl)-2-éthoxyéthan-1-one ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)- 1H-indol-7-yl)pipéridin-1-yl)-2-phénoxyéthan-1-one ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)- 1H-indol-7-yl)pipéridin-1-yl)-3-méthoxypropan-1-one ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridin-1-yl)-4-méthoxybutan-1-one ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)- 1H-indol-7-yl)pipéridin-1-yl)-2-méthoxypropan-1-one ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridin-1-yl)-2-isopropoxyéthan-1-one ;
1-(4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridin-1-yl)-2-hydroxyéthan-1-one ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)- 1H-indol-7-yl)-N-(4-fluorophényl)pipéridine-1-carboxamide ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-(3 - fluorophényl)pipéridine-1-carboxamide ;
4-(2-(5-((1R,4R,7R)-7-amino-2-azabicyclo[2.2.1]heptane-2-carbonyl)-7-méthoxy-1 -méthyl-1H-benzo[d]imidazol-2-yl)-1-(cyclopropylméthyl)- 1H-indol-7-yl)-N-(2-fluorophényl)pipéridine-1-carboxamide ;
4-(2-(5-((1R,4R,7R)-7-amino-2-azabicyclo[2.2.1]heptane-2-carbonyl)-7-méthoxy-1 -méthyl-1H-benzo[d]imidazol-2-yl)-1-(cyclopropylméthyl)- 1H-indol-7-yl)-N-(pyridin-3-yl)pipéridine-1-carboxamide ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-(pyridin-4-yl)pipéridine-1-carboxamide ;
4-(2-(5-((1R,4R,7R)-7-amino-2-azabicyclo[2.2.1]heptane-2-carbonyl)-7-méthoxy-1 -méthyl-1H-benzo[d]imidazol-2-yl)-1-(cyclopropylméthyl)- 1H-indol-7-yl)-N-(4-méthoxyphényl)pipéridine-1-carboxamide ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-(2-méthoxyphényl)pipéridine-1-carboxamide ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-(3 - méthoxyphényl)pipéridine-1-carboxamide ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-cyclopropylpipéridine-1-carboxamide ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-éthylpipéridine-1-carboxamide ;
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)pipéridine-1-carboxamide ; ou
4-(2-(5-((1R,4R,7R)-7-amino-2-azabicyclo[2.2.1]heptane-2-carbonyl)-7-méthoxy-1-méthyl-1H-benzo[d]imidazol-2-yl)-1-(cyclopropylméthyl)-1H-indol-7-yl)-N-isopropylpipéridine-1-carboxamide.

17. Composition pharmaceutiquement acceptable comprenant le composé selon l'une quelconque des revendications 1 à 16 et un excipient, un adjuvant ou un véhicule pharmaceutiquement acceptables, ladite composition étant facultativement combinée à un agent thérapeutique supplémentaire.

18. Procédé d'inhibition de PAD4 dans un échantillon biologique in vitro, comprenant l'étape consistant à mettre en contact la PAD4 avec un composé selon l'une quelconque des revendications 1 à 16.

19. Composé selon l'une quelconque des revendications 1 à 16 ou composition selon la revendication 17 pour utilisation en thérapie, de préférence pour utilisation dans un procédé de traitement d'une maladie, d'un trouble ou état médié par la PAD4 sélectionné dans le groupe constitué par une leucémie lymphocytaire aiguë, une spondylite ankylosante, un cancer, une leucémie lymphocytaire chronique, une colite, un lupus, une polyarthrite rhumatoïde, une sclérose en plaques et une colite ulcéreuse, chez un sujet ayant ladite maladie, ledit trouble ou état médié par la PAD4, le procédé comprenant l'étape de l'administration audit sujet du composé ou de la composition ; la maladie, le trouble ou l'état médié par la PAD4 étant plus préférablement sélectionné parmi une polyarthrite rhumatoïde, un lupus érythémateux disséminé, un lupus érythémateux cutané, une colite ulcéreuse et un cancer.
